# EUROPEAN PATENT APPLICATION

(11) **EP 4 070 820 A1**
(43) Date of publication of application: **12.10.2022**
(21) Application number: 20895027.9
(22) Date of filing: 11.03.2020
(51) Int. Cl.: A61K 47/55, A61K 47/60, A61K 31/5377, A61K 31/4184, A61K 31/519, A61K 31/517, A61P 35/00

(54) **POLYETHYLENE GLYCOL CONJUGATE DRUG, PREPARATION METHOD THEREFOR AND APPLICATION THEREOF**

(30) Priority: 06.12.2019 CN 201911242386
(71) Applicant: Chongqing Upgra Biotechnology Co., Ltd., Chongqing 401120 (CN)
(72) Inventor: LI, Gaoquan, Chongqing 401120 (CN); ZHANG, Qian, Chongqing 401120 (CN); LI, Dajun, Chongqing 401120 (CN); LI, Diedie, Chongqing 401120 (CN); GAO, Chengzhi, Chongqing 401120 (CN); DING, Xiaoling, Chongqing 401120 (CN); LIU, Yue, Chongqing 401120 (CN); WEI, Yusong, Chongqing 401120 (CN); YANG, Xiangwei, Chongqing 401120 (CN); PENG, Yongchen, Chongqing 401120 (CN); GAO, Jia, Chongqing 401120 (CN); LUO, Qiang, Chongqing 401120 (CN); HENG, Yanxia, Chongqing 401120 (CN); LIU, Mei, Chongqing 401120 (CN); YI, Yuyang, Chongqing 401120 (CN); ZENG, Xiafan, Chongqing 401120 (CN); TU, Tao, Chongqing 401120 (CN); TANG, Xiao, Chongqing 401120 (CN); LIU, Xi, Chongqing 401120 (CN); LI, Jianhuan, Chongqing 401120 (CN); WANG, Zhaojie, Chongqing 401120 (CN); WAN, Jinping, Chongqing 401120 (CN); HOU, Mingyang, Chongqing 401120 (CN); LOU, Jie, Chongqing 401120 (CN); CHEN, Huiyu, Chongqing 401120 (CN); YANG, Yue, Chongqing 401120 (CN); WANG, Yuanqiang, Chongqing 401120 (CN)
(74) Representative: Nony
(86) International application number: PCT/CN2020/078743
(87) International publication number: WO 2021/109351

(57) **Abstract**

The disclosure relates to the technical field of medicine, specifically to a polyethylene glycol conjugated drug, a preparation method therefor and use thereof, and relates in particular to a polyethylene glycol conjugated drug represented by formula (I) or a pharmaceutically acceptable salt thereof. The disclosure further relates to a method for preparation of the polyethylene glycol conjugated drug or a pharmaceutically acceptable salt thereof, a pharmaceutical composition comprising the polyethylene glycol conjugated drug or a pharmaceutically acceptable salt thereof, and use of the polyethylene glycol conjugated drug or a pharmaceutically acceptable salt thereof in preparation of a medicament.

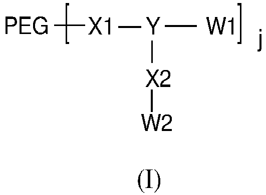

## Description

### TECHNICAL FIELD

The disclosure belongs to the technical field of medicine, and relates to a polyethylene glycol conjugated drug, and a preparation method therefor and use thereof.

### BACKGROUND

Compared with the original drug, PEGylated drugs have huge advantages. They can increase the water solubility of drug molecules and this is important for molecules with extremely low solubility such as paclitaxel, camptothecin, or platinum derivatives. The PEGylated drugs also can prevent or reduce the agglomeration, immunogenicity and antigenicity of drugs. Most small-molecule drugs can only stay in the blood circulation for a few minutes, while polymer-(anticancer drug) conjugates can stay for tens, hundreds of hours or even longer, which is conducive to "enhanced penetration and retention" effect, i.e., EPR effect, caused by leakage of tumor capillaries. Due to increase in hydrodynamic volume, the renal elimination of the drug can be weakened, the drug can be protected from enzymatic degradation, the half-life of the drug in the plasma can be extended, and the bioavailability of the drug can be increased. The anticancer drugs can be highly enriched in cancerous organs, tissues or cells through passive or active targeting of the polymer-(anticancer drug) conjugates, thus greatly reducing the toxic side effects caused by small molecule drugs spreading all over the body. The cell absorption of drugs can be limited to the endocytic pathway, which is conducive to drug delivery to the lysosome, thereby avoiding drug resistance caused by p-glycoprotein pumping. The polymer-(anticancer drug) conjugates can also stimulate or restore immune function, which is conducive to killing cancer cells.

The polyethylene glycol conjugated drug technology of NEKTAR and ENZON of the United States has achieved great success. So far, 15 drugs have been approved by the US FDA to enter the market. In addition, 36 new clinical drugs are undergoing phase I, phase II, and phase III clinical trials or entering the NDA phase. However, all of the above PEGylated drugs are PEGylated monodrugs.

As disclosed in Chinese patent ZL201510996205.4, a chemotherapeutic drug gemcitabine and a Chk1 inhibitor AZD7762 are simultaneously grafted onto a four-arm polyethylene glycol carrier. The Chk1 inhibitor itself has no anti-cancer effect, but it can enhance the efficacy of the chemotherapeutic drug when combined with the chemotherapy drug gemcitabine. As disclosed in Chinese patents ZL201710761441.7 and ZL201710761572.5, two anticancer drugs are simultaneously grafted onto a grafting site of polyethylene glycol to achieve inhibition of cancer cells through different biological signal pathways or targets, as well as free combination of different treatment modalities.

### SUMMARY

The inventors of the disclosure have uniquely designed the structure of polyethylene glycol conjugated drugs, especially selecting appropriate linking groups (including extended linking groups such as urea carbonyl, succinyl, etc.), which can conveniently increase the drug loading of the polyethylene glycol conjugated drugs and adjust the position of the drugs and the types and ratios of dual- or multi-dose drugs, thereby providing the following disclosure.

### BRIEF SUMMARY

In one aspect, the disclosure relates to a polyethylene glycol conjugated drug of formula (I) or a pharmaceutically acceptable salt thereof;

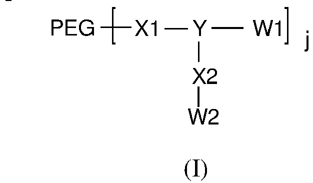

wherein PEG is a single-arm or multi-arm polyethylene glycol segment, j represents the number of arms of PEG1, and X1, X2, Y, W1, and W2 are defined as follows.

In one aspect, this disclosure provides a pharmaceutical composition, including the polyethylene glycol conjugated drug of the disclosure or a pharmaceutically acceptable salt thereof in a therapeutically and/or prophylactically effective amount.

In one aspect, the disclosure provides use of the polyethylene glycol conjugated drug of the disclosure or a pharmaceutically acceptable salt thereof in the preparation of a medicament for treating and/or preventing a disease (such as a cancer). The disease refers to a disease treated by an active ingredient in the polyethylene glycol conjugated drug.

In one aspect, the disclosure further provides an injection solution, comprising the polyethylene glycol conjugated drug of the disclosure or a pharmaceutically acceptable salt thereof, or the pharmaceutical composition of the disclosure.

The disclosure further provides a method and an intermediate for preparing the polyethylene glycol conjugated drug of the disclosure or a pharmaceutically acceptable salt thereof.

### DETAILED SUMMARY

### 1. Polyethylene glycol conjugated drug or pharmaceutically acceptable salt thereof

In one aspect, the disclosure provides a polyethylene glycol conjugated drug of formula (I) or a pharmaceutically acceptable salt thereof; 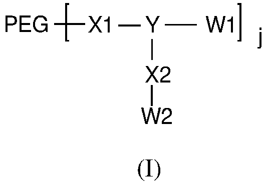 wherein PEG is a single-arm or multi-arm polyethylene glycol segment; j represents the number of arms of PEG1 (for example, 1, 2, 4, or 8);
X1 is selected from -NH-, 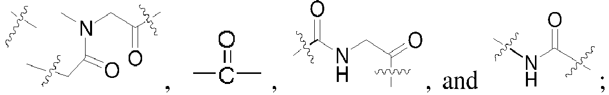 when j is greater than 1 (such as, 2, 4 or 8), there may be multiple (such as, 2, 4 or 8) X1s at the same time, and in this case, the X1s may be the same or different;
Y represents Lys (lysine residue) or Glu (glutamic acid residue); when j is greater than 1 (such as, 2, 4 or 8), there may be multiple (such as, 2, 4 or 8) Ys at the same time, and in this case, the Ys may be the same or different;
X2 is selected from 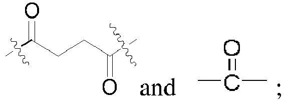 when j is greater than 1 (such as, 2, 4 or 8), there may be multiple (such as, 2, 4 or 8) X2s at the same time, and in this case, the X2s may be the same or different;
W1 is selected from N1-AC1, Q, 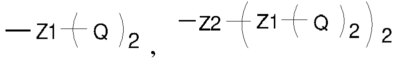 and 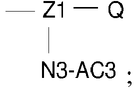 when j is greater than 1 (such as, 2, 4 or 8), there may be multiple (such as, 2, 4 or 8) W1s at the same time, and in this case, the W1s may be the same or different;
Q represents 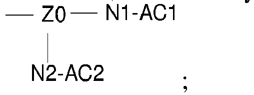 each of Z0, Z1, and Z2 is independently selected from 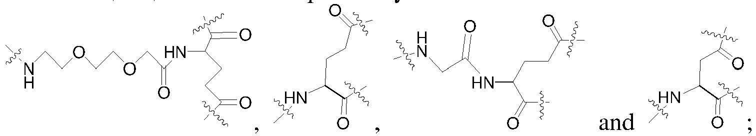
Z0, Z1 and Z2 may be the same or different, and when there are multiple Z0s, multiple Z1s or multiple Z2s at the same time, the Z0s are the same or different, the Z1s are the same or different, or the Z2s are the same or different;
each of N1, N2 and N3 independently may be G (glycine residue) or GFLG (glycine-phenylalanine-leucine-glycine); N1, N2 and N3 may be the same or different, and when there are multiple N1s, multiple N2s or multiple N3s at the same time, the N1s are the same or different, the N2s are the same or different, or the N3s are the same or different;
AC1, AC2 and AC3 are drug molecules (for example, drug molecules with anti-tumor activity); AC1, AC2 and AC3 may be the same or different, and when there are multiple AC1s, multiple AC2s or multiple AC3s at the same time, the AC1s are the same or different, the AC2s are the same or different, or the AC3s are the same or different;
W2 is selected from N1'-AC1', Q', 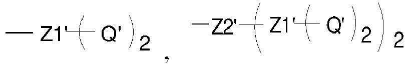 and 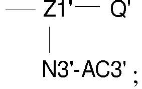
when j is greater than 1 (such as, 2, 4 or 8), there may be multiple (such as, 2, 4 or 8) W2s at the same time, and in this case, the W2s may be the same or different;
Q'represents 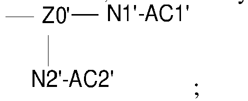
each of Z0', Z1' and Z2' is independently selected from: 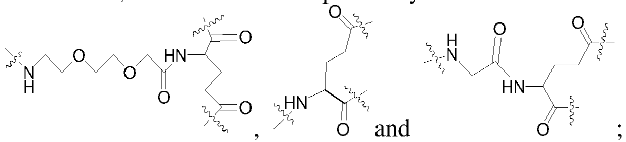
Z0', Z1' and Z2' may be the same or different, and when there are multiple Z0's, multiple Z1's or multiple Z2's at the same time, the Z0's are the same or different, the Z1's are the same or different, or the Z2's are the same or different;
each of N1', N2', and N3' independently may be G or GFLG; N1', N2', and N3' may be the same or different, and when there are multiple N1's, multiple N2's, or multiple N3's at the same time, the N1's are the same or different, the N2's are the same or different, or the N3's are the same or different;
AC1', AC2' and AC3' are drug molecules (for example, drug molecules with anti-tumor activity); AC1', AC2' and AC3' may be the same or different, and when there are multiple AC1's, multiple AC2's or multiple AC3's at the same time, the AC1's are the same or different, the AC2's are the same or different, or the AC3's are the same or different.

In some embodiments, the polyethylene glycol conjugated drug of the disclosure or a pharmaceutically acceptable salt thereof is characterized in one or more of the following:
(1) PEG is a single-arm or four-arm polyethylene glycol segment;
(2) PEG has a number-average molecular weight of 5k-10k, 10k-20k or 20k-40k;
(3) each of AC1, AC2, AC3, AC1', AC2', AC3' is independently selected from LPT, PCB, SB7, PKA, ABR, and SN38;
(4) when Y represents Lys, X1 is linked to an α-amino group on the Lys;
(5) when Y represents Lys, X1 is linked to an ε-amino group on the Lys;
(6) when Y represents Glu, X1 is linked to an α-carboxyl group on the Lys;
(7) when Y represents Glu, X1 is linked to a γ-carboxyl group on the Lys;
(8) N1 and N2 are both GFLG;
(9) N1, N2 and N3 are all GFLG;
(10) N1 and N2 are both G;
(11) N1, N2 and N3 are all G;
(12) N1' and N2' are both GFLG;
(13) N1', N2' and N3' are all GFLG;
(14) N1' and N2' are both G; and
(15) N1', N2' and N3' are all G.

In some embodiments, AC1 and AC2 are selected from:
(1) a combination in which AC1 and AC2 are both PCB;
(2) a combination in which AC1 and AC2 are both LPT; and
(3) a combination in which AC1 and AC2 are both SN38.

In some embodiments, AC1' and AC2' are selected from:
(4) a combination in which AC1' and AC2' are both LPT;
(5) a combination in which AC1' is SB7 and AC2' is LPT;
(6) a combination in which AC1' and AC2' are both PCB; and
(7) a combination in which AC1' and AC2' are both ABR.

In some embodiments, AC1, AC2 and AC3 are all SN38.

In some embodiments, AC1', AC2' and AC3' are all SN38.

In some embodiments, the polyethylene glycol conjugated drug of the disclosure or a pharmaceutically acceptable salt thereof is characterized in one or more of the following:
(1) N1 is GFLG and AC1 is SB7;
(2) N1 and N2 are both GFLG and AC1 and AC2 are both LPT;
(3) N1 and N2 are both GFLG and AC1 and AC2 are both PCB;
(4) N1, N2 and N3 are all G and AC1, AC2 and AC3 are all SN38;
(5) N1 is GFLG and AC1 is PCB;
(6) N1' and N2' are both GFLG and AC1' and AC2' are both LPT;
(7) N1' and N2' are both GFLG, AC1' is SB7, and AC2' is LPT;
(8) N1' and N2' are both GFLG and AC1' and AC2' are both PCB;
(9) N1' is GFLG and AC1' is PKA;
(10) N1' and N2' are both G and AC1' and AC2' are both ABR;
(11) N1', N2' and N3' are all G and AC1', AC2' and AC3' are all SN38;
(12) N1' and N2' are both GFLG, AC1' is SB7, and AC2' is PCB;
(13) N1 and N2 are both G and AC1 and AC2 are both SN38; and
(14) N1' is G and AC1' is SN38.

In some embodiments, in the polyethylene glycol conjugated drug of the disclosure or a pharmaceutically acceptable salt thereof, the PEG is a single-arm polyethylene glycol segment and X1 is linked to an end of the PEG or in the PEG. In some embodiments, when X1 is linked in PEG, X1 represents

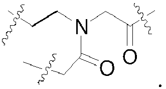

In some embodiments, in the polyethylene glycol conjugated drug of the disclosure or a pharmaceutically acceptable salt thereof, Y represents Lys.

In some embodiments, Y represents Lys and X2 is 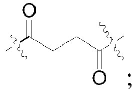
X1 is selected from 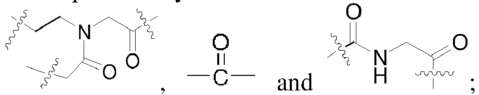
W1 is selected from N1-AC1, 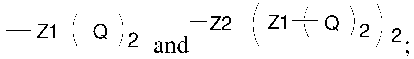
W2 is selected from Q',

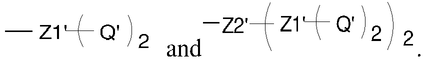

In some embodiments, the PEG is a single-arm polyethylene glycol segment with a number-average molecular weight of 10k-20k or 20k-40k.

In some embodiments, W1 is selected from:
(1) 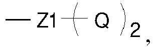 wherein Z1 is 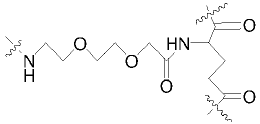 and Z0 is 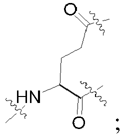 in some embodiments, N1 and N2 are both GFLG and AC1 and AC2 are both LPT;
(2) 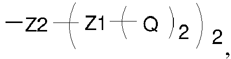 wherein Z2 is 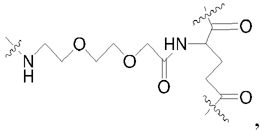 Z1 is 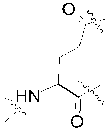 and Z0 is 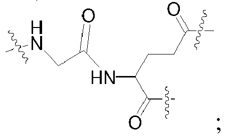 in some embodiments, N1 and N2 are both GFLG and AC1 and AC2 are both LPT; and
(3) N1-AC1, wherein N1 is GFLG; in some embodiments, AC1 is SB7.

In some embodiments, W2 is selected from:
(1) 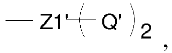 wherein Z1' is 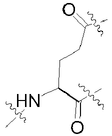 and Z0' is 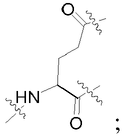 in some embodiments, N1' and N2' are both GFLG and AC1' and AC2' are both LPT;
(2) Q', wherein Z0' is 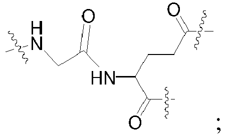 in some embodiments, N1' and N2' are both GFLG and AC1' is SB7, and AC2' is LPT;
(3) 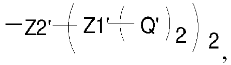 wherein Z2' is 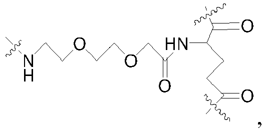 Z1' is 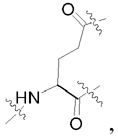 and Z0' is 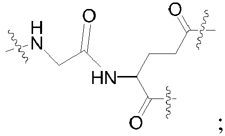 in some embodiments, N1' and N2' are both GFLG and AC1' and AC2' are both LPT; and
(4) 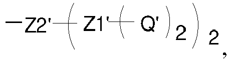 wherein Z2' is 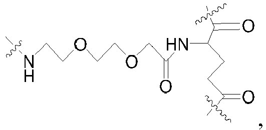 Z1' is 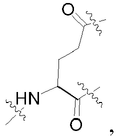 and Z0' is 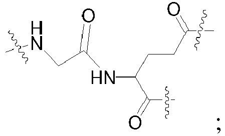 in some embodiments, N1' and N2' are both G and AC1' and AC2' are both ABR.

In some embodiments, the polyethylene glycol conjugated drug has a structure selected from: 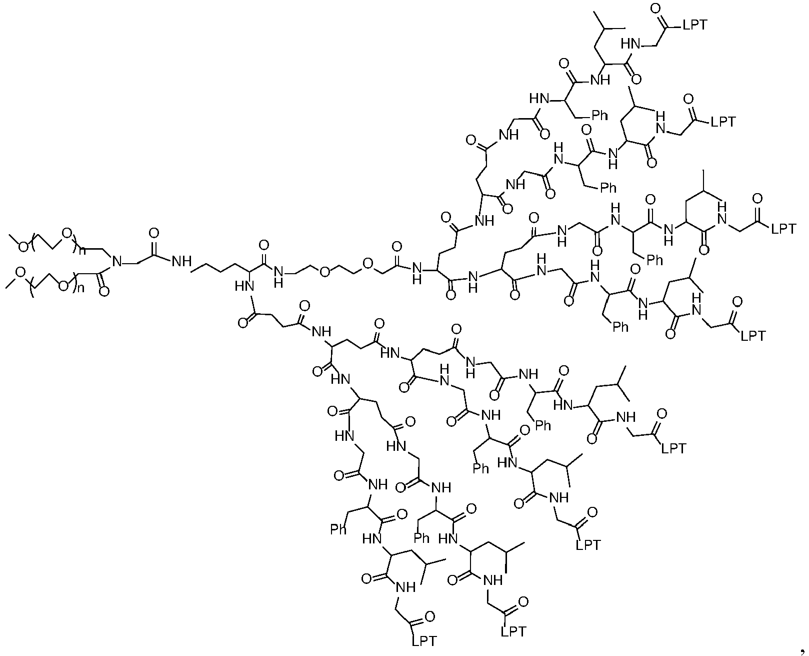 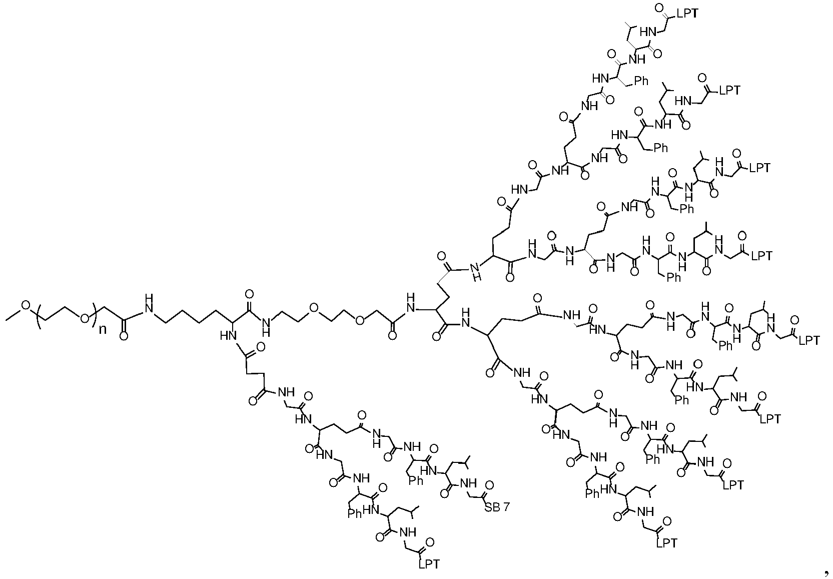 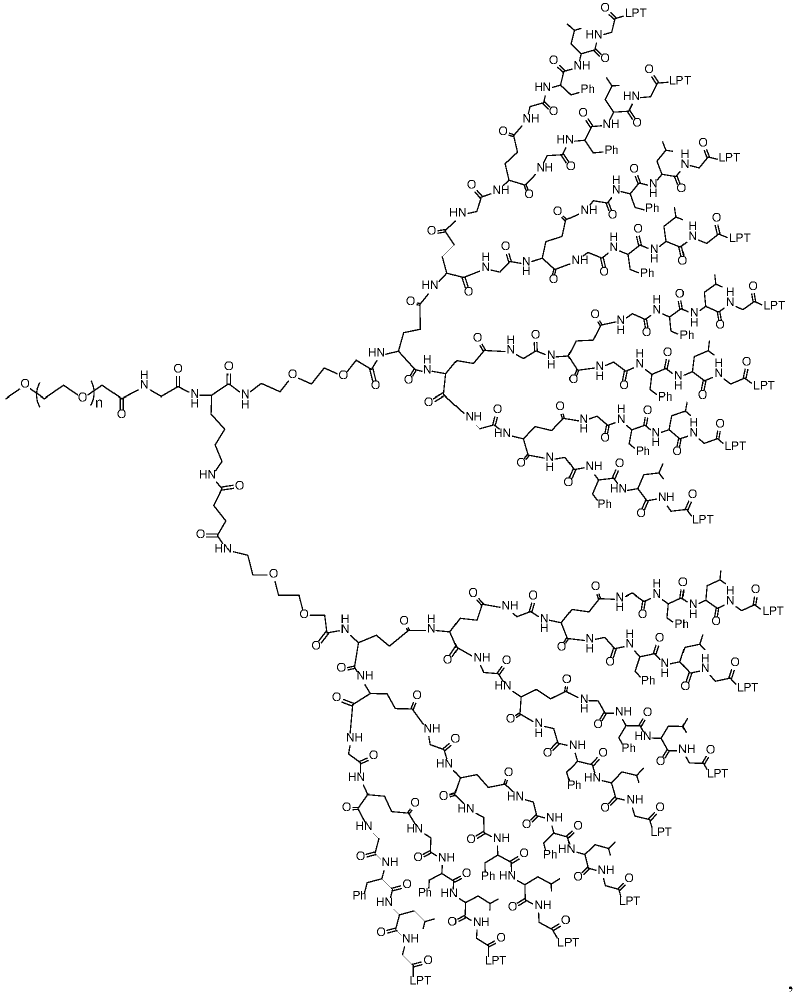 and

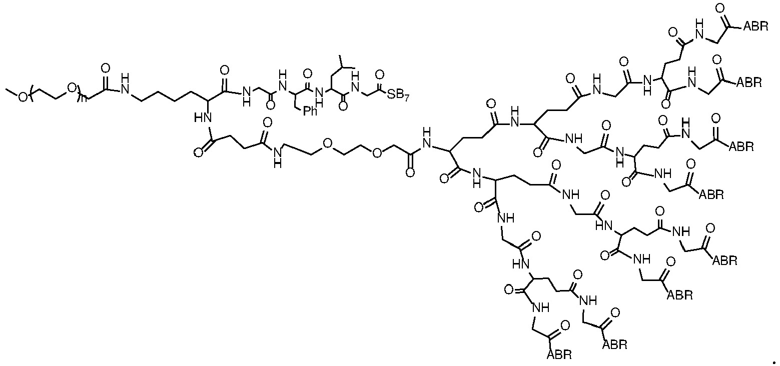

In some embodiments, Y represents Lys and X2 is 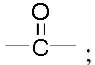
X1 is selected from 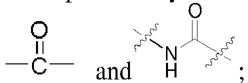
W1 is selected from N1-AC1, Q, 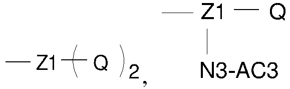 and 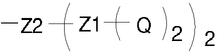
W2 is selected from N1'-AC1', Q' and

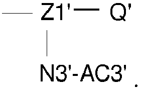

In some embodiments, the PEG is a single-arm or four-arm polyethylene glycol segment with a number-average molecular weight of 5k-20k or 20k-40k.

In some embodiments, W1 is selected from:
(1) Q, wherein Z0 is 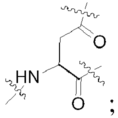 in some embodiments, N1 and N2 are both GFLG and AC1 and AC2 are both PCB;
(2) 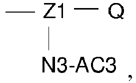 wherein Z1 is 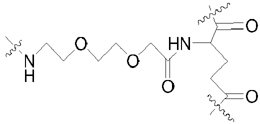 and Z0 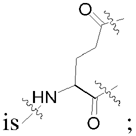 in some embodiments, N1, N2 and N3 are all G and AC1, AC2 and AC3 are all SN38;
(3) N1-AC1, wherein N1 is GFLG; in some embodiments, AC1 is PCB.
(4) 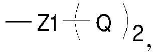 wherein Z1 is 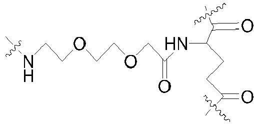 and Z0 is 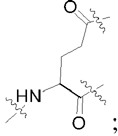 in some embodiments, N1 and N2 are both GFLG and AC1 and AC2 are both PCB; and
(5) 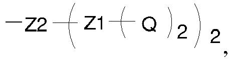 wherein Z2 and Z0 are both 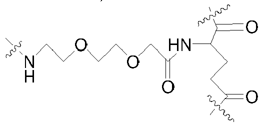 and Z1 is 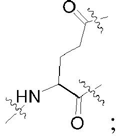 in some embodiments, N1 and N2 are both GFLG and AC1 and AC2 are both PCB.

In some embodiments, W2 is selected from:
(1) N1'-AC1', wherein N1' is GFLG; in some embodiments, AC1' is PKA;
(2) 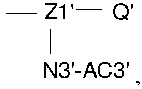 wherein Z1' is 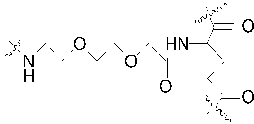 and Z0' is 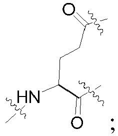 in some embodiments, N1', N2' and N3' are all G and AC1', AC2' and AC3' are all SN38;
(3) N1'-AC1', wherein N1' is GFLG; in some embodiments, AC1' is SB7; and
(4) Q', wherein Z0' is 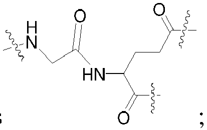 in some embodiments, N1' and N2' are both GFLG, AC1' is SB7 and AC2' is PCB.

In some embodiments, the polyethylene glycol conjugated drug has a structure selected from: 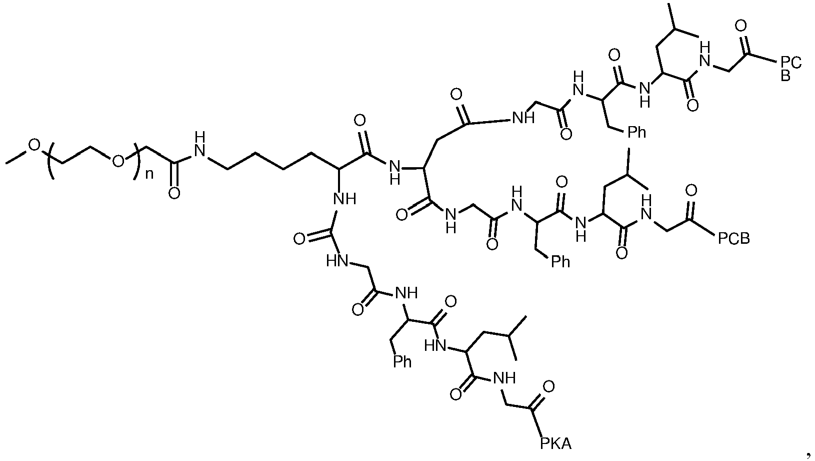 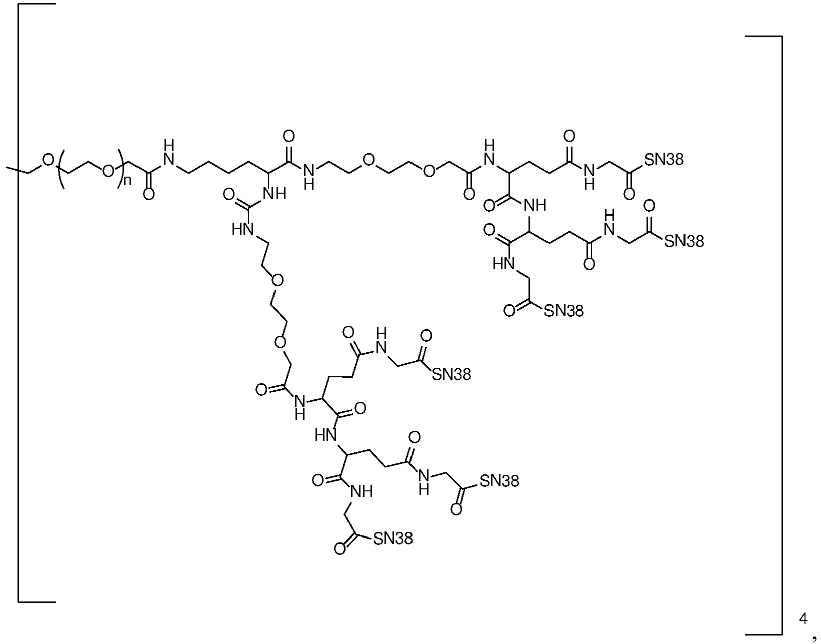 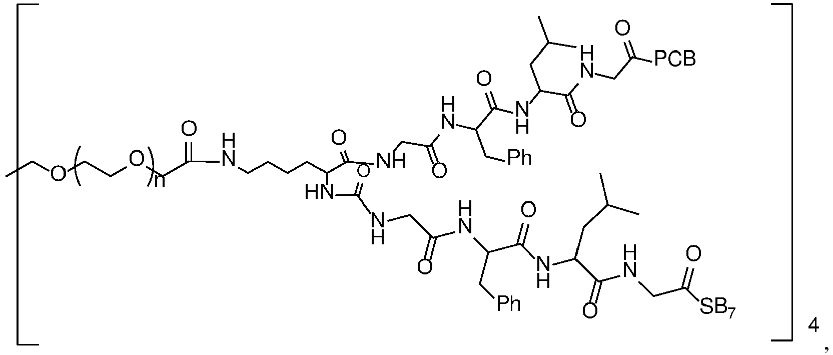 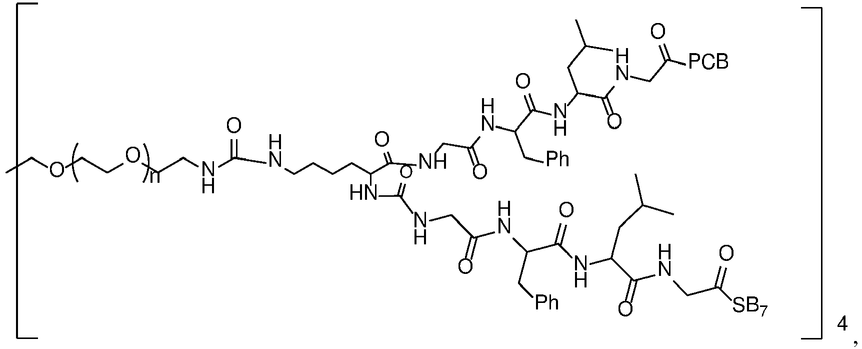 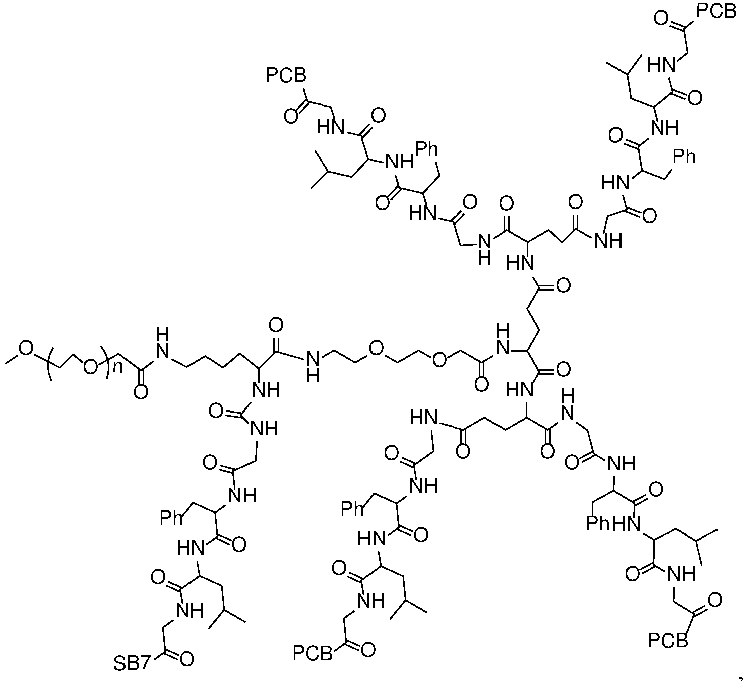 and

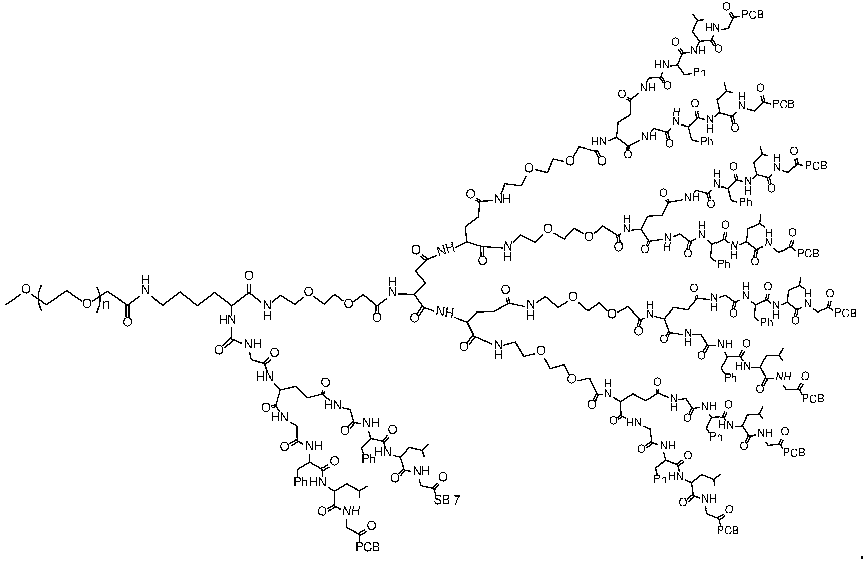

In some embodiments, in the polyethylene glycol conjugated drug of the disclosure or a pharmaceutically acceptable salt thereof, Y represents Glu.

In some embodiments, Y represents Glu and X2 is 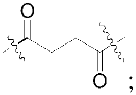 X1 is -NH-; W1 is N1-AC1; W2 represents

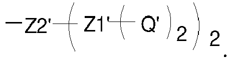

In some embodiments, the PEG is a single-arm polyethylene glycol segment with a number-average molecular weight of 10k-20k.

In some embodiments, N1 is GFLG and AC1 is SB7.

In some embodiments, Z2' is 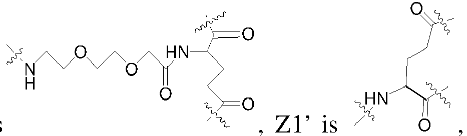 and Z0' is

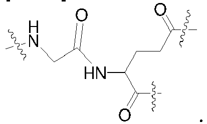

In some embodiments, N1' and N2' are both GFLG and AC1' and AC2' are both LPT.

In some embodiments, the polyethylene glycol conjugated drug has a structure as follows:

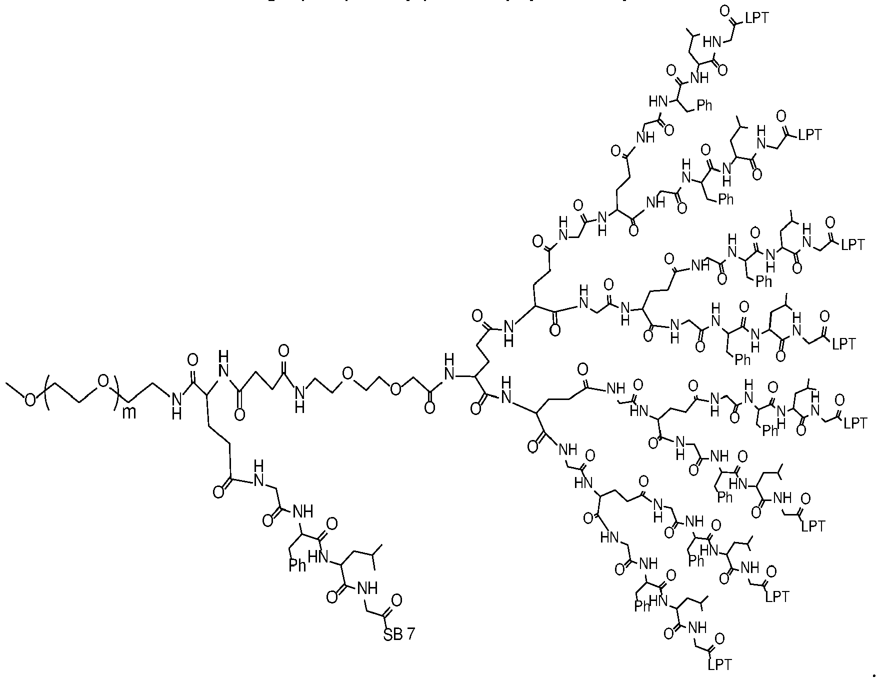

In some embodiments, Y represents Glu and X2 is 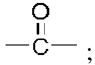 X1 is -NH-; W1 is N1-AC1; W2 represents

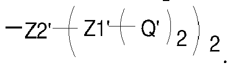

In some embodiments, the PEG is a single-arm polyethylene glycol segment with a number-average molecular weight of 10k-20k.

In some embodiments, N1 is GFLG and AC1 is SB7.

In some embodiments, Z2' and Z0' are 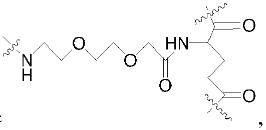 and Z1'

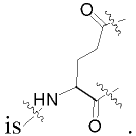

In some embodiments, N1' and N2' are both GFLG and AC1' and AC2' are both PCB.

In some embodiments, the polyethylene glycol conjugated drug has a structure as follows:

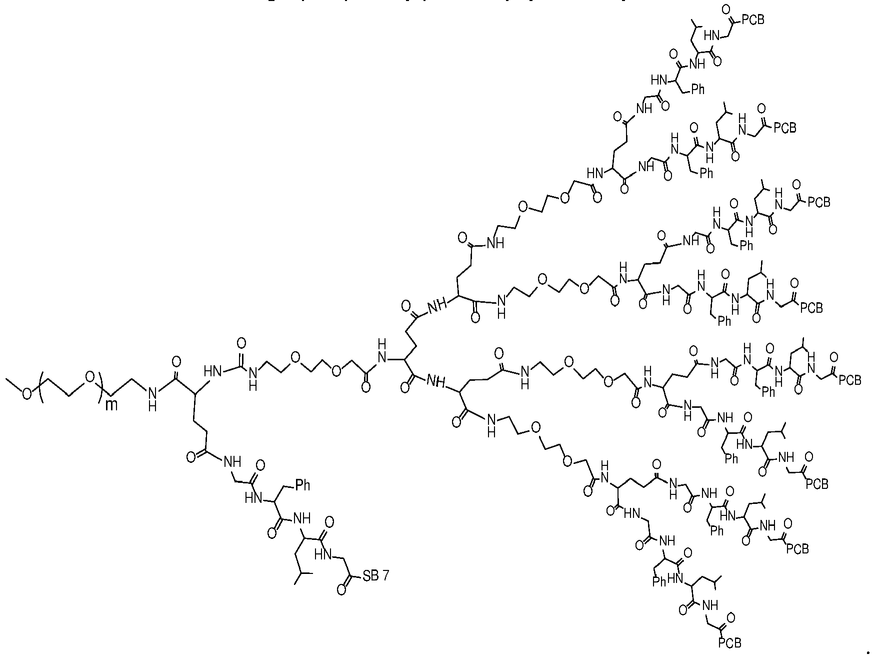

In some embodiments, Y represents Glu and X2 is 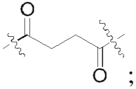 X1 is -NH-; W1 is Q; W2 is N1'-AC1'.

In some embodiments, the PEG is a four-arm polyethylene glycol segment with a number-average molecular weight of 20k-40k.

In some embodiments, N1 and N2 are both G and AC1 and AC2 are both SN38.

In some embodiments, N1' is G and AC1' is SN38.

In some embodiments, the polyethylene glycol conjugated drug has a structure as follows:

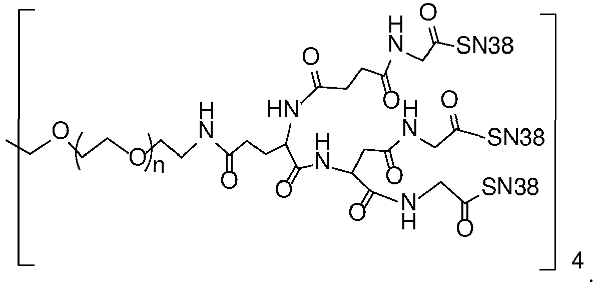

The polyethylene glycol conjugated drug of the disclosure includes but is not limited to:

| | |
|---|---|
| 1 | 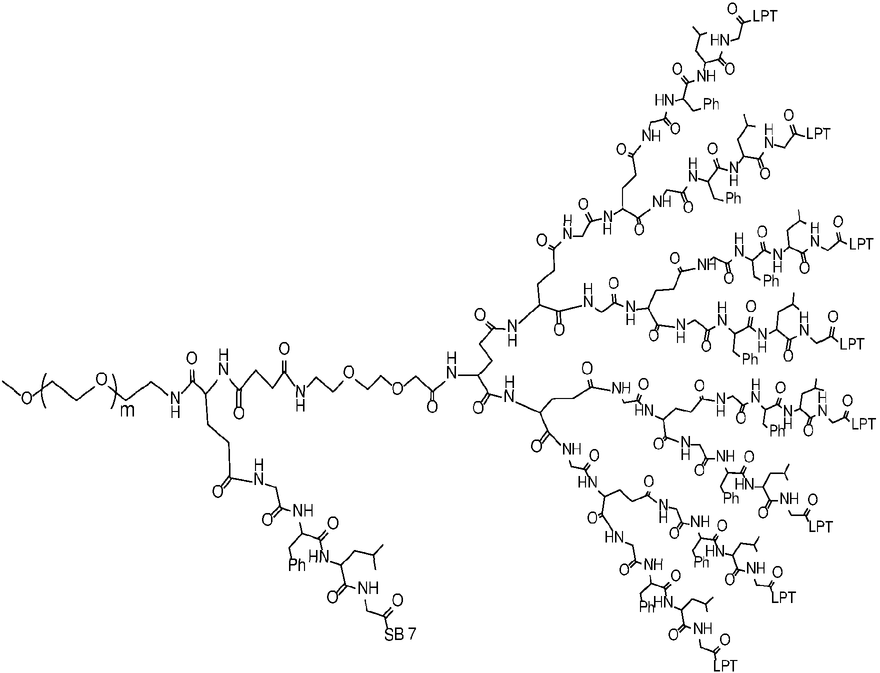 |
| | wherein PEG has a number-average molecular weight of 20k; |
| 2 | 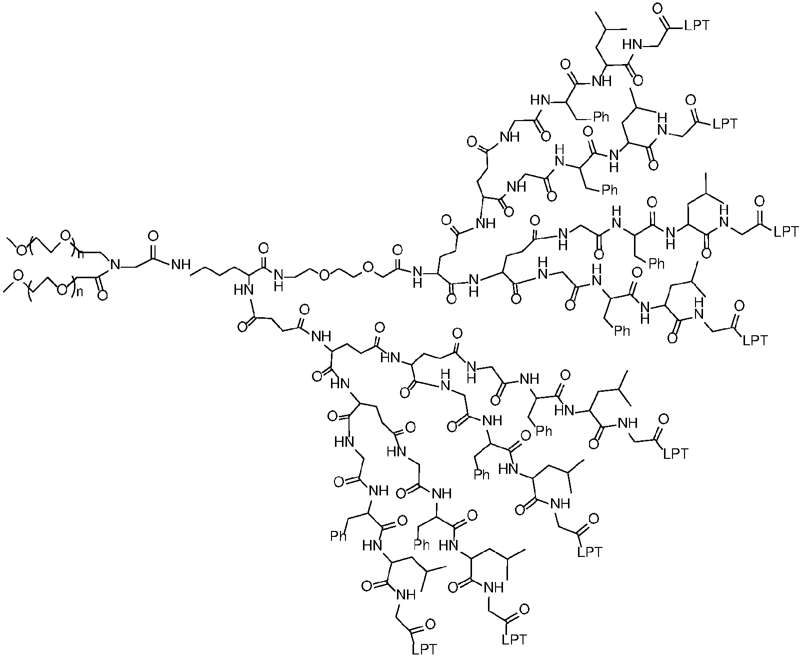 |
| | wherein PEG has a number-average molecular weight of 40k; |
| 3 | 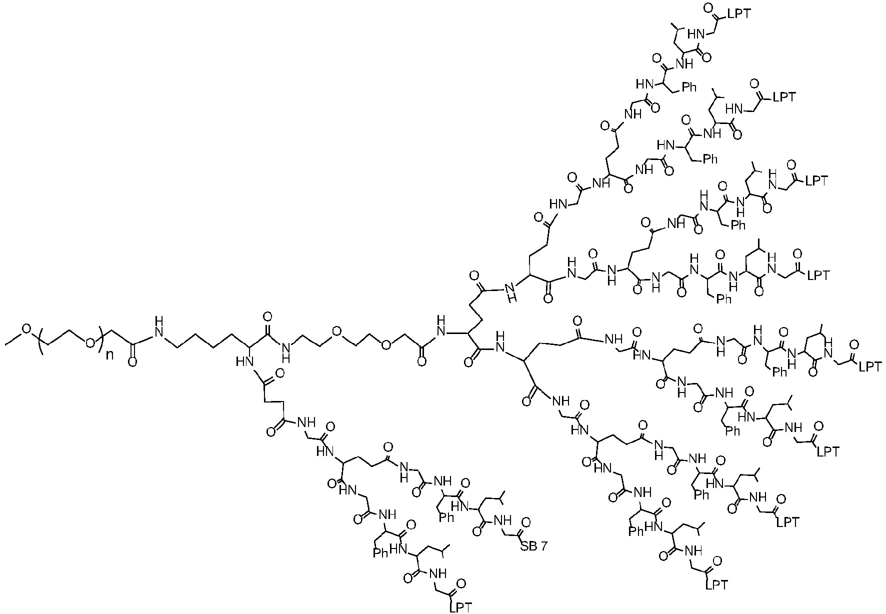 |
| | wherein PEG has a number-average molecular weight of 40k; |
| 4 | 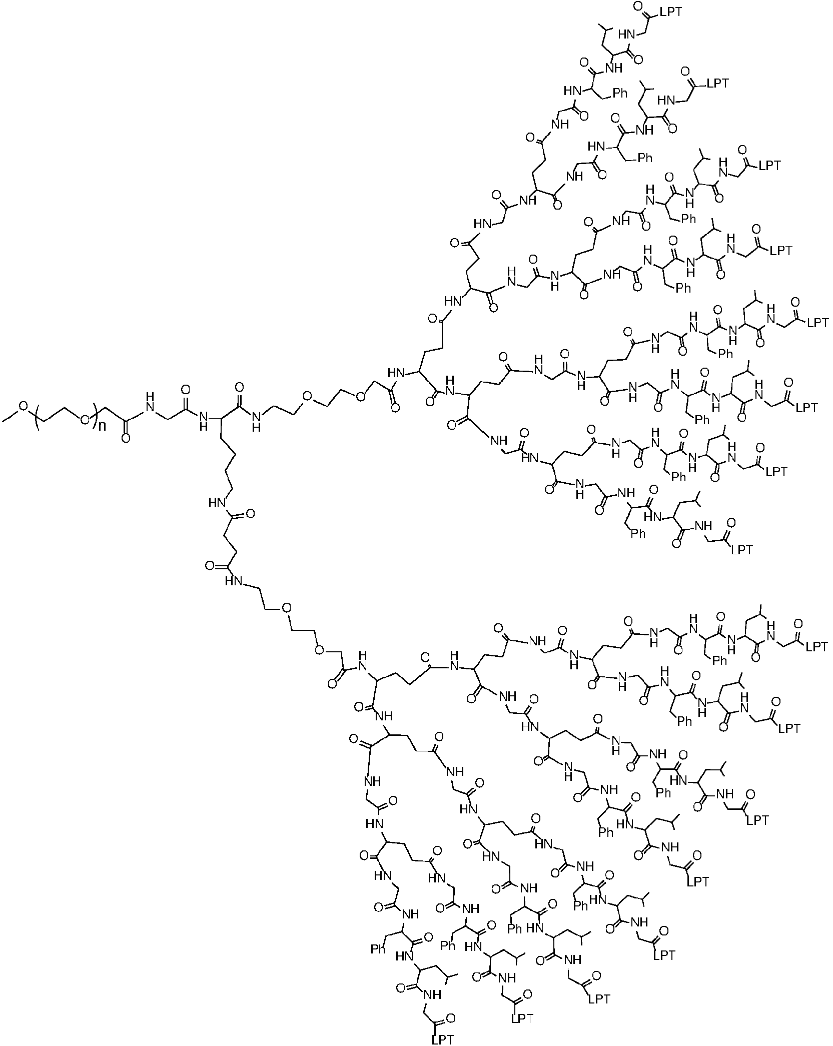 |
| | wherein PEG has a number-average molecular weight of 40k; |
| 5 | 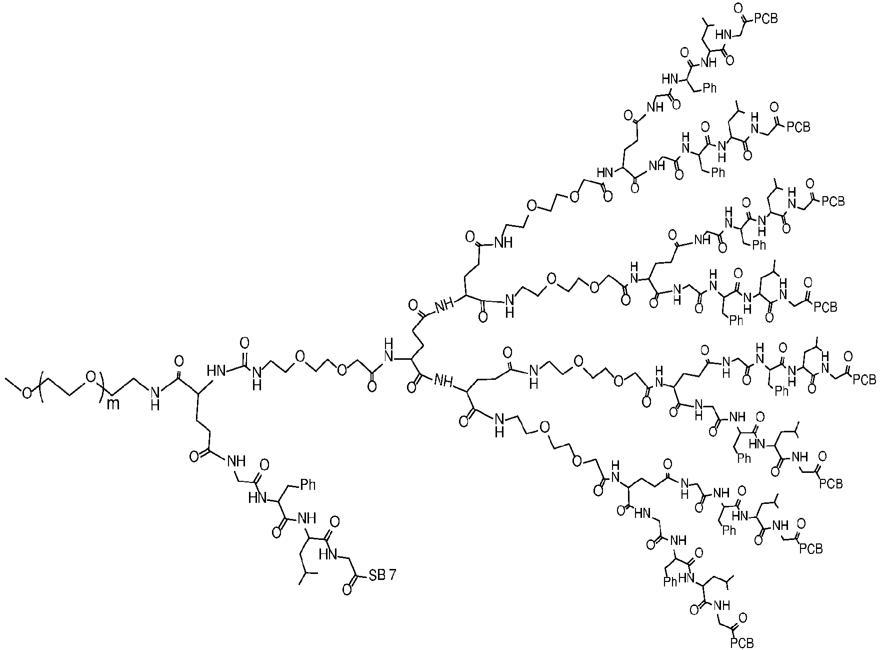 |
| | wherein PEG has a number-average molecular weight of 20k; |
| 6 | 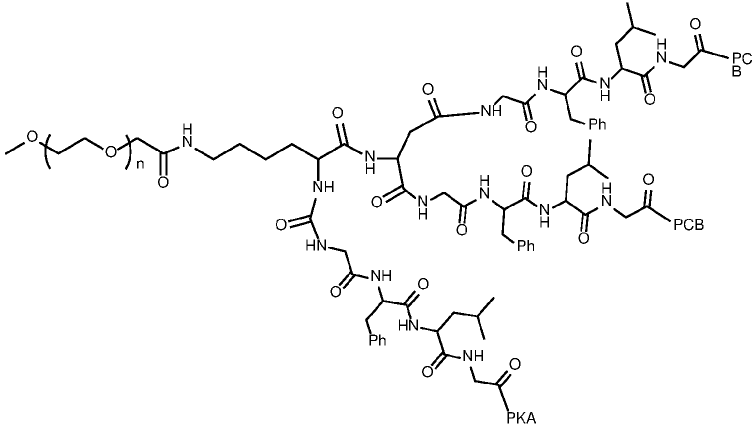 |
| | wherein PEG has a number-average molecular weight of 5k; |
| 7 | 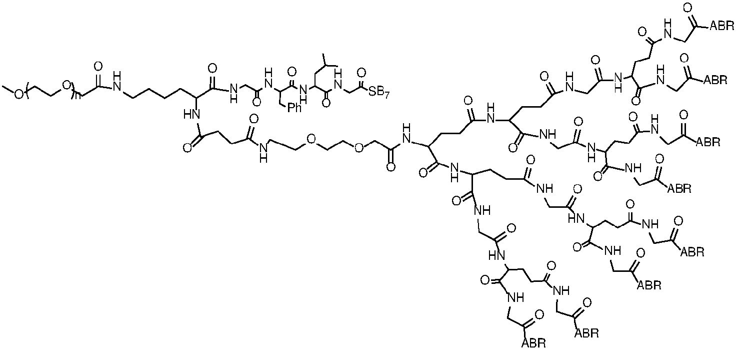 |
| | wherein PEG has a number-average molecular weight of 40k; |
| 8 | 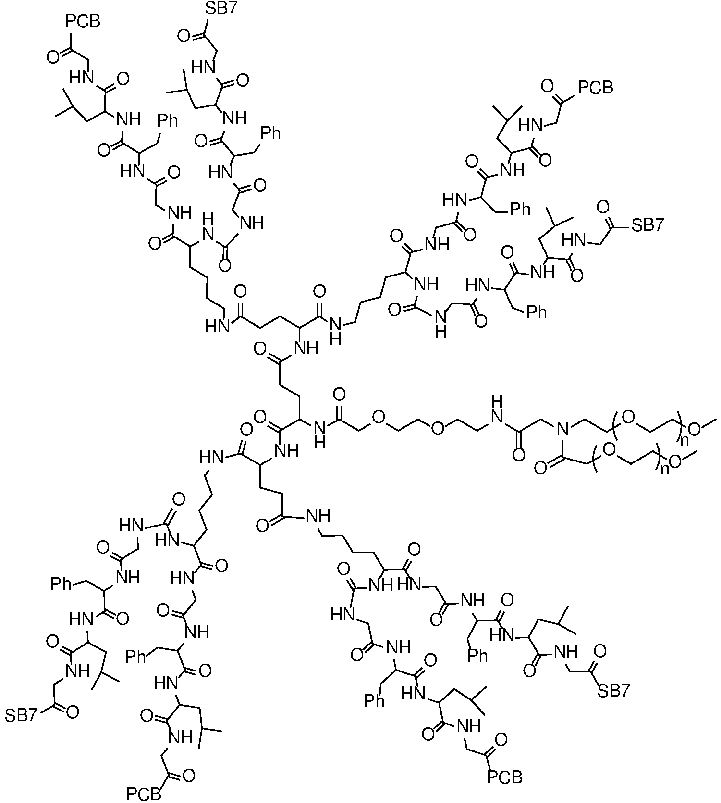 |
| | wherein PEG has a number-average molecular weight of 40k; |
| 9 | 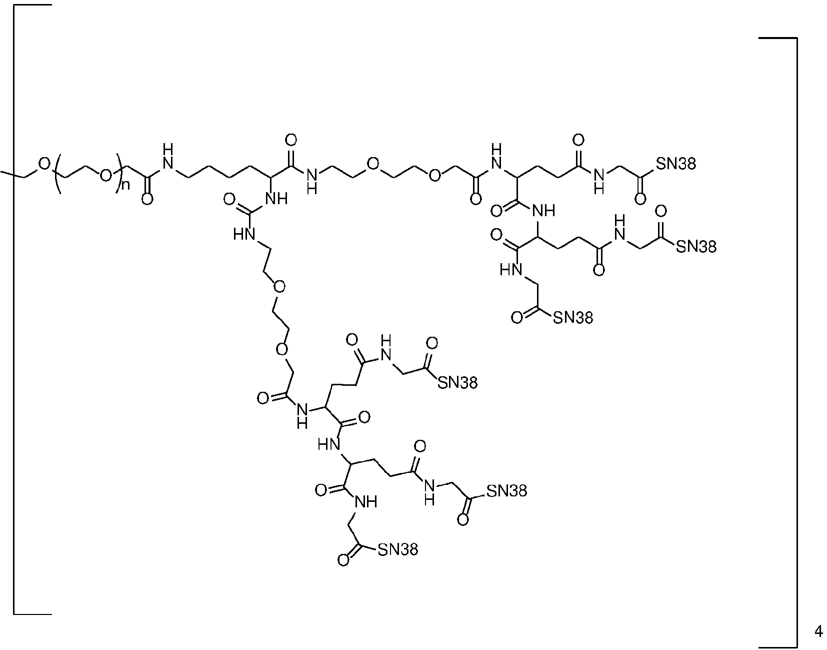 |
| | wherein PEG has a number-average molecular weight of 40k; |
| 10 | 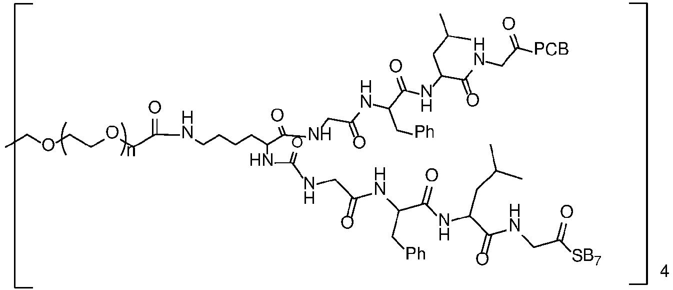 |
| | wherein PEG has a number-average molecular weight of 40k; |
| 11 | 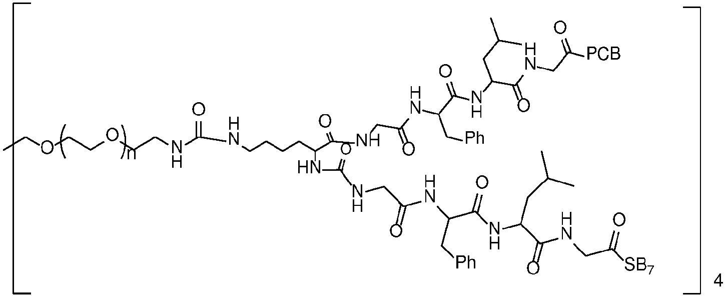 |
| | wherein PEG has a number-average molecular weight of 40k; |
| 12 | 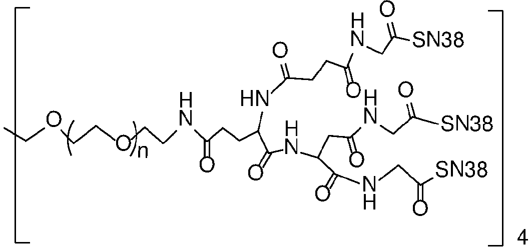 |
| | wherein PEG has a number-average molecular weight of 40k; |
| 13 | 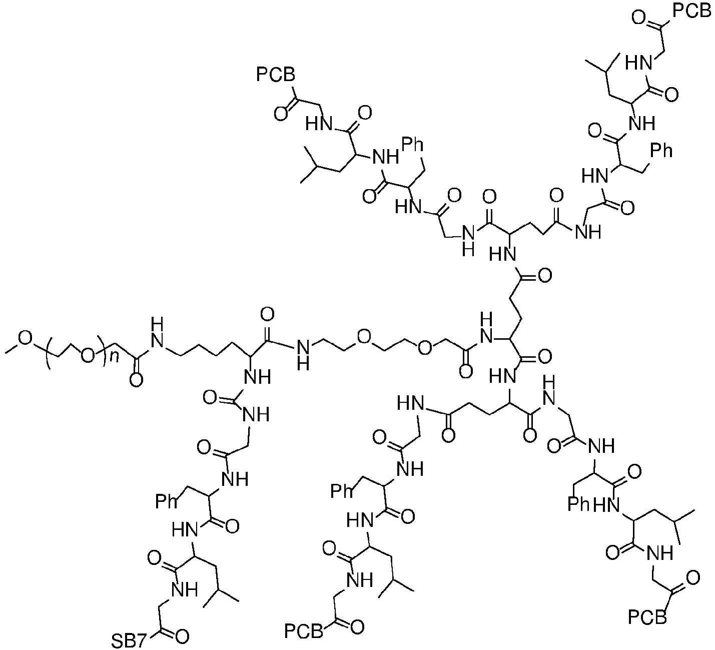 |
| | wherein PEG has a number-average molecular weight of 20k; |
| 14 | 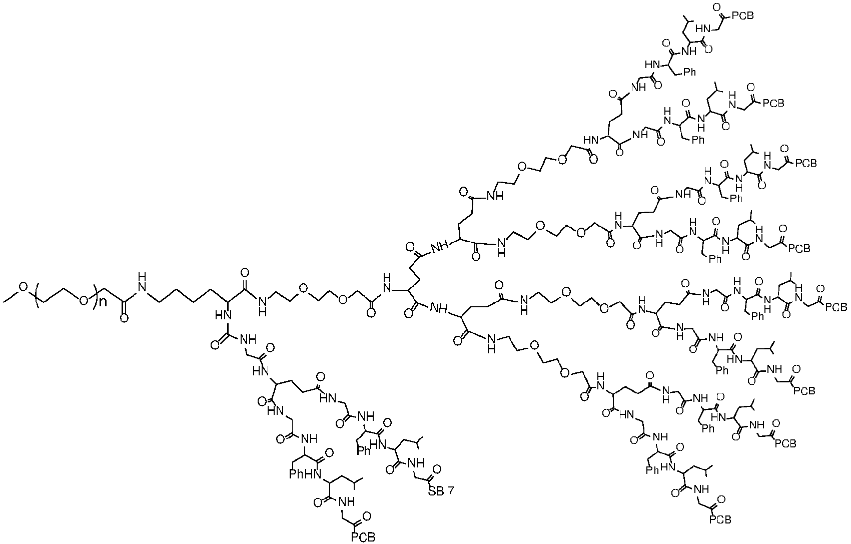 |
| | wherein PEG has a number-average molecular weight of 40k. |

### 2. Pharmaceutical composition and its use and preparations

In one aspect, this disclosure provides a pharmaceutical composition, including the polyethylene glycol conjugated drug of the disclosure or a pharmaceutically acceptable salt thereof in a therapeutically and/or prophylactically effective amount. In some embodiments, the composition further includes one or more pharmaceutically acceptable excipients.

The pharmaceutical composition of the disclosure may be prepared into any pharmaceutically acceptable dosage form. For example, the pharmaceutical composition of the disclosure may be prepared into tablets, capsules, pills, granules, solutions, suspensions, syrups, injection preparations (including injection solutions, sterile powders for injection, and concentrated solutions for injection), suppositories, inhalants or sprays.

In addition, the pharmaceutical composition of the disclosure may also be applied to patients or subjects in need of such treatment in any suitable way of administration, such as oral, parenteral, rectal or pulmonary administration. In the case of oral administration, the pharmaceutical composition may be prepared into oral preparations, for example, oral solid preparations, such as tablets, capsules, pills, granules, etc.; it may also be prepared into oral liquid preparations, such as oral solutions oral suspensions, syrups, etc. When the pharmaceutical composition is prepared into oral preparations, suitable fillers, binders, disintegrants, lubricants, etc. may be added. In the case of parenteral administration, the pharmaceutical composition may be prepared into injection preparations, including injection solutions, sterile powders for injection, and concentrated solutions for injection. When the pharmaceutical composition is prepared into injection preparations, they may be produced by a conventional method in the current pharmaceutical field. In the case of preparation of injection preparations, it is not required to add additives, or appropriate additives may be added according to the nature of the drug. In the case of rectal administration, the pharmaceutical composition may be prepared into suppositories and the like. In the case of pulmonary administration, the pharmaceutical composition can be made into an inhalant or a spray.

The subject of the disclosure is a mammal, such as a bovine, an equine, an ovine, a swine, a canine, a feline, a rodent, and a primate; more preferably, the subject is a human.

In some embodiments, the pharmaceutical composition is prepared in the form of an injection.

In some embodiments, the pharmaceutical composition is used to treat and/or prevent cancers.

In one aspect, the disclosure provides use of the polyethylene glycol conjugated drug of the disclosure or a pharmaceutically acceptable salt thereof in the preparation of a medicament for treating and/or preventing a disease (such as a cancer). The disease refers to a disease treated by an active ingredient in the polyethylene glycol conjugated drug.

In one aspect, this disclosure provides a method for treating and/or preventing a cancer, including administering an effective amount of the polyethylene glycol conjugated drug of the disclosure or a pharmaceutically acceptable salt thereof to a subject in need.

In some embodiments, the cancer is selected from colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, and sarcoma, as well as metastasis of these cancers.

In one aspect, the disclosure further provides an injection solution, including the polyethylene glycol conjugated drug of the disclosure or a pharmaceutically acceptable salt thereof, or the pharmaceutical composition of the disclosure. In some embodiments, saline (normal saline) is used as a carrier for the injection solution.

### 3. Preparation method and intermediates

In one aspect, this disclosure provides a method (Method 1) for preparing the polyethylene glycol conjugated drug of the disclosure or a pharmaceutically acceptable salt thereof, including the following steps:
step 1: preparing an intermediate W1-Y-X2-W2, wherein Y has a free or activated carboxyl group; and
step 2: carrying out an amidation reaction so that PEG with amino groups is linked to Y on the intermediate W1-Y-X2-W2 to obtain a polyethylene glycol conjugated drug of formula (I), wherein the number of amino groups is denoted as j, and the amino groups are free or activated amino groups;

PEG, X2, Y, W1, W2, and j are as defined above.

In some embodiments, in the polyethylene glycol conjugated drug, Y represents Glu, X1 represents -NH-, W1 represents N1-AC1, and W2 represents

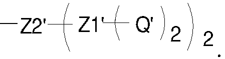

In some embodiments, the intermediate W1-Y-X2-W2 of Method 1 is prepared by a method including the following steps:
step 1: preparing an intermediate W1-Y-X2 and an intermediate W2 respectively, wherein X2 has a free or activated carboxyl group, Y has a protected carboxyl group, and W2 has a free or activated amino group;
step 2: causing the free or activated carboxyl group on X2 to react with the free or activated amino group on W2 so that W1-Y-X2 is linked to W2; and
step 3: deprotecting the protected carboxyl group on Y and optionally carrying out activation to obtain the intermediate W1-Y-X2-W2.

In one aspect, this disclosure provides a method (Method 2) for preparing the polyethylene glycol conjugated drug of the disclosure or a pharmaceutically acceptable salt thereof, including the following steps:
step 1: preparing an intermediate W1-Y-X2-W2, wherein Y has a free or activated amino group; and
step 2: carrying out an amidation reaction so that PEG with carboxyl groups is linked to Y on the intermediate W1-Y-X2-W2 to obtain a polyethylene glycol conjugated drug of formula (I), wherein the number of carboxyl groups is denoted as j, and the carboxyl groups are free or activated carboxyl groups;

PEG, X2, Y, W1, W2, and j are as defined above.

In some embodiments, Y in the polyethylene glycol conjugated drug refers to Lys.

In some embodiments, the intermediate W1-Y-X2-W2 of Method 2 is prepared by a method including the following steps:
step 1: preparing an intermediate W1-Y-X2 and an intermediate W2 respectively, wherein Y has a protected amino group, X2 has a free or activated carboxyl group, and W2 has a free or activated amino group;
step 2: causing the free or activated carboxyl group on X2 to react with the free or activated amino group on W2 so that W1-Y-X2 is linked to W2; and
step 3: deprotecting the protected amino group on Y and optionally carrying out activation to obtain the intermediate W1-Y-X2-W2.

In some embodiments, in the polyethylene glycol conjugated drug, Y refers to Lys and X1 and X2 are both

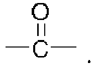

In some embodiments, in the polyethylene glycol conjugated drug, Y refers to Lys, X1 is 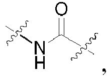 and X2 is

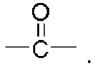

In some embodiments, in the polyethylene glycol conjugated drug, Y refers to Lys, X1 is 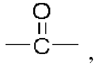 and X2 is

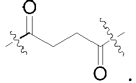

In some embodiments, the intermediate W1-Y-X2-W2 of Method 2 is prepared by a method including the following steps:
step 1: preparing an intermediate W1-Y and an intermediate X2-W2 respectively, wherein Y has 2 amino groups, one of which is a free or activated amino group and the other is a protected amino group, and X2 has a free or activated carboxyl group;
step 2: causing the free or activated amino group on Y to react with the free or activated carboxyl group on X2 so that W1-Y is linked to X2-W2; and
step 3: deprotecting the protected amino group on Y and optionally carrying out activation to obtain the intermediate W1-Y-X2-W2.

In some embodiments, in the polyethylene glycol conjugated drug, Y refers to Lys, X1 is 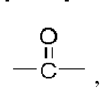 and X2 is

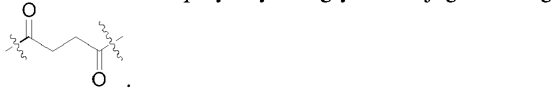

In some embodiments, N-ACs on W1 and W2 are the same. The intermediate W1-Y-X2-W2 of Method 2 is prepared by a method including the following steps:
step 1: preparing intermediates N-AC, W1'-Y and X2-W2' respectively, wherein Y has 2 amino groups, one of which is a free or activated amino group and the other is a protected amino group, X2 has a free or activated carboxyl group, W1' and W2' are respectively precursors of W1 and W2 and said W1' and W2' have not been linked to N-AC, W1' and W2' have the same group M (e.g., hydroxyl group) that can react with N-AC, and M is protected;
step 2: causing the free or activated amino group on Y to react with the free or activated carboxyl group on X2 to obtain an intermediate W1'-Y-X2-W2';
step 3: deprotecting M but not deprotecting the protected amino group;
step 4: linking N-AC to W1' and W2'; and
step 5: deprotecting the protected amino group on Y and optionally carrying out activation to obtain the intermediate W1-Y-X2-W2.

In some embodiments, in the polyethylene glycol conjugated drug, Y refers to Lys, X1 is 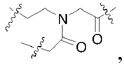 X2 is 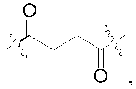 and the structure of the PEG is

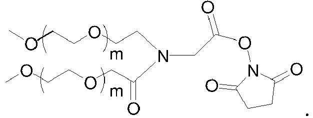

In some embodiments, N-ACs on W1 and W2 are the same. The intermediate W1-Y-X2-W2 of Method 2 is prepared by a method including the following steps:
step 1: preparing intermediates N-AC, W1'-Y-X2 and W2' respectively, wherein Y has a protected amino group, X2 has a free or activated carboxyl group, W2' has a free or activated amino group, W1' and W2' are respectively precursors of W1 and W2 and said W1' and W2' have not been linked to N-AC, each of W1' and W2' has a group M (e.g., hydroxyl group) that can react with N-AC, and M is protected;
step 2: causing the free or activated amino group on W2' to react with the free or activated carboxyl group on X2 to obtain an intermediate W1'-Y-X2-W2';
step 3: deprotecting M but not deprotecting the protected amino group;
step 4: linking N-AC to W1' and W2'; and
step 5: deprotecting the protected amino group on Y and optionally carrying out activation to obtain the intermediate W1-Y-X2-W2.

In the polyethylene glycol conjugated drug of the disclosure or a pharmaceutically acceptable salt thereof, N-ACs on W1 and W2 may be the same, and X1 may be 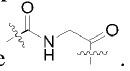 This disclosure provides a method (Method 3) for preparing the polyethylene glycol conjugated drug of the disclosure or a pharmaceutically acceptable salt thereof, including the following steps:
step 1: preparing intermediates N-AC, W1'-Y-X2 and W2' respectively, wherein X2 has a free or activated carboxyl group, Y has a protected amino group, W2' has a free or activated amino group, W1' and W2' are respectively precursors of W1 and W2 and said W1' and W2' have not been linked to N-AC, each of W1' and W2' has a group M (e.g., hydroxyl group) that can react with N-AC, and M is protected;
step 2: causing the free or activated amino group on W2' to react with the free or activated carboxyl group on X2 to obtain an intermediate W1'-Y-X2-W2';
step 3: deprotecting the amino group on Y but not deprotecting the protected group of M;
step 4: causing the amino group on Y to react with a carboxyl group on Boc-Gly-OH (glycine with a protected amino group) to obtain an intermediate 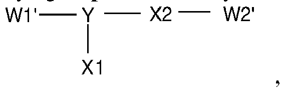 wherein X1 has a protected amino group;
step 5: deprotecting M but not deprotecting the protected amino group;
step 6: linking N-AC to W1' and W2';
step 7: deprotecting the protected amino group on X1 and optionally carrying out activation to obtain an intermediate 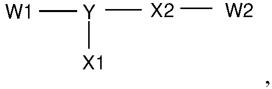 wherein X1 has a free or activated amino group; and
step 8: carrying out an amidation reaction so that PEG with carboxyl groups is linked to X1 on the intermediate 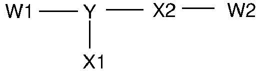 to obtain a polyethylene glycol conjugated drug of formula (I), wherein the number of carboxyl groups is denoted as j, and the carboxyl groups are free or activated carboxyl groups;
PEG, X2, Y, W1, W2, and j are as defined above.

In some embodiments, Y in the polyethylene glycol conjugated drug refers to Lys.

In some embodiments, in the polyethylene glycol conjugated drug, Y refers to Lys, X1 is 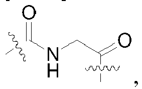 and X2 is

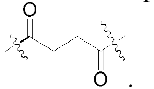

In the polyethylene glycol conjugated drug of the disclosure or a pharmaceutically acceptable salt thereof, N-ACs on W1 and W2 may be the same. This disclosure provides a method (Method 4) for preparing the polyethylene glycol conjugated drug of the disclosure or a pharmaceutically acceptable salt thereof, including the following steps:
step 1: preparing intermediates N-AC', W1'-Y-X2 and W2' respectively, wherein X2 has a free or activated carboxyl group, Y has a protected carboxyl group, W2' has a free or activated amino group, W1' and W2' are respectively precursors of W1 and W2 and said W1' and W2' have not been linked to N-AC', each of W1' and W2' has a group M (e.g., hydroxyl group) that can react with N-AC', M is protected, AC' is a precursor of AC and AC' is protected by a protecting group (e.g., TBDPS);
step 2: causing the free or activated amino group on W2' to react with the free or activated carboxyl group on X2 to obtain an intermediate W1'-Y-X2-W2';
step 3: deprotecting M but not deprotecting the protected carboxyl group;
step 4: linking N-AC' to W1' and W2';
step 5: deprotecting the protected carboxyl group on Y and optionally carrying out activation to obtain an intermediate W1"-Y-X2-W2", wherein Y has a free or activated carboxyl group, W1" is a precursor of W1 and W1" is the one without removing the protecting group on AC', and W2" is a precursor of W2 and W2" is the one without removing the protecting group on AC';
step 6: carrying out an amidation reaction so that PEG with amino groups is linked to the intermediate W1"-Y-X2-W2" to obtain an intermediate 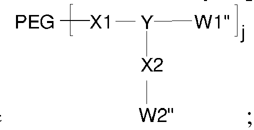 and
step 7: removing the protective groups of AC's on W1" and W2" to obtain a polyethylene glycol conjugated drug of formula (I),
wherein PEG, X1, X2, W1, W2, and Y are as defined above.

In some embodiments, in the polyethylene glycol conjugated drug, Y refers to Glu, X1 is -NH-, and X2 is

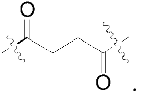

In some embodiments, in the polyethylene glycol conjugated drug, W1 is Q and W2 is N1'-AC1'.

In some embodiments, AC in the polyethylene glycol conjugated drug is SN38.

In the polyethylene glycol conjugated drug of the disclosure or a pharmaceutically acceptable salt thereof, N-ACs on W1 and W2 may be the same. This disclosure provides another method (Method 5) for preparing the polyethylene glycol conjugated drug of the disclosure or a pharmaceutically acceptable salt thereof, including the following steps:
step 1: preparing intermediates N-AC', W1'-Y and X2-W2' respectively, wherein X2 has a free or activated carboxyl group, Y has two amino groups, one of which is a free or activated amino group and the other is a protected amino group, W1' and W2' are respectively precursors of W1 and W2 and said W1' and W2' have not been linked to N-AC', each of W1' and W2' has a group M (e.g., hydroxyl group) that can react with N-AC', M is protected, AC' is a precursor of AC and AC' is protected by a protecting group (e.g., TBDPS);
step 2: causing the free or activated carboxyl group on X2 to react with the free or activated amino group on Y to obtain an intermediate W1'-Y-X2-W2';
step 3: deprotecting M but not deprotecting the protected amino group;
step 4: linking N-AC' to W1' and W2';
step 5: deprotecting the protected amino group on Y and optionally carrying out activation to obtain an intermediate W1"-Y-X2-W2", wherein Y has a free or activated amino group, W1" is a precursor of W1 and W1" is the one without removing the protecting group on AC', and W2" is a precursor of W2 and W2" is the one without removing the protecting group on AC';
step 6: carrying out an amidation reaction so that PEG with carboxyl groups is linked to Y on the intermediate W1"-Y-X2-W2" to obtain an intermediate 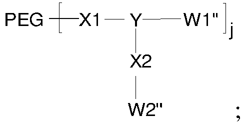 and
step 7: removing the protective groups of AC's on W1" and W2" to obtain a polyethylene glycol conjugated drug of formula (I),
wherein PEG, X1, X2, Y, W1, and W2 are as defined above.

In some embodiments, in the polyethylene glycol conjugated drug, Y refers to Lys and X1 and X2 are both

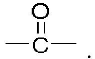

In some embodiments, in the polyethylene glycol conjugated drug, W1 is 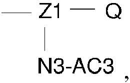 and W2 is

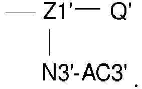

In some embodiments, AC in the polyethylene glycol conjugated drug is SN38.

In one aspect, this disclosure provides a compound having any of the following structures
W1-Y-X2-W2, W1'-Y-X2-W2', W1"-Y-X2-W2", 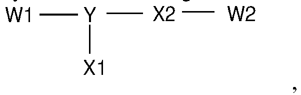 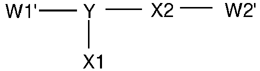 or 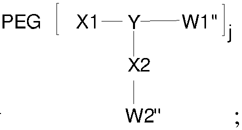
wherein X1, X2, Y, W1, W2, W1', W2', W1', W2' and PEG are as defined above.

The disclosure further provides the following compounds:

| | |
|---|---|
| 1 | 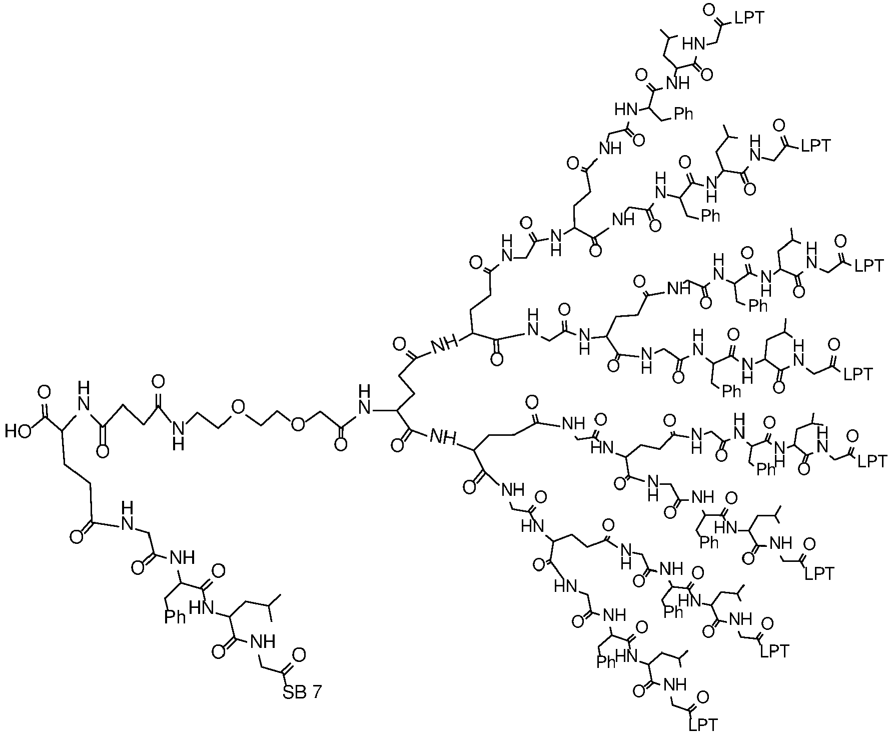 |
| 2 | 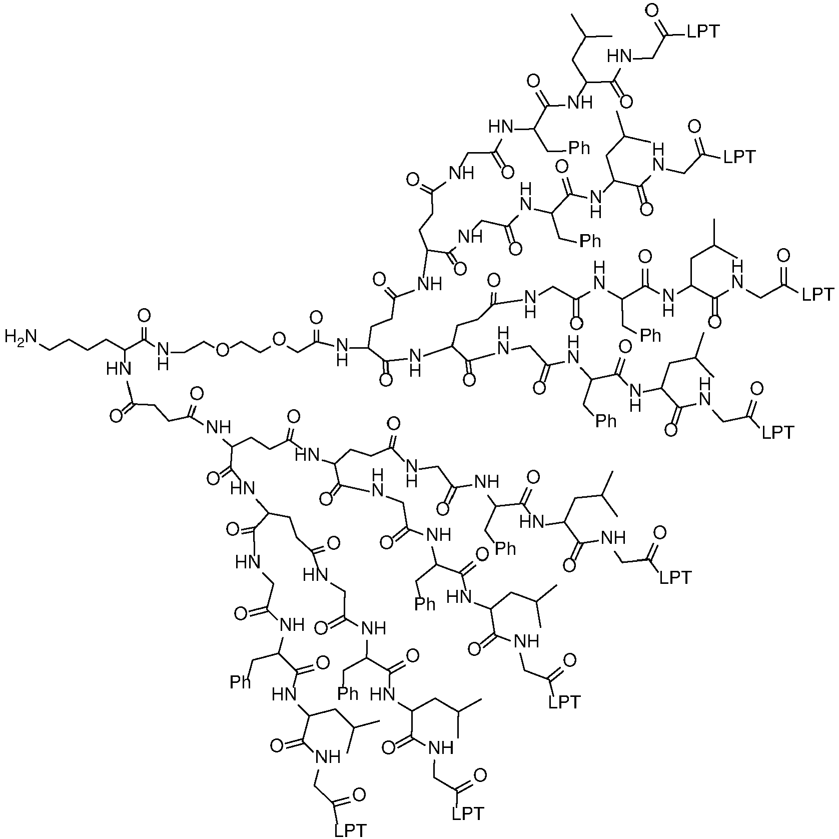 |
| 3 | 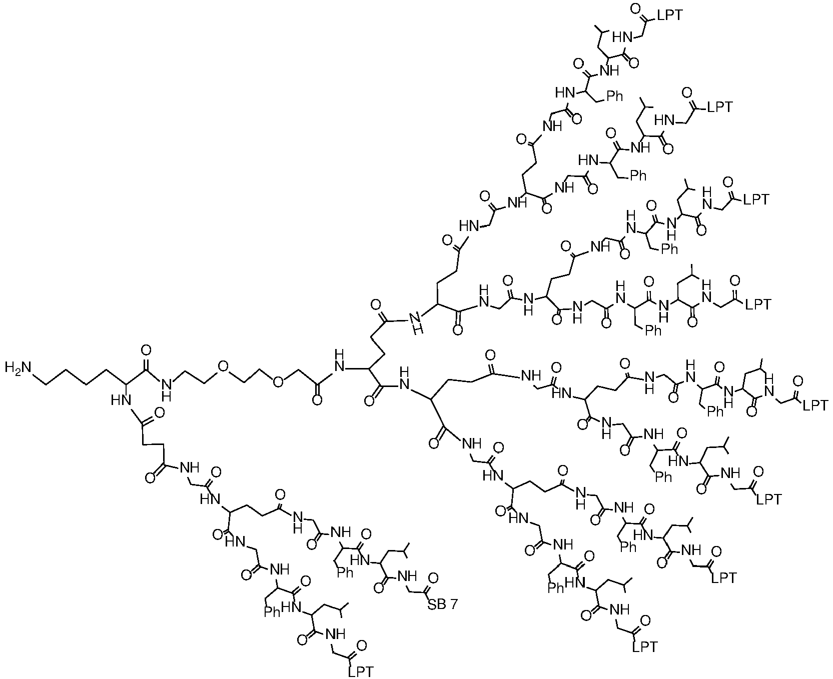 |
| 4 | 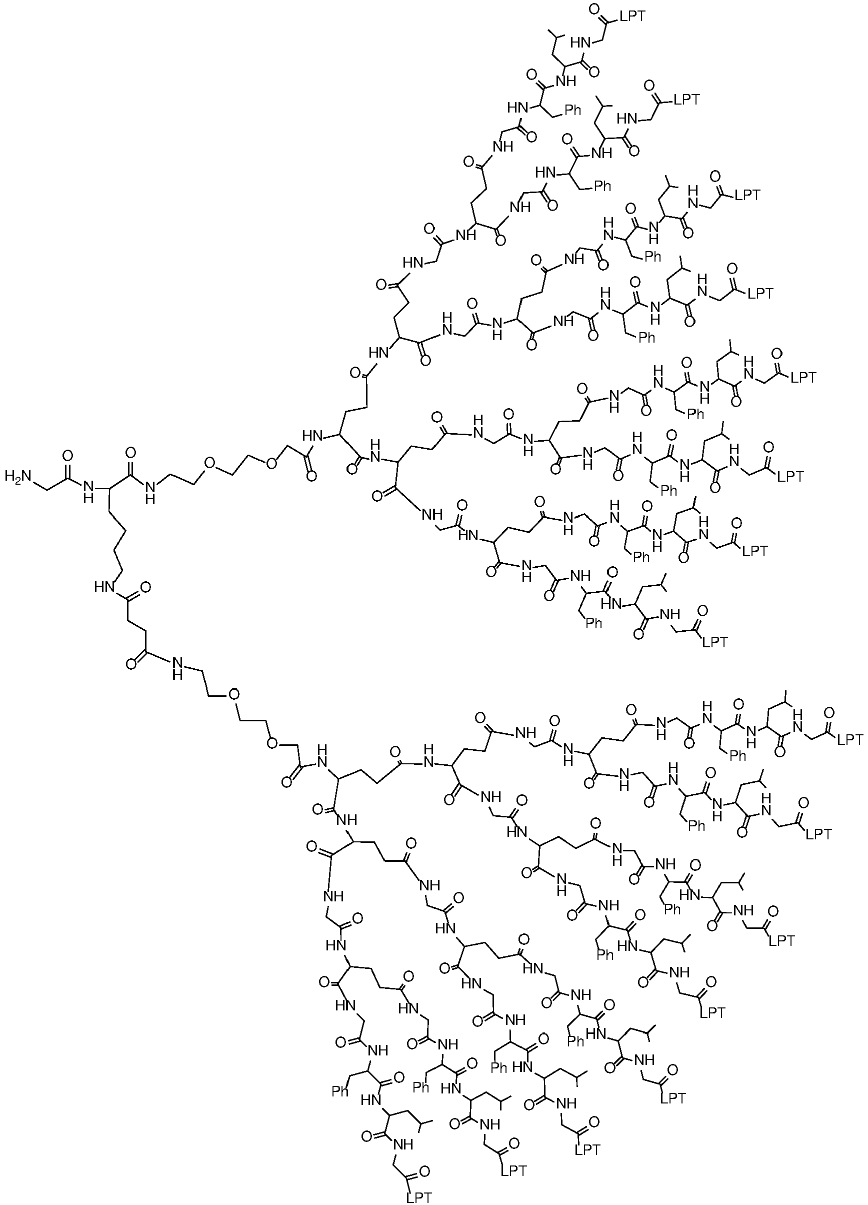 |
| 5 | 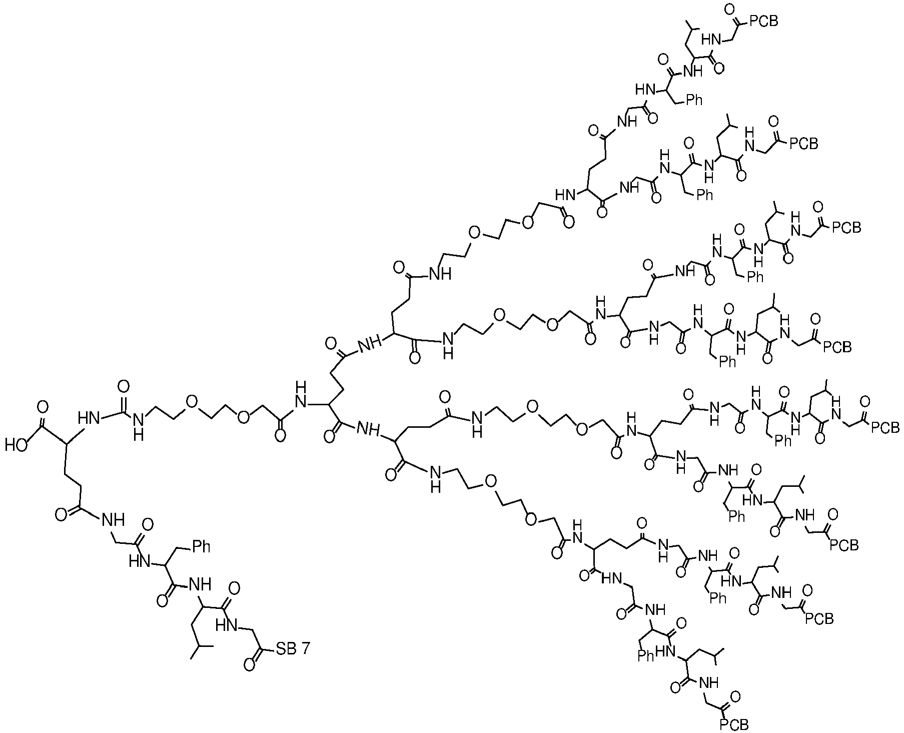 |
| 6 | 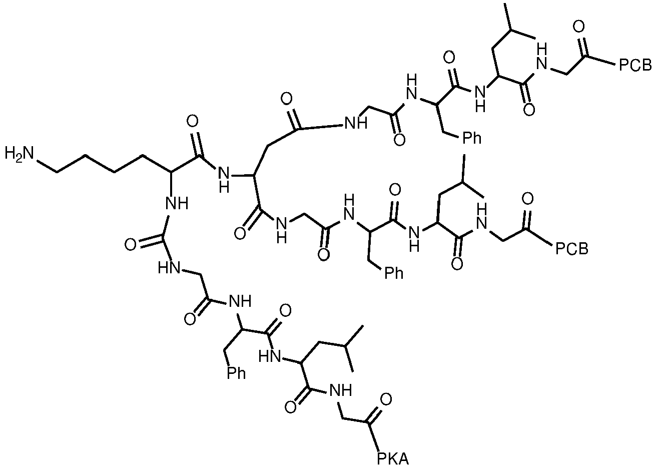 |
| 7 | 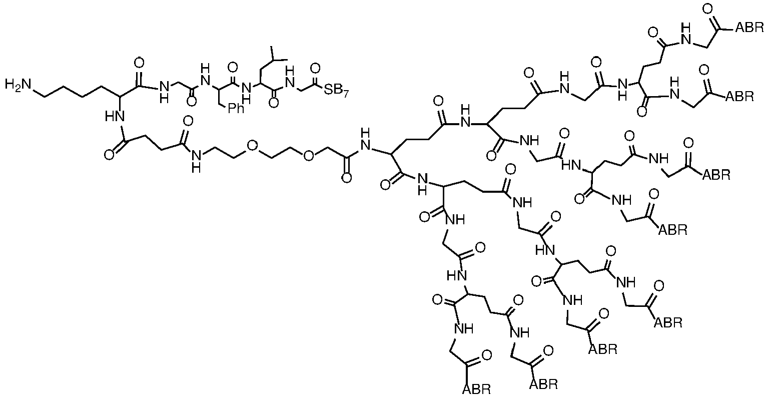 |
| 8 | 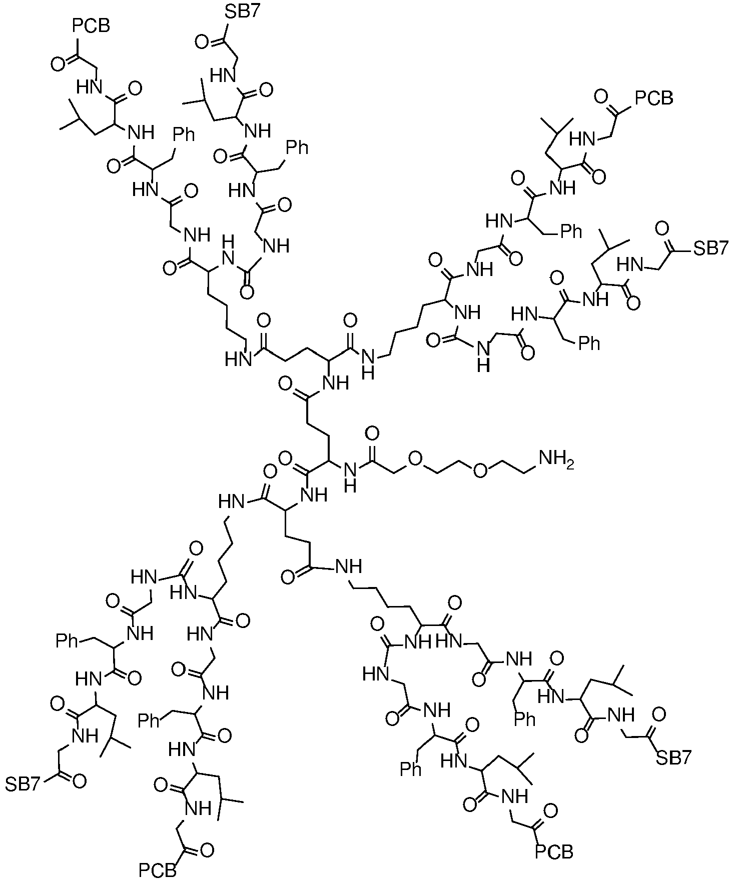 |
| 9 | 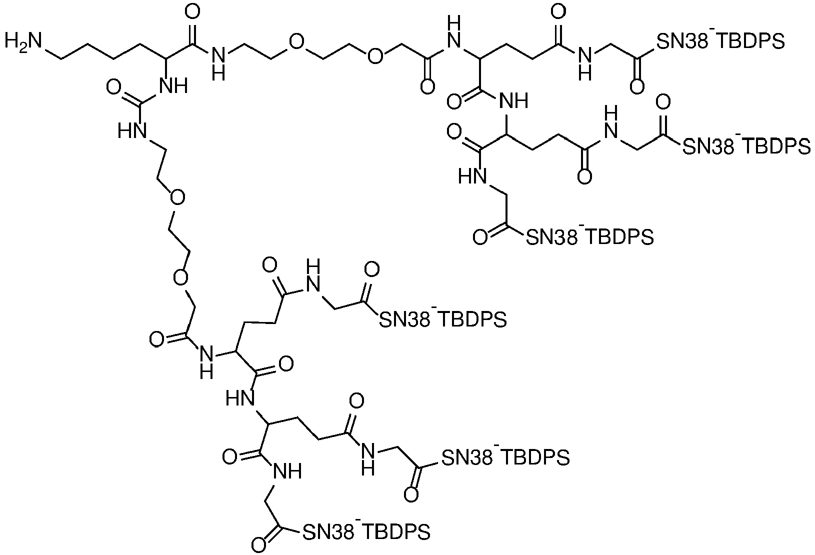 |
| 10 | 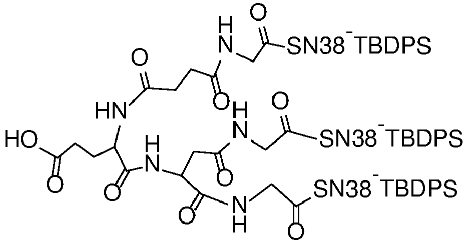 |
| 11 | 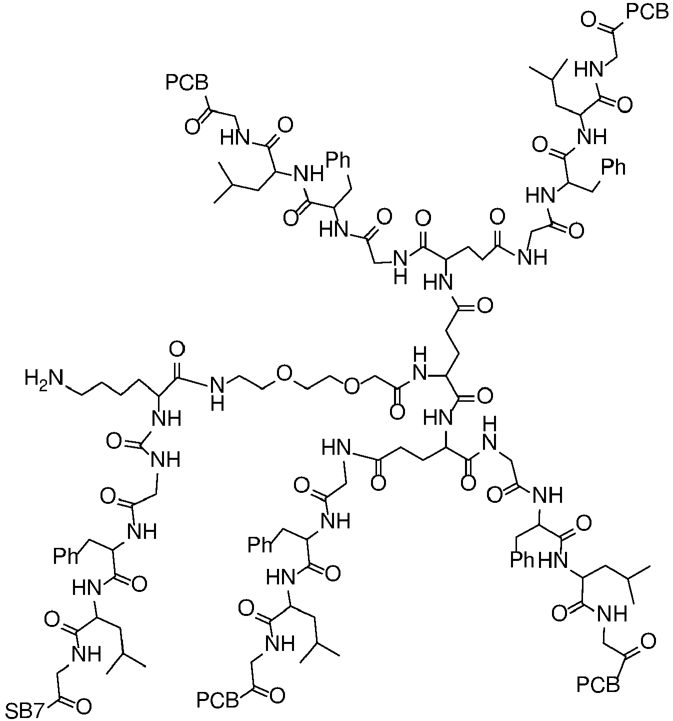 |
| 12 | 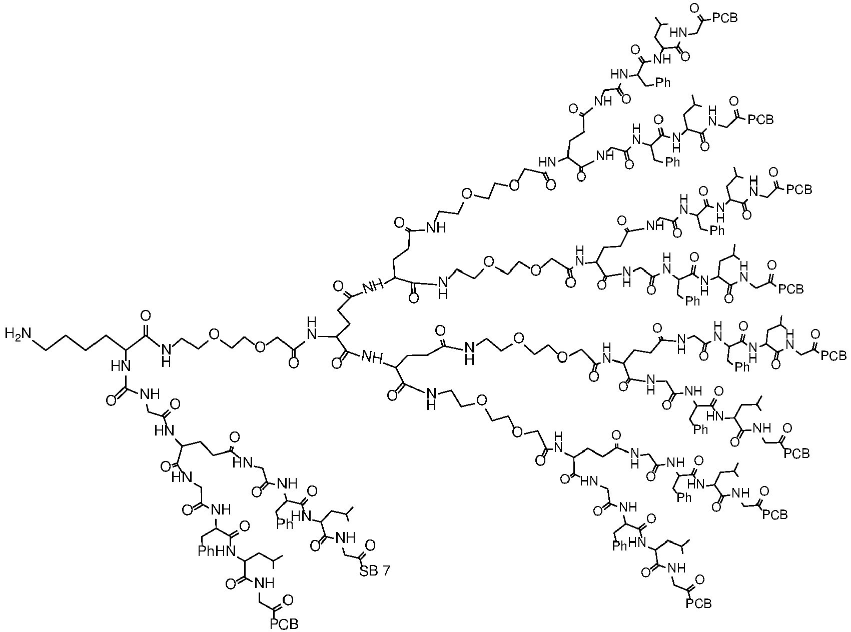 |

;

Optionally, the free amino group(s) or free carboxyl group(s) in the compounds are protected or activated.

In one aspect, the disclosure provides use of the compound (as an intermediate) in preparation of the polyethylene glycol conjugated drug of the disclosure or a pharmaceutically acceptable salt thereof.

### Definition of terms

Unless otherwise defined below, the meanings of all technical and scientific terms used herein are intended to be the same as those commonly understood by those skilled in the art. The reference to the technology used herein is intended to refer to the technology generally understood in the art, including those technical changes or equivalent technology substitutions that are obvious to those skilled in the art. Although it is believed that the following terms are well understood by those skilled in the art, the following definitions are still set forth to better explain the disclosure.

As used herein, "PEG" is an abbreviation for polyethylene glycol, which refers to a homopolymer with a repeating unit of -CH₂CH₂O-, including single-arm polyethylene glycol, multi-arm polyethylene glycol and their derivatives, such as derivatives with reactive functional groups such as amino or carboxyl group at the terminal. In the disclosure, the arms of the multi-arm polyethylene glycol preferably have the same degree of polymerization. When referring to the molecular weight of the multi-arm polyethylene glycol, the molecular weight means the total molecular weight of each arm. In the structural formula of the disclosure, the letter m or n in the subscript of the repeating unit of polyethylene glycol represents the degree of polymerization.

As used herein, the "pharmaceutically acceptable salt" of the polyethylene glycol conjugated drug includes an acid addition salt and base addition salt of the polyethylene glycol conjugated drug. such as hydrochloride, hexafluorophosphate, and meglumine salt.

As used herein, the wavy line " 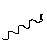 " in the structural formula means the position where other groups are bonded to the structure represented by the structural formula.

As used herein, the term "effective amount" refers to the amount of a compound that will relieve one or more symptoms of the disease being treated to a certain extent after being administered.

As used herein, the term "treating" means reversing, alleviating, or inhibiting the disease or condition to which such term is applied or the progression of one or more symptoms of such a disease or condition, or preventing such a disease or condition or one or more symptoms of such a disease or condition.

### Beneficial Effect

The polyethylene glycol conjugated drug of the disclosure may achieve high drug loading capacity and can achieve flexible adjustment of the location of the drug and the types and proportions of dual drugs or multiple drugs.

The embodiments of the disclosure will be described in detail below in conjunction with the accompanying drawings and examples. However, those skilled in the art will understand that the following drawings and examples are only used to illustrate the disclosure, not to limit the scope of the disclosure. Various objectives and advantageous aspects of the disclosure will become apparent to those skilled in the art based on the accompanying drawings and the following detailed description of the preferred embodiments.

### BRIEF DESCRIPTION OF THE DRAWINGS

FIG. 1 shows an average tumor volume growth trend of each group in Experimental Example 1.
FIG. 2 shows a schematic diagram showing a tumor weight inhibition rate of each group in Experimental Example 1.

### DETAILED DESCRIPTION

The embodiments of the disclosure will be described in detail below in conjunction with examples. However, those skilled in the art will understand that the following examples are only used to illustrate the disclosure, not to limit the scope of the disclosure. Those without specific conditions in the examples are generally implemented under conventional conditions or conditions recommended by the manufacturers. The reagents or instruments used without specifying the manufacturers are all conventional products that can be purchased commercially.

The meanings of abbreviations in the examples are as follows:

| | | | |
|---|---|---|---|
| G | Glycine residue | L | Leucine residue |
| F | Phenylalanine residue | Asp | Aspartic acid residue |
| E | Glutamate residue | Glu | Glutamate residue |
| Lys | Lvsine residue | TFA | Trifluoroacetate |
| DMF | N, N-dimethylformamide | Bn | Benzvl |
| Boc | Tert-butoxycarbonyl | Fmoc | Fluorenyl methoxycarbonyl |
| HOBT | 1-hvdroxvbenzotriazole | Ts | P-toluenesulfonyl |
| HBTU | O-benzotriazole-tetramethylurea | LPT | Lapatinib |
| | hexafluorophosphate | | |
| DIEA | N,N-diisopropylethylamine | SB7 | SB-743921 |
| EA | Ethyl acetate | PCB | Palbociclib |
| PKA | PKI-587 with unterminated dimethyl group (Gedatolisib) | ABR | Abitron |
| SN38 | 7-ethyl-10-hydroxycamptothecin | TBDPS | Tert-butyldiphenylsilyl |
| LC | NH₂-CH₂CH₂O-CH₂CH₂O-CH₂-COOH or -NH-CH₂CH₂O-CH₂CH₂O-CH₂-CO- | | |

The source and structure of some raw materials are as follows:
M-NH₂HCl-5K
JenKem, **mPEG-**CH₂CH₂-NH₂HCl
M-SCM-10K
JenKem, 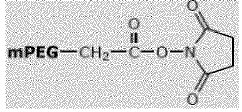
4ARM-SCM-40K/5K
JenKem, 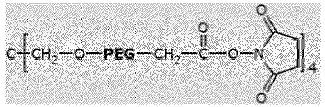
Y-SCM-40K
JenKem,

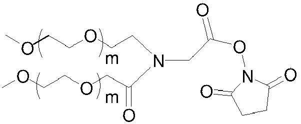

### Example 1: Synthesis of compound 24-231

30-28

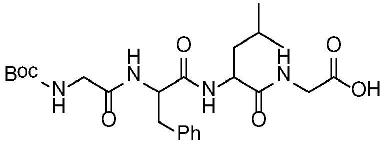

The raw material Boc-GFLG-OBn (synthesized as reported, 19.0 g, 32.6 mmoL) and 10% Pd/C (Palladium/Carbon) catalyst (300 mg) were added into a hydrogenation reactor and then dissolved with DMF (50 mL) where the level of the solvent was above a stirrer; the hydrogenation reaction device was then sealed to perform the "three pumping and three charging" operation (i.e., pumping the air from the reaction system with a vacuum water pump for about 3 min-charging hydrogen-pumping hydrogen-charging hydrogen---pumping hydrogen---charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (90 mL) until it did not contain any product, and then the reaction product was obtained.

30-29

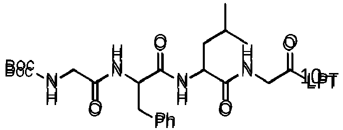

Compound 30-28 (17.9 mmoL), LPT (8 g, 13.77 mmoL), HBTU (7.83 g, 20.65 mmoL) and HOBT (2.79 g, 20.65 mmoL) were added in a 500 mL round-bottomed flask and then dissolved with DMF (100 mL), and the mixed solution was stirred at -5 °C for 30 min. Then DIEA (10.24 mL, 61.96 mmoL) was slowly added dropwise, and then the mixed solution reacted at a low temperature for 2 h; after that, the reaction device was placed at room temperature and the reaction solution was stirred overnight to react. At the end of the reaction, deionized water (1000 mL) was added to rinse DMF, and a pale yellow solid was precipitated and then dried to obtain 14.53 g of the product.

14-128

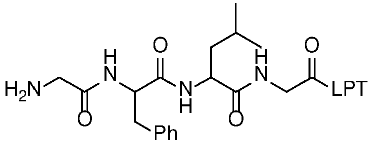

Compound 30-29 (14.53 g, 13.77 mmoL) was added in a 500 mL round-bottomed flask, and then dissolved with dichloromethane (150 mL), and then TFA (15.34 mL, 206.55 mmoL) was added and the obtained solution was stirred at room temperature to react overnight. At the end of the reaction, the reaction solution was concentrated under reduced pressure, saturated sodium bicarbonate (200 mL) was added to neutralize the TFA, the product in the aqueous phase was extracted three times with ethyl acetate (150 mL×3), and the obtained organic phases were combined, then dried with anhydrous sodium sulfate, filtered by suction, concentrated, and dried; the operations of dry sample loading, column chromatography, and elution with 5% methanol/0.5% ammonia water/dichloromethane were carried out, and then the elution product was then collected and concentrated, and 13.15 g of the product was obtained.

24-162

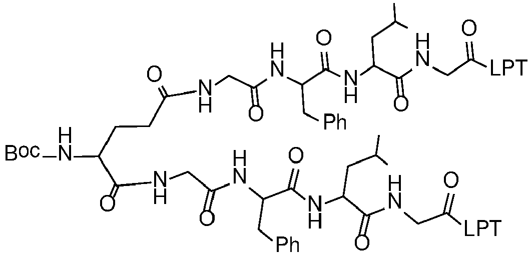

Boc-Glu-(OH) (0.8962 g, 3.6247 mmoL), GFLG-LPT (7.1 g, 7.4307 mmoL, synthesized according to the synthesis method of Compound 14-128), HBTU (4.1239 g, 10.8741 mmoL), and HOBT (1.4693 g, 10.8741 mmoL) were weighed and added in a 500 mL flask and then completely dissolved with DMF (30 mL) in a condition of ultrasonic. The obtained solution was stirred at -5 °C for 30 min. DIEA (5.3918 mL, 32.6223 mmoL) was then slowly added dropwise, and the mixed solution was stirred at a low temperature for 2 h and then placed at room temperature to react to the end. At the end of the reaction, the reaction solution was transferred to a 2 L separatory funnel, ethyl acetate (400 mL) and saturated sodium bicarbonate solution (300 mL) were then added, the organic phase was then separated, and the aqueous phase was extracted twice with ethyl acetate (100 mL × 2) until there was no product in the aqueous phase. The obtained organic phases were combined and washed once with saturated sodium chloride solution (100 mL), and then concentrated and evaporated to dryness. 9.8 g of the product was obtained with a yield of 100%, 2.2 g being extra-quota.

24-164

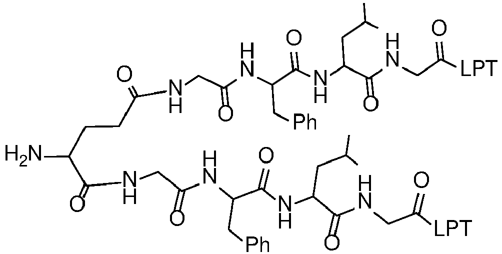

Compound 24-162 (7.69 g, 3.6247 mmoL) was weighed, dichloromethane (10 mL) and TFA (8.0768 mL, 108.741 mmoL) were then added sequentially, and the obtained solution was treated by ultrasonic until Compound 24-162 was completely dissolved; a ground glass stopper was used, and the mixed solution was stirred at room temperature to react. At the end of the reaction, the reaction solution was evaporated to dryness and the dichloromethane was removed; n-hexane (150 mL) was added and the obtained solution was treated by ultrasonic for 2 min and then rested for 10 min; the supernatant was discarded; ethyl acetate (10 mL) was added to the lower solution and the obtained solution was then thoroughly treated by ultrasonic; methyl tert-butyl ether (100 mL) and n-hexane (100 mL) were then added and the obtained solution was filtered by suction and the solid was dried. 10 g of the product was obtained with a yield of 100%, 3 g being extra-quota.

24-165

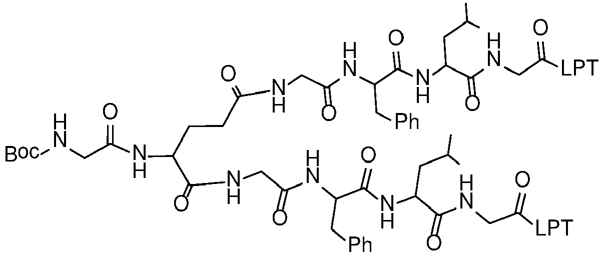

Compound 24-164 (7.3 g, 3.6247 mmoL), Boc-Gly-(OH) (0.6985 g, 3.9872 mmoL), HBTU (2.0619 g, 5.4371 mmoL), and HOBT (0.7347 g, 5.4371 mmoL) were weighed and added in a 500 mL flask and then completely dissolved with DMF (50 mL) in a condition of ultrasonic. The obtained solution was stirred at -5 °C for 30 min. DIEA (2.9771 mL, 17.9424 mmoL) was then slowly added dropwise, and the mixed solution was stirred at a low temperature for 2 h and then placed at room temperature to react to the end. At the end of the reaction, methyl tert-butyl ether (30 mL) and n-hexane (300 mL) were added to the reaction solution and the obtained solution was treated by ultrasonic for 2 min and then rested for 10 min in a refrigerator; the supernatant was discarded as impurities. The above operations were repeated twice. Ethyl acetate (20 mL), methyl tert-butyl ether (50 mL) and n-hexane (200 mL) were added to the lower solution and the obtained solution was then treated by ultrasonic for 2 min and filtered by suction; the obtained solid was dissolved with 20% methanol/dichloromethane (300 mL) by ultrasonic treatment, silica gel powder (30 g) was then added, and the obtained solution was evaporated to dryness with a rotary evaporator and then subjected to column chromatography. Elution with 1% ammonia water +4% methanol/dichloromethane was carried out. 5.8 g of the product was obtained with a yield of 74%.

24-170

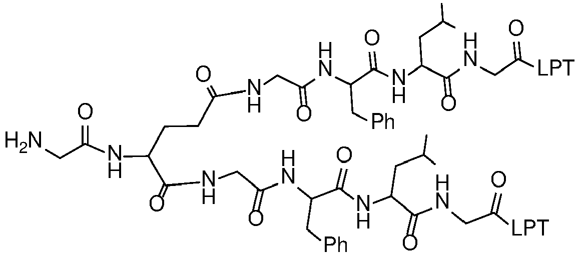

Compound 24-165 (5.8 g, 2.6614 mmol) was weighed, dichloromethane (15 mL) and TFA (4.9419 mL, 66.535 mmoL) were then added sequentially, and the obtained solution was treated by ultrasonic until Compound 24-165 was completely dissolved; a ground glass stopper was used, and the mixed solution was stirred at room temperature to react. At the end of the reaction, the reaction solution was evaporated to dryness and the dichloromethane was removed; methyl tert-butyl ether (150 mL) was added and the obtained solution was treated by ultrasonic for 2 min; n-hexane (50 mL) was then added and the obtained solution was filtered by suction; the obtained solid product was completely dissolved with 20% methanol/dichloromethane (70 mL), silica gel powder (25 g) was then added, and the obtained solution was evaporated to dryness with a rotary evaporator and then subjected to column chromatography. Gradient elution with 1% ammonia water +4%-5% methanol/dichloromethane was carried out. 4.8 g of the product was obtained with a yield of 87%.

16-34

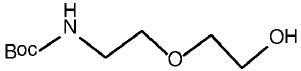

2-(2-aminoethoxy) ethanol (18.8680 g, 190.2226 mmoL) was weighed and poured into a 500 mL round-bottomed flask and then diluted with dichloromethane (100 mL), triethylamine (38.4972 mL, 380.4452 mmoL) was then added, and (Boc)₂O (49.8261 g, 228.2671 mmoL) was then added slowly with stirring, and the obtained solution was stirred at room temperature to react. At the end of the reaction, the reaction solution was evaporated to dryness, then sodium bicarbonate powder was added, the obtained mixture was diluted with dichloromethane, silica gel powder was added, and the operations of evaporating, dry sample loading, and column chromatography were carried out. Elution with 50% ethyl acetate/petroleum ether was carried out. 27.3 g of the product was obtained with a yield of 70%.

16-36

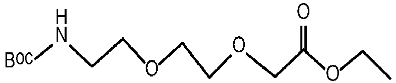

Compound 16-24 (27.3 g, 132.8144 mmoL) was added in a 500 mL flask, nitrogen was introduced in the flask for protective purpose, the THF solution of potassium tert-butoxide was added, the mixed solution was placed at 0 °C to react, ethyl bromoacetate (17.6265 mL, 159.3773 mmoL) was then added, and the obtained solution was first stirred for 3 h, and then moved to room temperature for reaction. At the end of the reaction, the reaction solution was first evaporated to dryness, then deionized water and ethyl acetate were added, and the organic phase was separated; the aqueous phase was extracted with ethyl acetate until there was no product in the aqueous phase; the organic phases were combined, dried with anhydrous sodium sulfate powder, and filtered by suction. The filtrate was subjected to dry sample loading and column chromatography. Gradient elution with 30%-100% ethyl acetate/petroleum ether was carried out. 20 g of the product was obtained with a yield of 52%.

24-36

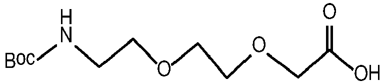

Compound 16-36 (17.9 g, 61.4402 mmoL) was added in a 250 mL flask, 1,4-dioxane was added, and lithium hydroxide (3.2386 g, 135.1685 mmoL) was further added with stirring, and 30 min later, deionized water was added until the solution became clear. At the end of the reaction, the reaction solution was extracted three times with a mixed solution of methyl tert-butyl ether and n-hexane (1:1) (100 mL×3). The aqueous phase was adjusted to pH=1 with concentrated hydrochloric acid, and then extracted three times with ethyl acetate (300 mL×3), the ethyl acetate phases were combined, the dissolution and washing with saturated sodium chloride was carried out three times (100 mL×3), the obtained solution was concentrated, and the operations of dry sample loading, column chromatography, and then gradient elution with 40%-100% ethyl acetate/petroleum ether were carried out. 10.1 g of the product was obtained with a yield of 62%.

14-154

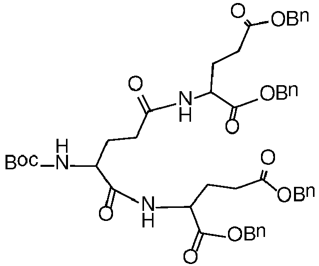

Compound Boc-Glu-OH (10 g, 40.5 mmoL), H-Glu-(OBn)₂·TsOH (42.5 g, 85.1 mmoL), HOBT (16.5 g, 121.5 mmoL) and HBTU (46.1 g, 121.5 mmoL) were added in a 500 mL round-bottomed flask and then dissolved with DMF (100 mL), and the mixed solution was stirred at -5 °C for 30 min. Then DIEA (66.2 mL, 400.5 mmoL) was slowly added dropwise and then the reaction device was placed at -5 °C and the reaction solution was stirred to react for 2 h; then the reaction solution further reacted overnight at room temperature. At the end of the reaction, the reaction product was washed with saturated sodium bicarbonate solution and then extracted three times with EA; the extract was washed twice with saturated sodium chloride solution, water was then removed with anhydrous sodium sulfate, filtering by suction was carried out, and the filtrate was then evaporated to dryness. The theoretical weight of the product was 35.07 g.

14-155

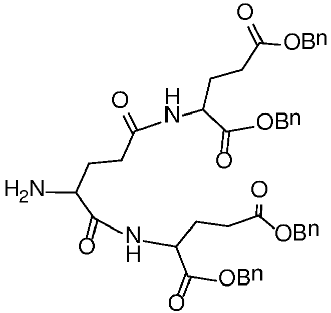

Compound 14-154 (35.1 g, 40.5 mmoL) was added in a 500 mL round-bottomed flask and then dissolved with CH₂Cl₂ (50 mL), and the mixed solution was stirred at room temperature. TFA (45 mL, 607.5 mmoL) was then added dropwise, and the mixed solution reacted at room temperature for 2 h. At the end of the reaction, the reaction solution was evaporated to dryness and the product was then dried in an oven. The operations of dry sample loading and column chromatography were carried out. Elution with 70% EA/PE was then carried out, and the elution product was collected and concentrated. 13.28 g (including extra-quota) of the product was obtained.

14-163

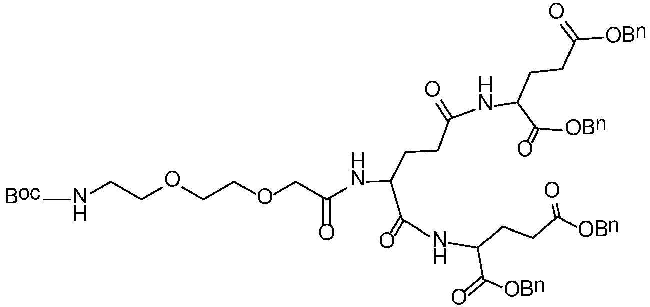

Compound 14-155 (31.06 g, 40.6 mmoL), Compound 10-102 (synthesized according to the synthesis method of Compound 24-36, 13.90 g, 52.8 mmoL), HOBT (8.23 g, 60.9 mmoL) and HBTU (23.10 g, 60.9 mmoL) were added in a 500 mL round-bottomed flask and then dissolved with DMF (150 mL), and the mixed solution was stirred at -5 °C for 30 min. Then DIEA (30.20 mL, 182.7 mmoL) was slowly added dropwise and then the reaction device was placed at -5 °C and the reaction solution was stirred to react for 2 h; then the reaction solution further reacted overnight at room temperature. At the end of the reaction, the reaction product was washed with saturated sodium bicarbonate solution and then extracted three times with EA; the extract was washed twice with saturated sodium chloride solution, the organic phase was then evaporated to dryness and dried in an oven. The operations of dry sample loading, column chromatography, and gradient elution with 0.2%-10% methanol/dichloromethane were carried out. 22.63 g of the product was obtained with a yield of 55.1%.

24-171

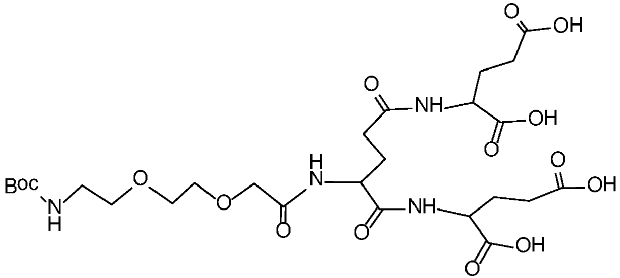

The reactant Boc-LC-E[E(OBn)₂]₂ (synthesized according to the synthesis method of Compound 14-163,0.5572 g, 0.5511 mmoL) and 10% Pd/C (50 mg) were added in a micro-reactor and then dissolved with DMF (30 mL); H₂ (20 psi) was introduced in the reactor, and the mixed solution was stirred to react. At the end of the reaction, the reaction solution was filtered by suction with diatomaceous earth as a filter cake to remove Pd/C; the diatomaceous earth was washed 3 to 4 times with DMF to obtain the DMF solution of the product for the next reaction step.

24-172

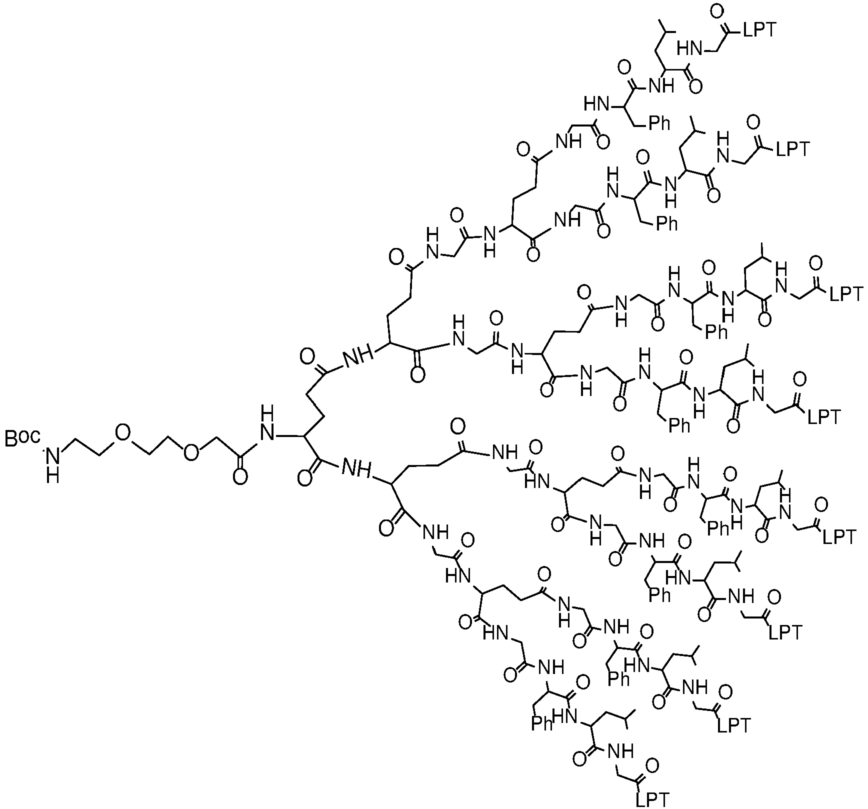

Compound 24-170 (5.5 g, 2.6453 mmoL), HBTU (1.2540 g, 3.3066 mmoL), and HOBT (0.4468 g, 3.3066 mmoL) were weighed and added into a 500 mL flask and then completely dissolved with the DMF solution of Compound 24-171 by ultrasonic treatment, the resulting solution was stirred at -5 °C for 30 min, DIEA (1.6396 mL, 9.9198 mmoL) was slowly added dropwise, and the mixed solution was stirred at a low temperature for 2 h and then placed at room temperature to react to the end. At the end of the reaction, methyl tert-butyl ether (150 mL) was added to the reaction solution, the mixed solution was treated by ultrasonic for 5 min and then filtered by suction, 20% methanol/dichloromethane (20 mL) was added, and then methyl tert-butyl ether (100 mL) and n-hexane (300 mL) were added; the resulting solution was then filtered by suction. 5.5 g of the product was obtained with a yield of 100%, 1.1 g being extra-quota.

24-173

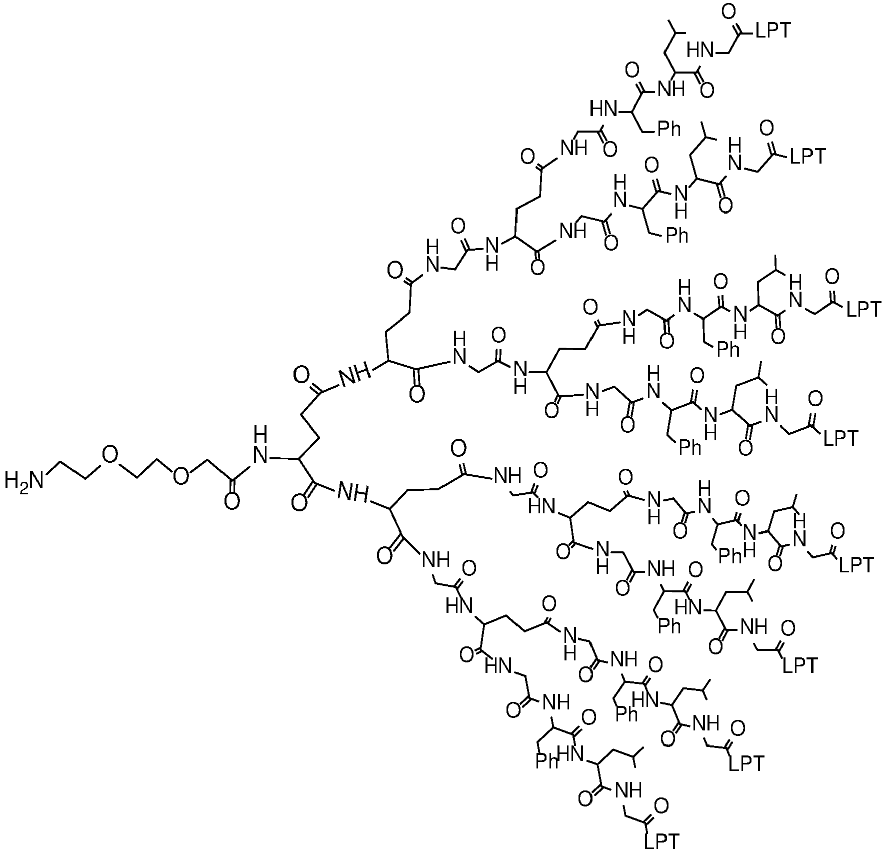

Compound 24-172 (4.9 g, 0.5509 mmoL) was weighed, dichloromethane (15 mL) and TFA (1.2275 mL, 16.5270 mmoL) were then added sequentially, and the obtained solution was treated by ultrasonic until Compound 24-172 was completely dissolved; a ground glass stopper was used, and the mixed solution was stirred at room temperature to react. At the end of the reaction, the reaction solution was evaporated to dryness and the dichloromethane was removed; ethyl acetate (200 mL) was added and the obtained solution was treated by ultrasonic for 2 min and then filtered by suction; the ethyl acetate phase was regarded as impurities and the obtained solid product was dissolved with 20% methanol/dichloromethane (70 mL) by ultrasonic; silica gel powder was then added, and the obtained mixture was evaporated to dryness with a rotary evaporator and then subjected to column chromatography. Gradient elution with 1% ammonia water +5%-20% methanol/dichloromethane was carried out. 2.7 g of the product was obtained with a yield of 56.25%.

¹H-NMR (400 MHz, DMSO-*d*₆) δ 10.61 (s, 4H), 8.76 (d, J = 29.9 Hz, 13H), 8.29-7.93 (m, 44H), 7.73 -7.48(m, 17H), 7.48 (m, 6H), 7.20 (m, 70H), 6.97 (s, 4H), 6.69 -6.55(m, 4H), 5.27 (s, 14H), 4.82 - 4.51 (m, 22H), 4.33-4.23 (m, 38H), 3.78 (s, 14H), 3.37 (s, 26H), 3.03 (d, J = 18.4 Hz, 41H), 2.88 (s, 13H), 2.73 (s, 28H), 2.13 (s, 13H), 1.64 (dd, J = 87.8, 49.1 Hz, 35H), 1.08 (s, 22H), 0.96 - 0.67 (m, 46H).

MALDI-TOF MS: [M+Na+]8808.90, [M+K+]8824.99.

3-13

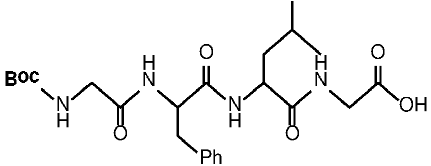

Boc-GFLG-OBn (synthesized as reported, 2.0036 g, 3.4323 mmoL) and Pd/C (0.05502 g) were added in a micro-reactor, DMF (30 mL) was then added to the micro-reactor, the device was set ready, air inlet and pump valves were opened to pump the internal environment to be vacuum, and then the pump valve was closed; H₂ (16 psi) was introduced, and after the reaction continued for 2 min, the air was pumped and H₂ was introduced again; pumping was repeated three times and air inlet and pump valves were closed (it should be noted that the vacuum pump was not closed during the whole operation) to seal H₂ in the reactor, and the reaction solution reacted overnight. At the end of the reaction, a funnel was filled with diatomaceous earth (compacted) to a half, the product was transferred drop by drop into the funnel with a dropper and filtered by suction, and then the reactor and the funnel were washed with DMF three times (no more than 20 mL of DMF each time), and the solution was transferred in a 250 mL flask as the raw material for Product 3-14.

3-14

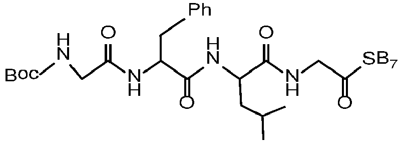

SB7 (SB-743921) (1.4790 g, 2.8603 mmoL), HBTU (1.6270 g, 4.2905 mmoL) and HOBT (0.5797 g, 4.2905 mmoL) were weighed and added to the flask with Compound 3-13, and the reaction solution was placed in a low-temperature constant temperature reaction bath (-5 °C) to react for 20 min, DIEA (2.2 mL, 12.8714 mmoL) was added dropwise, and the mixed solution first reacted for 2 h and then taken out and placed at room temperature overnight on a magnetic stirrer. At the end of the reaction, the reaction solution was transferred to a 2 L separatory funnel, saturated sodium bicarbonate solution (300 mL) and ethyl acetate (400 mL) were added to the separatory funnel, the obtained solution was shaken thoroughly to separate the organic phase, and the aqueous phase was extracted three times with ethyl acetate (150 mL×3); the obtained organic phases were combined and washing with a saturated sodium chloride solution (300 mL) was carried out three times; the obtained organic phase was poured into a 2 L Erlenmeyer flask, dried with anhydrous magnesium sulfate, and then filtered and concentrated, 12 g of silica gel powder was added to the concentrated solution to obtain a solid solution; the operations of dry sample loading and column chromatography were carried out, and elution is performed with a mixed solution (2% methanol: 1% ammonia water: 97% dichloromethane) and a mixed solution (5% methanol: 1% ammonia water: 94% dichloromethane) respectively. 3.7 g of the product was obtained with a yield of 100%.

MALDI-TOFMS: [M+H⁺]991.45, [M+Na⁺]1013.40

3-16

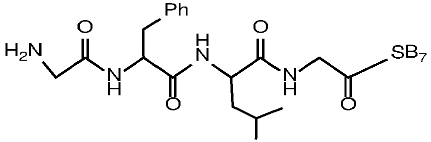

Compound 3-14 (3.6 g, 3.6304 mmoL) was added in a 250 mL flask and dissolved with dichloromethane (15 mL), TFA (2.7 mL, 36.304 mmoL) was then added to the solution, and the obtained solution was stirred overnight at room temperature. At the end of the reaction, the reaction solution was evaporated to remove the solvent; the obtained product was dissolved with ethyl acetate and the obtained solution was then transferred to a 2 L separatory funnel; saturated sodium bicarbonate solution (300 mL, the aqueous phase was alkaline) and ethyl acetate (400 mL) were added to the separatory funnel, the obtained solution was shaken thoroughly to separate the organic phase, and the aqueous phase was extracted three times with ethyl acetate (100 mL× 3); the obtained organic phases were combined, washing with a saturated sodium bicarbonate solution (100 mL) was carried out once, and washing with a saturated sodium chloride solution was carried out twice (100 mL×2); the obtained organic phase was poured into a 2 L Erlenmeyer flask, dried with anhydrous magnesium sulfate, and then filtered and concentrated; 10 g of silica gel powder was added to the concentrated solution and the obtained solution was evaporated to dryness to obtain a solid solution; the operations of dry sample loading, column chromatography and elution with eluent (5% methanol: 1% ammonia water: 94% dichloromethane) were carried out; the elution product was collected and evaporated to dryness. 1.7587 g of the product was obtained with a yield of 54.33%.

24-197

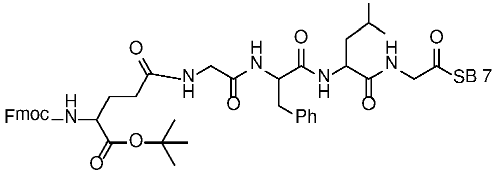

Compound 3-16 (0.5 g, 0.5608 mmoL), Boc-Glu-OtBu (0.2505 g, 0.5888 mmoL), HBTU (0.3190 g, 0.8412 mmoL) and HOBT (0.1137g, 0.8412 mmoL) were weighed and added in a 100 mL flask and then completely dissolved with DMF (20 mL) in a condition of ultrasonic. The obtained solution was stirred at -5 °C for 30 min. DIEA (0.4170 mL, 2.5236 mmoL) was then slowly added dropwise, and the mixed solution reacted at a low temperature to the end. At the end of the reaction, the reaction solution was transferred to a 1 L separatory funnel, ethyl acetate (200 mL) and deionized water (150 mL) were then added, the organic phase was then separated, and the aqueous phase was extracted twice with ethyl acetate (100 mL×2) until there was no product in the aqueous phase. The obtained organic phases were combined and washed once with saturated sodium chloride solution (100 mL), and then concentrated and evaporated to dryness. 1.2 g of the product was obtained with a yield of 100%, 0.48 g being extra-quota.

24-203

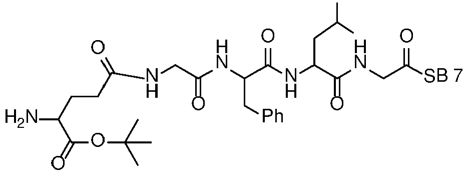

Compound 24-197 (0.72 g, 0.5543 mmoL) was added in a 250 mL round-bottomed flask and then dissolved with DMF (10 mL), and then morpholine (1.4495 mL,16.6288 mmoL) was added and the obtained solution was stirred at room temperature to react for 120 min. At the end of the reaction, the reaction solution was transferred to a 1 L separatory funnel, ethyl acetate (200 mL) and deionized water (150 mL) were then added, the organic phase was then separated, and the aqueous phase was extracted twice with ethyl acetate (100 mL×2) until there was no product in the aqueous phase. The obtained organic phases were combined and washed once with saturated sodium chloride solution (100 mL), and then concentrated and evaporated to dryness. 1 g of the product was obtained with a yield of 100%, 0.41 g being extra-quota.

24-204

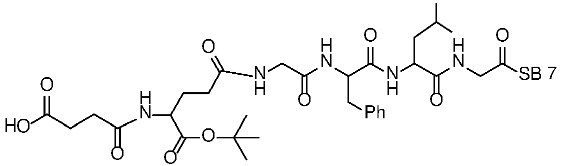

Compound 24-203 (0.6 g, 0.5573 mmoL) was added to a 250 ml round-bottomed flask and dissolved with DMF (10 mL); the reaction solution was placed in a low-temperature constant temperature bath (-5 °C), DIEA (0.4606 mL, 2.7865 mmoL) was added and the mixed solution was stirred at room temperature to react for 30 min; and then succinic anhydride (0.1673 mL, 1.6718 mmoL) was added to the reaction solution. At the end of the reaction, the reaction solution was transferred to a 1 L separatory funnel, ethyl acetate (200 mL) and deionized water (150 mL) were then added, the organic phase was then separated, and the aqueous phase was extracted twice with ethyl acetate (100 mL×2) until there was no product in the aqueous phase. The obtained organic phases were combined and washed once with saturated sodium chloride solution (100 mL), and then concentrated and evaporated to dryness. 1 g of the product was obtained with a yield of 100%, 0.35 g being extra-quota.

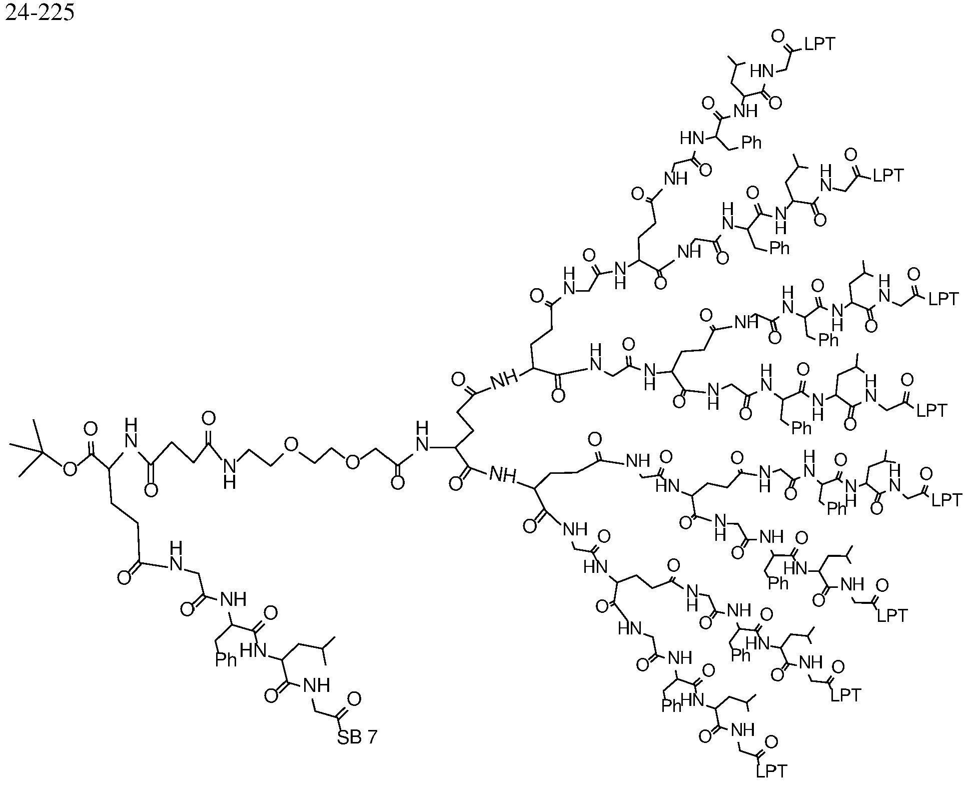

Compound 24-173 (0.5 g, 0.0569 mmoL), Compound 24-204 (0.0803 g, 0.0682 mmoL), HBTU (0.0324 g, 0.0854 mmoL) and HOBT (0.0115 g, 0.0854 mmoL) were weighed and added in a 250 mL flask and then completely dissolved with DMF (20 mL) in a condition of ultrasonic. The obtained solution was stirred at -5 °C for 30 min. DIEA (0.0424 mL, 0.2561 mmoL) was then slowly added dropwise, and the mixed solution reacted at a low temperature to the end. At the end of the reaction, the reaction solution was transferred to a 1 L separatory funnel, ethyl acetate (20 mL) and deionized water (150 mL) were then added. The mixed solution was ultrafiltered. The obtained solid product was dissolved with 20% methanol/dichloromethane, silica gel powder was then added to the obtained solution, themixture was evaporated to dryness and then subjected to column chromatography. Gradient elution with 1% ammonia water +5%-7% methanol/dichloromethane was carried out. 0.3 g of the product was obtained with a yield of 60%.

24-227

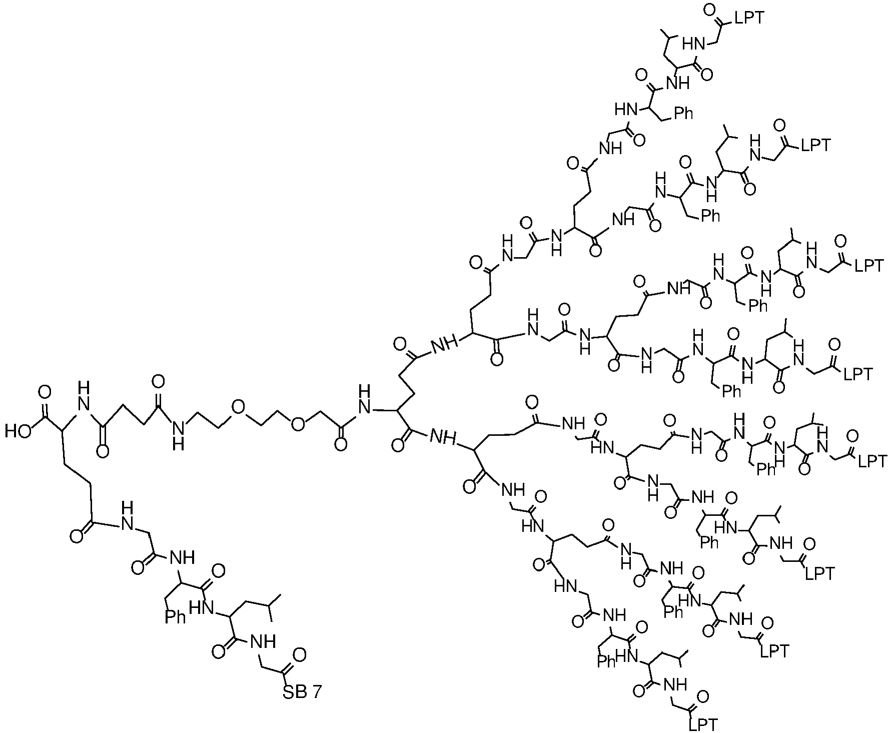

Compound 24-225 (0.5 g, 0.0502 mmoL) was weighed, dichloromethane (10 mL) and TFA (0.0933 mL, 1.2558 mmoL) were then added sequentially, and the obtained solution was treated by ultrasonic until Compound 24-225 was completely dissolved; a ground glass stopper was used, and the mixed solution was stirred at room temperature to react. At the end of the reaction, methyl tert-butyl ether (100 mL) and n-hexane (150 mL) were directly added to the reaction solution and the obtained solution was filtered by suction; the obtained solid product was dissolved with 20% methanol/dichloromethane (50 mL) by ultrasonic, silica gel powder (3 g) was then added, and the obtained solution was evaporated to dryness with a rotary evaporator and then subjected to column chromatography. Gradient elution with 1% ammonia water +6%-7% methanol/dichloromethane was carried out. 0.3 g of the product was obtained with a yield of 60%.

24-231

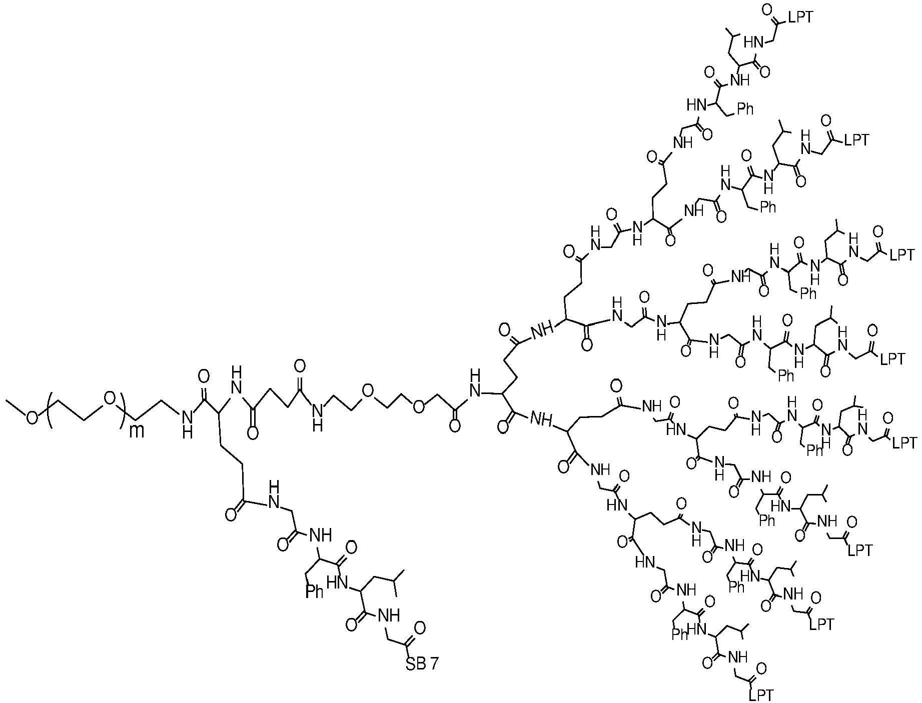

The reactants, Compound 24-227 (0.3 g, 0.0303 mmoL), M-NH₂HCL-20K (0.6245 g, 0.0303 mmoL), HBTU (0.0230 g, 0.0606 mmoL) and HOBT (0.0082 g, 0.0606 mmoL) were weighed and added in a 250 mL reaction flask and dissolved with DMF (15 mL) by ultrasonic; the obtained solution was stirred at -5 °C for 30 min; DIEA (0.0300 mL, 0.1818 mmoL) was slowly added dropwise and the obtained solution was stirred for 1 h and then slowly stirred at room temperature to react in the dark. At the end of the reaction, methyl tert-butyl ether (200 mL) was added to precipitate the reaction solution, suction filtering was carried out to obtain a powder product, the powder product was dissolved with a mixed solvent of 20% methanol and dichloromethane, silica gel (3 g) was added to the obtained solution, and the operations of evaporation, dry sample loading and column chromatography were carried out. Elution with 1% ammonia water+6% methanol/dichloromethane was carried out, the product was collected and evaporated to dryness, the solid product was dissolved with dichloromethane (5 mL) by ultrasonic, methyl tert-butyl ether (150 mL) and n-hexane (50 mL) were added sequentially; suction filtering was carried out; dichloromethane was added to dissolve the obtained product; the obtained solution was precipitated with methyl tert-butyl ether and n-hexane; the process of dissolution and precipitation was repeated three times. 0.59 g of the product was obtained with a yield of 66%.

¹H-NMR (400 MHz, DMSO-*d*₆) δ 8.74-8.62(m, 1H), 8.30 - 7.94 (m, 36H), 7.75-7.73 (m, 6H), 7.48 (s, 8H),7.20-7.15(m,96H),6.95(s,11H),6.67-6.51 (m, 7H), 5.76 (s, 9H), 5.26 (s, 9H), 4.78-4.57 (m, 20H), 4.36-4.14 (m, 49H), 3.51 (s, 1820H), 3.06-2.89 (m, 90H), 2.72-2.67 (m, 34H), 2.33-2.11 (m, 26H), 1.59-1.48 (m, 23H), 1.30-1.23 (m, 58H), 0.84 (s, 58H).

Example 2: Synthesis of compound 31-188

33-22

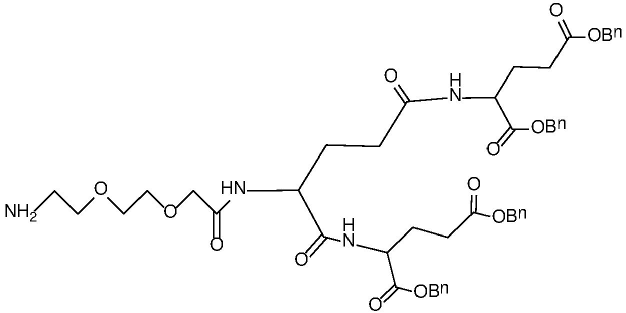

Compound 14-163 (4 g, 3.95 mmoL) was weighed and then dissolved with TFA (8.8 mL, 118.6 mmoL) and dichloromethane (10 mL). The obtained solution was stirred to react. At the end of the reaction, the reaction solution was concentrated, then extracted with ethyl acetate (100 mL) and saturated NaHCO₃ solution (70 mL), the organic phase was separated, and the aqueous phase was extracted three times with ethyl acetate (50 mL×3). The organic phases were combined and evaporated to dryness for the next step. The product was obtained with a yield of 100%.

31-121

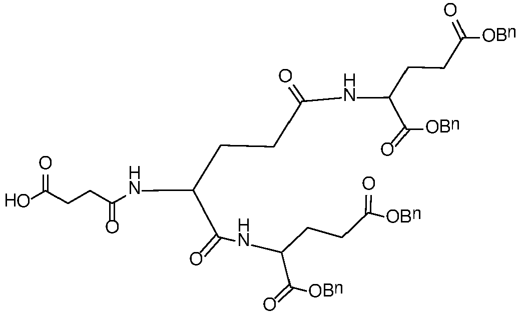

Compound 14-155 (7.0 g, 9.14 mmoL) was added to a 500 mL flask and then dissolved with DMF (10 mL), and then DIEA (6.04 mL, 36.56 mmoL) and succinoside (2.74 g, 27.42 mmoL) were slowly added dropwise to continue the reaction, and the reaction was stirred at room temperature to react overnight. At the end of the reaction, the reaction solution was washed with saturated NaCl (400 mL) and then extracted three times with EA (300 mL), the EA phase was then evaporated to dryness with a rotary evaporator, the operations of dry sample loading, column chromatography, gradient elution with 2% methanol/dichloromethane-10% methanol/dichloromethane were carried out, and then the elution product was then collected, concentrated, evaporated to dryness, and dried in a vacuum oven. 5.1 g of the pure product 31-121 was obtained with a yield of 99%.

31-149

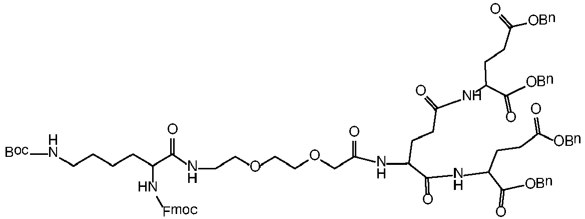

Compound 31-146 (synthesized according to the synthesis method of Compound 33-22, 13.76 g, 15.1 mmoL), Fmoc-Lys(Boc)-OH (7.78 g, 16.61 mmoL), HOBT (3.06 g, 22.65 mmoL) and HBTU (8.59 g, 22.65 mmoL) were added in a 500 mL round-bottomed flask and then dissolved with DMF (30 mL), and the mixed solution was stirred at -5 °C for 30 min. Then DIEA (15 mL, 90.75 mmoL) was slowly added dropwise, and then the mixed solution reacted at a low temperature for 2 h; after that, the reaction device was placed at room temperature and the reaction solution was stirred overnight to react. At the end of the reaction, the reaction solution was transferred to a 2 L separatory funnel, washed with saturated NaCl (400 mL) and then extracted three times with EA (300 mL), the EA phase was then evaporated to dryness with a rotary evaporator, the operations of dry sample loading, column chromatography, gradient elution with 1% methanol/dichloromethane-3% methanol/dichloromethane were carried out, and then the elution product was then collected, concentrated, evaporated to dryness, and dried in a vacuum oven. 7.7 g of a pure product and 13.9 g of a mixed product was obtained.

31-155

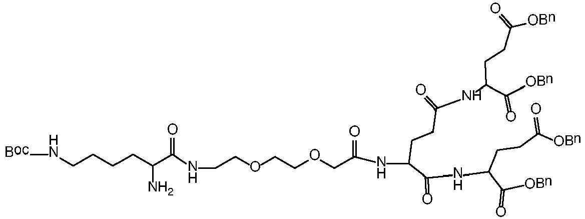

Compound 31-149 (5.1 g, 3.74 mmoL) was added in a 250 mL round-bottomed flask and then dissolved with DMF (15 mL), and the mixed solution was stirred at room temperature. Morpholine (9.79 mL, 112.37 mmoL) was then added dropwise, and the mixed solution reacted at room temperature for 2 h. At the end of the reaction, the reaction solution was washed with saturated NaCl (200 mL) and then extracted three times with EA (200 mL), the organic phase was then evaporated to dryness with a rotary evaporator, the operations of dry sample loading, column chromatography, gradient elution with 1% methanol/0.5% ammonia water/dichloromethane-2% methanol/0.5% ammonia water/dichloromethane were carried out, and then the elution product was then collected, concentrated, evaporated to dryness, and dried in a vacuum oven. 2.31 g of the product was obtained with a yield of 54.1%.

31-165

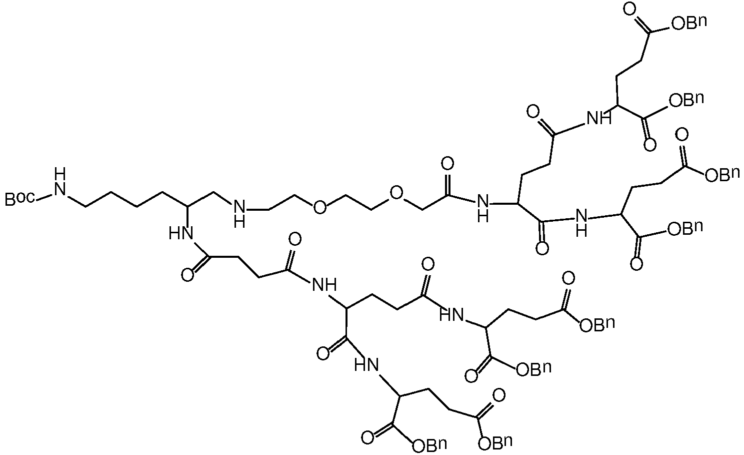

Compound 31-121 (1.84 g, 2.13 mmoL), Compound 31-155 (2.31 g, 2.03 mmoL), HOBT (0.41 g, 3.04 mmoL) and HBTU (1.15 g, 3.04 mmoL) were added in a 500 mL round-bottomed flask and then dissolved with DMF (25 mL), and the mixed solution was stirred at -5 °C for 30 min. Then DIEA (1.51 mL, 9.12 mmoL) was slowly added dropwise, and then the mixed solution reacted at a low temperature for 2 h; after that, the reaction device was placed at room temperature and the reaction solution was stirred overnight to react. At the end of the reaction, the reaction solution was transferred to a 2 L separatory funnel, washed with saturated NaHCO₃ (200 mL) and then extracted three times with EA (100 mL); the extract was washed with saturated NaCl (300 mL) and then extracted once with EA; the EA phase was then evaporated to dryness with a rotary evaporator, the operations of dry sample loading, column chromatography, gradient elution with 2% methanol/dichloromethane-4% methanol/dichloromethane were carried out, and then the elution product was then collected, concentrated, evaporated to dryness, and dried in a vacuum oven. 2.16 g of the product was obtained with a yield of 53.6%.

31-172

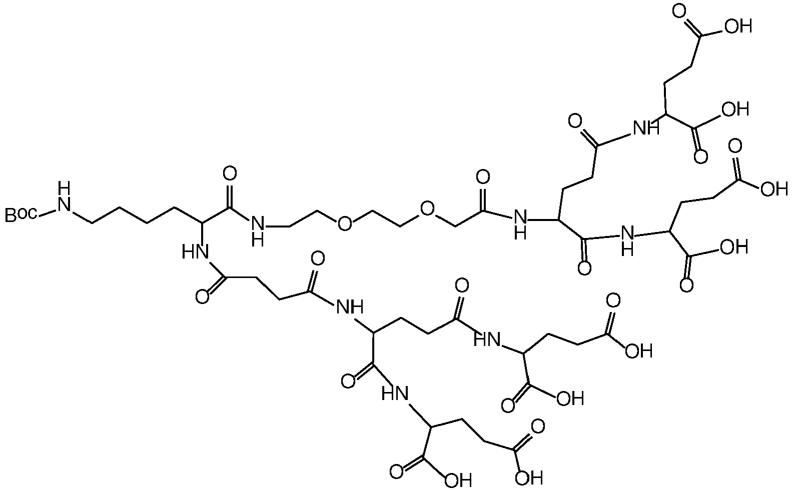

The raw material Compound 31-165 (0.59 g, 0.30 mmoL) and 10% Pd/C catalyst (100 mg) were added into a hydrogenation reactor and then dissolved with DMF (40 mL) where the level of the solvent was above a stirrer; the hydrogenation reaction device was then sealed to perform the "three pumping and three charging" operation (i.e., pumping the air from the reaction system with a vacuum pump for about 3 min-charging hydrogen-pumping hydrogen-charging hydrogen---pumping hydrogen---charging hydrogen) so that the pressure on the hydrogenation reaction device was read as 30 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (30 mL) until it did not contain any product, and then the reaction product was obtained.

31-173

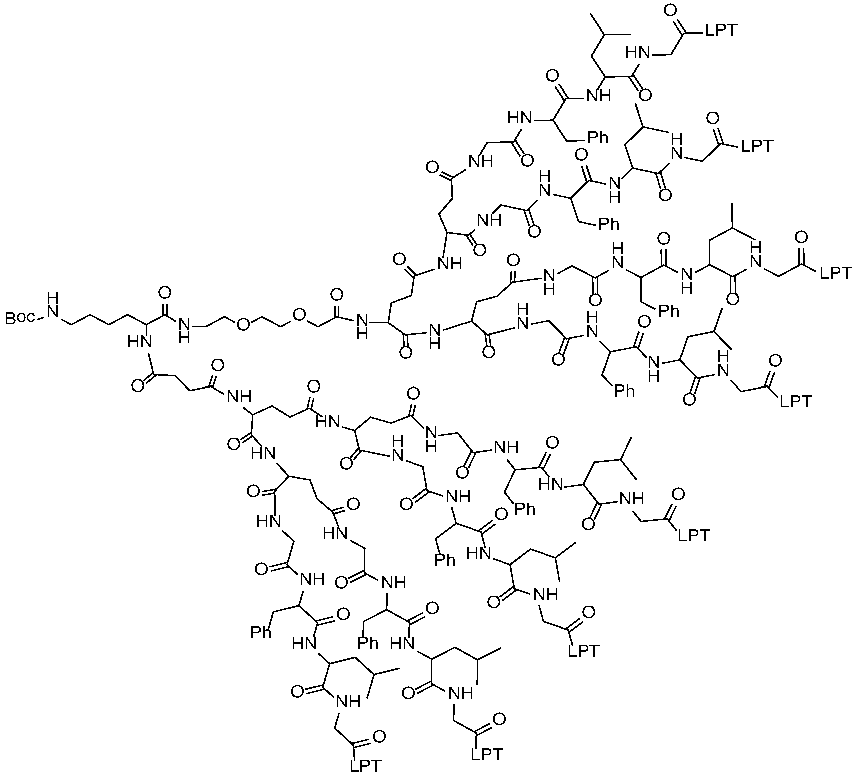

Compound 31-172 (0.30 mmoL), Compound 14-128 (2.5 g, 2.62 mmoL), HOBT (0.49 g, 3.6 mmoL) and HBTU (1.36 g, 3.6 mmoL) were added in a 500 mL round-bottomed flask and then dissolved with DMF (90 mL), and the mixed solution was stirred at -5 °C for 30 min. Then DIEA (1.78 mL, 10.8 mmoL) was slowly added dropwise, and then the mixed solution reacted at a low temperature for 2 h; after that, the reaction device was placed at room temperature and the reaction solution was stirred overnight to react. At the end of the reaction, the reaction solution was precipitated with n-hexane (500 mL) and methyl tert-butyl ether (500 mL) and then filtered to obtain a solid product; the solid product was then dried. The product was obtained with a theoretical weight of 2.63 g.

31-179

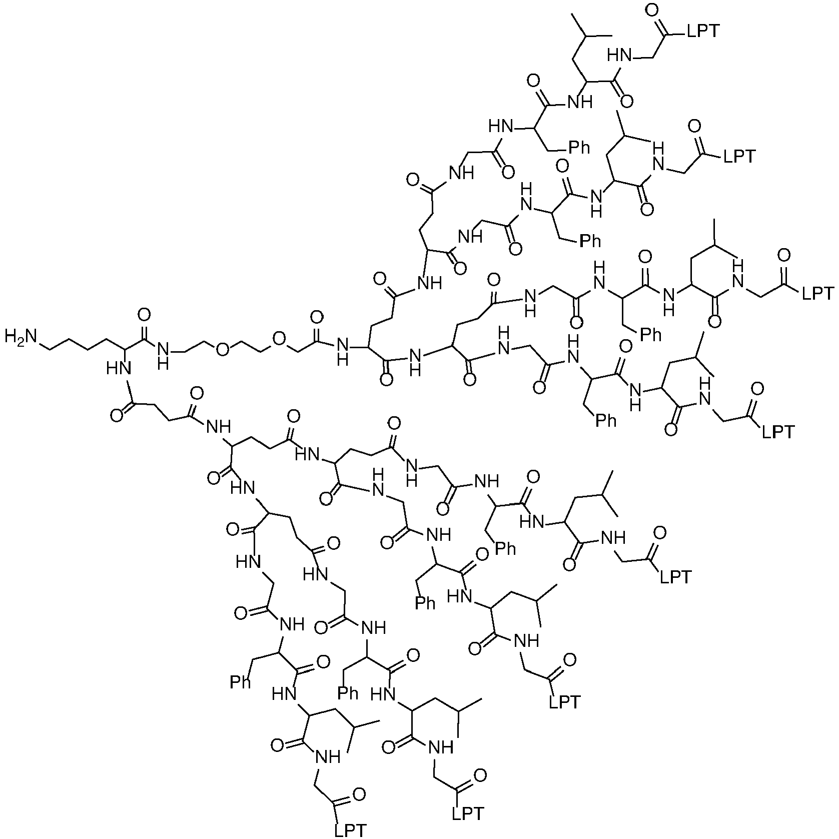

Compound 31-173 (2.63 g, 0.30 mmoL) was added in a 500 mL round-bottomed flask and then dissolved with dichloromethane (20 mL), TFA (0.33 mL, 4.5 mmoL) was then added and the obtained solution was stirred at room temperature to react for 2 h; at the end of the reaction, the reaction solution was evaporated to dryness with a rotary evaporator, then precipitated with methyl tert-butyl ether (150 mL) and filtered by suction to obtain a solid product; the obtained solid product was dissolved with dichloromethane (100 mL) and methanol (10 mL); the operations of dry sample loading, column chromatography and gradient elution with 6% methanol/1% ammonia water/dichloromethane-9% methanol/1% ammonia water/dichloromethane were carried out; the elution product was then collected, concentrated, evaporated to dryness, and dried in a vacuum oven. 1.16 g of the product was obtained with a yield of 44.6%.

31-188

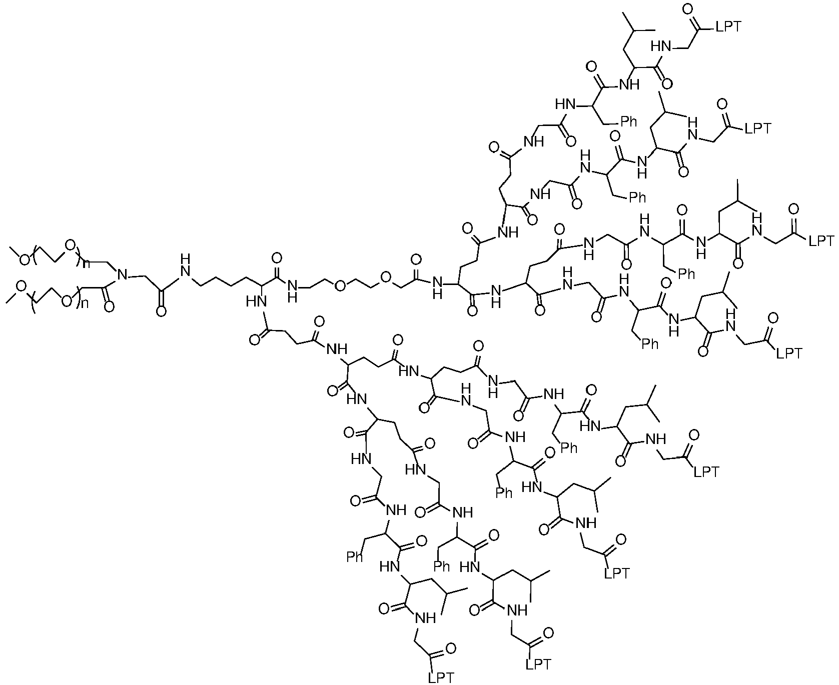

Compound 31-179 (0.39 g, 0.045 mmoL) and Y-NHS-40K (1.8 g, 0.041 mmoL) were added in a 250 mL round-bottomed flask and then dissolved with DMF (20 mL), and the mixed solution was stirred slowly in the dark to react for one week. The reaction solution was applied to the TLC plate every day for monitoring. At the end of the reaction, the reaction solution was precipitated with n-hexane (500 mL) and methyl tert-butyl ether (200 mL) and then filtered by suction; the solid was taken, and then dried in an oven. The operations of dry sample loading, column chromatography, and gradient elution with 5% methanol/1% ammonia water/dichloromethane-8% methanol/1% ammonia water/dichloromethane were carried out. The elution product was then collected, concentrated, evaporated to dryness with a rotary evaporator, and dried in an oven. The product was then dissolved with absolute ethanol (50 mL), the resulting solution was precipitated with methyl tert-butyl ether (100 mL) and n-hexane (50 mL) and filtered by suction; the obtained solid was taken and dried in an oven. 1.58 g of the product was obtained with a yield of 72.1%.

¹H-NMR (400 MHz, DMSO-d₆) δ 9.84 (s, 9H), 8.73 (s, 8H), 8.54 (s, 4H), 8.22-8.06 (m, 31H), 8.00 (s, 13H), 7.80 - 7.70 (m, 21H), 7.56-7.43 (m,28H), 7.31-7.09 (m, 65H), 6.66 (s, 9H), 6.53 (s, 5H), 5.32-5.21 (m, 26H), 4.71 (d, *J* = 18.6 Hz, 18H), 4.58-4.44 (m,41H), 4.24-4.12(m, 39H), 4.05 - 3.46 (m, 3974H), 3.158-3.12(m, 22H), 3.03 (d, *J* = 17.6 Hz, 34H), 2.76-2.65 (m, 20H), 1.56-1.44 (m, 30H), 0.88 - 0.75 (m, 48H).

### Example 3: Synthesis of compound 24-243

24-205

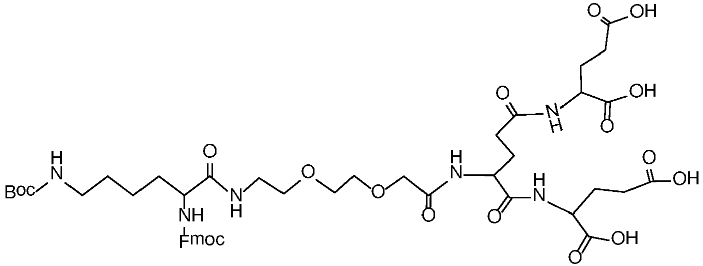

The reactant Compound 15-91 (synthesized according to the synthesis method of Compound 31-149, 0.33 g, 0.1503 mmoL) and 10% Pd/C (30 mg) were added in a micro-reactor and then dissolved with DMF (30 mL); H₂ (20 psi) was introduced in the reactor, and the mixed solution was stirred to react. At the end of the reaction, the reaction solution was filtered by suction with diatomaceous earth as a filter cake to remove Pd/C; the diatomaceous earth was washed four times with DMF to obtain the DMF solution of Compound 24-205 for the next reaction step.

24-206

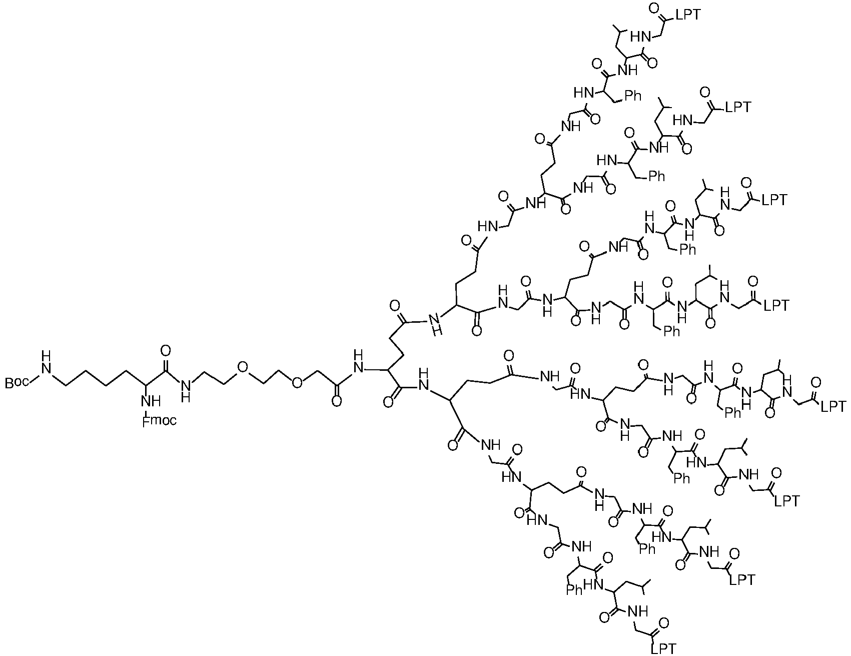

Compound 24-170 (1.5 g, 0.721 mmoL), HBTU (0.3420 g, 0.9018 mmoL) and HOBT(0.1219 g, 0.9018 mmoL) were weighed and added into a 250 mL flask and then completely dissolved with the DMF solution of Compound 24-205 by ultrasonic, the resulting solution was stirred at -5 °C for 30 min, DIEA (0.4472 mL, 2.7054 mmoL) was slowly added dropwise, and the mixed solution was stirred at a low temperature for 2 h and then placed at room temperature to react to the end. At the end of the reaction, methyl tert-butyl ether (100 mL) and n-hexane (150 mL) were added to the reaction solution and the obtained solution was treated by ultrasonic for 5 min and then rested for 20 min in a refrigerator; the supernatant was discarded. Ethyl acetate (20 mL) was added to the lower solution and the obtained solution was then treated by ultrasonic for 2 min; n-hexane (100 mL) was added and the obtained solution was filtered by suction; the obtained solid was dissolved with 20% methanol/dichloromethane (70 mL); silica gel powder was then added, and the obtained solution was evaporated to dryness and then subjected to column chromatography. Gradient elution with 1% ammonia water +5%-7% methanol/dichloromethane was carried out. 1 g of the product was obtained with a yield of 76.9%.

24-223

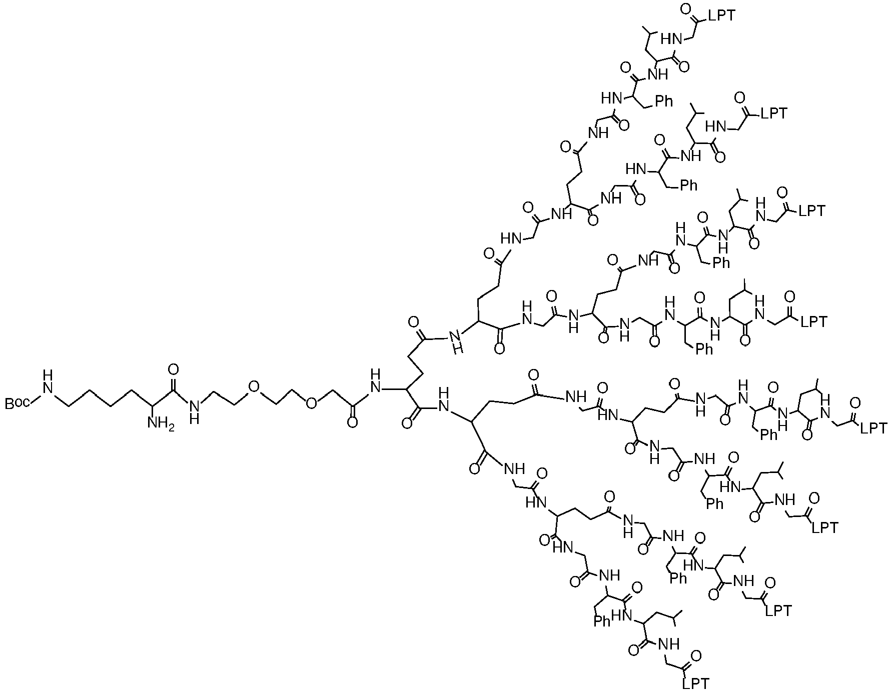

Compound 24-206 (1 g, 0.1082 mmoL) was added in a 250 mL round-bottomed flask and then dissolved with DMF (10 mL), and then morpholine (0.2827 mL, 3.2448 mmoL) was added and the obtained solution was stirred at room temperature to react. At the end of the reaction, methyl tert-butyl ether (150 mL) and n-hexane (100 mL) were added to the reaction solution and the obtained solution was treated by ultrasonic for 5 min and then filtered by suction, and the product was dissolved with 20% methanol/dichloromethane (50 mL); silica gel powder was added, and the obtained solution was evaporated to dryness and then subjected to column chromatography. Elution with 1% ammonia water +7% methanol/dichloromethane was carried out. 0.7 g of the product was obtained with a yield of 70%.

¹H-NMR (400MHz, DMSO-*d*₆) δ 8.77 (s,6H), 8.55(s,7H), 8.25-7.97(m,49H), 7.79-7.71 (m, 14H), 7.47 (m, 8H), 7.20-7.16 (m, 72H), 7.00 (s, 5H), 6.60 (d, *J* = 56.3 Hz, 6H), 5.25 (s, 14H), 4.82 - 4.51 (m, 20H), 4.37-4.21 (m, 28H), 3.90 - 3.47 (m, 55H), 3.03 (d, *J* = 18.0 Hz, 30H), 2.89-2.73 (m, 19H),2.11- 2.06 (m, 15H), 1.84-1.43 (m, 36H), 1.40-1.23 (m, 50H), 0.85-0.77 (m, 47H).

19-107

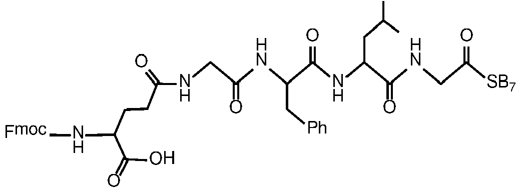

Compound 19-100 (synthesized according to the synthesis method of 24-197, 4 g, 3.0794 mmoL) was added in a 500 mL round-bottomed flask, and then dissolved with dichloromethane (50 mL), TFA (6.8617 mL, 92.3823 mmoL) was then added, and the obtained solution was stirred overnight at room temperature to react; at the end of the reaction, the reaction solution was concentrated under reduced pressure, saturated sodium bicarbonate (200 mL) was added to neutralize the TFA, the product in the aqueous phase was extracted three times with ethyl acetate (150 mL×3); the organic phases were combined and then dried with anhydrous sodium sulfate, and then filtered by suction, concentrated, and dried in an oven. 4 g of the product was obtained with a yield of 100%.

15-98

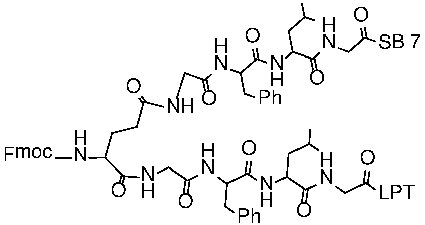

The reactants, Compound 19-107 (1 g, 0.8033 mmoL), Compound 14-128 (0.7675 g, 0.8033 mmoL), HBTU (0.4570 g, 1.2050 mmoL), HOBT (0.1628 g, 1.2050 mmoL) were added in a 250 mL reaction flask, and the obtained solution was stirred at a low temperature of 0 °C for 30 min, and then DIEA (0.5975 mL, 3.6149 mmoL) was slowly added dropwise. Under this condition, the solution was further stirred for 3 h and then stirred at room temperature to react. The reaction solution was transferred to a 1 L separatory funnel, ethyl acetate (200 mL) and deionized water (150 mL) were then added, the organic phase was then separated, and the aqueous phase was extracted twice with ethyl acetate (100 mL×2) until there was no product in the aqueous phase. The obtained organic phases were combined and washed once with saturated sodium chloride solution (100 mL), and then concentrated; silica gel powder (10 g) was then added, and the obtained solution was evaporated to dryness and then subjected to column chromatography. Gradient elution with 1% ammonia water +4%-9% methanol/dichloromethane was carried out. 1.3 g of the product was obtained with a yield of 76.3%.

15-100

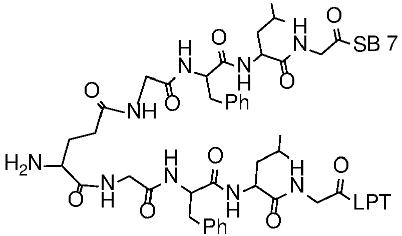

Compound 15-98 (0.3 g, 0.6249 mmoL) was weighed and dissolved with DMF, and then morpholine (1.6 mL, 18.7471 mmoL) was added, and the obtained solution was stirred at room temperature to react for 1 h. At the end of the reaction, the reaction solution was precipitated with methyl tert-butyl ether (30 mL) and n-hexane (250 mL) and then filtered by suction to obtain a powder product; the powder product was dried to obtain 1.1 g of the product, with a yield of 90.2%.

15-102

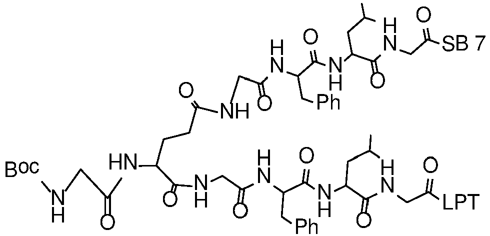

Compound 15-100 (1.1 g, 0.5618 mmoL), Boc-Gly-OH (0.0984 g, 0.5618 mmoL), HBTU (0.3196 g, 0.8427 mmoL), and HOBT (0.1139 g, 0.8427 mmoL) were added to a 250 mL reaction flask in sequence and then dissolved with DMF (30 mL). The resulting solution was stirred at a low temperature of 0 °C to react for 30 min; DIEA (0.4 mL, 2.5280 mmoL) was then slowly added dropwise in the reaction solution, and the mixed solution was further stirred to react for 3 h under this condition, and then stirred to react at room temperature. At the end of the reaction, the reaction solution was precipitated with methyl tert-butyl ether (30 mL) and n-hexane (200 mL) and then filtered by suction to obtain a powder product; the powder product was dried to obtain 1.3 g of the product, with a yield of 100%.

15-103

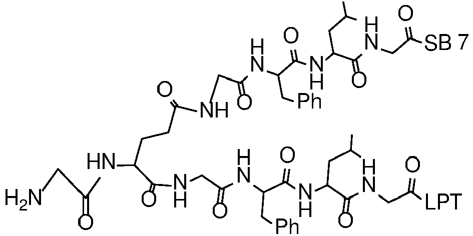

Compound 15-102 (1.2 g, 0.5618 mmoL) was weighed and dichloromethane (10 mL) and TFA (1.3 mL, 16.8540 mmoL) were then sequentially added to a reaction flask. The resulting solution was stirred at room temperature to react for 3 h. At the end of the reaction, the reaction solution was precipitated with methyl tert-butyl ether (30 mL) and n-hexane (200 mL) and then filtered by suction and dried to obtain 1.1 g of the product, with a yield of 100%.

15-105

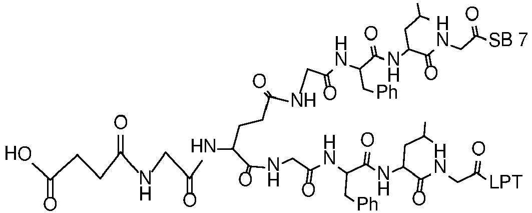

Compound 15-103 (1.2 g, 0.5618 mmoL) was weighed and then dissolved with DMF (20 mL); succinic anhydride (0.1687 mL, 1.6854 mmoL) was then added to the resulting solution and the obtained solution was stirred at a low temperature to react for 30 min. DIEA (0.5 mL, 2.8090 mmol) was slowly added dropwise to the reaction solution and 30 min later, the mixed solution was stirred at room temperature for 3 h. At the end of the reaction, the reaction solution was precipitated with methyl tert-butyl ether (30 mL) and n-hexane (200 mL) and then filtered by suction to obtain a solid product; the obtained solid product was dissolved with 20% methanol/dichloromethane, silica gel powder was then added, and the obtained solution was evaporated to dryness and then subjected to column chromatography. Gradient elution with 1% ammonia water +4%-9% methanol/dichloromethane was carried out. 0.9 g of the product was obtained with a yield of 76.3%.

24-237

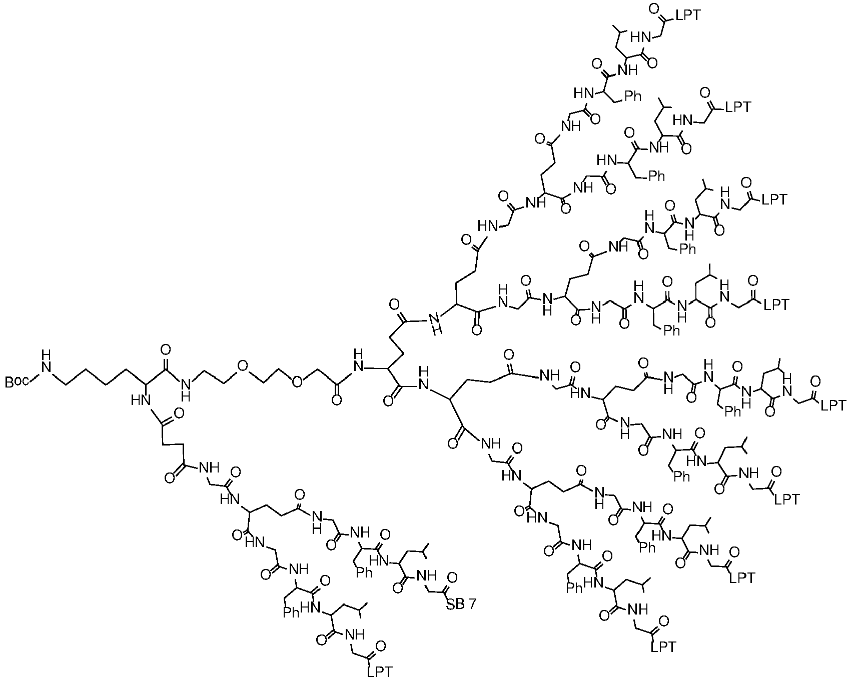

Compound 24-223 (0.7 g, 0.0776 mmoL), Compound 15-105 (0.1969 g, 0.0931 mmoL), HBTU (0.0441 g, 0.1164 mmoL) and HOBT (0.0157 g, 0.1164 mmoL) were weighed and added in a 250 mL flask and then completely dissolved with DMF (40 mL) in a condition of ultrasonic. The obtained solution was stirred at -5 °C for 30 min. DIEA (0.0577 mL, 0.3492 mmoL) was then slowly added dropwise, and the mixed solution reacted at a low temperature to the end. At the end of the reaction, methyl tert-butyl ether (150 mL) and n-hexane (150 mL) were added to the reaction solution and the obtained solution was treated by ultrasonic for 5 min; the supernatant was discarded; ethyl acetate (10 mL) was added to the lower solution and the obtained solution was then treated by ultrasonic for 2 min; methyl tert-butyl ether (100 mL) and n-hexane (50 mL) were added and the obtained solution was filtered by suction; the obtained product was dissolved with 20% methanol/dichloromethane (50 mL); silica gel powder was then added, and the obtained solution was evaporated to dryness and then subjected to column chromatography. Elution with 1% ammonia water +6%-8% methanol/dichloromethane was carried out. 0.5 g of the product was obtained with a yield of 62%.

24-241

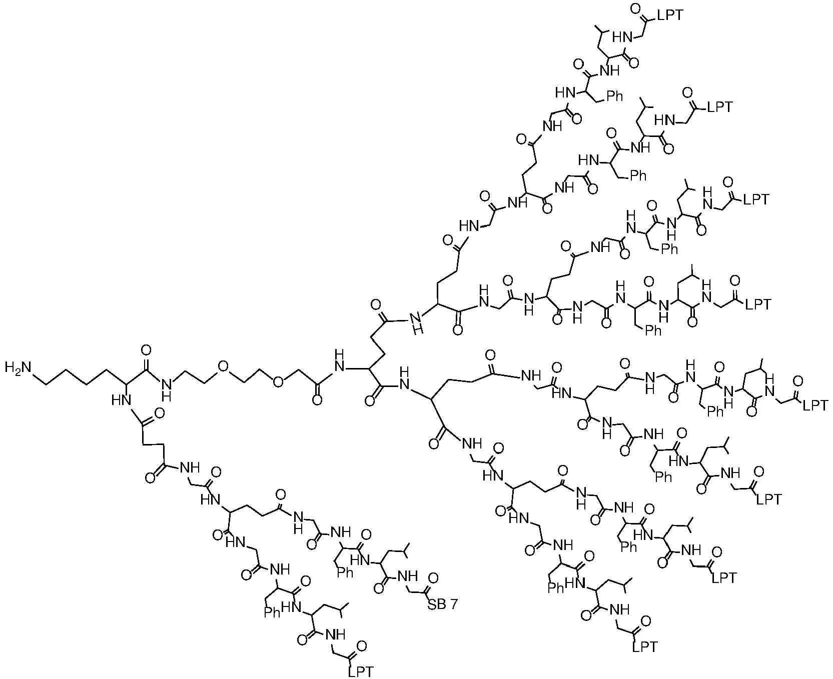

Compound 24-237 (0.5 g, 0.0450 mmoL) was weighed, dichloromethane (10 mL) and TFA (0.0835 mL, 1.1240 mmoL) were then added sequentially, and the obtained solution was treated by ultrasonic until Compound 24-237 was completely dissolved; a ground glass stopper was used, and the mixed solution was stirred at room temperature to react. At the end of the reaction, methyl tert-butyl ether (150 mL) and n-hexane (100 mL) were directly added to the reaction solution and the obtained solution was filtered by suction; the obtained solid product was dissolved with 20% methanol/dichloromethane (50 mL) by ultrasonic, silica gel powder (3 g) was then added, and the obtained solution was evaporated to dryness with a rotary evaporator and then subjected to column chromatography. Gradient elution with 1% ammonia water +6%-8% methanol/dichloromethane was carried out. 0.2 g of the product was obtained with a yield of 40%.

24-243

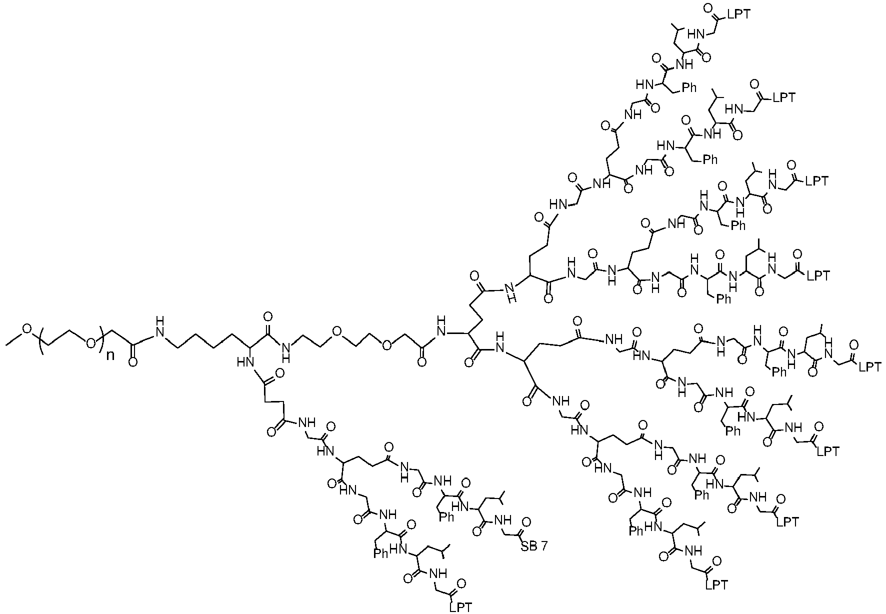

Compound 24-241 (0.2 g, 0.0180 mmoL) was weighed and then dissolved with DMF (20 mL) and then M-SCM-40K (0.7419 g, 0.01798 mmoL) was added and dissolved by ultrasonic. The obtained solution reacted in the dark at a low speed stirring. At the end of the reaction, the reaction solution was precipitated with methyl tert-butyl ether (150 mL) and n-hexane (70 mL) and then filtered by suction to obtain a solid product; the obtained solid product was dissolved with 20% methanol/dichloromethane, silica gel powder (3 g) was then added, and the obtained solution was evaporated to dryness and then subjected to column chromatography. Gradient elution with 1% ammonia water+6%-7% methanol/dichloromethane was carried out, the product was collected and evaporated to dryness, the obtained product was thoroughly dissolved with absolute ethanol (3 mL) by ultrasonic, methyl tert-butyl ether (150 mL) and n-hexane (50 mL) were added sequentially; suction filtering was carried out; the obtained product was dissolved with absolute ethanol (3 mL), and the obtained solution was precipitated with methyl tert-butyl ether and n-hexane; the process of dissolution and precipitation was repeated three times, suction filtering was carried out, and the obtained product was dried in an oven. 0.66 g of the product was obtained with a yield of 73%.

¹H-NMR (400 MHz, DMSO-*d*₆) δ 8.75 - 8.71 (m,6H), 8.53-8.51 (m, 6H), 8.22 - 8.15 (m, 4H), 7.95-7.90 (m, 19H), 7.68-7.65 (m,17H), 7.45-7.19 (m, 141H), 6.67 - 6.66 (m, 4H), 6.54 - 6.53 (m, 4H), 5.26 (s, 2H), 5.10-5.05 (m, 5H), 4.77 - 4.60 (m, 21H), 4.58 (s, 4H), 4.35 - 4.21 (m,4H), 4.14-4.00 (m,10H), 3.50 (m,3659H), 3.05-2.89 (m, 90H), 2.67 -2.62(m, 96H), 2.33 (s, 35H), 2.17-2.03 (m,31H), 2.00 - 1.47 (m, 14H), 1.45-1.15 (m, 30H), 0.86-0.78(m, 60H).

### Example 4: Synthesis of compound 33-82

33-41

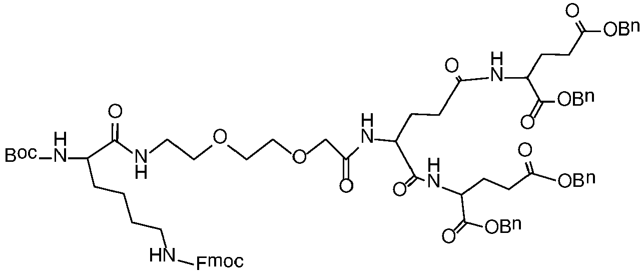

Boc-L-Lys (Fmoc)-OH (1.42 g, 1.49 mmoL), Compound 33-22 (2.6 g, 2.9 mmoL), HOBT (0.589 g, 4.365 mmoL), and HBTU (1.65 g, 4.36 mmoL) were weighed and added in a 250 mL flask and then dissolved with a DMF solution (20 mL). The obtained solution was placed in a low-temperature constant-temperature bath (-5 °C) and stirred for 30 min. DIEA (2.1 mL, 13.08 mmoL) was then added dropwise and the mixed solution reacted for 2 h at a low temperature, and then the reaction solution was stirred at room temperature to react. At the end of the reaction, the reaction solution was extracted with ethyl acetate (100 mL) and saturated NaCl solution (70 mL), the organic phase was separated, and the aqueous phase was extracted three times with ethyl acetate (50 mL×3). The organic phases were combined and evaporated to dryness. The operations of column chromatography and gradient elution with 50%-100% ethyl acetate/petroleum ether were carried out. 3.9 g of the product was obtained with a yield of 100%.

33-44

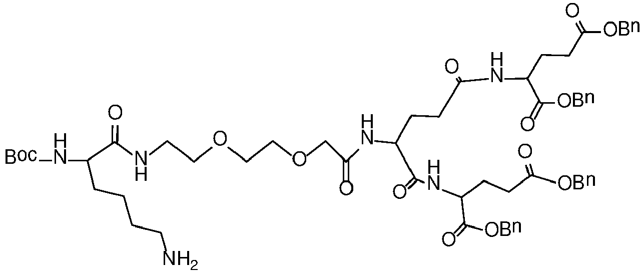

Compound 33-41 (2.9 mmoL) was weighed and added in a 250 mL flask and then dissolved with DMF solution (25 mL) and then morpholine (5.0 mL, 58 mmoL) was added, and the obtained solution was stirred at room temperature to react for 3 h. At the end of the reaction, the reaction solution was extracted with ethyl acetate (100 mL) and deionized aqueous solution (70 mL), the organic phase was separated, and the aqueous phase was extracted three times with ethyl acetate (50 mL×3). The organic phases were combined and evaporated to dryness for the next step. 1.7 g of the product was obtained with a yield of 51%.

33-49

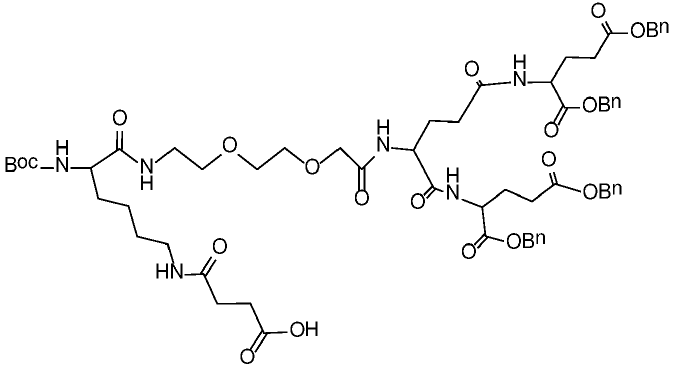

The reactant Compound 33-44 (1.7 g, 1.49 mmoL) was weighed and then dissolved with DMF solution (30 mL). DIEA (0.985 mL, 5.96 mmoL) was added dropwise, and succinic anhydride (0.447g, 4.47 mmoL) was added 30 min later and dissolved by ultrasonic. The obtained solution was stirred to react. At the end of the reaction, the reaction solution was extracted with ethyl acetate (100 mL) and deionized aqueous solution (70 mL), the organic phase was separated, and the aqueous phase was extracted three times with ethyl acetate (50 mL×3). The organic phases were combined and evaporated to dryness for the next step. The product was obtained with a yield of 100%.

33-55

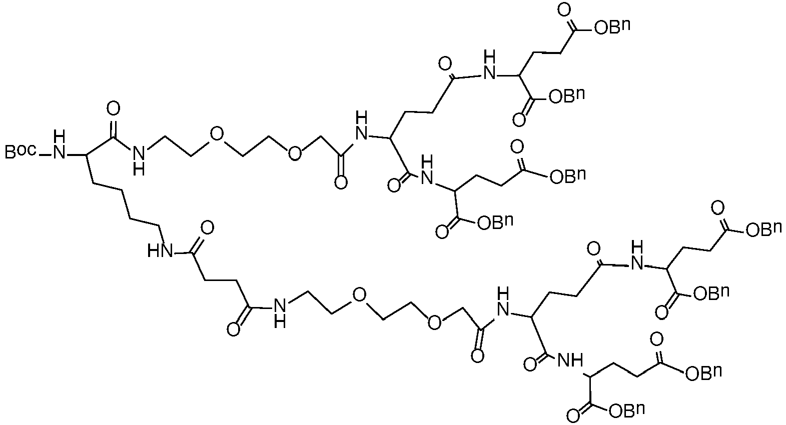

Compound 33-49 (0.26 g, 1.49 mmoL), Compound 33-22 (1.49 mmoL), HOBT (0.3 g, 2.235 mmoL), and HBTU (0.84 g, 2.235 mmoL) were weighed and added in a 250 mL flask and then dissolved with a DMF solution (20 mL); the obtained solution was placed in a low-temperature constant temperature bath (-5 °C) and stirred for 30 min, DIEA (1.18 mL, 6.705 mmoL) was added dropwise, and the mixed solution first reacted at a low temperature for 2 h and then was stirred at room temperature to react. At the end of the reaction, the reaction solution was extracted with ethyl acetate (100 mL) and saturated NaCl solution (70 mL), the organic phase was separated, and the aqueous phase was extracted three times with ethyl acetate (50 mL×3). The organic phases were combined and evaporated to dryness for the next step. The product was obtained with a yield of 100%.

33-58

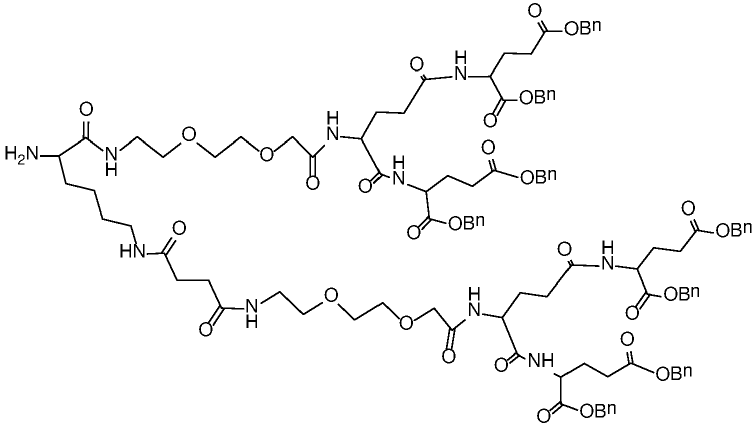

The reactant Compound 33-55 (1.49 mmoL) was measured. TFA (2.7 mL, 37.25 mmoL) and dichloromethane (10 mL) were added sequentially to dissolve the Compound 33-55. The obtained solution was stirred to react. At the end of the reaction, the reaction solution was concentrated and then precipitated with methyl tert-butyl ether (50 mL) and n-hexane (100 mL) and filtered by suction. The obtained product was evaporated to dryness and the product was obtained with a yield of 100%.

33-60

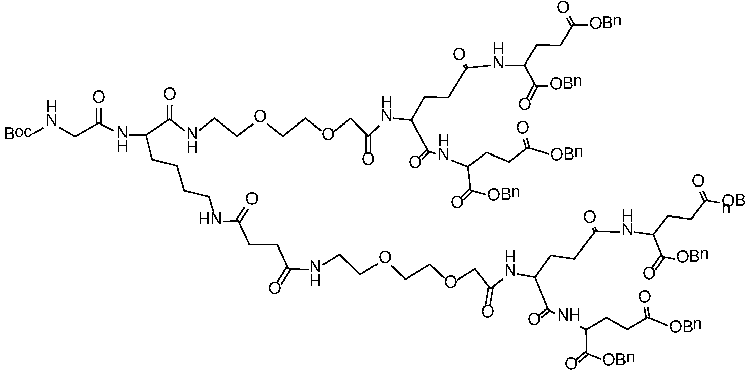

Boc-Gly-OH (0.26 g, 1.49 mmoL), Compound 33-58 (1.49 mmoL), HOBT (0.3 g, 2.235 mmoL), and HBTU (0.84 g, 2.235 mmoL) were weighed and added in a 250 mL flask and then dissolved with a DMF solution (80 mL); the obtained solution was placed in a low-temperature constant temperature bath (-5 °C) and stirred for 30 min, DIEA(1.18 mL, 6.705 mmoL) was added dropwise, and the mixed solution first reacted at a low temperature for 2 h and then was stirred at room temperature to react. At the end of the reaction, ethyl acetate (100 mL) and purified water (70 mL) were added, the organic phase was separated, and the aqueous phase was extracted three times with ethyl acetate (50 mL×3). The operations of column chromatography, dry sample loading and gradient elution with 2% methanol/dichloromethane-5% methanol/dichloromethane were carried out. 1 g of the product was obtained with a yield of 33%.

### MALDI-TOF MS: [M+H⁺] 2189.66, [M+Na⁺] 2210.76, [M+K⁺] 2227.06

33-70

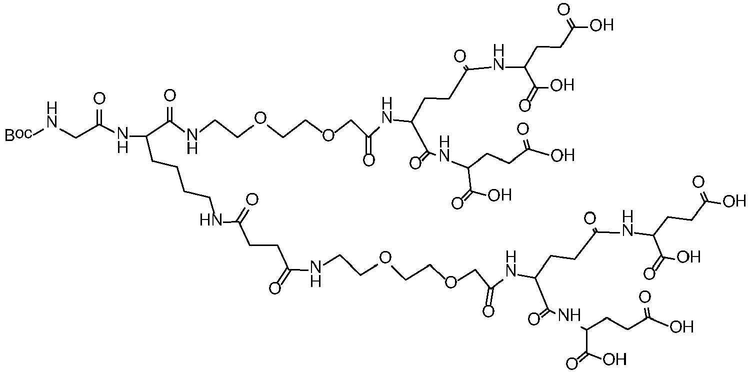

Compound 33-60 (0.2448 g,0.1118 mmoL) and 10% Pd/C (70 mg) were added in a micro-reactor and then DMF (30 mL) was added; the device was set ready, H₂ (20 psi) was introduced, and the mixed solution was placed at room temperature overnight. At the end of the reaction, a funnel was filled with diatomaceous earth (compacted) to a half, the product was transferred drop by drop into the funnel with a dropper and filtered by suction, and then the reactor and the funnel were washed with DMF three times (25 mL×3), and then the solution was transferred in a 250 mL flask for the next step.

33-73

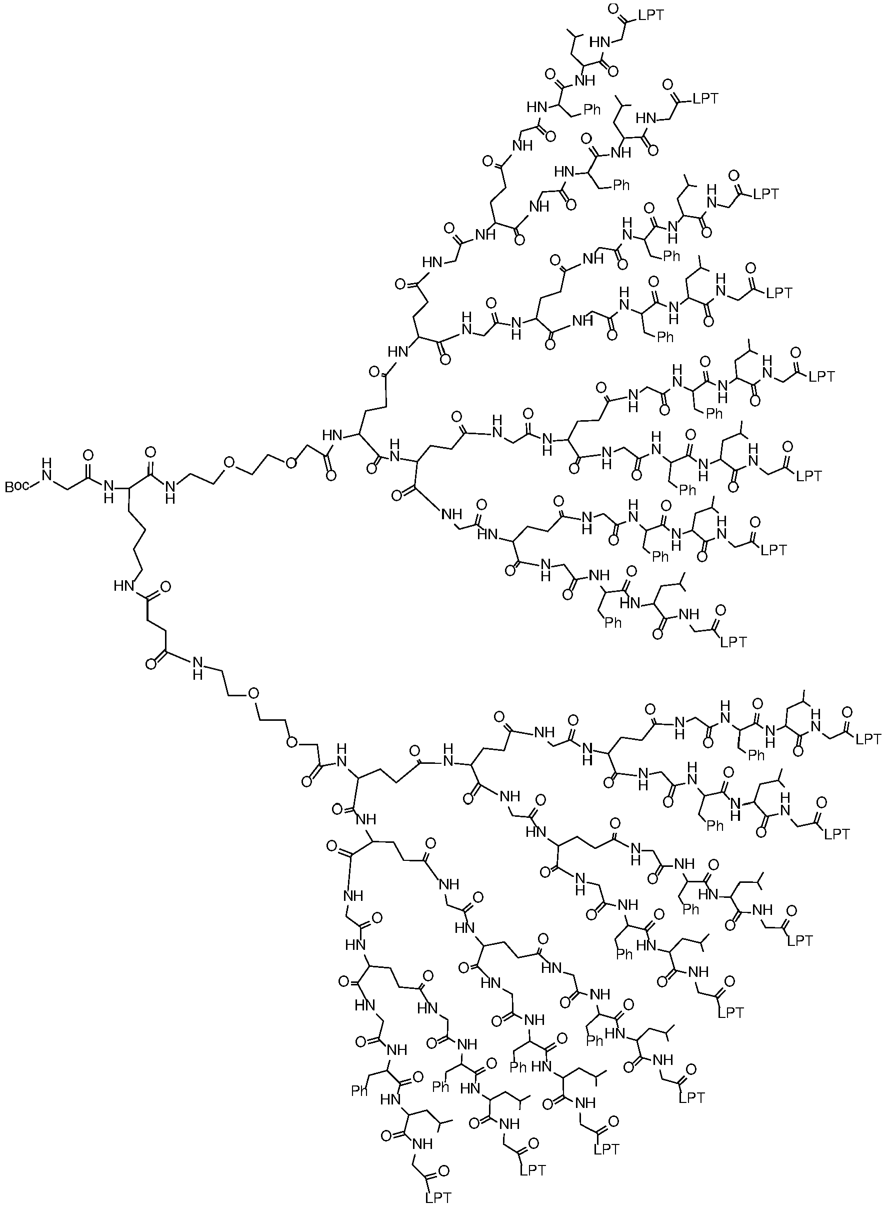

Compound 24-170 (2.0 g, 0.9619 mmoL), Compound 33-70 (0.098 g ,0.0.1118 mmoL), HOBT (0.18 g, 1.34 mmoL), and HBTU (0.508 g, 1.34 mmoL) were weighed and added in a 250 mL flask and then dissolved with a DMF solution (80 mL); the obtained solution was placed in a low-temperature constant temperature bath (-5 °C) and stirred for 30 min, DIEA (0.069 mL, 0.383 mmoL) was added dropwise, and the mixed solution first reacted at a low temperature for 2 h and then was stirred at room temperature to react. At the end of the reaction, the reaction solution was precipitated with methyl tert-butyl ether (50 mL) and n-hexane (100 mL). The operations of column chromatography, dry sample loading and gradient elution with 1% ammonia water: 5% methanol/dichloromethane-1% ammonia water: 10% methanol/dichloromethane were carried out. 0.5 g of the product was obtained.

### MALDI-TOFMS: [M+K⁺] 17976.20

33-77

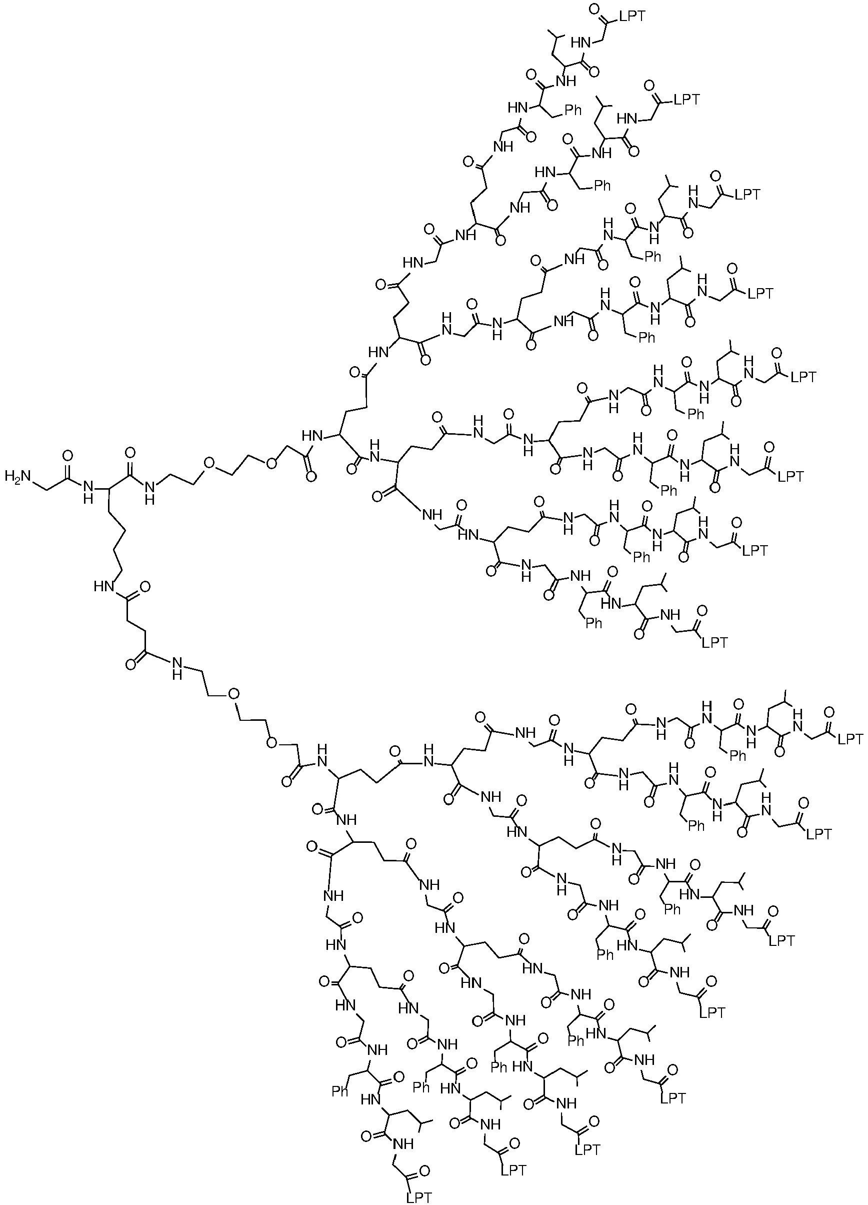

The reactant Compound 33-73 (0.5 g, 0.0278 mmoL) was measured. TFA (0.06 mL, 0.835 mmoL) and dichloromethane (10 mL) were added sequentially to dissolve the Compound 33-73. The obtained solution was stirred to react. At the end of the reaction, the reaction solution was concentrated and then precipitated with methyl tert-butyl ether (50 mL) and n-hexane (100 mL) and filtered by suction. The operations of column chromatography, dry sample loading and gradient elution with 1% ammonia water: 4% methanol/dichloromethane-1% ammonia water: 5% methanol/dichloromethane were carried out. 0.2 g of the product was obtained.

¹H-NMR (400 MHz, DMSO-d₆) δ 9.91 - 9.79 (m, 13H), 8.78 - 8.70 (m, 12H), 8.55 (s, 14H), 8.33 - 7.89 (m, 108H), 7.83 - 7.66 (m, 27H), 7.52 - 7.41 (m, 16H), 7.40 - 6.84 (m, 170H), 6.76 - 6.62 (m, 13H), 6.59 - 6.48(m, 7H), 5.26 (s, 20H), 4.78 - 4.52 (m, 46H), 4.39 - 4.07 (m, 58H), 3.86 - 3.55 (m, 86H), 3.50 (s, 5H), 3.20 - 2.96 (m, 73H), 2.89 (s, 6H), 2.83 - 2.66 (m, 41H), 2.30 (s, 7H), 2.16 - 1.84 (m, 34H), 1.63 - 1.31 (m, 79H), 1.23 (s, 34H), 0.88 - 0.78 (m, 96H).

33-82

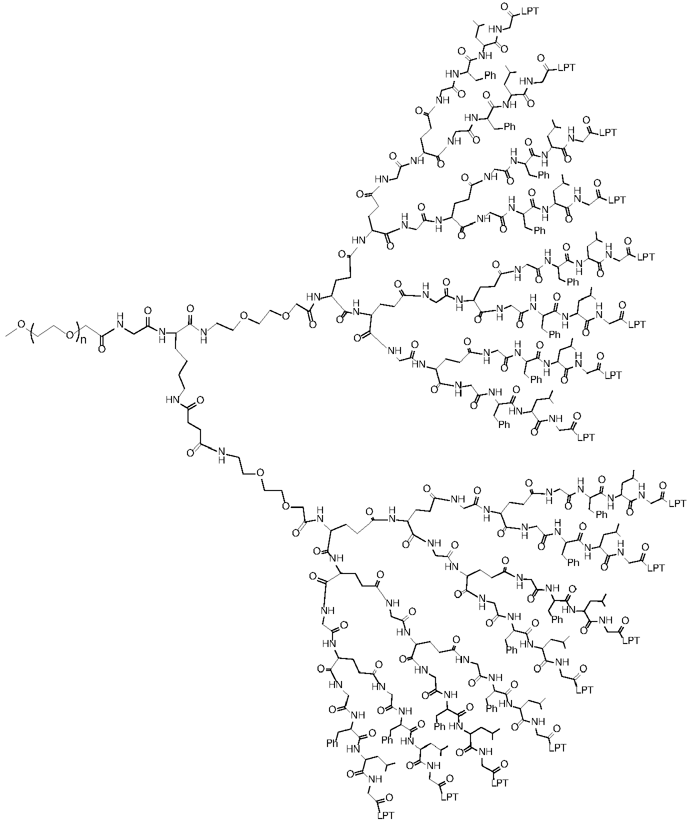

The reactant Compound 33-77 (0.2 g) was added to M-SCM-40K (0.44 g) and the mixture was then dissolved with DMF solution (20 mL). The obtained solution was stirred slowly to react in the dark. At the end of the reaction, the reaction solution was precipitated with methyl tert-butyl ether (50 mL) and n-hexane (100 mL) and then filtered by suction. The operations of column chromatography, dry sample loading and gradient elution with dichloromethane-1% ammonia water: 6% methanol/dichloromethane were carried out. The obtained product was evaporated to dryness; absolute ethanol (10 mL) was then added and the solution was treated by ultrasonic to obtain a homogeneous phase, and the homogeneous phase was precipitated with n-hexane (50 mL). The process of precipitation was repeated three times. The obtained product was dried in vacuum. 0.3 g of the product was obtained finally with a yield of 48%.

¹H-NMR (400 MHz, DMSO-d₆) δ 9.91 - 9.79 (m, 12H), 8.78 - 8.70 (m, 11H), 8.55 (s, 15H), 8.39 - 7.93 (m, 115H), 7.83 - 7.68 (m, 31H), 7.52 - 7.41 (m, 14H), 7.35 - 7.02 (m, 176H), 6.76 - 6.62 (m, 7H), 6.59 - 6.48(m, 7H), 5.26 (s, 24H), 4.83 - 4.49 (m, 42H), 4.42 - 3.82 (m, 153H), 3.58 - 3.44 (m, 5938H), 3.21 - 2.85 (m, 112H), 2.79 - 2.63 (m, 51H), 2.33 (s, 5H), 2.20 - 2.00 (m, 14H), 1.92 - 1.38 (m, 79H), 1.30 - 1.09 (m,10H), 0.87 - 0.79 (m, 96H).

### Example 5: Synthesis of compound 15-139

15-125

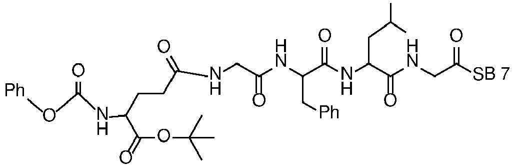

Compound 24-203 (0.5 g, 0.4644 mmoL) was added in a 250 mL round-bottomed flask and then dissolved with dichloromethane (10 mL) by ultrasonic; TEA (0.26 mL, 1.8576 mmoL) was then added to the obtained solution and the obtained solution was then stirred for 30 min at 0 °C to react. Then, phenyl chloroformate (0.1167 mL, 0.9288 mmoL) was slowly added dropwise and the obtained solution reacted at a low temperature. 2 hours later, the reaction ended and methyl tert-butyl ether and n-hexane were then added. The obtained solution was then filtered by suction to obtain 0.4 g of the product.

30-28

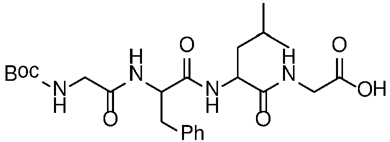

The raw material Boc-GFLG-OBn (synthesized as reported, 19.0 g, 32.6 mmoL) and 10% Pd/C (Palladium/Carbon) catalyst (300 mg) were added into a hydrogenation reactor and then dissolved with DMF (50 mL) where the level of the solvent was above a stirrer; the hydrogenation reaction device was then sealed to perform the "three pumping and three charging" operation (i.e., pumping the air from the reaction system with a vacuum water pump for about 3 min-charging hydrogen-pumping hydrogen-charging hydrogen---pumping hydrogen---charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (90 mL) until it did not contain any product, and then the reaction product was obtained.

30-30

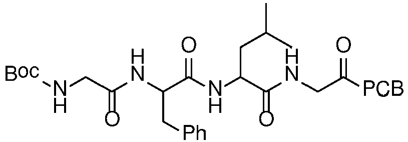

Compound 30-28 (13.97 mmoL), PCB (5 g, 11.17 mmoL), HBTU (6.35 g, 16.76 mmoL) and HOBT (2.26g, 16.76 mmoL) were added in a 500 mL round-bottomed flask and then dissolved with DMF (100 mL), and the mixed solution was stirred at -5 °C for 30 min. Then DIEA (8.31 mL, 50.28 mmoL) was slowly added dropwise, and then the mixed solution reacted at a low temperature for 2 h; after that, the reaction device was placed at room temperature and the reaction solution was stirred overnight to react. At the end of the reaction, deionized water (1000 mL) was added to rinse DMF, and a pale yellow solid was precipitated and then dried to obtain 10.3 g of the product.

30-33

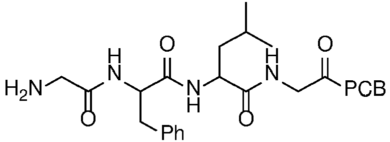

Compound 30-30 (10.3 g, 11.17 mmoL) was added in a 500 mL round-bottomed flask and then dissolved with dichloromethane (50 mL), TFA (12.45 mL, 167.55 mmoL) was then added and the obtained solution was stirred at room temperature to react overnight; at the end of the reaction, the reaction solution was concentrated at reduced pressure and then precipitated three times with n-hexane (50 mL) and methyl tert-butyl ether (400 mL), and the operation of precipitation was repeated three times; suction filtering was then carried out to obtain a solid product; the obtained solid product was dissolved with dichloromethane and methanol; the operations of dry sample loading, column chromatography and elution with 5% methanol/1% ammonia/dichloromethane were carried out; the elution product was then collected, concentrated, and dried. 8.6 g of the product was obtained with a yield of 94%.

41-1

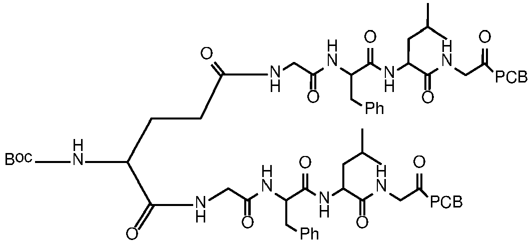

Boc-Glu-OH (1.2 g, 4.8 mmoL), GFLF-PCB (synthesized according to the synthesis method of Compound 30-33, 8.2 g, 9.7 mmoL), HBTU (5.5 g, 14.4 mmoL) and HOBT (1.9 g, 14.4 mmoL) were added in a 500 mL round-bottomed flask and then dissolved with DMF (50 mL), and the mixed solution was stirred at -5 °C for 30 min. Then DIEA (7.1 mL, 43.2 mmoL) was slowly added dropwise to continue the reaction, and 2 h later, the reaction solution was stirred at room temperature overnight. At the end of the reaction, deionized water (200 mL) was added to rinse DMF, and a pale yellow solid was precipitated and then dried to obtain 22 g of the product (including extra-quota).

41-2

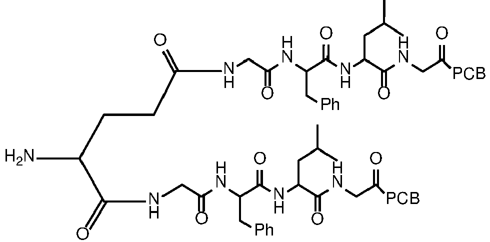

Compound 41-1 (22 g, 11.8 mmoL) was added in a 500 mL round-bottomed flask and then dissolved with dichloromethane (10 mL), and then TFA (26.3 mL, 354 mmoL) was added dropwise and the obtained solution was stirred overnight at room temperature. At the end of the reaction, the reaction solution was concentrated, precipitated with n-hexane (100 mL) and methyl tert-butyl ether (300 mL) and then filtered by suction; the solid was taken, and then dried for later use. The operations of dry sample loading, column chromatography, and gradient elution with 1% ammonia water/3% methanol/dichloromethane-1% ammonia water/5% methanol/dichloromethane were carried out. The elution product was collected and concentrated. 5.9 g of the product was obtained with a yield of 70.2%.

41-4

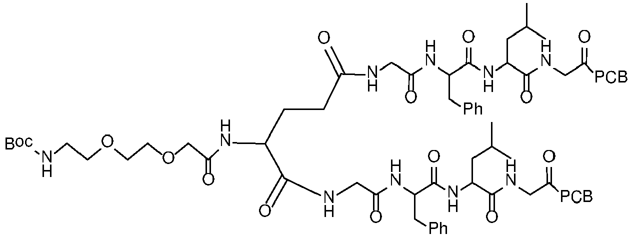

Compound 41-2 (5.9 g, 3.36 mmoL), Boc-LC-OH (synthesized according to the synthesis method of Compound 24-36, 0.88 g, 3.36 mmoL), HBTU (1.9 g, 5.04 mmoL) and HOBT (0.68 g, 5.04 mmoL) were added in a 250 mL round-bottomed flask and then dissolved with DMF (20 mL), and the mixed solution was stirred at -5 °C for 30 min. Then DIEA (2.5 mL, 15.12 mmoL) was slowly added dropwise to continue the reaction, and 2 h later, the reaction solution was stirred at room temperature overnight. At the end of the reaction, the reaction solution was precipitated with n-hexane (100 mL) and methyl tert-butyl ether (300 mL) and then filtered by suction; the solid was taken, and then dried for later use in the next step.

41-6

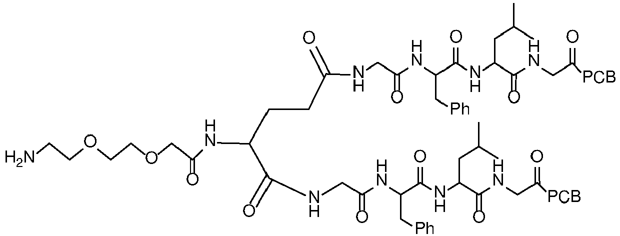

Compound 41-4 (7.9 g, 3.9 mmoL) was added in a 250 mL round-bottomed flask and then dissolved with dichloromethane (10 mL), and then TFA (8.7 mL, 117 mmoL) was added dropwise and the obtained solution was stirred overnight at room temperature. At the end of the reaction, the reaction solution was concentrated, precipitated with n-hexane (100 mL) and methyl tert-butyl ether (300 mL) and then filtered by suction; the solid was taken, and then dried for later use. The operations of dry sample loading, column chromatography, and gradient elution with 1% ammonia water/3% methanol/dichloromethane-1% ammonia water/4% methanol/dichloromethane-1% ammonia water/5% methanol/dichloromethane were carried out. The elution product was collected and concentrated to obtain the final product.

24-1

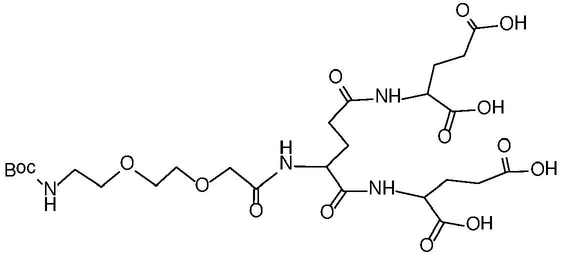

The reactant Compound 14-163 (0.2 g, 0.2104 mmoL) and 10% Pd/C (30 mg) were added in a micro-reactor and then dissolved with DMF (30 mL); H₂ (20 psi) was introduced in the reactor, and the mixed solution was stirred to react. At the end of the reaction, the reaction solution was filtered by suction with diatomaceous earth as a filter cake to remove Pd/C; the diatomaceous earth was washed 3 to 4 times with DMF to obtain the DMF solution of the product for the next reaction step.

15-131

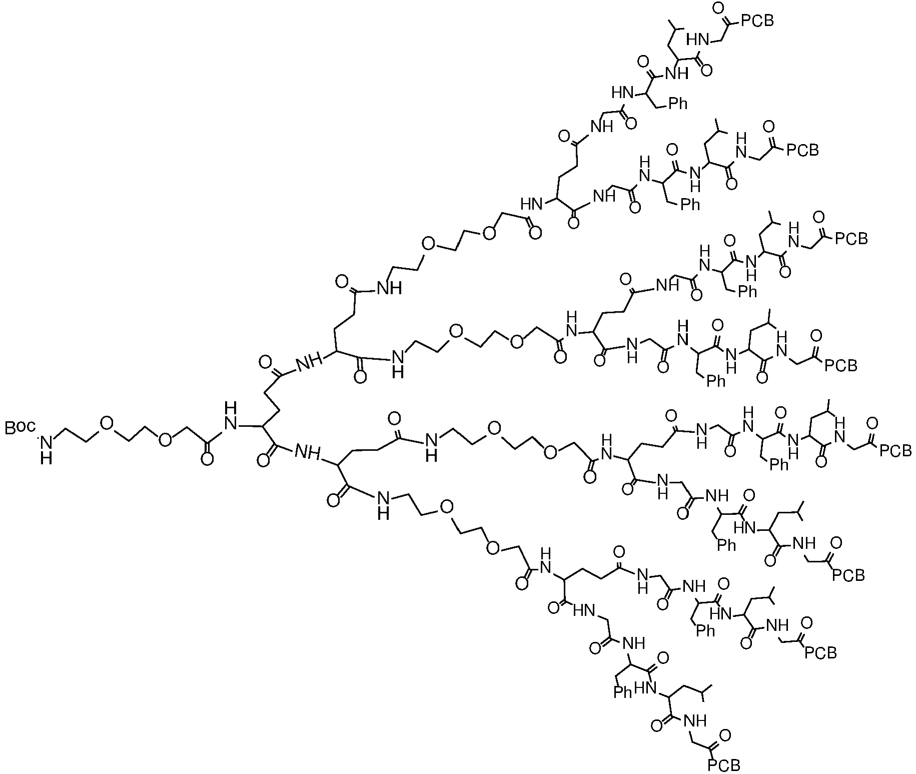

Compound 41-6 (2 g, 1.0525 mmoL), HBTU (0.4170 g, 1.2630 mmoL), and HOBT (0.1707 g, 1.2630 mmoL) were weighed and added into a 250 mL flask and then completely dissolved with the DMF solution of Compound 24-1 by ultrasonic, the resulting solution was stirred at -5 °C for 30 min, DIEA (0.6 mL, 3.7891 mmoL) was slowly added dropwise, and the mixed solution was stirred at a low temperature for 2 h and then placed at room temperature to react to the end. At the end of the reaction, methyl tert-butyl ether (150 mL) was added to the reaction solution and the obtained solution was treated by ultrasonic for 5 min and then filtered by suction, and the product was dissolved with 20% methanol/dichloromethane (20 mL); silica gel powder was added, and the obtained solution was evaporated to dryness and then subjected to column chromatography. Gradient elution with 1% ammonia water +6%-15% methanol/dichloromethane was carried out. 1.7 g of the product was obtained with a yield of 98%.

15-133

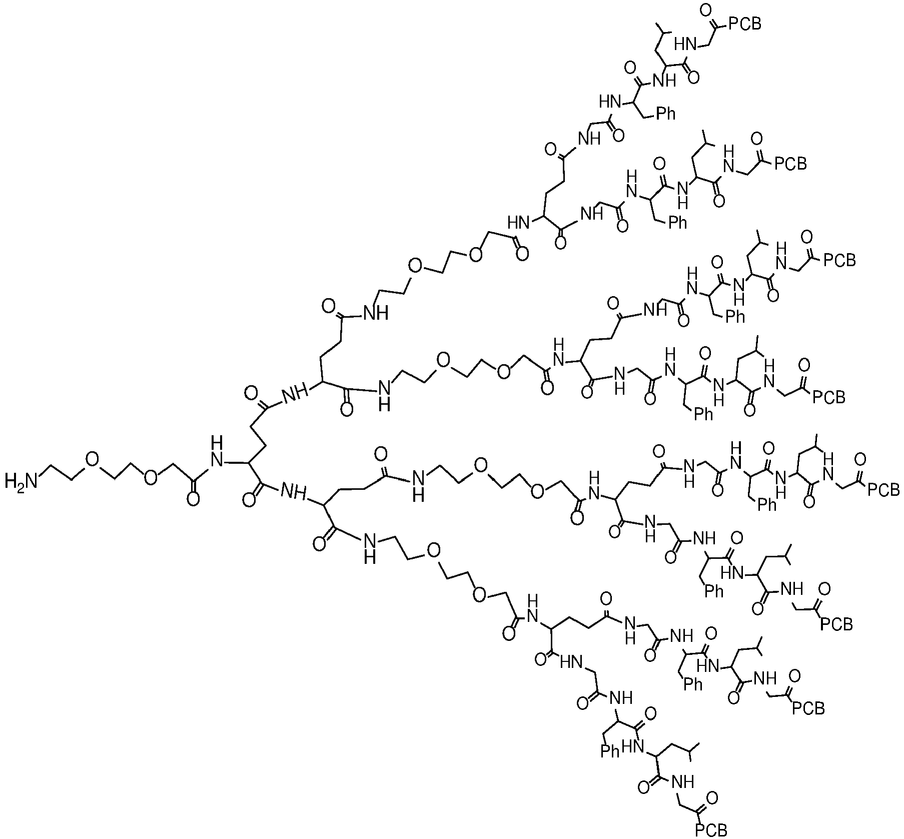

Compound 15-131 (1.7 g, 0.2105 mmoL) was weighed, dichloromethane (10 mL) and TFA (0.47 mL, 6.3300 mmoL) were then added sequentially, and the obtained solution was treated by ultrasonic until Compound 15-131 was completely dissolved; a ground glass stopper was used, and the mixed solution was stirred at room temperature to react. At the end of the reaction, the reaction solution was evaporated to dryness and the dichloromethane was removed; ethyl acetate (20 mL) was added and the obtained solution was treated by ultrasonic for 2 min; methyl tert-butyl ether (150 mL) and n-hexane (70 mL) were added and the obtained solution was then filtered by suction; the obtained solid product was dissolved with 20% methanol/dichloromethane by ultrasonic; silica gel powder was then added, and the obtained solution was evaporated to dryness with a rotary evaporator and then subjected to column chromatography. Gradient elution with 1% ammonia water +4%-6% methanol/dichloromethane was carried out. 0.7 g of the product was obtained with a yield of 43.8%.

15-135

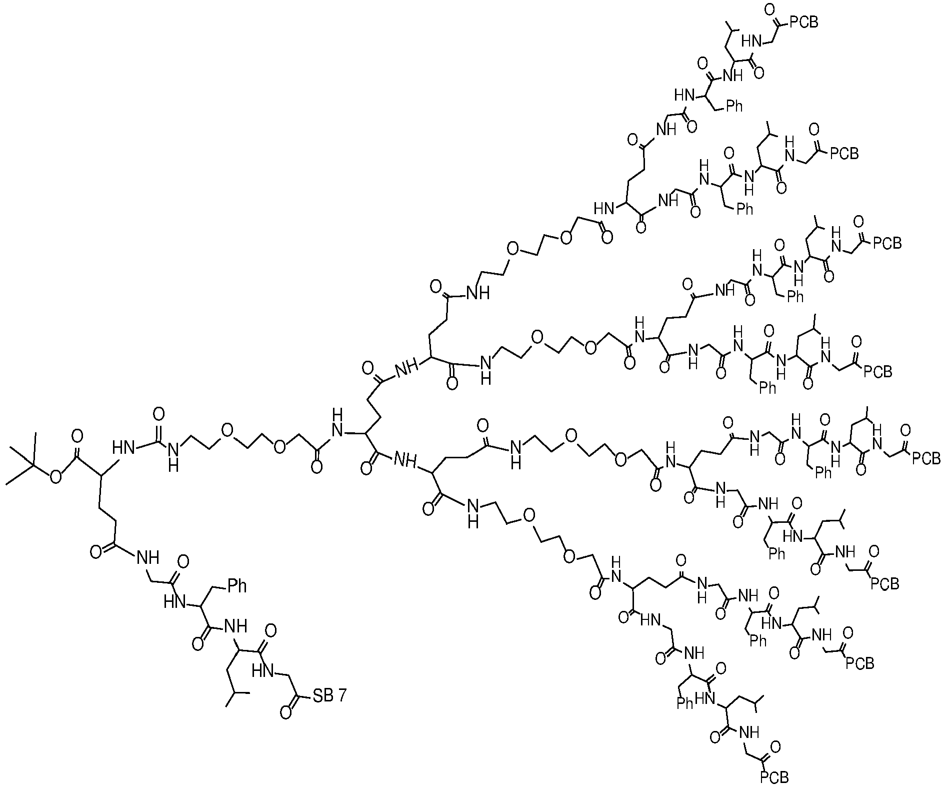

Compound 15-133 (0.6 g, 0.0743 mmoL) and Compound 15-125 (0.1333 g, 0.1114 mmoL) were added into a 250 mL reaction flask, DMF (20 mL) solution and DIEA (0.1 mL) were added sequentially, and the mixed solution was stirred at 100 °C to react to the end. At the end of the reaction, methyl tert-butyl ether (150 mL) was added to the reaction solution and the obtained solution was treated by ultrasonic for 5 min and then filtered by suction, and the resulting product was then dried to obtain 0.6 g of a final product.

15-136

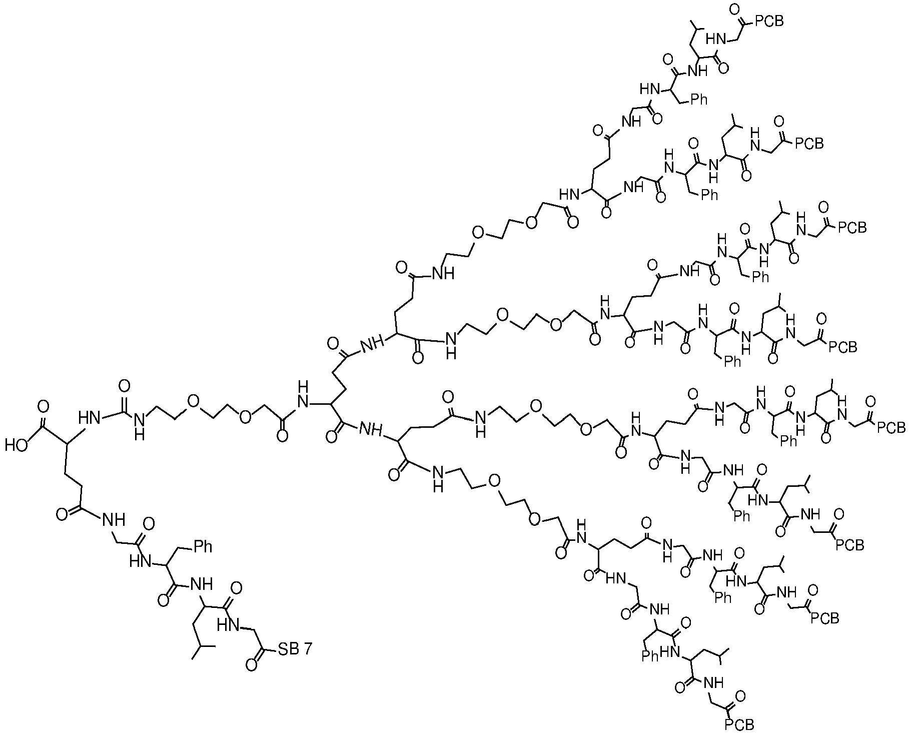

Compound 15-135 (0.6 g, 0.0649 mmoL) was weighed, dichloromethane (10 mL) and TFA (0.1446 mL, 1.9463 mmoL) were then added sequentially, and the obtained solution was treated by ultrasonic until Compound 15-135 was completely dissolved; a ground glass stopper was used, and the mixed solution was stirred at room temperature to react. At the end of the reaction, methyl tert-butyl ether (100 mL) and n-hexane (150 mL) were directly added to the reaction solution and the obtained solution was filtered by suction; the obtained solid product was dissolved with 20% methanol/dichloromethane (50 mL) by ultrasonic, silica gel powder (3 g) was then added, and the obtained solution was evaporated to dryness with a rotary evaporator and then subjected to column chromatography. Gradient elution with 1% ammonia water +3%-8% methanol/dichloromethane was carried out. 0.2 g of the product was obtained with a yield of 30%.

15-139

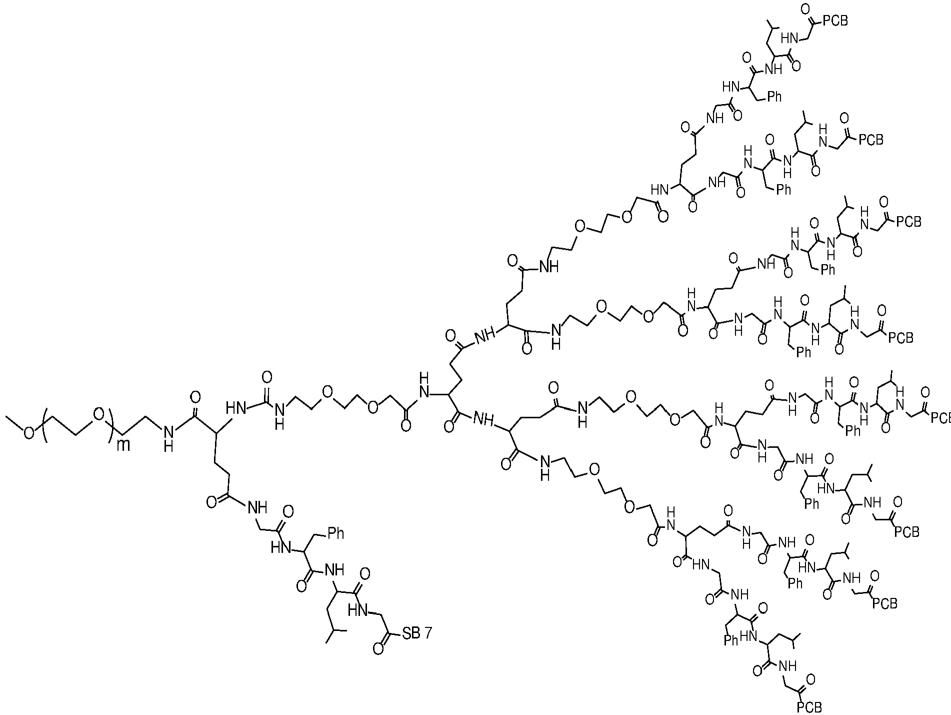

The reactants, Compound 15-136 (0.2 g, 0.0222 mmoL), M-NH₂HCL-20K (0.4570 g, 0.0222 mmoL), HBTU (0.0168 g, 0.0444 mmoL) and HOBT (0.0060 g, 0.0444 mmoL) were weighed and added in a 250 mL reaction flask and dissolved with DMF (15 mL) by ultrasonic; the obtained solution was stirred at -5 °C for 30 min; DIEA (0.0220 mL, 0.1332 mmoL) was slowly added dropwise and the obtained solution was stirred for 1 h and then slowly stirred at room temperature to react in the dark. At the end of the reaction, methyl tert-butyl ether (200 mL) was added to precipitate the reaction solution, suction filtering was carried out to obtain a powder product, the powder product was dissolved with a mixed solvent of 20% methanol and dichloromethane, silica gel (3 g) was added to the obtained solution, and the operations of evaporation, dry sample loading and column chromatography were carried out. Gradient elution with 1% ammonia water+6%-10% methanol/dichloromethane was carried out, the product was collected and evaporated to dryness, the solid product was dissolved with dichloromethane (5 mL) by ultrasonic, methyl tert-butyl ether (150 mL) and n-hexane (50 mL) were added sequentially; suction filtering was carried out; dichloromethane was added to dissolve the obtained product; the obtained solution was precipitated with methyl tert-butyl ether and n-hexane; the process of dissolution and precipitation was repeated three times. 0.2 g of the product was obtained with a yield of 30%.

¹H-NMR (400 MHz, DMSO-d₆) δ 10.18-10.12 (m, 6H), 9.00-8.91 (m, 8H), 8.25 - 7.84 (m, 68H),7.57-7.46 (m, 9H), 7.28-7.10 (m, 46H), 4.64-4.18 (m, 33H), 3.53-3.48 (m, 1054H), 3.22-3.00 (m,71H), 2.94-2.65 (m, 77H), 2.43-2.04 (m, 109H), 1.96-1.42 (m, 114H), 0.91-0.78 (m, 54H).

### Example 6: Synthesis of compound 29-138

24-58

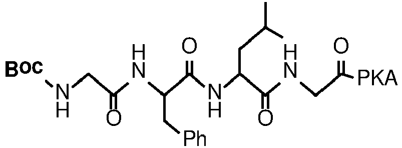

PKA (18.3 g, 31.2028 mmoL), HBTU (17.75 g, 46.8042 mmoL), and HOBT (6.3241 g, 46.8042 mmoL) were added in a round-bottomed flask and then dissolved with the DMF solution of Boc-GFLG-OH; the obtained solution was then stirred for 30 min at -5 °C and DIEA (23.2076 mL, 140.4126 mmoL) was then slowly added dropwise to the mixed solution to react for 2 h; then, the reaction solution was stirred overnight at room temperature to react At the end of the reaction, methyl tert-butyl ether and n-hexane were added to the reaction solution and the obtained solution was then rested; the supernatant was discarded and there was no product in the supernatant. A small amount of ethyl acetate was added to the oily product, the mixed solution was treated by ultrasonic and then precipitated with methyl tert-butyl ether and n-hexane, and the process of dissolution and precipitation by using ethyl acetate, methyl tert-butyl ether and n-hexane was repeated four times, and the product was evaporated to dryness. 40 g of product was obtained, 7 g being extra-quota. The obtained product was directly used for the next reaction step.

24-60

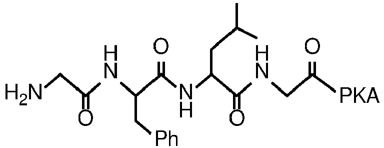

Compound 24-58 (33 g, 31.2028 mmoL) was weighed, dichloromethane (20 mL) and TFA (69.5279 mL, 936.084 mmoL) were then added sequentially, and the obtained solution was treated by ultrasonic until Compound 24-58 was completely dissolved; a ground glass stopper was used, and the mixed solution was stirred at room temperature to react. At the end of the reaction, the reaction solution was evaporated to dryness and dichloromethane was removed; n-hexane (200 mL)was then added and the obtained solution was treated by ultrasonic for 10 min and then rested for 20 min; the supernate was discarded; methyl tert-butyl ether was added to the lower solution until a solid was precipitated, and suction filtering was then carried out; next, methyl tert-butyl ether was added and the obtained solution was treated by ultrasonic, and then suction filtering was carried out; washing with methyl tert-butyl ether was carried out four times; the obtained solution was filtered by suction to obtain 35 g of the product with a yield of 100%.

29-110

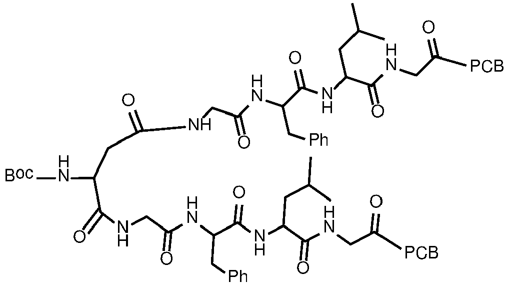

Compound 30-33 (4.0 g, 4.87 mmoL), Boc-Asp-OH (0.5 g, 2.21 mmoL), HBTU (0.90 g, 6.64 mmoL), and HOBT (2.52 g, 6.64 mmoL) were added in a 500 mL flask and then dissolved with a proper amount of DMF (50 mL), and the mixed solution was stirred for 30 min at -5 °C to react. DIEA (3.3 ml, 19.9 mmoL) was then slowly dropwise over 5 min. The reaction solution was first stirred at -5 °C to react for 1 h, and then stirred at room temperature to further react overnight. At the end of the reaction, n-hexane (100 mL) was added, the reaction solution was then shaken, and the supernatant was discard; the above operations were repeated three times. Methyl tert-butyl ether (80 mL) and a small amount of n-hexane (10 mL) were then added, the obtained solution was then shaken, and the supernatant was discarded; the above operations were repeated three times. The reaction solution precipitated to obtain a powdery solid; suction filtering was carried out, the filter cake was washed with methyl tert-butyl ether (40 mL×3) and then collected and dried in a vacuum oven. Crude product 29-110 was obtained.

29-112

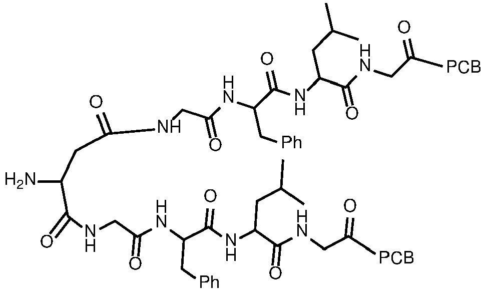

Compound 29-110 (4.17 g, 2.21 mmoL) was added in a 250 mL flask and then dissolved with dichloromethane (8 mL) and TFA (2.5 mL, 33.15 mmoL), and the mixed solution was stirred at room temperature to react overnight. At the end of the reaction, the reaction solution was evaporated with a rotary evaporator to obtain an oily product. Methyl tert-butyl ether (60 mL) was then added; the reaction solution was precipitated to obtain a powdery solid; suction filtering was carried out, the filter cake was washed with methyl tert-butyl ether (40 mL×3) and then collected, and dried in a vacuum oven. 3.84 g of crude product 29-112 was obtained with a yield of 100%.

29-114

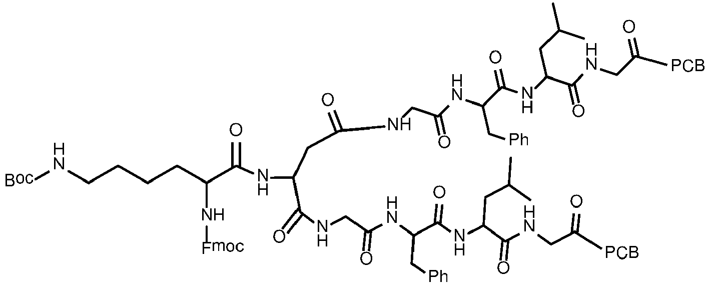

Compound 29-112 (3.84 g, 2.21 mmoL), Fmoc -Lys (Boc)-OH (1.14 g, 2.43 mmoL), HBTU (1.26 g, 3.32 mmoL), and HOBT (0.45 g, 3.32 mmoL) were added in a 500 mL flask and then dissolved with a proper amount of DMF (50 mL), and the mixed solution was stirred for 30 min at 0 °C to react. Then DIEA (1.6 mL, 9.95 mmoL) was slowly added dropwise over 3 min. The reaction solution was further stirred at 0 °C to react overnight. At the end of the reaction, n-hexane (100 mL) was added, the reaction solution was then shaken, and the supernatant was discard; the above operations were repeated three times. Methyl tert-butyl ether (80 mL) and a small amount of n-hexane (10 mL) were then added, the obtained solution was then shaken, and the supernatant was discarded; the above operations were repeated three times. The reaction solution precipitated to obtain a powdery solid; suction filtering was carried out, the filter cake was washed with methyl tert-butyl ether (40 mL×3) and then collected and dried in a vacuum oven. 4.8 g of crude product 29-112 was obtained with a yield of 100%.

29-117

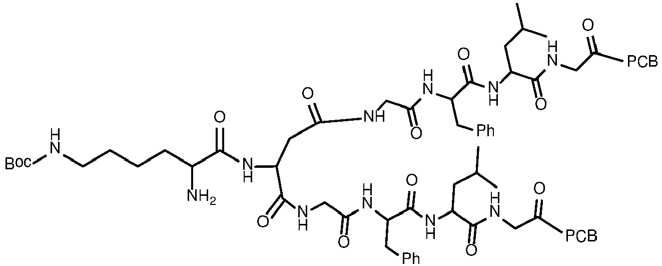

Compound 29-114 (4.8 g, 2.21 mmoL) was added in a 250 mL flask and then dissolved with DMF (10 mL), morpholine (5.8 mL, 66.3 mmoL) was then added to the obtained solution, and the mixed solution was stirred at room temperature to react for 1 h. At the end of the reaction, n-hexane (100 mL) was added, the reaction solution was then shaken, and the supernatant was discard; the above operations were repeated three times. Methyl tert-butyl ether (80 mL) and a small amount of n-hexane (10 mL) were then added, the obtained solution was then shaken, and the supernatant was discarded; the above operations were repeated three times. The reaction solution precipitated to obtain a powdery solid; then, suction filtering was carried out, the filter cake was washed with methyl tert-butyl ether (40 mL×3) and then collected and dissolved with a methanol/dichloromethane (1:4) solution (100 mL); silica gel powder (20 g) was then added to the obtained solution and the solution was evaporated to be powder; the operations of dry sample loading, column chromatography and gradient elution with a mixed solution (5.5%-8% methanol/dichloromethane) were carried out; the elution product was collected, concentrated and dried in a vacuum oven. 2.9 g of product 29-117 was obtained with a yield of 67%.

26-124

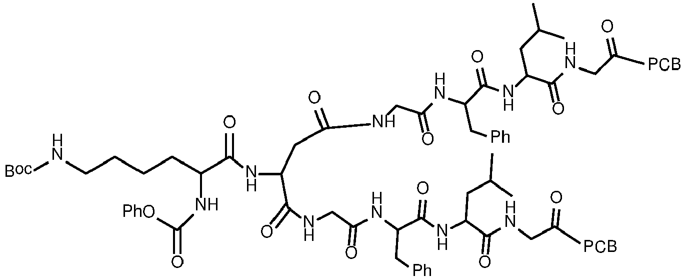

Compound 29-117 was added to a 500 mL flask and then dissolved with dichloromethane by ultrasonic, DIEA (0.56 mL, 3.43 mmoL) was then added to the obtained solution, the mixed solution was then stirred for 20 min at 0 °C, and then phenyl chloroformate (0.2 mL, 1.65 mmoL) was slowly added dropwise, and then the obtained solution reacted at 0 °C for 1 h. At the end of the reaction, n-hexane (100 mL) was added, the reaction solution was then shaken, and the supernatant was discard; the above operations were repeated three times. Methyl tert-butyl ether (80 mL) and a small amount of n-hexane (10 mL) were then added, the obtained solution was then shaken, and the supernatant was discarded; the above operations were repeated three times. The reaction solution precipitated to obtain a powdery solid; suction filtering was carried out, the filter cake was washed with methyl tert-butyl ether (40 mL×3) and then collected and dried in a vacuum oven. 2.7 g of crude product 29-124 was obtained with a yield of 100%.

26-135

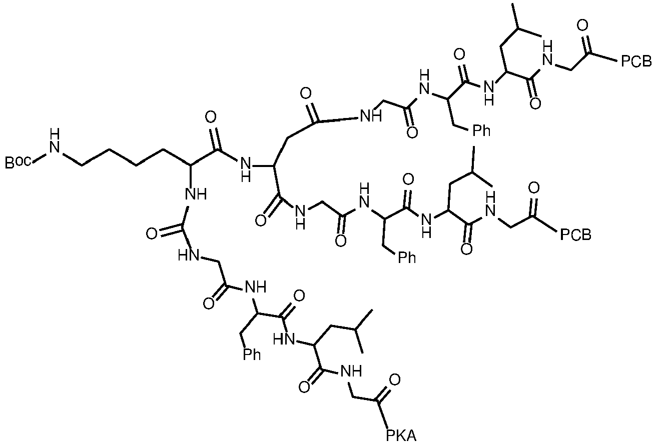

Compound 29-124 (1.8 g, 0.8 mmoL) and Compound 24-60 (1.3 g, 1.2 mmoL) were added into a 500 mL flask and then dissolved with DMF (15 mL), and the obtained solution was stirred at 80 °C to react overnight. At the end of the reaction, n-hexane (100 mL) was added, the reaction solution was then shaken, and the supernatant was discard; the above operations were repeated three times. Methyl tert-butyl ether (80 mL) and a small amount of n-hexane (10 mL) were then added, the obtained solution was then shaken, and the supernatant was discarded; the above operations were repeated three times. The reaction solution precipitated to obtain a powdery solid; then, suction filtering was carried out, the filter cake was washed with methyl tert-butyl ether (40 mL × 3) and then collected and dissolved with a methanol/dichloromethane (1:4) solution (100 mL); silica gel powder (20 g) was then added to the obtained solution and the solution was evaporated to be powder; the operations of dry sample loading, column chromatography and gradient elution with a mixed solution (4%-8% methanol/dichloromethane) were carried out; the elution product was collected, concentrated and dried in a vacuum oven. 1.2 g of product 29-135 was obtained with a yield of 52%.

26-137

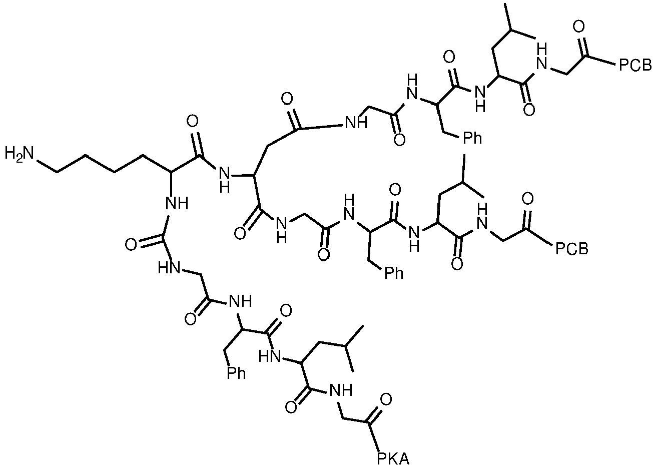

Compound 29-135 (1.2 g, 0.41 mmoL) was added in a 250 mL flask and then dissolved with dichloromethane (8 mL) and TFA (0.5 mL), and the mixed solution was stirred at room temperature to react overnight. At the end of the reaction, the reaction solution was evaporated with a rotary evaporator to obtain an oily product. Methyl tert-butyl ether (60 mL) was then added; the reaction solution was precipitated to obtain a powdery solid; suction filtering was carried out, the filter cake was washed with methyl tert-butyl ether (40 mL×3) and then collected and dissolved with a methanol/dichloromethane (1:4) solution (100 mL); silica gel powder (10 g) was then added to the obtained solution and the solution was evaporated to be powder; the operations of dry sample loading, column chromatography and gradient elution with a mixed solution (1% ammonia water/4%-7% methanol/dichloromethane) were carried out; the elution product was collected, concentrated and dried in a vacuum oven. 0.8 g of product 29-137 was obtained with a yield of 67%.

29-138

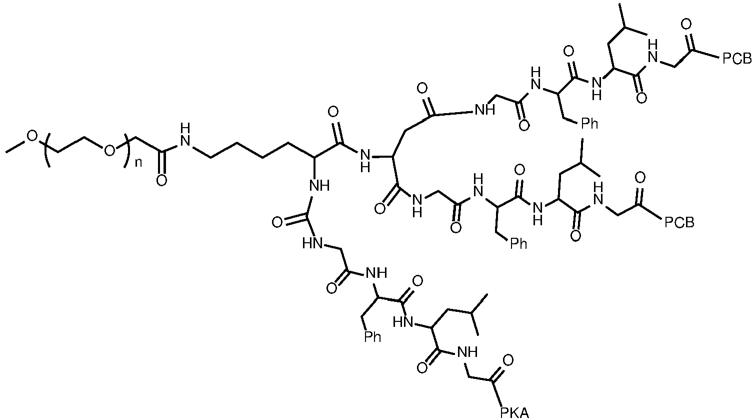

Compound 29-137 (0.4 g, 0.14 mmoL) and M-SCM-5K (purchased from JenKem) (0.77 g, 0.15 mmoL) were added into a 250 mL flask and then dissolved with DMF (15 mL), and the obtained solution was slowly stirred at room temperature to react for one week in the dark. At the end of the reaction, methyl tert-butyl ether (30 mL) were added, the reaction solution was then shaken and then precipitated to obtain a solid product; suction filtering was then carried out, the resulting filter cake was washed with methyl tert-butyl ether (40 mL×3) and dissolved with a methanol/dichloromethane (1: 4) solution (100 mL), silica gel powder (10 g) was added to the obtained solution, and the solution was then evaporated to dryness to obtain a powdery solid; the operations of dry sample loading, column chromatography, and gradient elution with a mixed solution (1% ammonia water/5%-7% methanol/dichloromethane) were carried out; the elution product was then collected, concentrated, and evaporated into a solid, and the solid was dried in a vacuum oven, thus obtaining a crude product; the crude product was then dissolved with absolute ethanol (15 mL) and dichloromethane (5 mL), and methyl tert-butyl ether (100 mL × 3) was added and a powdery solid was then precipitated; the suction filtering was carried out and the filter cake was washed with methyl tert-butyl ether (40 mL×3) and dried in the vacuum oven. 0.5 g of Product 29-138 was obtained with a yield of 45%.

¹H-NMR (400 MHz, DMSO-d₆) δ 10.16 (s, 3H), 8.96 (s,3H), 8.17 - 7.76 (m, 23H), 7.50 (d, *J* = 9.1 Hz, 4H), 7.26-7.08 (m,18H), 4.03-3.83 (m, 24H), 3.60-3.51 (m, 476H), 3.22-3.08 (m, 17H), 2.89 (s, 5H), 2.78-2.66 (m, 14H), 2.33 - 2.18 (m, 6H), 1.89-1.44(m, 41H), 0.91 - 0.79 (m, 18H).

### Example 7: Synthesis of compound 26-256

26-217

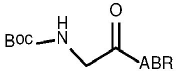

Abiraterone (ABR, 8.0 g, 22.89 mmoL), Boc-Gly-OH (5.2 g, 29.76 mmoL), and DMAP (0.28 g, 2.3 mmoL) were added into a 1 L round-bottomed flask and then dissolved with dichloromethane (400 mL); the obtained solution reacted at a low temperature for 0.5 h; DCC (11.8 g, 57.2 mmoL) was then added and the obtained solution was covered with cloth and stirred at 0 °C to react for 2 h in the dark, and then stirred at room temperature to react overnight. At the end of the reaction, the reaction solution was filtered by suction, the filtrate was evaporated to dryness and dried in a vacuum oven. 11.6 g of crude Product 26-217 was obtained with a yield of 100%.

26-218

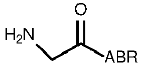

Compound 26-217 (11.6 g, 22.89 mmoL) was added in a 250 mL flask and then dissolved with dichloromethane (8 mL) and TFA (25.4980 mL, 343.35 mmoL), and the mixed solution was stirred at room temperature to react overnight. At the end of the reaction, the reaction solution was evaporated with a rotary evaporator to obtain an oily product. Ethyl acetate (50 mL) was then added and the oily product was dissolved by ultrasonic; then, petroleum ether (80 mL) was added to the solution and a solid was precipitated; suction filtering was carried out. The above operations were repeated 5 to 6 times. The filter cake was collected and dried in a vacuum oven. 9.3 g of crude Product 26-218 was obtained with a yield of 100%.

38-9

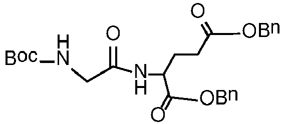

Boc-Gly-OH (4.0 g, 15.1924 mmoL), E(OBn)₂ (6.9 g, 13.8112 mmoL), HBTU (7.8567 g, 20.7169 mmoL), and HOBT (2.8 g, 20.7169 mmoL) were added in a 500 mL flask and then dissolved with a proper amount of DMF (100 mL), and the mixed solution was stirred for 30 min at -5 °C to react. Then DIEA (10.2723 mL, 62.1506 mmoL) was slowly added dropwise over 10 min. The reaction solution was first stirred at -5 °C to react for 1 h, and then stirred at room temperature to further react overnight. At the end of the reaction, the reaction solution was transferred to a 2 L separatory funnel and extracted with pure water (300 mL) and ethyl acetate (200 mL) to obtain the organic phase; the aqueous phase was extracted with ethyl acetate (200 mL×3), and the obtained organic phases were combined, washed with saturated sodium chloride solution (150 mL×3), then concentrated and evaporated to dryness, the solid was then dissolved with a methanol/dichloromethane (1:4) solution (200 mL), silica gel powder (30 g) was added to the obtained solution, and the solution was then evaporated to dryness to obtain a powdery solid; the operations of dry sample loading, column chromatography, and gradient elution with a mixed solution (50%-70% petroleum ether/ethyl acetate) were carried out; the elution product was then collected, concentrated and evaporated to obtain a solid, and the solid was then dried in a vacuum oven. 5.4 g of Product 38-9 was obtained with a yield of 62%.

26-221

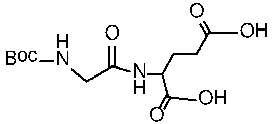

Product 38-9 (7.0276 mmoL) and 10% Pd/C (0.03 g) were added into a hydrogenation reactor and then dissolved with DMF (30 mL); the hydrogenation reaction device was then sealed to perform the "three pumping and three charging" operation (i.e., pumping the air from the reaction system with a vacuum water pump for about 3 min-charging hydrogen-pumping hydrogen-charging hydrogen---pumping hydrogen---charging hydrogen) so that the pressure on the hydrogenation reaction device was read as 0.18 MPa, and then the obtained solution reacted overnight at room temperature. At the end of the reaction, the reaction solution was filtered with diatomaceous earth, the filter cake was washed with DMF (20 mL×3). Product 26-221 was obtained with a yield of 100%.

26-222

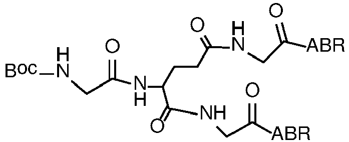

Compound 26-218 (6.0 g, 14.7689 mmoL), Compound 26-221 (7.0276 mmoL), HBTU (8.0 g, 21.0828 mmoL), and HOBT (2.85 g, 21.0828 mmoL) were added in a 500 mL flask and then dissolved with a proper amount of DMF (50 mL), and the mixed solution was stirred for 30 min at -5 °C to react. Then DIEA (10.45 mL, 63.2484 mmoL) was slowly added dropwise over 5 min. The reaction solution was first stirred at -5 °C to react for 1 h, and then stirred at room temperature to further react overnight. At the end of the reaction, n-hexane (100 mL) was added, the reaction solution was then shaken, and the supernatant was discard; the above operations were repeated three times. Methyl tert-butyl ether (80 mL) and a small amount of n-hexane (10 mL) were then added, the obtained solution was then shaken, and the supernatant was discarded; the above operations were repeated three times. The reaction solution precipitated to obtain a powdery solid; suction filtering was carried out, the filter cake was washed with methyl tert-butyl ether (40 mL×3) and then collected and dried in a vacuum oven. Crude Product 26-222 was obtained with a yield of 100%.

26-227

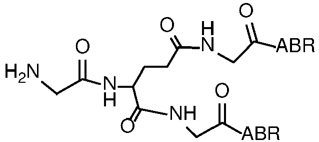

Compound 26-222 (9.1 g, 7.0276 mmoL) was added in a 250 mL flask and then dissolved with dichloromethane (8 mL) and TFA (7.8 mL, 105.414 mmoL), and the mixed solution was stirred at room temperature to react overnight. At the end of the reaction, the reaction solution was evaporated with a rotary evaporator to obtain an oily product. Methyl tert-butyl ether (60 mL) was then added; the reaction solution was precipitated to obtain a powdery solid; suction filtering was carried out, the filter cake was washed with methyl tert-butyl ether (40 mL×3) and then collected and dissolved with a methanol/dichloromethane (1:4) solution (100 mL); silica gel powder (20 g) was then added to the obtained solution and the solution was evaporated to be powder; the operations of dry sample loading, column chromatography and gradient elution with a mixed solution (3%-6% methanol/dichloromethane) were carried out; the elution product was collected, concentrated and dried in a vacuum oven. 4.3 g of product 26-227 was obtained with a yield of 52%.

26-229

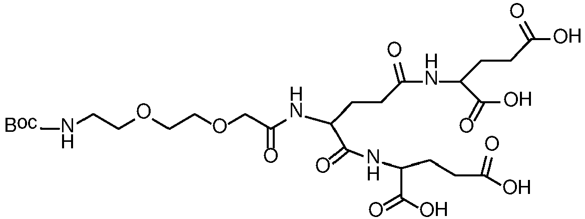

Product 14-163 (0.8961 g, 0.8862 mmoL) and 10% Pd/C (0.03 g) were added into a hydrogenation reactor and then dissolved with DMF (30 mL); the hydrogenation reaction device was then sealed to perform the "three pumping and three charging" operation (i.e., pumping the air from the reaction system with a vacuum water pump for about 3 min-charging hydrogen-pumping hydrogen-charging hydrogen---pumping hydrogen---charging hydrogen) so that the pressure on the hydrogenation reaction device was read as 0.18 MPa, and then the obtained solution reacted overnight at room temperature. At the end of the reaction, the reaction solution was filtered with diatomaceous earth, and the filter cake was washed with DMF (20 mL×3). Product 26-229 was obtained with a yield of 100%.

26-230

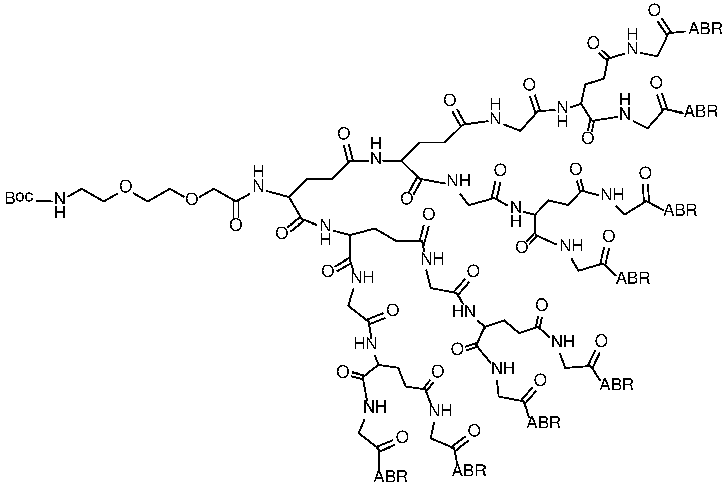

Compound 26-227 (4.3 g, 3.6333 mmoL), Compound 26-229 (0.8862 mmoL), HBTU (2.0165 g, 5.3171 mmoL), and HOBT (0.7185 g, 5.3171 mmoL) were added in a 500 mL flask and then dissolved with a proper amount of DMF (50 mL), and the mixed solution was stirred for 30 min at -5 °C to react. Then DIEA (10.45 mL, 63.2484 mmoL) was slowly added dropwise over 5 min. The reaction solution was first stirred at -5 °C to react for 1 h, and then stirred at room temperature to further react overnight. At the end of the reaction, n-hexane (100 mL) was added, the reaction solution was then shaken, and the supernatant was discard; the above operations were repeated three times. Methyl tert-butyl ether (80 mL) and a small amount of n-hexane (10 mL) were then added, the obtained solution was then shaken, and the supernatant was discarded; the above operations were repeated three times. The reaction solution precipitated to obtain a powdery solid; suction filtering was carried out, the filter cake was washed with methyl tert-butyl ether (40 mL × 3) and then collected and dried in a vacuum oven. Crude Product 26-230 was obtained with a yield of 100%.

26-240

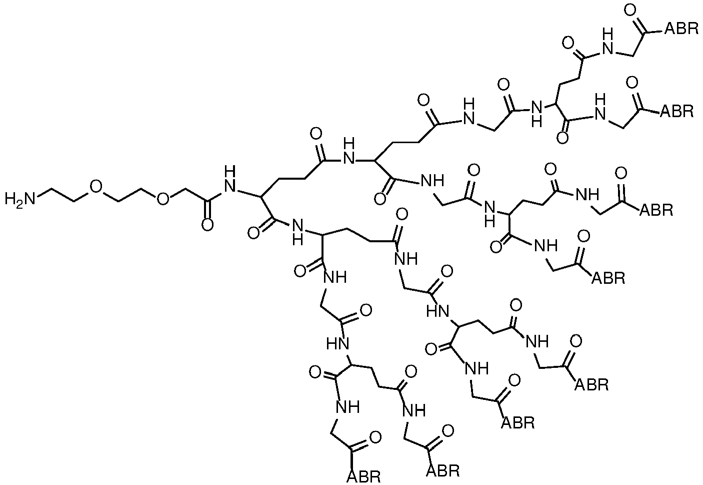

Compound 26-230 (4.7 g, 0.8862 mmoL) was added in a 250 mL flask and then dissolved with dichloromethane (8 mL) and TFA (0.9872 mL, 13.293 mmoL), and the mixed solution was stirred at room temperature to react overnight. At the end of the reaction, the reaction solution was evaporated with a rotary evaporator to obtain an oily product. Methyl tert-butyl ether (60 mL) was then added; the reaction solution was precipitated to obtain a powdery solid; suction filtering was carried out, the filter cake was washed with methyl tert-butyl ether (40 mL×3) and then collected and dissolved with a methanol/dichloromethane (1:4) solution (100 mL); silica gel powder (20 g) was then added to the obtained solution and the solution was evaporated to be powder; the operations of dry sample loading, column chromatography and gradient elution with a mixed solution (4%-8% methanol/dichloromethane) were carried out; the elution product was collected, concentrated and dried in a vacuum oven. 3.3 g of product 26-240 was obtained with a yield of 72%.

40-5

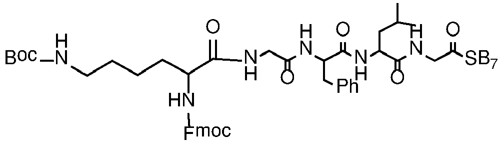

Compound 3-16 (2.0 g, 2.24 mmoL), Fmoc-L-Lys (Boc)-OH (1.15 g, 2.46 mmoL), HBTU (1.27 g, 3.36 mmoL), and HOBT (0.45 g, 3.36 mmoL) were added in a 250 mL flask and then dissolved with a proper amount of DMF (30 mL), and the mixed solution was stirred for 30 min at 0 °C to react. DIEA (1.67 ml, 10.08 mmoL) was then slowly dropwise over 5 min. The reaction solution was further stirred at 0 °C to react overnight. At the end of the reaction, the reaction solution was transferred to a 1 L separatory funnel and extracted with pure water (100 mL) and ethyl acetate (80 mL) to obtain the organic phase; the aqueous phase was extracted with ethyl acetate (80 mL×3), and the obtained organic phases were combined and washed with saturated sodium chloride solution (70 mL × 3); the obtained solution was then concentrated, evaporated to dryness, and dried in a vacuum oven. Crude Product 40-5 was obtained with a yield of 100%.

40-6

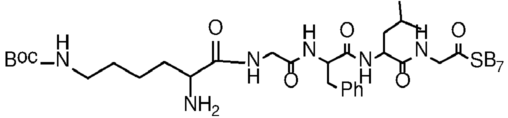

Compound 40-5 (3.01 g, 2.24 mmoL) was added in a 250 mL flask and then dissolved with DMF (10 mL), morpholine (3.9 mL, 44.8 mmoL) was then added to the obtained solution, and the mixed solution was stirred at room temperature to react for 1h. At the end of the reaction, the reaction solution was transferred to a 1 L separatory funnel and extracted with pure water (100 mL) and ethyl acetate (80 mL) to obtain the organic phase; the aqueous phase was extracted with ethyl acetate (80 mL×3), and the obtained organic phases were combined and washed with saturated sodium chloride solution (70 mL×3); the obtained solution was then concentrated, evaporated to dryness, and dried in a vacuum oven. Crude Product 40-6 was obtained with a yield of 100%.

40-7

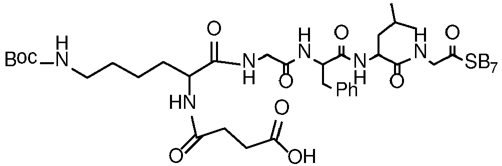

Compound 40-6 (2.51 g, 2.24 mmoL) was added to a 500 mL flask and then dissolved with DMF (10 mL), and then DIEA (1.48 mL, 8.96 mmoL) and succinoside (0.67 g, 6.72 mmoL) were slowly added dropwise to continue the reaction, and the reaction was stirred at room temperature to react overnight. At the end of the reaction, n-hexane (100 mL) was added, the reaction solution was then shaken, and the supernatant was discard; the above operations were repeated three times. Methyl tert-butyl ether (80 mL) and a small amount of n-hexane (10 mL) were then added, the obtained solution was then shaken, and the supernatant was discarded; the above operations were repeated three times. The reaction solution precipitated to obtain a powdery solid; then, suction filtering was carried out, the filter cake was washed with methyl tert-butyl ether (40 mL × 3) and then collected and dissolved with a methanol/dichloromethane (1:4) solution (100 mL); silica gel powder (8 g) was then added to the obtained solution and the solution was evaporated to be powder; the operations of dry sample loading, column chromatography and gradient elution with a mixed solution (1% ammonia water/5%-7% methanol/dichloromethane) were carried out; the elution product was collected, concentrated and dried in a vacuum oven. 1.2 g of product 40-7 was obtained with a yield of 48%.

26-242

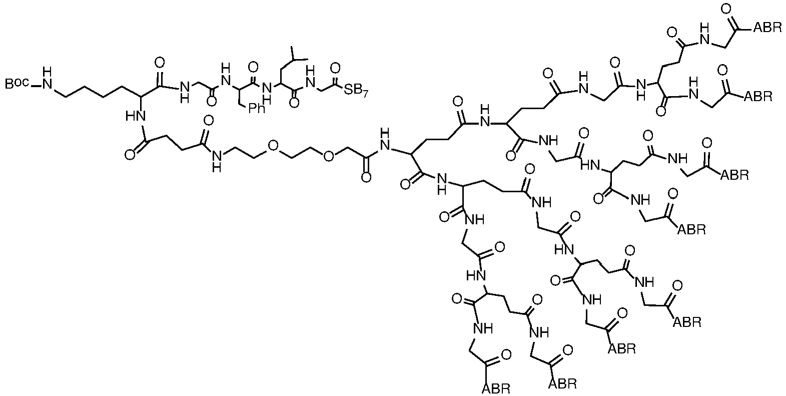

Compound 40-7 (0.6 g, 0.5276 mmoL), Compound 26-240 (2.5 g,0.4796 mmoL), HBTU (0.2728 g, 0.7194 mmoL), and HOBT (0.1 g, 0.7194 mmoL) were added in a 250 mL flask and then dissolved with a proper amount of DMF (50 mL), and the mixed solution was stirred for 30 min at -5 °C to react. Then DIEA (0.3573 mL, 2.1583 mmoL) was slowly added dropwise over 2 min. The reaction solution was first stirred at -5 °C to react for 1 h, and then stirred at room temperature to further react overnight. At the end of the reaction, n-hexane (100 mL) was added, the reaction solution was then shaken, and the supernatant was discard; the above operations were repeated three times. Methyl tert-butyl ether (80 mL) and a small amount of n-hexane (10 mL) were then added, the obtained solution was then shaken, and the supernatant was discarded; the above operations were repeated three times. The reaction solution precipitated to obtain a powdery solid; suction filtering was carried out, the filter cake was washed with methyl tert-butyl ether (40 mL×3) and then collected and dried in a vacuum oven. Crude Product 26-242 was obtained with a yield of 100%.

26-248

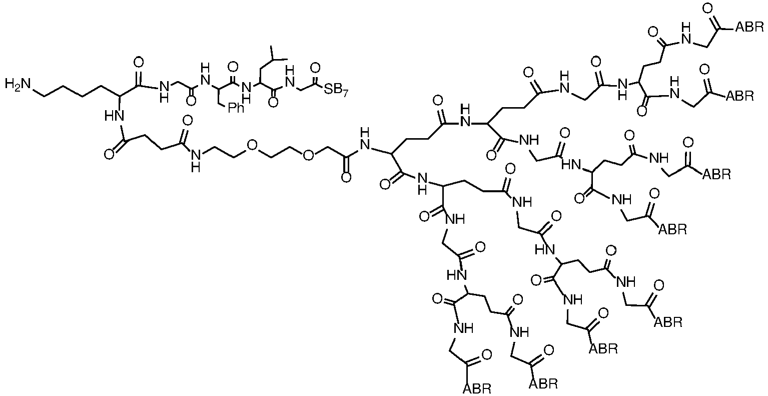

Compound 26-242 (3.2 g, 0.5276 mmoL) was added in a 250 mL flask and then dissolved with dichloromethane (8 mL) and TFA (0.5877 mL, 7.914 mmoL), and the mixed solution was stirred at room temperature to react overnight. At the end of the reaction, the reaction solution was evaporated with a rotary evaporator to obtain an oily product. Methyl tert-butyl ether (60 mL) was then added; the reaction solution was precipitated to obtain a powdery solid; suction filtering was carried out, the filter cake was washed with methyl tert-butyl ether (40 mL×3) and then collected and dissolved with a methanol/dichloromethane (1:4) solution (100 mL); silica gel powder (20 g) was then added to the obtained solution and the solution was evaporated to be powder; the operations of dry sample loading, column chromatography and gradient elution with a mixed solution (3%-8% methanol/dichloromethane) were carried out; the elution product was collected, concentrated and dried in a vacuum oven. 1.7 g of product 26-248 was obtained with a yield of 55%.

26-256

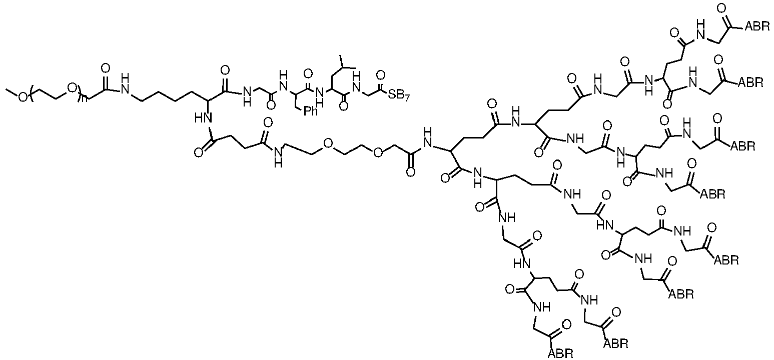

Compound 26-242 (0.2110 g, 0. 3571 mmoL), M-CM-40K (purchased from JenKem, 1.5 g, 0. 3752 mmoL), HBTU (0.2031 g, 0.5357 mmoL), and HOBT (0.072 g, 0.5357 mmoL) were added in a 250 mL flask and then dissolved with a proper amount of DMF (40 mL), and the mixed solution was stirred for 30 min at 0 °C to react. DIEA (0.5312 mL, 3.2139 mmoL) was then slowly dropwise over 30 min. Then, the reaction solution was slowly stirred at room temperature in the dark to react for one week. At the end of the reaction, methyl tert-butyl ether (30 mL) were added, the reaction solution was then shaken and then precipitated to obtain a solid product; suction filtering was then carried out, the resulting filter cake was washed with methyl tert-butyl ether (40 mL×3) and dissolved with a methanol/dichloromethane (1: 4) solution (100 mL), silica gel powder (8 g) was added to the obtained solution, and the solution was then evaporated to dryness to obtain a powdery solid; the operations of dry sample loading, column chromatography, and gradient elution with a mixed solution (5%-9% methanol/dichloromethane) were carried out; the elution product was then collected, concentrated, and evaporated into a solid, and the solid was dried in a vacuum oven, thus obtaining a crude product; the crude product was then dissolved with absolute ethanol (15 mL) and dichloromethane (5 mL), and methyl tert-butyl ether (100 mL×3) was added and a powdery solid was then precipitated; the suction filtering was carried out and the filter cake was washed with methyl tert-butyl ether (40 mL×3) and dried in the vacuum oven. 1.2 g of Product 26-242 was obtained with a yield of 71%.

¹H-NMR (400 MHz, DMSO-d₆) δ 8.57 (s, 6H), 8.45-8.30 (m, 13H), 8.25 - 8.02 (m, 23H), 7.76-7.68 (m, 9H), 7.35 - 7.15 (m, 22H), 6.10 (s, 10H), 5.46-5.32 (m, 14H), 4.61 - 4.07 (m, 70H), 3.50 (s,3747H), 3.26 - 2.64 (m, 65H), 2.37 - 1.17 (m,137H), 1.08-0.82(m, 34H).

### Example 8: Synthesis of compound 28-229

22-262

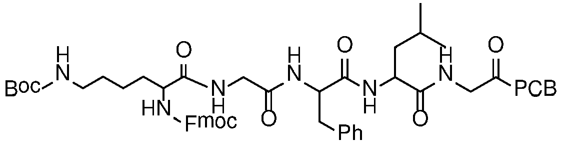

Product 25-254 (synthesized according to the synthesis method of Compound 30-33, 3.86 g, 4.6954 mmoL), Fmoc-L-Lys (Boc)-OH (2 g, 4.2686 mmoL), HBTU (2.43 g, 6.4029 mmoL), and HOBT (0.87 g, 6.4029 mmoL) were added in a 500 mL flask and dissolved with 20 mL of DMF; the obtained solution was stirred at -5 °C to react for 30 min, and DIEA (3.2 mL, 19.2086 mmoL) was then slowly added dropwise; the obtained solution further reacted at for 1 h, and then stirred at room temperature to react overnight. At the end of the reaction, n-hexane (100 mLx3) was added for precipitation, and the lower oily solid was dissolved with a small amount of dichloromethane; methyl tert-butyl ether was added, and a solid product was precipitated and then dried in an oven. 9.1 g of the Product 22-262 was obtained with a yield of 100%.

22-263

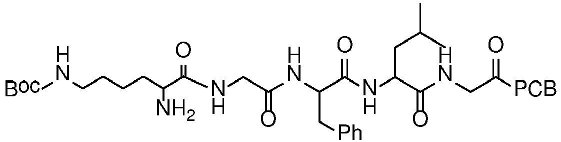

Product 22-262 (9.1 g, 4.2686 mmoL) was added in a 250 mL flask and then dissolved with DMF (20 mL), morpholine (3.72 ml, 42.686 mmoL) was added to the obtained solution, and the mixed solution was stirred at room temperature to react for 1 h. At the end of the reaction, n-hexane (100 mLx3) was added for precipitation, and the lower oily solid was dissolved with a small amount of dichloromethane; methyl tert-butyl ether was added, and a solid product was precipitated; the filter cake was dissolved with a methanol/dichloromethane (1:5) solution, 40 mL of silica gel powder was added to the obtained solution, and the solution was then evaporated to dryness; the operations of dry sample loading, column chromatography, and gradient elution with an eluent (0%-0.5% ammonia water: 2%-4% methanol: 98%-95.5% dichloromethane) were carried out; the filtrate was then collected, concentrated and evaporated to dryness. 5.8 g of Product 22-263 was obtained with a yield of 100%.

### MALDI-TOF MS: [M+Na⁺]1072.45

22-267

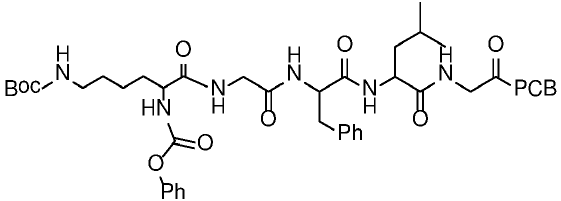

Compound 22-263 (5.8 g, 4.2686 mmoL) was added into a 250 mL flask and then dissolved with 20 mL of dichloromethane, triethylamine (3 ml, 21.343 mmoL) was then added, the reaction solution was then stirred at 0 °C for 20 to 30 min, and phenyl chloroformate (1.6 ml, 12.8058 mmoL) was then slowly added dropwise to the reaction solution. After the phenyl chloroformate was added completely, the solution reacted at 0 °C for 2 to 3 h. At the end of the reaction, when the reaction solution was evaporated with a rotary evaporator to one-half of the original volume, 100 mL of n-hexane and 200 mL of methyl tert-butyl ether were added, and a solid product was precipitated; the filter cake was dissolved with a methanol/dichloromethane (1:5) solution, 60 mL of silica gel powder was added to the obtained solution, and the solution was then evaporated to dryness; the operations of dry sample loading, column chromatography, and gradient elution with an eluent (1%-4% methanol: 99%-96% dichloromethane) were carried out; the filtrate was then collected, concentrated and evaporated to dryness. 5.7 g of Product 22-267 was obtained with a yield of 100%.

22-273

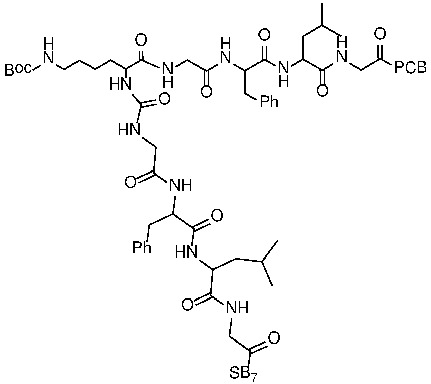

Compound 22-267 (4.99 g, 4.2686 mmoL) and Compound 22-272 (synthesized according to the synthesis method of Compound 3-16, 3.8 g, 4.2686 mmoL) were added in a 500 mL flask and then dissolved with DMF (20 mL), and the mixed solution was stirred for 3 to 4 h in an oil bath at 45 to 80 °C to react. At the end of the reaction, n-hexane (100 mLx3) was added for precipitation, and the lower oily solid was dissolved with a small amount of dichloromethane; methyl tert-butyl ether was added, and a solid product was precipitated; the filter cake was dissolved with a methanol/dichloromethane (1:5) solution, 40 mL of silica gel powder was added to the obtained solution, and the solution was then evaporated to dryness; the operations of dry sample loading, column chromatography, and gradient elution with an eluent (0.5% ammonia water: 1%-5% methanol: 98.5%-94.5% dichloromethane) were carried out; the filtrate was then collected, concentrated and evaporated to dryness. 7.5 g of Product 22-273 was obtained with a yield of 89.3%.

### MALDI-TOF MS: [M+Na⁺] 1988.92

22-277

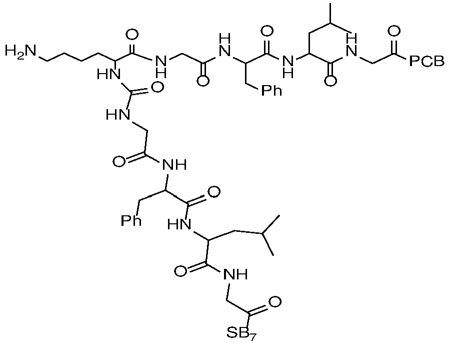

Product 22-273 (7.5 g, 3.8115 mmoL) was added in a 500 mL flask and then dissolved with dichloromethane (10 mL), TFA (2.8 mL, 38.1148 mmoL) was then added in the flask, and the mixed solution was stirred at room temperature to react overnight. At the end of the reaction, the reaction solution was concentrated to 10 mL, methyl tert-butyl ether (200 mL) was then added and the powdery product was precipitated; suction filtering was then carried out, and the filter cake was washed with methyl tert-butyl ether (50 mL × 3) and then dissolved with 200 mL of a mixed solution (20% methanol: 80% dichloromethane; silica gel powder (60 g) was added and the obtained solution was then evaporated to dryness to obtain a powdery solid; the operations of dry sample loading, column chromatography, and gradient elution with a mixed solution (1% ammonia water: 4%-7% methanol/dichloromethane) were carried out; the elution product was then collected, concentrated, and evaporated to dryness. 2.4 g of Product 22-277 was obtained with a yield of 34%.

### MALDI-TOF MS: [M+H⁺] 1966.72, [M+Na⁺] 1988.67

28-211

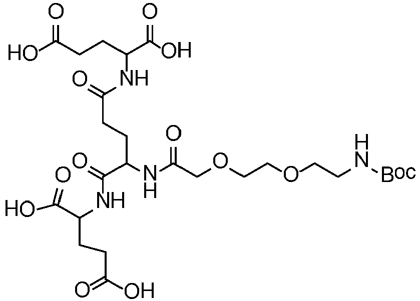

Compound 25-75 (0.1839 g, 0.1819 mmoL, synthesized according to the synthesis method of Compound 14-163) and 10% Pd/C (0.0100 g) were sequentially added into a hydrogenation reactor and then dissolved with DMF (30 mL), hydrogen gas (Pa=1.6 MPa) was then introduced into the reactor, and the obtained solution was stirred overnight at room temperature to react. At the end of the reaction, the reactor was taken out, the reaction solution was filtered with diatomaceous earth, and washing with DMF (20 mL × 3) was then carried out. 0.1183 g of Product 28-211 was obtained with a yield of 100%.

28-212

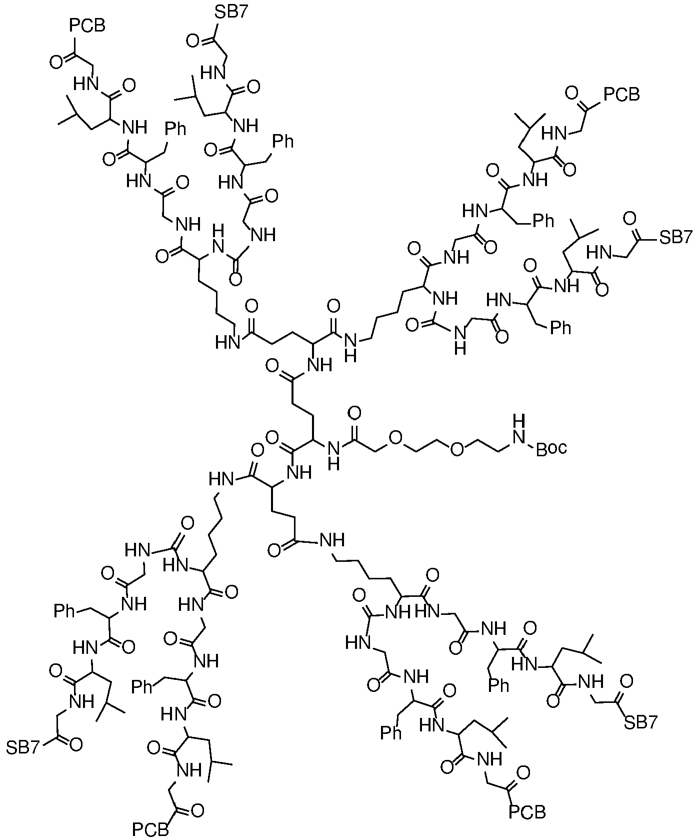

Compound 28-211 (0.1183 g, 0.1819 mmoL), Compound 22-277 (1.56 g, 0.8353 mmoL), HBTU (0.4139 g, 1.0914 mmoL), and HOBT (0.1475 g, 1.0914 mmoL) were added into a 250 mL flask and the mixed solution was stirred at -5 °C for about 10 min to react; DIEA (0.54 mL, 3.2742 mmoL) was then slowly added dropwise, and the obtained solution further reacted at -5 °C for 1 h; and the solution was stirred overnight at room temperature to react. At the end of the reaction, n-hexane (150 mL) and methyl tert-butyl ether (40 mL) were added for precipitation, supernatant was discarded, and n-hexane (150 mL) and methyl tert-butyl ether (40 mL) were added to the lower oily solution; such operations were repeated four times to obtain a viscous oily product; methyl tert-butyl ether (100 mL) was added to precipitate a solid product; suction filtering was then carried out and the filter cake was dissolved with a mixed solution of methanol (40 mL) and dichloromethane (160 mL), silica gel powder (15 g) was then added to the obtained solution, and the solution was then evaporated to dryness to obtain a powdery solid; the operations of dry sample loading, column chromatography, and gradient elution with a mixed solution (1% ammonia water: 5-7% methanol/dichloromethane) were carried out; the elution product was then collected, concentrated, and evaporated to dryness. 1.1 g of Product 28-212 with a yield of 75%.

28-218

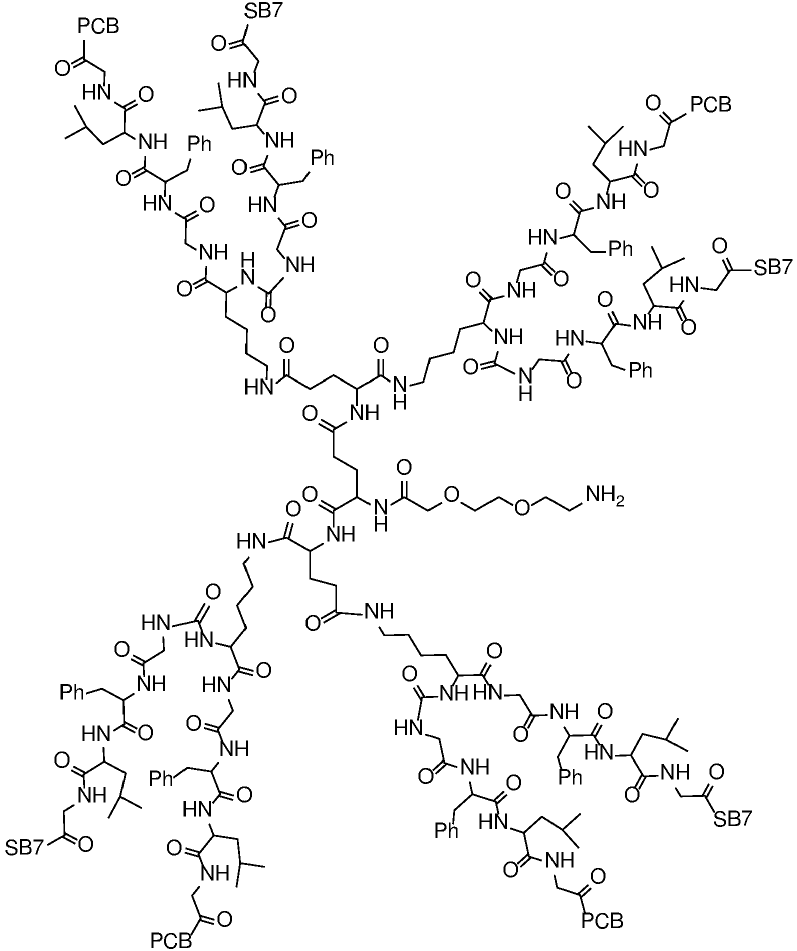

Product 28-212 (1.1 g, 0.1367 mmoL) was added in a 250 mL flask and then dissolved with dichloromethane (15 mL), TFA (2.0152 mL, 13.67 mmoL) was then added in the flask, and the mixed solution was stirred overnight at room temperature to react. At the end of the reaction, the reaction solution was concentrated to a small amount, methyl tert-butyl ether (150 mL) was then added and the powdery product was precipitated; suction filtering was then carried out, and the filter cake was washed with methyl tert-butyl ether (50 mL×3) and then dissolved with a mixed solutionof methanol (30 mL) and dichloromethane (120 mL); silica gel powder (15 g) was added and the obtained solution was then evaporated to dryness to obtain a powdery solid; the operations of dry sample loading, column chromatography, and gradient elution with a mixed solution (1% ammonia water: 6%-8% methanol/dichloromethane) were carried out; the elution product was then collected, concentrated, and evaporated to dryness. 0.75 g of Product 28-218 was obtained with a yield of 69%.

28-229

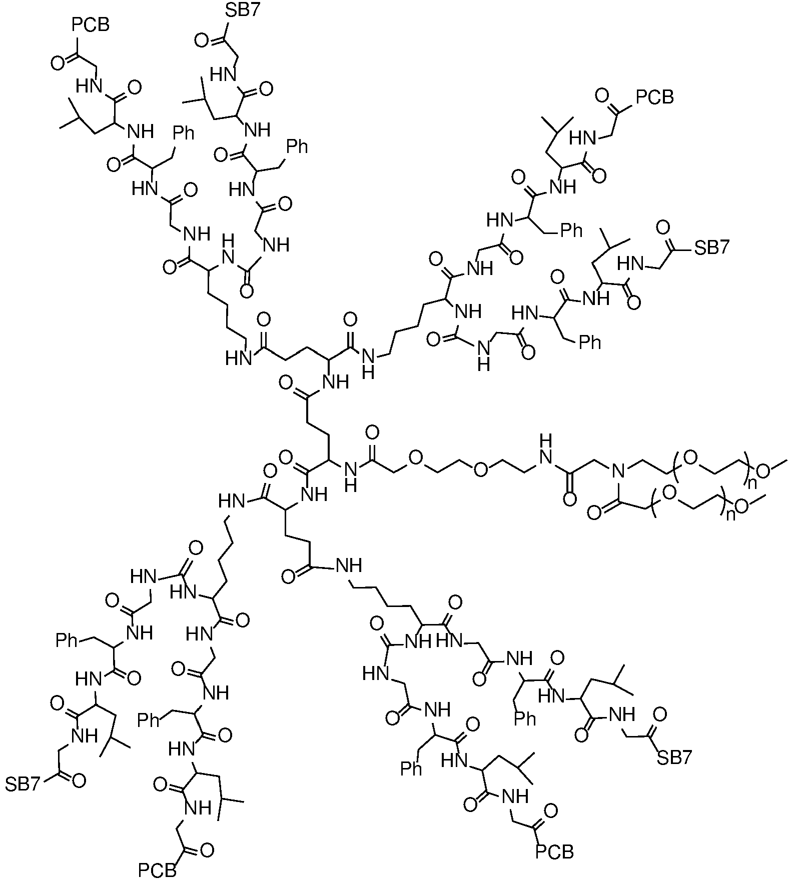

Compound 28-218 (0.3771 g, 0.0474 mmoL) was added into 250 mL and then dissolved with DMF (25 mL); Y-SCM-40K (1.8748 g, 0.0431 mmoL) was then added to the mixed solution and dissolved by ultrasonic; the obtained solution was slowly stirred for 7 days at room temperature in the dark to react. At the end of the reaction, n-hexane (150 mL) and methyl tert-butyl ether (50 mL) were added to the solution, the supernatant was discarded, and n-hexane (150 mL) and methyl tert-butyl ether (50 mL) were added to the lower liquid; such operations were repeated four times to obtain a viscous oily product; the viscous oily product was then dissolved with a mixed solution of methanol (30 mL) and dichloromethane (120 mL); silica gel powder (25 g) was added to the obtained solution, and the solution was then evaporated to dryness to obtain a powdery solid; the operations of dry sample loading, column chromatography, and gradient elution with a mixed solution (1% ammonia water: 6%-8% methanol/dichloromethane) were carried out; the elution product was then collected, concentrated and evaporated to dryness to obtain a solid, and the solid was dried for 1 h in a vacuum oven; the product was dissolved with absolute ethanol (10 mL) and dichloromethane (20 mL), and methyl tert-butyl ether (30 mL) and n-hexane (100 mL) were added to the obtained solution to separate out a solid product by precipitation; the solution was filtered and the filter cake was washed with methyl tert-butyl ether (50 mL × 2) and dried in the vacuum oven. 1.53 g of Product 28-229 was obtained with a yield of 68%.

¹H-NMR (400 MHz, DMSO-d6) δ 10.15 (s, 3H), 9.04 (s, 2H), 8.95 (s, 2H), 8.04 (m, 52H), 7.53 (m, 10H), 7.17 (m, 89H), 5.79 (m, 2H), 4.53 (s, 9H), 4.36 (s, 7H), 4.24 (s, 15H), 3.51 (s, 3976H), 2.89 (s, 6H), 2.73 (s, 12H), 2.41 (s, 12H), 2.30 (s, 23H), 2.03 - 1.82 (m, 5H), 1.49 (s, 33H), 1.34 (m, 56H), 1.23 (m, 116H), 0.86 (m, 67H), 0.49 (s, 6H).

### Example 9: Synthesis of compound 43-8

28-252

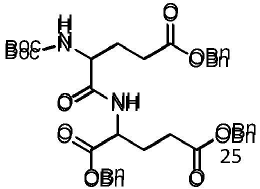

Boc-Glu-(OH) (OBn) (5 g, 14.82 mmoL), H-Glu-(OBn) ₂ (7.40 g, 14.82 mmoL), HBTU (8.43 g, 22.23 mmoL), and HOBT (3.0 g, 22.23 mmoL) were added into a 500 mL flask and then dissolved with DMF (55 mL); the obtained solution was stirred at -5 °C for about 30 min to react, and then DIEA (13.47 mL, 81.51 mmoL) was slowly added dropwise; and then the obtained solution was further stirred at -5 °C for 1 h to react, and then stirred overnight at room temperature to react. At the end of the reaction, the reaction solution was transferred to a 1 L separatory funnel, saturated NaHCO₃ (250 mL) and ethyl acetate (200 mL) were then added, and the obtained solution was shaken and extracted. The aqueous phase was washed with ethyl acetate (150 mL×2), the organic phases were combined, washed with saturated sodium chloride solution (200 mL×2), concentrated, evaporated to dryness, and dried in a vacuum oven. 9.5 g of Product 28-252 was obtained with a yield of 100%.

28-253

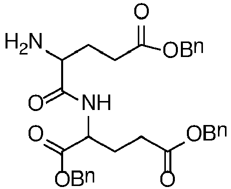

Compound 28-252 (9.58 g, 14.82 mmoL) was added in a 500 mL flask and then dissolved with dichloromethane (30 mL), TFA (13.68 mL, 148.2 mmoL) was then added with stirring, and the mixed solution was stirred overnight at room temperature to react. At the end of the reaction, the reaction solution was concentrated and then transferred to a 1 L separatory funnel and a saturated NaHCO₃ solution (300 mL) and ethyl acetate (200 mL) were added; the obtained solution was then shaken and extracted to obtain an organic phase; the aqueous phase was washed with ethyl acetate (200 mL × 2), and the obtained organic phases were combined and washed with saturated sodium chloride solution (200 mL × 2); the obtained solution was then concentrated, evaporated to dryness, and dried in a vacuum oven. 8.1 g of Product 28-253 was obtained with a yield of 100%.

28-254

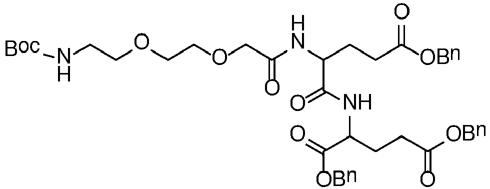

Boc-LC-OH (3.55 g, 13.47 mmoL, synthesized according to the synthesis method of Compound 24-36), Compound 28-253 (8.1 g, 14.82 mmoL), HBTU (7.61 g, 20.21 mmoL), and HOBT (2.73 g, 20.21 mmoL) were added in a 500 mL flask and dissolved with DMF (50 mL), and the obtained solution was stirred for about 30 min at -5 °C; then, DIEA (10.02 mL, 60.62 mmoL) was slowly added dropwise; the obtained solution was further stirred at -5 °C for 1 h, and then the reaction solution was stirred overnight at room temperature to further react. At the end of the reaction, the reaction solution was transferred to a 1 L separatory funnel, saturated NaHCO₃ (250 mL) and ethyl acetate (200 mL) were then added, and the obtained solution was shaken and extracted. The aqueous phase was washed with ethyl acetate (150 mL×2),the organic phases were combined, washed with saturated sodium chloride solution (200 mL×2),concentrated, evaporated to dryness, and dried in a vacuum oven. 10.7 g of Product 28-254 was obtained with a yield of 100%.

28-256

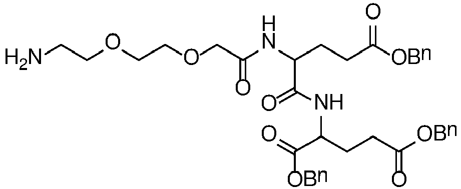

Compound 28-254 (10.66 g, 13.47 mmoL) was added in a 500 mL flask and then dissolved with dichloromethane (30 mL), TFA (15 mL, 202.05 mmoL) was then added with stirring, and the mixed solution was stirred overnight at room temperature to react. At the end of the reaction, the reaction solution was concentrated and then transferred to a 1 L separatory funnel; a saturated sodium bicarbonate solution (300 mL) and ethyl acetate (200 mL) were then added and the obtained solution was shaken and extracted to obtain an organic phase; the aqueous phase was washed with ethyl acetate (150 mL × 2), and the obtained organic phases were combined, washed with saturated sodium chloride solution (200 mL × 2), then concentrated and evaporated to dryness; the solid product was then dissolved with methanol (30 mL) and dichloromethane (120 mL), silica gel powder (25 g) was added to the obtained solution, and the solution was then evaporated to dryness to obtain a powdery solid; the operations of dry sample loading, column chromatography, and gradient elution with a mixed solution (0.5% ammonia water : 3%methanol/dichloromethane) were carried out. 7.8 g of Product 28-256 was obtained with a yield of 84%.

28-263

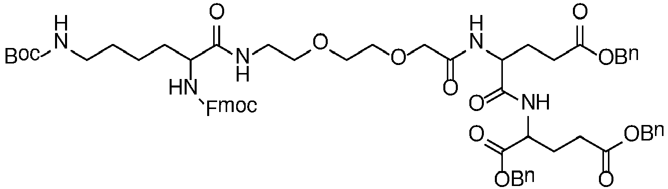

Fmoc-L-Lys(Boc)-OH (1.76 g, 3.76 mmoL), Compound 28-256 (2.6 g, 3.76 mmoL), HBTU (2.14 g, 5.64 mmoL), HOBT (0.76 g, 5.64 mmoL) were added in a 500 mL flask and then dissolved with DMF (50 mL), and the obtained solution was stirred for about 30 min at -5 °C; then, DIEA (2.8 mL, 16.92 mmoL) was slowly added dropwise; the obtained solution was further stirred at -5 °C for 3 h to react. At the end of the reaction, the reaction solution was transferred to a 1 L separatory funnel, deionized water (200 mL) and ethyl acetate (200 mL) were then added, and the obtained solution was shaken and extracted. The aqueous phase was washed with ethyl acetate (150 mL×2), the organic phases were combined, washed with saturated sodium chloride solution (200 mL×2), concentrated, evaporated to dryness, and dried in a vacuum oven. 4.2 g of Product 28-263 was obtained with a yield of 100%.

37-3

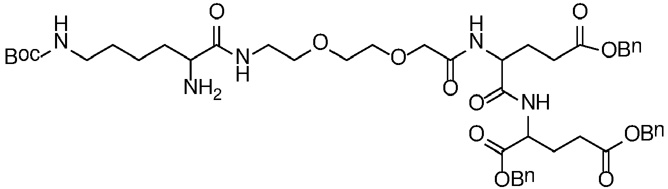

Compound 28-263 (5.7 g, 4.99 mmoL) was added into a 250 mL flask and then dissolved with DMF (20 mL); morpholine (8.69 mL, 99.8 mmoL) was added to the obtained solution, and the obtained solution was stirred at room temperature for 1 h to react. At the end of the reaction, the reaction solution was transferred to a 1 L separatory funnel and extracted with deionized water (200 mL) and ethyl acetate (200 mL) to obtain an organic phase; the aqueous phase was washed with ethyl acetate (200 mL×2), and the obtained organic phases were combined, washed with saturated sodium chloride solution (200 mL×2), then concentrated and evaporated to dryness; the solid product was then dissolved with methanol (30 mL) and dichloromethane (120 mL), silica gel powder (20 g) was added to the obtained solution, and the solution was then evaporated to dryness to obtain a powdery solid; the operations of dry sample loading, column chromatography, and elution with a mixed solution (0.5% ammonia water : 3% methanol/dichloromethane) were carried out. 2.7 g of Product 37-3 was obtained with a yield of 58%.

28-262

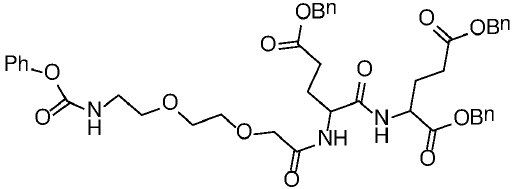

Compound 28-256 (5.2 g, 7.5 mmoL) was added in a 250 mL round-bottomed flask and then dissolved with dichloromethane (15 mL) by ultrasonic; TEA (2.1 mL, 15 mmoL) was then added to the obtained solution and the obtained solution was then stirred for 30 min at 0 °C to react. Then, phenyl chloroformate (0.94 mL, 7.5 mmoL) was slowly added dropwise and the obtained solution reacted at a low temperature. Two hours later, the reaction ended, the reaction solution was dissolved and extracted with dichloromethane and saturated sodium chloride solution, and the organic phase was then evaporated to dryness. 5 g of the product was obtained with a yield of 83%.

37-8

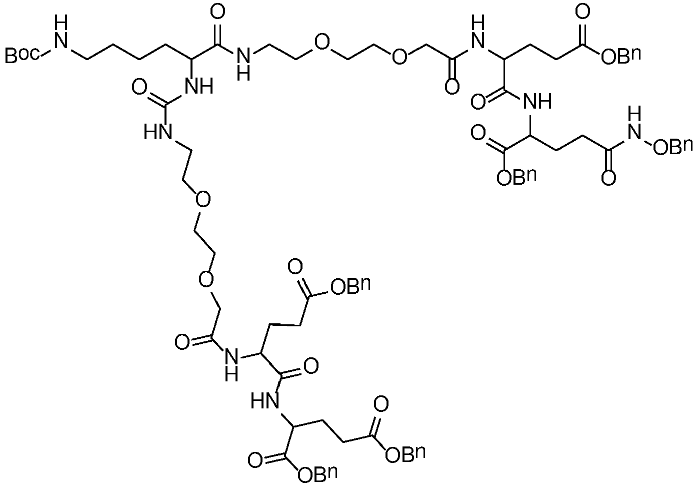

Compound 37-3 (2.7 g , 2.9 mmoL)and Compound 28-262 (2.4 g, 2.9 mmoL) was added into a 250 mL flask and then dissolved with DMF (35 mL); the obtained solution was stirred overnight at 80 °C to react. At the end of the reaction, the reaction solution was transferred to a 1 L separatory funnel and extracted with ethyl acetate (200 mL) and saturated sodium bicarbonate solution (200 mL) to obtain an organic phase; the aqueous phase was washed with ethyl acetate (150 mL×3); silica gel powder (20 g) was then added to the obtained solution, and the solution was then evaporated to dryness to obtain a powdery solid; the operations of dry sample loading, column chromatography, and elution with a mixed solution (0.5% ammonia water : 1% methanol/dichloromethane) were carried out. 1.3 g of Product 37-8 was obtained with a yield of 28%.

43-1

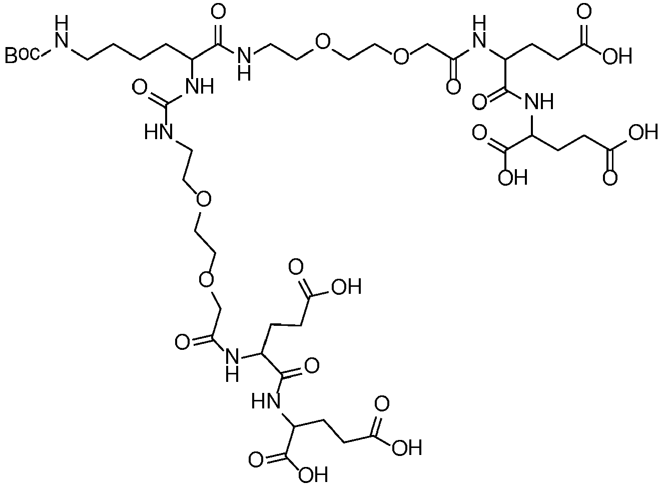

Compound 37-8 (1.3 g, 0.79 mmoL) and Pd/C (0.0300 g) were sequentially added into a hydrogenation reactor and then dissolved with DMF (30 mL), hydrogen gas (Pa=1.8 MPa) was then introduced into the reactor, and the obtained solution was stirred overnight at room temperature to react. At the end of the reaction, the reactor was taken out, the reaction solution was filtered with diatomaceous earth, and washing with DMF (20 mL×3) was then carried out to obtain the product as a raw material of the next reaction step.

43-2

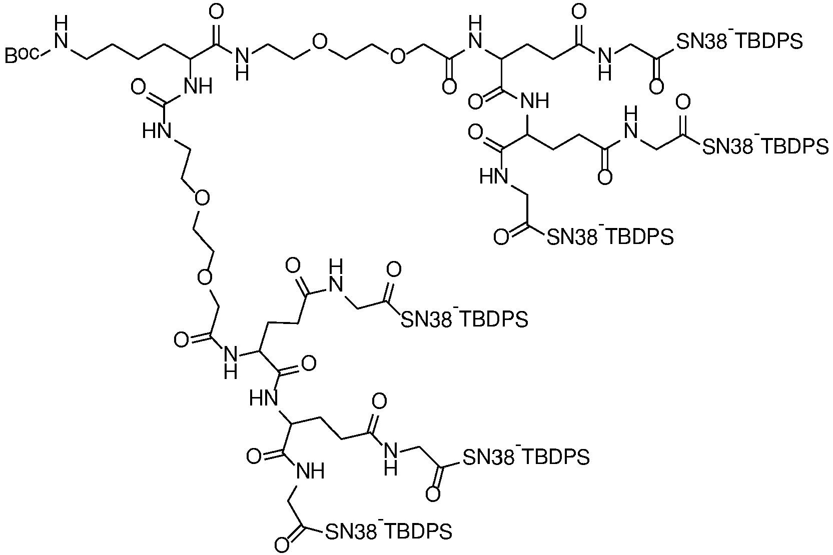

G-SN38-TBDPS (3.314 g, 4.819 mmoL), Compound 43-1 (0.86 g, 0.79 mmoL), HBTU (2.7 g, 7.11 mmoL), and HOBT (0.96 g, 7.11 mmoL) were added into a 250 mL flask and the mixed solution was stirred at 0 °C for about 30 min to react; DIEA (3.5 mL, 21.33 mmoL) was then slowly added dropwise, and the obtained solution further reacted at 0 °C for 3 h. At the end of the reaction, the reaction solution was transferred to a 1 L separatory funnel and then extracted with a saturated sodium chloride solution (200 mL) and ethyl acetate (250 mL) to obtain an organic phase; the aqueous phase was washed with ethyl acetate (200 mL × 1), and the obtained organic phases were combined, washed with saturated sodium chloride solution (200 mL×1), then concentrated and evaporated to dryness; the solid product was then dissolved with methanol (20 mL) and dichloromethane (80 mL), silica gel powder (15 g) was added to the obtained solution, and the solution was then evaporated to dryness to obtain a powdery solid; the operations of dry sample loading, column chromatography, and gradient elution with a mixed solution (4%-5% methanol/dichloromethane) were carried out. 2.1 g of Product 43-2 was obtained with a yield of 53%.

43-4

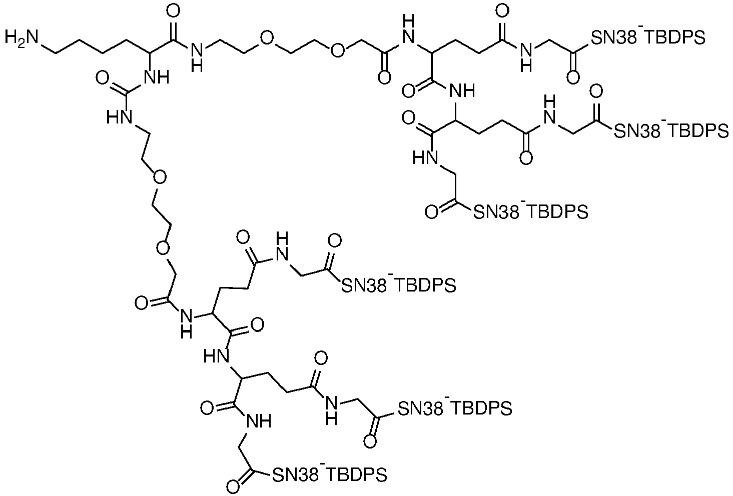

Compound 43-2 (2.1 g, 0.412 mmoL) was added in a 250 mL flask and then dissolved with dichloromethane (5 mL), TFA (9.3 mL, 125.5545 mmoL) was then added with stirring, and the mixed solution was stirred overnight at room temperature to react. At the end of the reaction, the reaction solution was first concentrated to a small amount, and then precipitated with methyl tert-butyl ether (150 mL) to obtain a powdery solid; filtering was then carried out and the filter cake was washed with methyl tert-butyl ether (20 mL × 3). The filter cake was then dissolved with a methanol (20 mL) and dichloromethane (80 mL), silica gel powder (10 g) was then added to the obtained solution, and the solution was then evaporated to dryness to obtain a powdery solid; the operations of dry sample loading, column chromatography, and elution with a mixed solution (6% methanol/dichloromethane) were carried out; the elution product was then collected, concentrated, evaporated to dryness, and dried in a vacuum oven. 1.3 g of Product 43-4 was obtained with a yield of 62%.

43-6

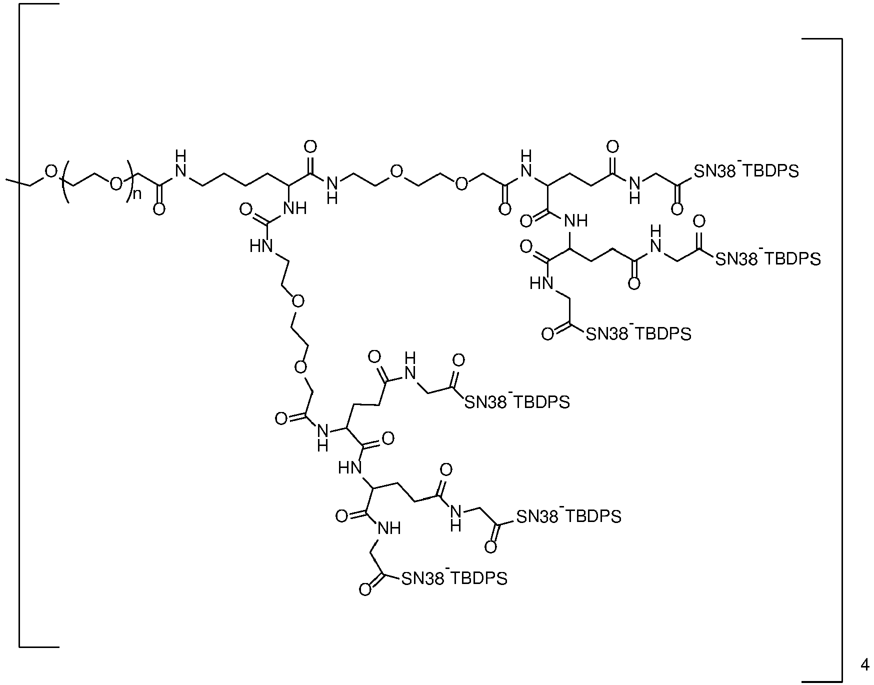

Compound 43-4 (1.3 g, 0.2592 mmoL) was added into a 250 mL flask and then dissolved with DMF (20 mL), and then 4ARM-CM-40K (2.5512 g, 0.0642 mmoL, purchased from JenKem), HBTU (0.2191 g, 0.5778 mmoL), and HOBT (0.0781 g, 0.5778 mmoL) were then added to the obtained solution; the solution was stirred at -5 °C for 20 min, and DIEA (0.493 mL, 2.9853 mmoL) was slowly added dropwise for titration; after reaction for 30 min, the reaction solution was taken out and then slowly stirred for a week in the dark at room temperature to react. At the end of the reaction, n-hexane (150 mL) and methyl tert-butyl ether (40 mL) were added to the solution, and the supernatant was discarded; such operations were repeated four times to obtain a viscous oily product and the viscous oily product was then dissolved with dichloromethane (10 mL); methyl tert-butyl ether (150 mL) was added to the reaction solution to obtain a solid product; suction filtering was then carried out, the filter cake was washed with methyl tert-butyl ether (40 mL×3) and then dissolved with methanol (20 mL) and dichloromethane (80 mL), silica gel powder (10 g) was then added to the obtained solution, and the solution was then evaporated to dryness to obtain a powdery solid; the operations of dry sample loading, column chromatography, and gradient elution with a mixed solution (6%-7% methanol/dichloromethane) were carried out; the elution product was then collected, concentrated, evaporated to dryness, and dried in a vacuum oven. 2.8 g of Product 43-6 was obtained with a yield of 75%.

43-8

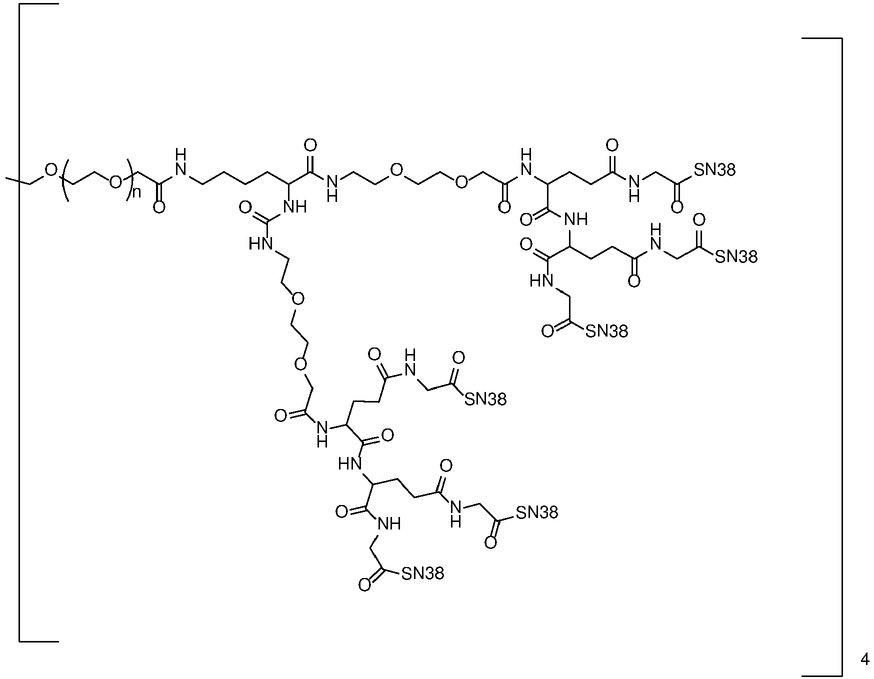

Compound 43-6 (2.8 g, 0.047 mmoL) was dissolved with tetrahydrofuran (25 mL), tetrabutylammonium fluoride trihydrate (TBAF·3H₂O, 0.0418 g, 0.1325 mmoL) and dilute hydrochloric acid solution (45 mL, 0.05 mol/L) were then added sequentially, and the mixed solution was then stirred overnight in the dark at room temperature to react. At the end of the reaction, the reaction solution was first evaporated to dryness, ethanol (20 mL) was then added, and the obtained solution was evaporated to dryness, and then ethanol (20 mL) was added; such operations were repeated three times to finally obtain a solid powder; then, the solid powder was dissolved with DMF (5 mL), and isopropanol (50 mL) was then added to the obtained solution to obtain a solid product by precipitation; then, filtering was carried out, the filter cake was dissolved with DMF (3.0 mL) again, and isopropanol (50 mL) was then added to the obtain solution to obtain a solid product by precipitation; then, filtering was carried out and the filter cake was washed three times with isopropanol ( 10 mL × 3) to obtain a solid product; the solid product was then dissolved with dichloromethane (6 mL), and methyl tert-butyl ether (50 mL) was then added to the obtained solution to obtain a solid product by precipitation; then, filtering was carried out, the filter cake was dissolved with dichloromethane (6 mL) again, and methyl tert-butyl ether (50 mL) was then added to obtain a solid product by precipitation; then, filtering was carried out, the filter cake was washed with methyl tert-butyl ether (10 mL×3) and then dried. 2.26 g of Product 43-8 was obtained with a yield of 90%.

¹H-NMR (400 MHz, DMSO-d6) δ 11.02 (s, 1H), 10.34 (s, 18H), 8.74 - 8.15 (m, 33H), 8.13 - 7.86 (m, 22H), 7.71 - 7.44 (m, 11H), 7.22 (m, 46H), 7.09 - 6.77 (m, 19H), 6.42 (m, 3H), 5.56 - 5.04 (m, 87H), 4.25 - 3.80 (m, 96H), 3.64 - 3.32 (m, 3570H), 3.20 - 3.01 (m, 93H), 2.68 (m, 25H), 2.26 - 2.03 (m, 70H), 1.72 (m, 32H), 1.28 (d, J = 6.8 Hz, 64H), 1.15 - 1.04 (m, 14H), 0.95-0.73 (m, 68H).

### Example 10: Synthesis of Compound 22-278

22-278

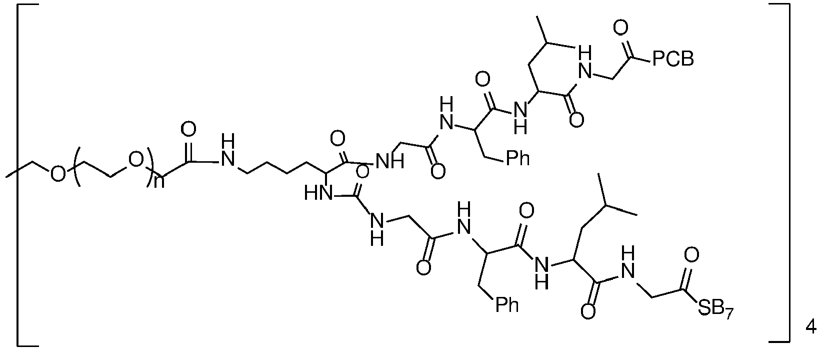

Compound 22-277 (0.5 g, 0.2677 mmoL) was added to a 500 mL flask and then dissolved with 10 mL of DMF, the obtained solution was stirred at -5 °C to react for 30 min, and DIEA (0.09 mL, 0.5354 mmoL) was then slowly added dropwise; then, 4ARM-SCM-40K (2.25 g) was added, and the obtained solution was slowly stirred at room temperature for a week to react. At the end of the reaction, n-hexane (50 mL × 3) was first added; and when there was little lower oily product, methyl tert-butyl ether (20 mL) was added for precipitation, and a solid product was precipitated; the filter cake was then dissolved with a methanol/dichloromethane (1:5) solution, 30 mL of silica gel powder was then added to the obtained solution, and the solution was then evaporated to dryness; the operations of dry sample loading, column chromatography, and gradient elution with an eluent (0.5%-1% ammonia water: 4%-6% methanol: 95.5%-93% dichloromethane) were carried out; the filtrate was then collected, concentrated and evaporated to dryness. 1.745 g of Product 22-278 was obtained with a yield of 67%.

¹H-NMR (400 MHz, DMSO-d6) δ 10.16 (s, 4H), 8.96 (s, 4H), 7.99 (m, 44H), 7.65 (s, 5H), 7.50 (s, 10H), 7.20 (s,71H), 6.49 (s, 2H), 6.24 (s, 2H), 5.79 (m, 6H), 4.53 (s, 8H), 3.84 (s, 19H), 3.69 (s, 52H), 3.51 (s, 3719H), 3.06 (s, 49H), 2.89 (s, 4H), 2.76 (m, 31H), 2.42 (s, 19H), 2.31 (s, 37H), 1.82 (m, 26H), 1.28 (m, 124H), 0.84 (m, 60H), 0.50 (s, 9H).

### Example 11: Synthesis of compound 22-284

22-279

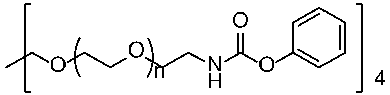

4ARM-NH₂·HCl-40K (2.97 g, 0.07585 mmoL) was added into a 250 mL flask and then dissolved with 20 mL of dichloromethane, triethylamine (0.26 ml, 1.8177 mmoL) was then added, the reaction solution was then stirred at 0 °C for 20 to 30 min, and phenyl chloroformate (0.05 ml, 0.3793 mmoL) was then slowly added dropwise to the reaction solution. After the phenyl chloroformate was added completely, the solution reacted at 0 °C for 2 to 3 h. At the end of the reaction, when the reaction solution was evaporated with a rotary evaporator to one-half of the original volume, 200 mL of methyl tert-butyl ether was added, and a solid product was precipitated; the solid product was washed twice with 100 mL of methyl tert-butyl ether and then dried. 2.9 g of Product 22-279 was obtained with a yield of 96.7%.

22-284

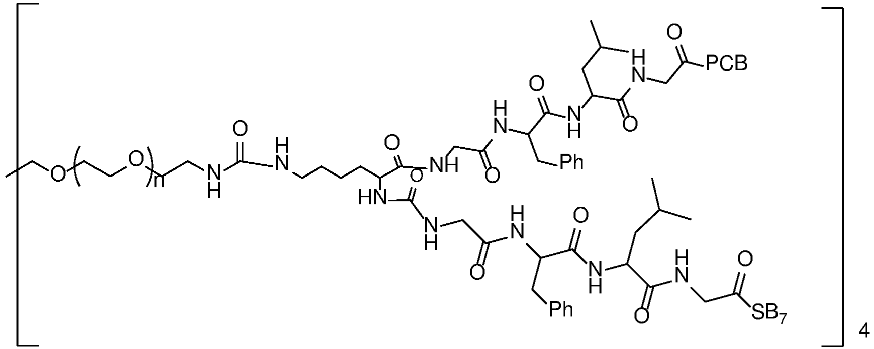

Compound 22-279 (1.925 g, 0.04868 mmoL) and Compound 22-277 (0.4 g, 0.2142 mmoL) were added to a 500 mL flask and then dissolved with 10 mL of DMF. The obtained solution was slowly stirred in the dark at 70 °C for one week. At the end of the reaction, n-hexane (50 mL × 3) was first added; and when there was little lower oily product, methyl tert-butyl ether (20 mL) was added for precipitation, and a solid product was precipitated; the filter cake was then dissolved with a methanol/dichloromethane (1:5) solution, 30 mL of silica gel powder was then added to the obtained solution, and the solution was then evaporated to dryness; the operations of dry sample loading, column chromatography, and gradient elution with an eluent (1% ammonia water: 5%-8% methanol: 94%-91% dichloromethane) were carried out; the filtrate was then collected, concentrated and evaporated to dryness. 1 g of Product 22-284 was obtained with a yield of 44%.

¹H-NMR (400 MHz, DMSO-d6) δ 8.96 (s, 3H), 8.09 (s, 10H), 7.87 (s, 2H), 7.52 (s, 56H), 7.26 (m, 77H), 6.26 (s, 4H), 5.17 (s, 2H), 4.47 (s, 45H), 3.68 (s, 130H), 3.51 (s, 3167H), 3.15 (m, 174H), 2.87 (s, 15H), 2.67 (s, 15H), 2.31 (m, 18H), 1.31 (m, 100H), 1.17 (m, 117H), 0.99 (s, 9H), 0.85 (s, 34H), 0.50 (s, 3H).

### Example 12: Synthesis of Compound 44-10

25-229

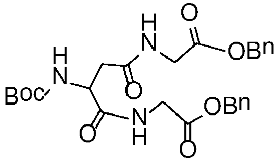

Boc-Asp-OH (6.0 g, 25.7268 mmoL), HBTU (29.2699 g, 77.1803 mmoL), HOBT (10.4286 g, 77.1803 mmoL) and H-Gly-OBn·TosOH (17.3599 g, 51.4536 mmoL) were added in a 500 mL round-bottomed flask and dissolved with DMF (100 mL), and the obtained solution was stirred for about 30 min at -5 °C; then, DIEA (38.3 mL, 231.5410 mmoL) was slowly added dropwise; the obtained solution was further stirred at -5 °C for 1 h, and then the reaction solution was stirred for 2 h at room temperature to further react. At the end of the reaction, the reaction solution was transferred to a 2 L separatory funnel, saturated NaHCO₃ solution (400 mL) and ethyl acetate (300 mL) were then added, and the obtained solution was shaken and extracted. Saturated sodium chloride solution (300 mL) was further added to the organic phase, and the obtained solution was then shaken and extracted. The organic phase was then evaporated to dryness and then dried in a vacuum oven. 13.5727 g of Product 25-229 was obtained with a yield of 100%.

25-230

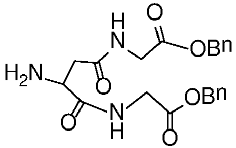

Compound 25-229 (13.5727 g, 25.7268 mmoL) was added in a 100 mL round-bottomed flask and then dissolved with dichloromethane (10 mL), TFA (19.1 mL, 257.268 mmoL) was then added with stirring, and the mixed solution was stirred overnight at room temperature to react. At the end of the reaction, the reaction solution was first concentrated and evaporated to remove dichloromethane. The reaction solution was then transferred to a 2 L separatory funnel, saturated NaHCO₃ solution (400 mL) and ethyl acetate (300 mL) were then added, and the obtained solution was shaken and extracted. Saturated sodium chloride solution (300 mL) was further added to the organic phase, and the obtained solution was then shaken and extracted. The organic phase was then evaporated to dryness and then dried in a vacuum oven. 9.3 g of Product 25-230 was obtained with a yield of 84.57%.

25-231

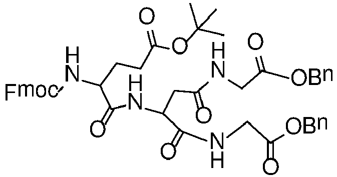

Compound 25-230 (9.3 g, 21.7569 mmoL), HBTU (11.2515 g, 29.6685 mmoL), HOBT (4.0088 g, 29.6685 mmoL) and Fmoc-Glu(OtBu)-OH (8.4154 g, 19.7790 mmoL) were added in a 500 mL round-bottomed flask and dissolved with DMF (50 mL), and the obtained solution was stirred for about 30 min at -5 °C; then, DIEA (19.6 mL, 118.6740 mmoL) was slowly added dropwise; and then the reaction solution was stirred for 3 h at -5 °C to further react. At the end of the reaction, the reaction solution was transferred to a 2 L separatory funnel, saturated sodium chloride solution (400 mL) and ethyl acetate (300 mL) were then added, and the obtained solution was shaken and extracted. Saturated sodium chloride solution (300 mL) was further added to the organic phase, and the obtained solution was then shaken and extracted. The organic phase was then evaporated to dryness and then dried in a vacuum oven. 16.5137 g of Product 25-231 was obtained with a yield of 100%.

25-232

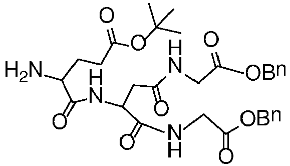

Compound 25-231 (16.5137 g, 19.779 mmoL) was added in a 500 mL round-bottomed flask and then dissolved with dichloromethane (20 mL), morpholine (25.8 mL, 296.6850 mmoL) was then added with stirring, and the mixed solution was stirred for 2 h at room temperature to react. At the end of the reaction, the reaction solution was first transferred to a 2 L separatory funnel, saturated sodium chloride solution (300 mL) and ethyl acetate (200 mL) were then added, and the obtained solution was shaken and extracted. Saturated sodium chloride solution (300 mL) was further added to the organic phase, and the obtained solution was then shaken and extracted. Then, deionized water (300 mL) was added to the organic phase and the obtained solution was then shaken and extracted. Finally, the organic phase was concentrated and evaporated to dryness and then dried in a vacuum oven. 12.1303 g of Product 25-232 was obtained with a yield of 100%.

25-246

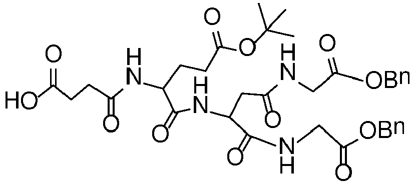

Compound 25-232 (12.1303 g, 19.779 mmoL) was dissolved with DMF (50 mL), and the obtained solution was stirred for about 10 min at -5 °C to react; then, DIEA (19.6 mL, 118.6740 mmoL) was slowly added dropwise; and then the reaction solution was stirred for 30 min at -5 °C to further react; the reaction solution was then taken out and succinic anhydride (5.9379 g, 59.337 mmoL) was then added to the solution to react at room temperature for 3 h. At the end of the reaction, the reaction solution was transferred to a 2 L separatory funnel, saturated sodium chloride solution (400 mL) and ethyl acetate (300 mL) were then added, and the obtained solution was shaken and extracted. Saturated sodium chloride solution (300 mL) was further added to the organic phase, and the obtained solution was then shaken and extracted. The organic phase was then evaporated to dryness and then dried in a vacuum oven. 14.0973 g of Product 25-246 was obtained with a yield of 100%.

25-249

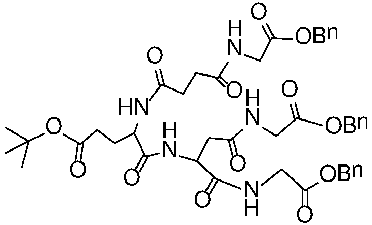

Compound 25-246 (14.0973 g, 19.7790 mmoL), HBTU (11.2515 g, 29.6685 mmoL), HOBT (4.0088 g, 29.6685 mmoL) and H-Gly-OBn·TosOH (6.9402 g, 20.5702 mmoL) were added in a 500 mL round-bottomed flask and dissolved with DMF (100 mL), and the obtained solution was stirred for about 30 min at -5 °C; then, DIEA (19.6 mL, 118.674 mmoL) was slowly added dropwise; the obtained solution was further stirred at -5 °C for 1 h, and then the reaction solution was stirred overnight at room temperature to further react. At the end of the reaction, the reaction solution was transferred to a 2 L separatory funnel, saturated NaHCO₃ solution (400 mL) and ethyl acetate (300 mL) were then added, and the obtained solution was shaken and extracted. Saturated sodium chloride solution (300 mL) was further added to the organic phase, and the obtained solution was then shaken and extracted. The organic phase was evaporated to dryness. The solid product was then dissolved with a mixed solvent (100 mL) (20% methanol/dichloromethane), 80 mL of silica gel powder was then added, and the obtained solution was then evaporated to dryness. The operations of dry sample loading and column chromatography were carried out. Elution was then carried out with eluent (1%-3% methanol: 99%-97% ethyl acetate), and the elution solution was collected, concentrated and evaporated to dryness. 9.8 g of Product 25-249 was obtained with a yield of 73.52%.

25-258

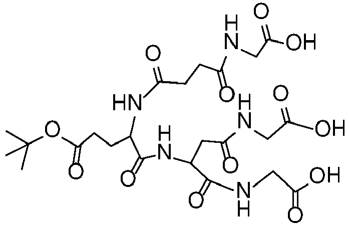

Compound 25-249 (4.0482 g, 5.0061 mmoL) and 0.1 mg of Pd/C were sequentially added into a hydrogenation reactor and then dissolved with DMF (40 mL), and finally hydrogen gas (Pa=1.6 MPa) was introduced into the reactor; the obtained solution was stirred overnight at room temperature to react. At the end of the reaction, the reactor was taken out, the reaction solution was filtered with diatomaceous earth, and the reactor was then washed with DMF (20 mL × 3). Then, n-hexane (150 mL) and methyl tert-butyl ether (30 mL) were added to precipitate the filtrate three times, and the lower oily product was evaporated to dryness and dried in a vacuum oven. 2.9513 g of Product 25-258 was obtained with a yield of 100%.

25-259

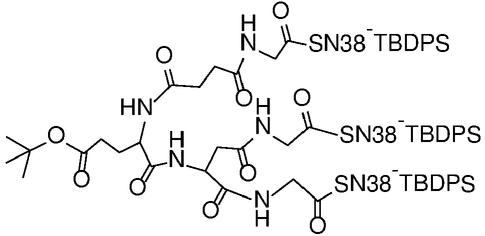

Compound 25-258 (0.9921 g, 1.6828 mmoL), G-SN38-TBDPS (3.5 g, 5.5534 mmoL) and 4-dimethylaminopyridine (DMAP, 0.1234 g, 1.0097 mmoL) were added into a 500 mL round-bottomed flask and then dissolved with dichloromethane (100 mL); the reaction solution was stirred at 0 °C for about 30 min, dicyclohexylcarbodiimide (DCC, 2.0833 g, 10.0968 mmoL) was then added to the reaction solution, and the mixed solution was stirred overnight at 0 °C to react. At the end of the reaction, the reaction solution was first filtered to remove DCC, and then the filter cake was washed three times with dichloromethane (20 mL). Silica gel powder (50 mL) was added, and the obtained solution was then evaporated to dryness; operations of dry sample loading and column chromatography were carried out. Elution was carried out with eluent (3%-7% methanol: 93%-97% dichloromethane). 1.5191 g of Product 25-259 was obtained with a yield of 37.18%.

### MALDI-TOF MS: [M+Na⁺] 2449.34.

25-280

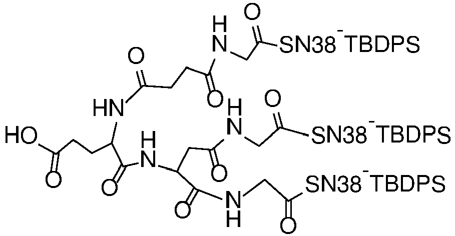

Compound 25-259 (1.51 g, 0.6219 mmoL) was added in a 100 mL round-bottomed flask and then dissolved with dichloromethane (5 mL), TFA (0.7 mL, 9.3290 mmoL) was then added with stirring, and the mixed solution was stirred overnight at room temperature to react. At the end of the reaction, the reaction solution was first concentrated and evaporated to remove dichloromethane. The reaction solution was first transferred to a 1 L separatory funnel, saturated sodium chloride solution (300 mL) and ethyl acetate (200 mL) were then added, and the obtained solution was shaken and extracted. Saturated sodium chloride solution (300 mL) was further added to the organic phase, and the obtained solution was then shaken and extracted. Then, deionized water (300 mL) was added to the organic phase and the obtained solution was then shaken and extracted. Finally, the organic phase was concentrated and evaporated to dryness. The solid product was then dissolved with a mixed solvent (30 mL) (20% methanol/dichloromethane), 30 mL of silica gel powder was then added, and the obtained solution was then evaporated to dryness. The operations of dry sample loading and column chromatography were carried out. Elution was then carried out with eluent (1%-8% methanol: 99%-92% dichloromethane), and the elution solution was collected, concentrated and evaporated to dryness. 0.7586 g of Product 25-280 was obtained with a yield of 51.43%.

### MALDI-TOF MS: [M+H⁺] 2372.83.

25-283

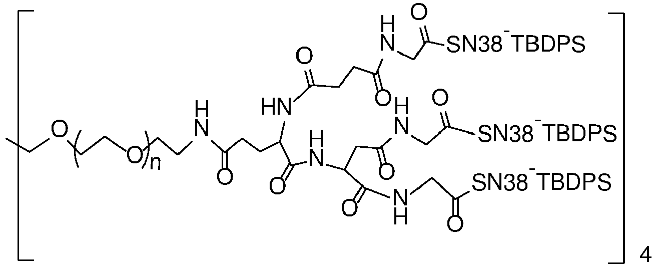

Compound 25-280 (0.7497 g, 0.3161 mmoL), HBTU (0.2451 g, 0.6462 mmoL), HOBT (0.0873 g, 0.6462 mmoL) and 4ARM-NH₂·HCl-40K (2.8153 g, 0.0718 mmoL) were added in a 100 mL round-bottomed flask and dissolved with DMF (20 mL), and the obtained solution was stirred for about 30 min at -5 °C ; then, DIEA (0.5 mL, 2.9438 mmoL) was slowly added dropwise; the obtained solution was further stirred at -5 °C for 10 min to react, and then the reaction solution was moved to a room temperature environment to react for one week. At the end of the reaction, the reaction solution was precipitated three times with n-hexane (60 mL) and methyl tert-butyl ether (10 mL) and then the supernatant was discarded. Then, methyl tert-butyl ether (60 mL) was added for precipitation, a powdery solid was obtained, and the filtering was carried out. The filter cake was washed with methyl tert-butyl ether (20 mL × 3). The filter cake was then dissolved with a mixed solvent (30 mL) (20% methanol/dichloromethane), 30 mL of silica gel powder was then added, and the obtained solution was then evaporated to dryness. The operations of dry sample loading and column chromatography were carried out. Elution was then carried out with eluent (1%-12% methanol: 99%-88% dichloromethane), and the elution solution was collected, concentrate, evaporated to dryness, and dried in a vacuum oven. 1.9752 g of Product 25-283 was obtained with a yield of 56.74%.

44-10

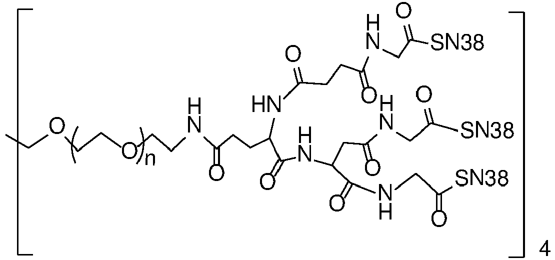

Compound 25-283 (1.9752 g, 0.0407 mmoL) was dissolved with THF (35 mL); tetrabutylammonium fluoride trihydrate (TBAF·3H₂O, 0.3082 g, 0.9768 mmoL) was obtained and dissolved in THF (10 mL); he THF solution containing TBAF was added to the THF solution containing Compound 25-283, and a certain amount of dilute hydrochloric acid solution (45 mL, 0.05 mol/L) was added to the mixed solution obtained, and the solution was then stirred overnight in the dark at room temperature to react. At the end of the reaction, the reaction solution was first evaporated to dryness, absolute ethanol (20 mL×3) was then added to remove water, and the obtained solution was evaporated to dryness again; the obtained solid product was then dissolved with DMF (1.0 mL), and isopropanol (50 mL) was then added to the obtained solution to obtain a powdery solid product by precipitation; then, filtering was carried out, the filter cake was dissolved with DMF (3.0 mL) again, and isopropanol (50 mL) was then added to the obtain solution to obtain a powdery solid product by precipitation; then, filtering was carried out and the filter cake was washed three times with isopropanol ( 10 mL×3) to obtain a solid product; the obtained solid product was then dissolved with dichloromethane (2.0 mL), and methyl tert-butyl ether (50 mL) was then added to the obtained solution to obtain a solid product by precipitation; then, filtering was carried out, the filter cake was dissolved with dichloromethane (2.0 mL) again, and methyl tert-butyl ether (50 mL) was then added to obtain a powdery solid product by precipitation; then, filtering was carried out, the filter cake was washed with methyl tert-butyl ether (10 mL × 3) and then dried. 1.125 g of Product 44-10 was obtained with a yield of 60.52%.

¹H-NMR (400 MHz, DMSO-d6) δ 10.28 (s, 42H), 8.62 - 7.63 (m, 69H), 7.40 (m, 31H), 7.15 - 6.89 (m, 19H), 5.96 - 4.99 (m, 81H), 4.53 (m, 24H), 4.35 - 3.28 (m, 3319H), 3.22 - 2.80 (m, 80H), 2.80 - 2.61 (m, 24H), 2.34 (s, 25H), 2.22 - 1.50 (m, 77H), 1.45 - 0.62 (m, 115H).

### Example 13: Synthesis of Compound 42-7

25-256

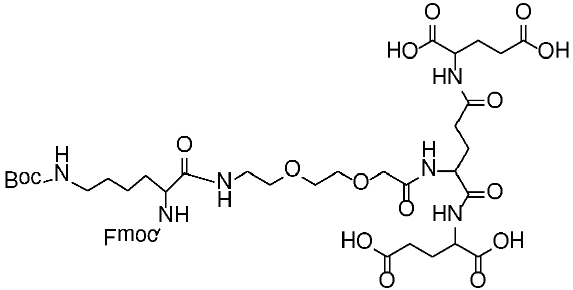

Compound 28-65 (1.8784 g, 1.3796 mmoL, synthesized according to the synthesis method of Compound 31-149) and 100 mg of Pd/C were sequentially added into a hydrogenation reactor and then dissolved with DMF (30 mL), hydrogen gas (Pa=1.6 MPa) was then introduced into the reactor, and the obtained solution was stirred overnight at room temperature to react. At the end of the reaction, the reactor was taken out, the reaction solution was filtered with diatomaceous earth, and then the reactor was washed with DMF (20 mL × 3). 1.3852 g of Product 25-256 was obtained with a yield of 100%.

25-257

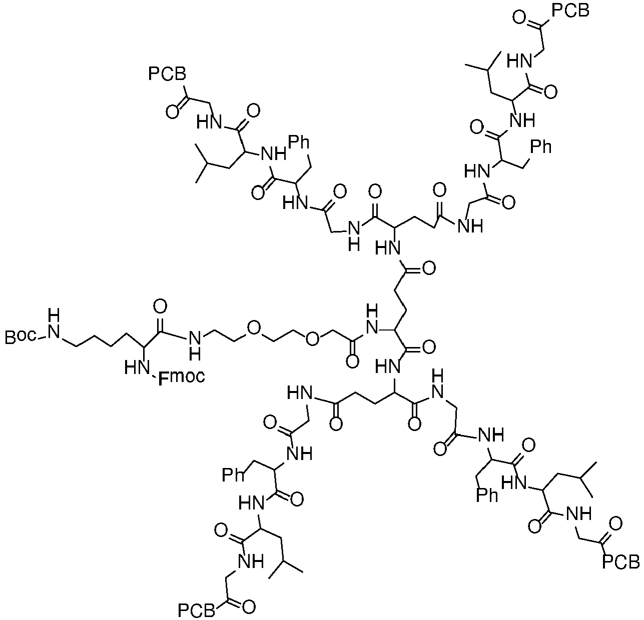

Compound 25-256 (1.3852 g, 1.3796 mmoL), HBTU (3.1390 g, 8.2776 mmoL), HOBT (1.1180 g, 8.2776 mmoL), and Compound 25-254 (5.8 g, 7.0562 mmoL, synthesized according to the synthesis method Compound 30-33) were added in a 500 mL round-bottomed flask and then dissolved with DMF (60 mL), and the obtained solution was stirred for about 30 min at -5 °C; then, DIEA (4.1 mL, 24.8328 mmoL) was slowly added dropwise; and then the reaction solution was stirred for 3 h at -5 °C to further react. At the end of the reaction, the reaction solution was precipitated five times with n-hexane (150 mL) and methyl tert-butyl ether (30 mL) and then the supernatant was discarded. Then, the lower oily product was dissolved with dichloromethane, methyl tert-butyl ether (100 mL) was then added for precipitation and a powdery solid was obtained: filtering was then carried out, and the filter cake was washed with methyl tert-butyl ether (20 mL×3) and then dried in a vacuum oven. 5.8 g of Product 25-257 was obtained with a yield of 100%.

25-267

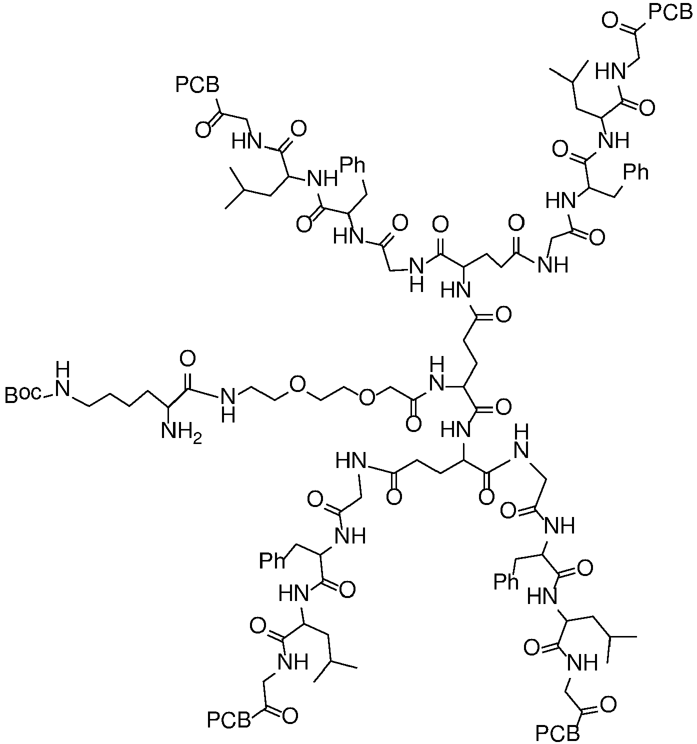

Compound 25-257 (5.8176 g, 1.3796 mmoL) was added in a 500 mL round-bottomed flask and then dissolved with dichloromethane (50 mL), morpholine (1.8 mL, 20.6940 mmoL) was then added with stirring, and the mixed solution was stirred for 3 h at room temperature to react. At the end of the reaction, the reaction solution was precipitated five times with n-hexane (150 mL) and methyl tert-butyl ether (30 mL) and then the supernatant was discarded. Then, the lower oily product was dissolved with dichloromethane, methyl tert-butyl ether (100 mL) was then added for precipitation and a powdery solid was obtained: filtering was then carried out, and the filter cake was washed with methyl tert-butyl ether (20 mL × 3) and then dried in a vacuum oven. 3.1 g of Product 25-267 was obtained with a yield of 56.26%.

25-270

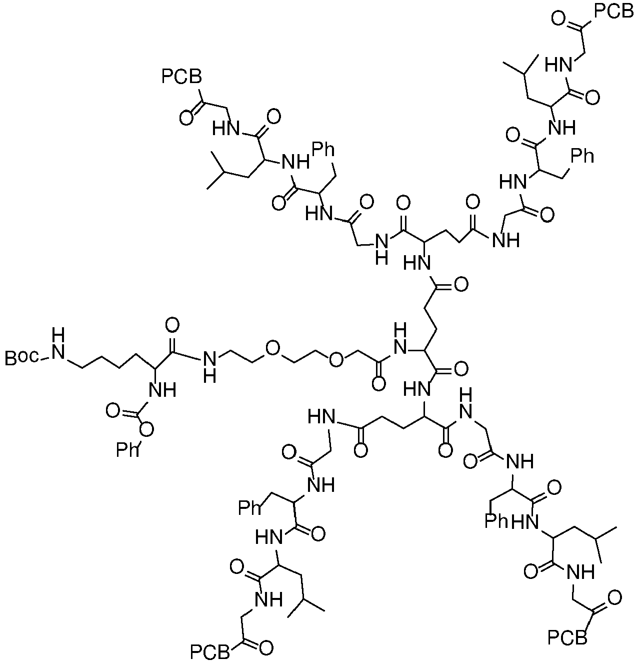

Compound 25-267 (3.1 g, 0.7761 mmoL) was dissolved with dichloromethane (50 mL), triethylamine (0.55 mL, 3.8800 mmoL) was added to the mixed solution and the obtained solution was stirred for about 20 min at -5 °C to react; then, phenyl chloroformate (0.3 mL, 2.3280 mmoL) was slowly added dropwise; and then the reaction solution was stirred for 3 h at -5 °C to further react At the end of the reaction, most of the dichloromethane was first evaporated out of the reaction solution; then, methyl tert-butyl ether (100 mL) was then added for precipitation and a powdery solid was obtained: filtering was then carried out, and the filter cake was washed three times with methyl tert-butyl ether (20 mL) and then dried in a vacuum oven. 3.1934 g of Product 25-270 was obtained with a yield of 100%.

25-277

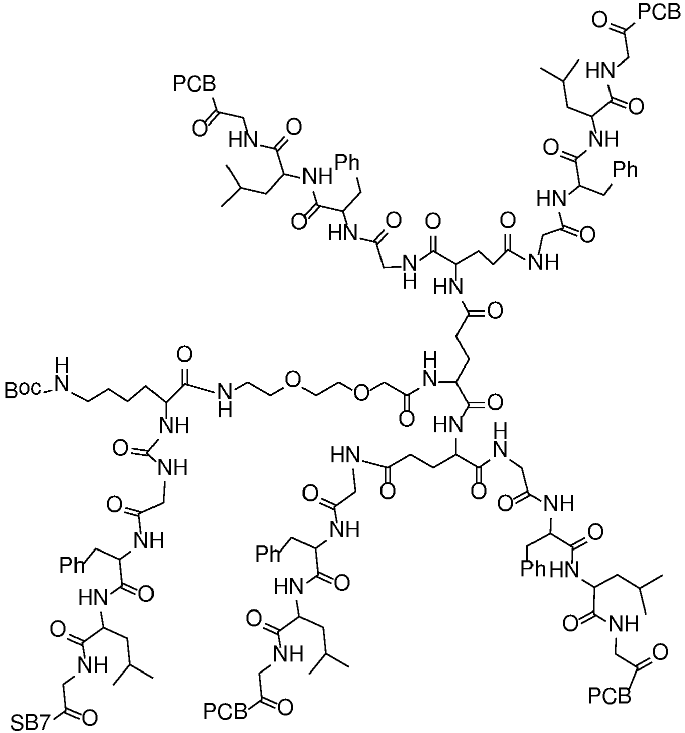

Compound 25-270 (3.1 g, 0.7761 mmoL) and Compound 25-132 (0.6919 g, 0.7761 mmoL, synthesized according to the synhesis method of Compound 3-16) were added into a 250 mL round-bottomed flask and then dissolved with DMF (30 mL), and the obtained solution was stirred overnight in an 80 °C oil bath to react. At the end of the reaction, the reaction solution was precipitated five times with n-hexane (150 mL) and methyl tert-butyl ether (30 mL) and then the supernatant was discarded. Then, the lower oily product was dissolved with dichloromethane, methyl tert-butyl ether (100 mL) was then added for precipitation and a powdery solid was obtained: filtering was then carried out, and the filter cake was washed with methyl tert-butyl ether (20 mL×3). The filter cake was then dissolved with a mixed solvent (60 mL) (20% methanol/dichloromethane), 40 mL of silica gel powder was then added, and the obtained solution was then evaporated to dryness. The operations of dry sample loading and column chromatography were carried out. Elution was then carried out with eluent (1% ammonia water: 1%-8% methanol: 98%-91% dichloromethane), and the elution solution was collected, concentrated and evaporated to dryness. 2.0245 g of Product 25-277 was obtained with a yield of 80.76%.

42-5

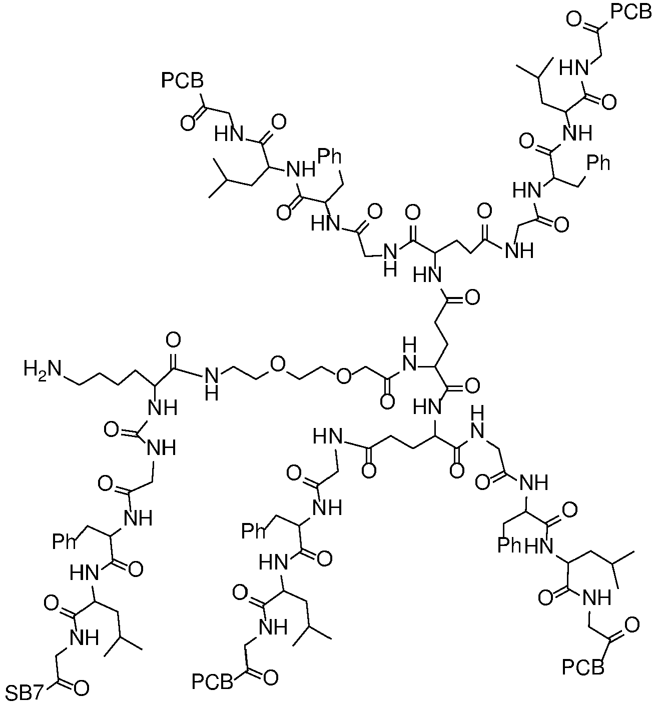

Compound 25-277 (2.0245 g, 0.4121 mmoL) was added in a 100 mL round-bottomed flask and then dissolved with dichloromethane (10 mL), TFA (0.6 mL, 6.1822 mmoL) was then added with stirring, and the mixed solution was stirred overnight at room temperature to react. At the end of the reaction, the reaction solution was first concentrated and evaporated to remove dichloromethane. Then, methyl tert-butyl ether (100 mL) was added for precipitation and a powdery solid was obtained: filtering was then carried out, and the filter cake was washed with methyl tert-butyl ether (20 mL×3). The filter cake was then dissolved with a mixed solvent (60 mL) (20% methanol/dichloromethane), 40 mL of silica gel powder was then added, and the obtained solution was then evaporated to dryness. The operations of dry sample loading and column chromatography were carried out. Elution was then carried out with eluent (1% ammonia water: 1%-8% methanol: 98%-91% dichloromethane), and the elution solution was collected, concentrated and evaporated to dryness. 1.3568 g of Product 42-5 was obtained with a yield of 68.41%.

42-7

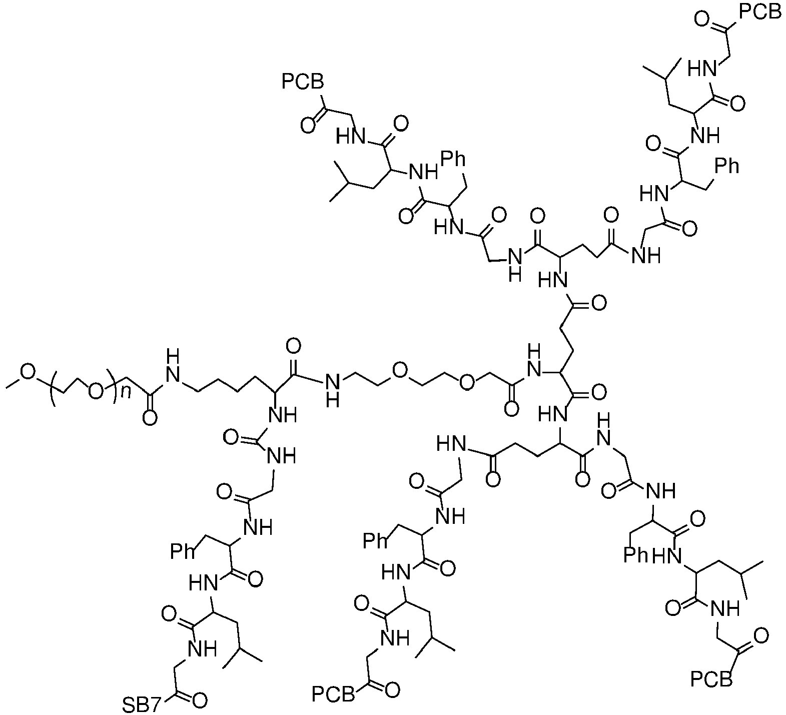

Compound 42-5 (0.9995 g, 0.2077 mmoL) and M-SCM-20K (2.0 g, 0.1888 mmoL) were added into a 500 mL round-bottomed flask and then dissolved with DMF (20 mL), and the obtained solution reacted for one week at room temperature in the dark. At the end of the reaction, the reaction solution was precipitated three times with n-hexane (100 mL) and methyl tert-butyl ether (10 mL) and then the supernatant was discarded. Then, dichloromethane (5.0 mL) was added, methyl tert-butyl ether (60 mL) was added for precipitation, a powdery solid was obtained, and the filtering was carried out. The filter cake was washed three times with methyl tert-butyl ether (20 mL). The filter cake was then dissolved with a mixed solvent (50 mL) (20% methanol/dichloromethane), 40 mL of silica gel powder was then added, and the obtained solution was then evaporated to dryness. The operations of dry sample loading and column chromatography were carried out. Elution was then carried out with eluent (1% ammonia water: 4%-10% methanol: 95%-89% dichloromethane), and the elution solution was collected, concentrated and evaporated to dryness. 1.3454 g of Product 42-7 was obtained with a yield of 46.66%.

### Example 14: Synthesis of Compound 24-266

33-124

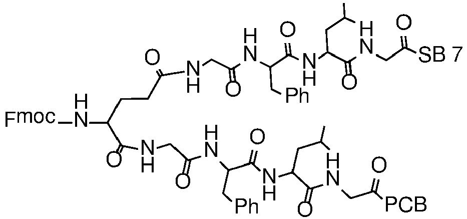

Compound 19-107 (1 g, 0.8 mmoL), Compound 30-33 (0.7 g, 0.88 mmoL), HBTU (0.45 g, 1.2 mmoL) and HOBT (0.16 g, 1.2 mmoL) were added in a 250 mL round-bottomed flask and then dissolved with DMF (20 mL), and the mixed solution was stirred at -5 °C for 30 min. Then DIEA (0.59 mL, 3.6 mmoL) was slowly added dropwise to continue the reaction, and 2 h later, the reaction solution was stirred at room temperature overnight. At the end of the reaction, the reaction solution was taken out, deionized water (200 mL) was added to the reaction solution, the obtained solution was then extracted three times with ethyl acetate (100 mL×3), and the organic phases were combined and then washed twice with saturated sodium chloride solution (200 mL); the obtained solution was then concentrated to dryness; the operations of dry sample loading, column chromatography, and gradient elution with eluent (1% ammonia water: 3% methanol/dichloromethane-1% ammonia water: 5% methanol/dichloromethane) were carried out; the product was concentrated and evaporated to dryness to obtain the final product. The actual output of the final product was 0.8 g, with a yield of 50%.

33-127

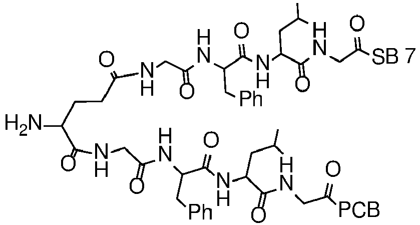

Compound 33-124 (0.8 g, 0.39 mmoL) was added in a 250 mL round-bottomed flask and then dissolved with DMF (20 mL), and then morpholine (1.01 mL, 11.7 mmoL) was added and the obtained solution was stirred for 1 h at room temperature to react. At the end of the reaction, methyl tert-butyl ether (100 mL) and n-hexane (200 mL) were added to precipitate the reaction solution and then a powder product was obtained. The actual output of the product was 0.5 g, with a yield of 71.4%.

33-130

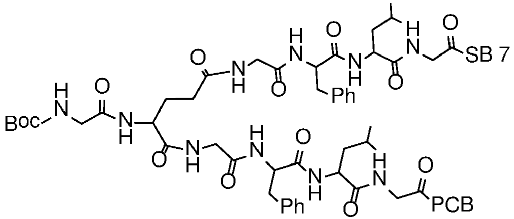

Compound 33-127 (0.5 g, 0.27 mmoL), Boc-Gly-OH (0.049 g, 0.28 mmoL), HBTU (0.15 g, 0.4 mmoL) and HOBT (0.054 g, 0.4 mmoL) were added in a 250 mL round-bottomed flask and then dissolved with DMF (20 mL), and the mixed solution was stirred at -5 °C for 30 min to react. Then DIEA (0.19 mL, 1.21 mmoL) was slowly added dropwise to continue the reaction, and 2 h later, the reaction solution was stirred at room temperature overnight to react further. At the end of the reaction, methyl tert-butyl ether (100 mL) and n-hexane (200 mL) were added to precipitate the reaction solution and then a powder product was obtained. The actual output of the product was 0.6g, with a yield of 100%.

33-131

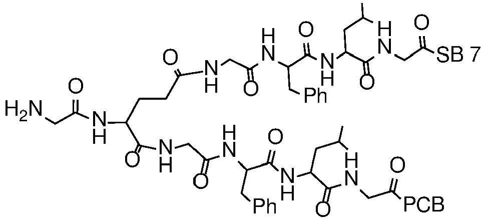

Compound 33-130 (0.5 g, 0.27 mmoL) was added in a 250 mL round-bottomed flask and then dissolved with dichloromethane (20 mL), and then TFA (0.6 mL, 8.1 mmoL) was added and the obtained solution was stirred at room temperature to react overnight. At the end of the reaction, the reaction solution was concentrated at reduced pressure and evaporated to dryness; methyl tert-butyl ether (100 mL) and n-hexane (200 mL) were then added for precipitation and then a powder product was obtained. The actual output of the product was 0.5 g, with a yield of 100%.

24-262

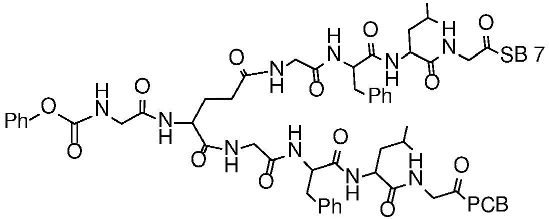

Compound 33-131 (0.5 g, 0.2657 mmoL) was added in a 250 mL round-bottomed flask and then dissolved with dichloromethane (15 mL) by ultrasonic; TEA (0.1494 mL, 1.0628 mmoL) was then added to the obtained solution and the obtained solution was then stirred for 30 min at 0 °C to react. Then, phenyl chloroformate (0.0668 mL, 0.5314 mmoL) was slowly added dropwise and the obtained solution reacted at a low temperature. Two hours later, the reaction ended, the reaction solution was evaporated to dryness, and the dichloromethane was removed; methyl tert-butyl ether (20 mL) was added and the obtained solution was treated by ultrasonic for 2 min; n-hexane (100 mL) was then added, and the obtained solution was filtered by suction to obtain 0.5 g of the product with a yield of 100%.

¹H-NMR (400 MHz, DMSO-*d*₆) δ 10.16 (s, 1H), 9.60 (s,2H),8.94(s,1H),8.22-8.03 (m, 6H), 7.89 (s, 2H), 7.58 - 7.46 (m, 2H), 7.40 - 7.30 (m, 2H),7.21-7.04 (m, 14H), 5.74 (s, 3H), 4.55-4.41 (m, 1H), 4.23-4.20 (m, 1H), 4.08 - 3.83 (m, 1H), 3.72-3.58 (m, 6H), 3.04 (s, 18H), 2.78-2.70(m, 2H), 2.42 (s, 3H), 2.31 (s, 7H), 2.14 (d, *J* = 14.5 Hz, 3H), 1.82 -1.78(m, 5H), 1.52-1.49 (m, 5H), 1.20 -1.18(m,27H), 0.94-0.77 (m, 12H), 0.50 (s, 3H).

24-205

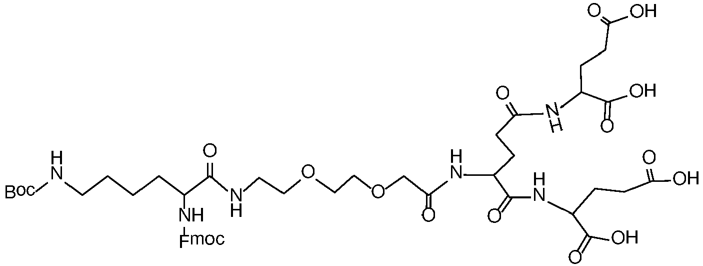

The reactant Compound 15-91 (synthesized according to the synthesis method of Compound 31-149, 0.33 g, 0.1503 mmoL) and 10% Pd/C (30 mg) were added in a micro-reactor and then dissolved with DMF (30 mL); H₂ (20 psi) was introduced in the reactor, and the mixed solution was stirred to react. At the end of the reaction, the reaction solution was filtered by suction with diatomaceous earth as a filter cake to remove Pd/C; the diatomaceous earth was washed four times with DMF to obtain the DMF solution of Compound 24-205 for the next reaction step.

41-7

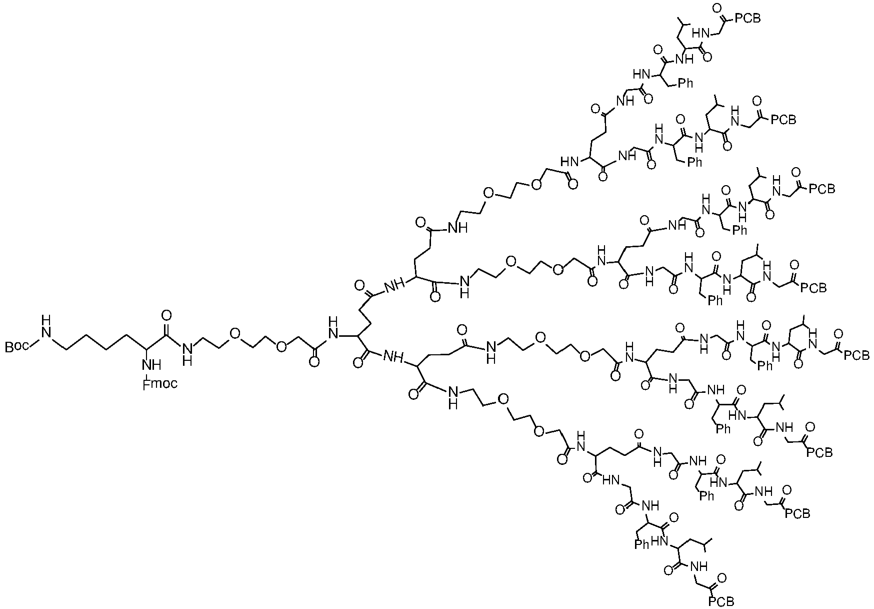

Compound 41-6 (0.84 g, 0.44 mmoL), Compound 24-205 (0.091 mmoL), HBTU (0.07 g, 0.546 mmoL) and HOBT (0.2 g, 0.546 mmoL) were added in a 250 mL round-bottomed flask and then dissolved with DMF (20 mL), and the mixed solution was stirred at -5 °C for 30 min. Then DIEA (0.27 mL, 1.638 mmoL) was slowly added dropwise to continue the reaction, and 2 h later, the reaction solution was stirred at room temperature overnight.

41-9

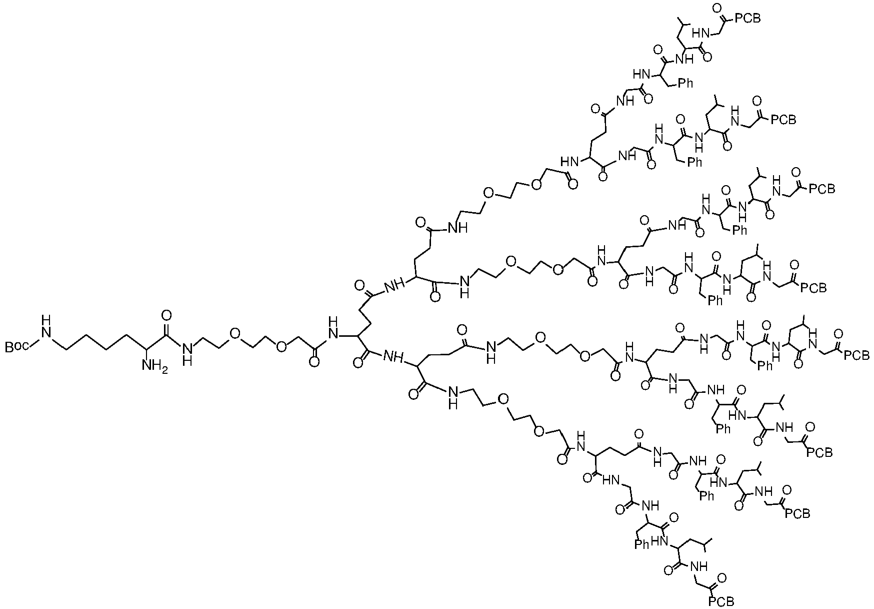

Reactant Compound 41-7 (0.7 g, 0.082 mmoL) was dissolved with DMF (30 mL), morpholine (0.214 mL, 2.46 mmoL) was added, and the obtained solution was stirred to react. At the end of the reaction, methyl tert-butyl ether (100 mL) and n-hexane (200 mL) were added to precipitate the reaction solution, and a powder product was obtained. The operations of column chromatography, dry sample loading and gradient elution with 1% ammonia water: 5% methanol/dichloromethane-1% ammonia water: 12% methanol/dichloromethane were carried out. 0.5 g of the product was obtained.

24-261

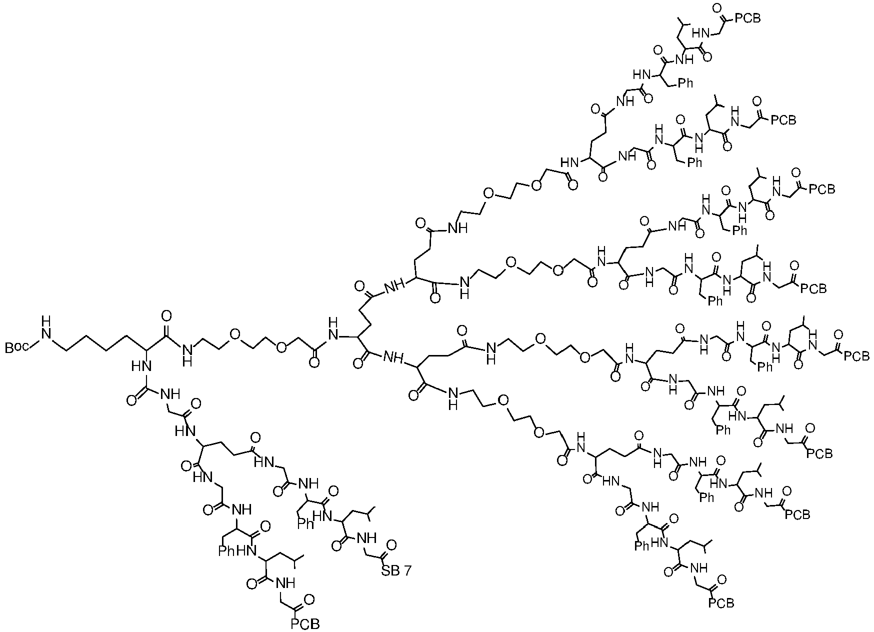

Compound 41-9 (0.5 g, 0.0602 mmoL) and Compound 24-262 (0.1446 g, 0.0722 mmoL) were added into a 250 mL reaction flask, DMF (20 mL) and DIEA (0.1 mL) were added sequentially, and the mixed solution was stirred at 100 °C to react to the end. At the end of the reaction, methyl tert-butyl ether (150 mL) was added to the reaction solution and the obtained solution was treated by ultrasonic for 5 min and then filtered by suction, and the resulting product was then dried to obtain 0.5 g of a final product with a yield of 100%.

24-264

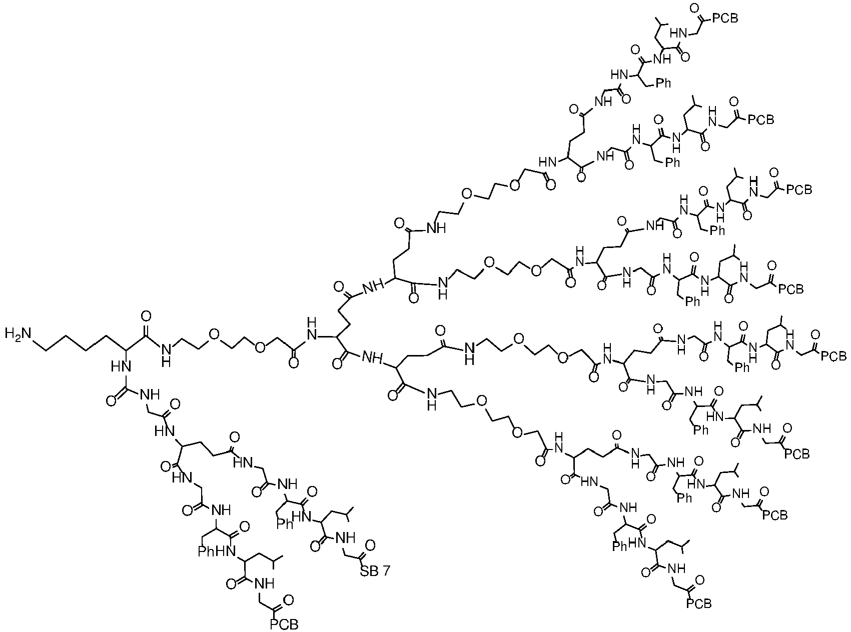

Compound 24-261 (0.5 g, 0.0489 mmoL) was weighed, dichloromethane (10 mL) and TFA (0.1 mL, 1.4654 mmoL) were then added sequentially, and the obtained solution was treated by ultrasonic until Compound 24-261 was completely dissolved; a ground glass stopper was used, and the mixed solution was stirred at room temperature to react. At the end of the reaction, methyl tert-butyl ether (150 mL) and n-hexane (100 mL) were directly added to the reaction solution and the obtained solution was filtered by suction; the obtained solid product was dissolved with 20% methanol/dichloromethane (50 mL) by ultrasonic, silica gel powder (3 g) was then added, and the obtained solution was evaporated to dryness with a rotary evaporator and then subjected to column chromatography. Gradient elution with 1% ammonia water +7%-10% methanol/dichloromethane was carried out. 0.2 g of the product was obtained with a yield of 46%.

¹H-NMR (400 MHz, DMSO-*d*₆) δ 10.16 (s, 6H), 8.95 (s, 6H),8.31-8.20 (m, 10H), 8.07 (s, 13H), 7.89 (m, 14H), 7.72 (s, 3H), 7.50 (m, 5H), 7.25-7.09 (m, 64H), 5.76 (s, 6H), 4.56-4.28 (m, 18H), 3.96 -3.90(m, 25H), 3.58-3.53 (m, 83H), 3.18-3.02 (m, 58H), 2.76-2.72 (s, 20H), 2.42 (s, 27H), 2.35 - 2.01 (m, 97H), 1.83-1.75 (m, 46H), 1.55-1.50 (m, 50H), 1.38-1.23 (m, 44H), 0.87-0.82(m, 62H).

24-266

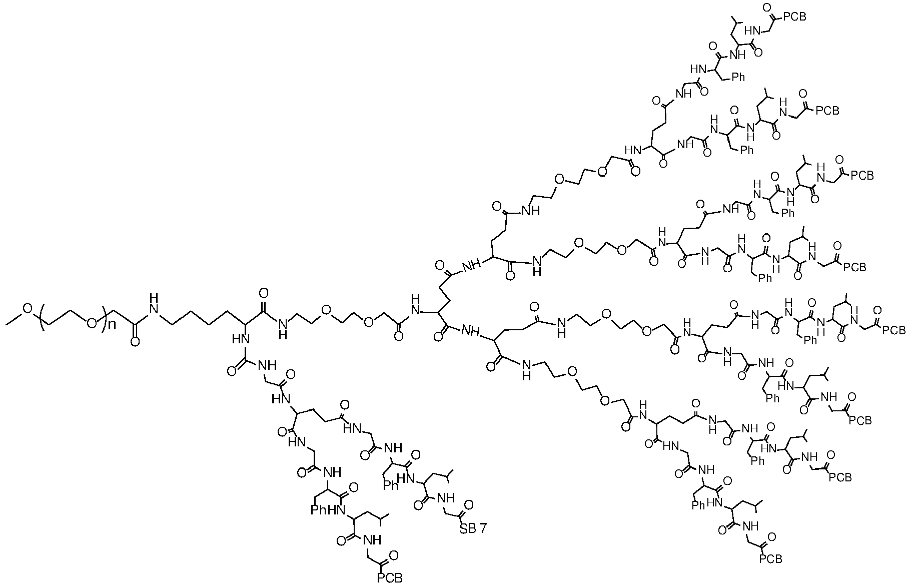

Compound 24-264 (0.2 g, 0.0198 mmoL) was weighed and then dissolved with DMF (20 mL) and then M-SCM-40K (0.799 g, 0.0189 mmoL) was then added and dissolved by ultrasonic. The obtained solution reacted in the dark at a low speed stirring. At the end of the reaction, the reaction solution was precipitated with methyl tert-butyl ether (150 mL) and n-hexane (70 mL) and then filtered by suction to obtain a solid product; the obtained solid product was dissolved with 20% methanol/dichloromethane, silica gel powder (3 g) was then added, and the obtained solution was evaporated to dryness and then subjected to column chromatography. Elution with 1% ammonia water+7% methanol/dichloromethane was carried out, the product was collected and evaporated to dryness, the obtained product was thoroughly dissolved with absolute ethanol (3 mL) by ultrasonic, methyl tert-butyl ether (150 mL) and n-hexane (50 mL) were added sequentially; suction filtering was carried out; the obtained product was dissolved with absolute ethanol (3 mL), and the obtained solution was precipitated with methyl tert-butyl ether and n-hexane; the process of dissolution and precipitation was repeated three times, suction filtering was carried out, and the obtained product was dried in an oven. 0.54 g of the product was obtained with a yield of 56%.

¹H-NMR (400 MHz, DMSO-*d*₆) δ 10.14 (s, 5H), 8.96 (s, 9H), 8.40-7.82 (s, 47H), 7.75-7.44 (m, 56H), 7.30-7.14 (m, 38H), 5.86 (s, 2H),4.57-4.28 (m, 32H), 4.18-3.98 (m, 32H), 3.51 (s, 3781H), 2.88-2.66 - 2.64 (m, 82H), 2.32-2.10 (m,78H),1.87-1.61 (m, 55H), 1.61-1.47(m, 42H), 1.30-1.17 (m, 150H), 0.88-0.77 (34H).

Experimental Example 1 In vivo anti-tumor efficacy test of the compound of the disclosure on subcutaneously transplanted tumor model of human colon cancer COLO-205 cells in BALB/c nude mice
I. Experimental purpose
   A subcutaneously transplanted tumor model of human colon cancer COLO-205 cells in BALB/c nude mice was established to investigate the anti-tumor effect of the test sample on the subcutaneously transplanted tumor.
II. Information of test sample and control
   1. Test sample
      Compound 22-278
   2. Control
      Component 1: SB7 HCL (PharmaBlock Sciences (Nanjing), Inc.)
      Component 2: Palbociclib (PCB, purity: 99.5%, Tianjin Pharmacn Medical Technology Co., Ltd)
   3. Solvent/negative control
   Sodium chloride injection (Shandong Qidu Pharmaceutical Co., Ltd., batch number: 4B19030803, concentration: 0.9%, specification: 100 mL: 0.9 g)
III. Preparation of test sample/control
   1. Test sample:
      An appropriate volume of Compound 22-278 was weighed, an appropriate amount of normal saline was added to Compound 22-278 to prepare a solution with a concentration of 9.47 mg/mL.
   3. Small-molecule control:
      An appropriate amount of SB7 (conversion coefficient: 93.4%) was weighed and dissolved with a certain volume of ethanol (5%, V/V); after SB7 was completely dissolved, and an appropriate amount of sodium chloride injection was added to prepare a solution with a concentration of 0.4 mg/mL.
      An appropriate amount of PCB was weighed and a certain volume of 0.5% CMC-Na solution was added; the obtained solution was then well stirred by a magnetic stirrer to prepare a solution with a concentration of 0.35 mg/mL.
   4. Negative control: normal saline was used directly.
IV. Experiment system
   1. Information of tumor cell line
      Human colon cancer cell COLO-205: sourced from Cell Resource Center, Institute of Basic Medical Sciences, Chinese Academy of Medical Sciences, and the culture conditions were RPMI1640 + 10% FBS, 37°C, 5% CO₂.
   2. Experimental animals
      SPF-level male BALB/c nude mice, 4 weeks old, with weight of 11.0-14.9 g
      Source of laboratory animal: Beijing Vitalstar Biotechnology Co., Ltd, production license number: SCXK (Beijing) 2016-0011, certificate number: No.1100111911049640.
   3. Animal rearing and management
      Rearing environment: animal facility with SPF-level environment
      License number for animal use: SYXK (Beijing) 2016-0029
      Feed: Animals were given qualified feed (provided by Beijing Keao Xieli Feed Co., Ltd., batch numbers: 19063113, 19063123, 19073113, production license number: SCXK (Beijing) 2014-0010), and the animals were allowed to eat the feed freely.
      Drinking water: during the quarantine and test process, animals were given purified water (filtered with secondary reverse osmosis RO membrane), supplied with a drinking bottle. The animals were allowed to drink the water freely.
      Rearing condition: Animals were raised in an independent air supply system (IVC), with 3 to 5 animals in each cage. The environmental conditions: temperature: 20 °C to 26 °C; relative humidity: 40% to 70%; actual temperature: 22°C to 26°C: actual relative humidity: 42% to 59%. The animals were raised under light and in the dark alternately for about 12 hours.
V. Experimental design

COLO-205 cells were thawed for cell subculture and amplification. When the cells were amplified to a sufficient number, the cells in the logarithmic growth phase were collected for cell inoculation. The cell concentration was adjusted to 5×10⁷ cells/mL, and the cells were inoculated subcutaneously in the right axilla of mice with a dose of 0.2 mL/mouse. After the animals were inoculated, the tumor growth was observed, and 30 qualified tumor-bearing mice with tumor volume within the range of 162.96 mm³ to 470.56 mm³ were selected and grouped randomly according to the tumor size.

According to the size of the tumor, the mice were randomly divided into 5 groups with 6 animals in each group. The dosage and animal number of each group are shown in Table 1. Negative control, SB7, and test samples were injected intravenously, and PCB was given by intragastric administration. Administration was consecutively carried out for two weeks, once a week.

**Table 1 Animal grouping and dosage**

| Test sample/ Control | Administration dose (mg/kg) | Administration concentration (mg/kg) | Administration capacity (mL/kg) | Number of animals |
|---|---|---|---|---|
| Negative control | - | - | 10 | 6 |
| SB7+PCB | 4+3.5 | 0.4+0.35 | 10+10 | 6 |
| 22-278 | 94.7 | 9.47 | 10 | 6 |

### VI. Indicator measurement

1. During the administration period, general clinical observations were performed twice a day, and body weight and tumor diameter measurements were performed twice a week. After euthanasia of the animal, the tumor was removed and weighed. After the animal was euthanized, gross anatomy was performed to observe the changes in the main organs.
2. Tumor diameter measurement
   Tumor diameters were measured in all animals with a vernier caliper on the grouping date (the day of the first administration was regarded as D1), twice a week after the first administration and the day before euthanasia; long and short diameters of the tumors were recorded, the tumor volumes were calculated, and the tumor growth curves were made according to the tumor volumes. The tumor volume was calculated as follows: V=1/2×long diameterxshort diameter².
3. Evaluation of curative effect based on tumor volume

Relative tumor volume (RTV) and relative tumor proliferation rate T/C% were calculated as follows:
$RTV = {V}_{t} / {V}_{0}$
Vₜ: Tumor volume obtained by measuring the tumor every day
Vo: initial tumor volume (before administration)
T/C%=average RTV of the treatment group/average RTV of the control group×100%
If T/C%≤40% and *P*≤0.05 in statistics for the RTV of the experimental group and the RTV of the model group, it means effective in tumor growth inhibition; on the contrary, if T/C%>40%, it means ineffective in tumor growth inhibition.

### 4. Evaluation of curative effect based on tumor weight

At the end of the experiment on D16, the tumor nodules were taken and weighed, and the difference in tumor weight between the groups was compared to further calculate the tumor inhibition rate IR_{TW}. IR_{TW}≥60% was used as a reference indicator for effectiveness. The calculation formula was as follows:
${IR}_{TW} \left(%\right) = \left({W}_{model group}-{W}_{treatment group}\right) / {W}_{model group} \times 100 %$

### VII. Statistical analysis

The statistical software SPSS13.0 was used to process the data, and the measurement data were expressed in the form of "mean±standard error". The specific analysis process was as follows: one-way analysis of variance (ANOVA) was adopted for statistical analysis, if ANOVA was statistically significant (*P*≤0.05) and the variance was homogeneous, Tukey test was carried out for comparative analysis between groups; if the variance was not homogeneous, Dunnett's T3 test was adopted for comparative analysis between groups.

### VIII. Results

### 1. Body weight and clinical observation:

The animals in the experiment showed no obvious abnormalities. Throughout the experiment, the body weights of mice in the negative control group were significantly lower than those before the grouping, and the animal weights of the other groups were slightly higher than those before the grouping. Body weights of animals in each group are shown in Table 2.

**Table 2 Statistical data of bodv weight (x̅ ± s )**

| Test sample/ Control | Weight (g) | | | | |
|---|---|---|---|---|---|
| | D1 | D5 | D8 | D12 | D15 |
| Negative control | 17.7±1.7 | 17.0±1.9 | 16.2±1.4 | 15.3±1.3 | 15.5±1.2 |
| SB7+PCB | 18.3±1.0 | 18.4±1.3 | 18.7±1.9 | 18.4±1.7^{a} | 19.3±1.9 |
| 22-278 | 18.3±1.3 | 19.0±1.6 | 20.0±1.7^{a} | 18.4±1.5^{a} | 19.4±1.7^{a} |

| | | | | | |
|---|---|---|---|---|---|
| Note: a means *P*≤0.05 relative to group 1 | | | | | |

### 2. Tumor volume

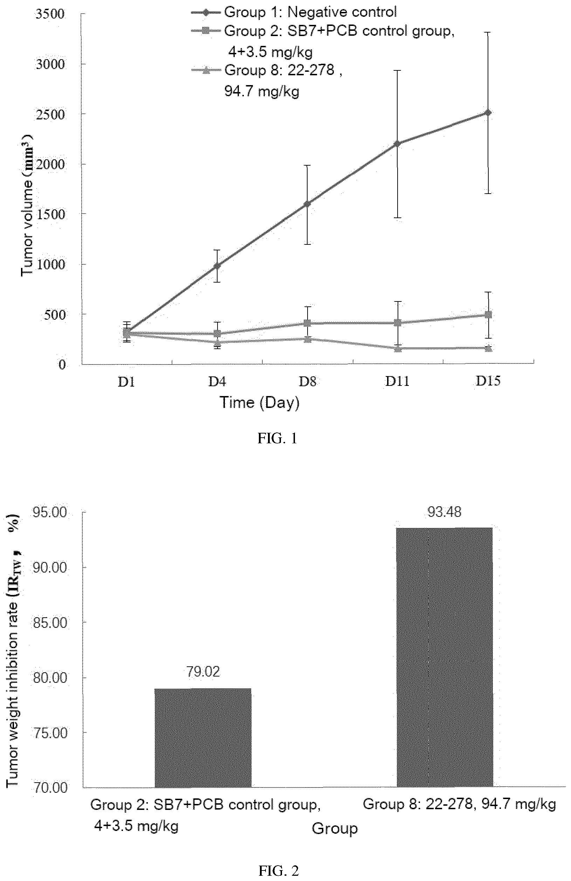

There was no significant difference in tumor volume among the groups in grouping on D1 (*P*>0.05)*.* The average tumor volume and average RTV of groups 2-10 were lower than those of the negative control group at each time point after administration. The tumor nodules in the negative control group maintained stable growth. The tumor volume and RTV of each group changed with time. The changes and the statistical differences among groups are shown in Table 3. The trend of average tumor volume growth of each group is shown in FIG. 1.

**Table 3 Statistical data of tumor volume and RTV**

| Test sample/ Control | Tumor volume (mm³) | | | | |
|---|---|---|---|---|---|
| | D1 | D5 | D8 | D12 | D15 |
| Negative control | 323.10 | 978.16 | 1590.65 | 2191.65 | 2498.27 |
| RTV | - | 3.30 | 5.43 | 7.41 | 8.32 |
| SB7+PCB | 326.47 | 297.00^{a} | 405.97^{a} | 407.50^{a} | 479.71^{a} |
| RTV | - | 0.89 | 1.21 | 1.21 | 1.43^{a} |
| 22-278 | 318.69 | 219.23^{a} | 250.34^{a} | 153.00^{a} | 152.29^{a} |
| RTV | - | 0.69 | 0.79 | 0.48 | 0.47^{a} |

| | | | | | |
|---|---|---|---|---|---|
| Note: a means *P*≤0.05 relative to group 1 | | | | | |

It can be seen from Table 3 and FIG. 1 that SB7+PCB and 22-278 have obvious inhibitory effect on tumor growth. The anti-tumor effect of 22-278 is better than that of the SB7+PCB combination.

### 3. Relative tumor proliferation rate T/C%

The statistical data of tumor T/C% and IR_{TV}% are shown in Table 4 and Table 5.

**Table 4 Statistical data of Relative tumor proliferation rate (T/C%)**

| Test sample/ Control | Relative tumor proliferation rate (T/C%) | | | |
|---|---|---|---|---|
| | D5 | D8 | D12 | D15 |
| SB7+PCB | 26.96 | 22.35 | 16.40 | 17.22^{∗} |
| 22-278 | 20.83 | 14.47 | 6.46 | 5.67^{∗} |

| | | | | |
|---|---|---|---|---|
| Note: "^{∗}" means that T/C%≤40%, and for RTV, *P*≤0.05 relative to group 1. | | | | |

**Table 5 Statistical data of tumor volume inhibition rate (IR_{TV}, %)**

| Test sample/ Control | Tumor volume inhibition rate (IR_{TV}, %) | | | |
|---|---|---|---|---|
| | D5 | D8 | D12 | D15 |
| SB7+PCB | 73.04 | 77.65 | 83.60 | 82.78^{∗} |
| 22-278 | 79.17 | 85.53 | 93.54 | 94.33^{∗} |

| | | | | |
|---|---|---|---|---|
| Note: "^{∗}" means that T/C%≤40%, and for RTV, *P*≤0.05 relative to group 1. | | | | |

It can be seen from Table 4 and Table 5 that on D15 (the day before euthanasia), the T/C% values of 37-26 group, 28-126 group and 28-206 group were 17.22%, 2.87%, 6.40%, and 9.58%, respectively and their IR_{TV}% values were 82.78%, 97.13%, 93.60%, and 90.42%, respectively. The T/C% of the 28-206 group on D15, the T/C% of the 37-26 group during the period of D8 to D15, and the T/C% of the 28-126 group on D8 and D15 fell below 40%, and their mean RTV values were significantly lower than the mean RTV of the negative control group (*P*≤0.05).

### 4. Tumor weight

The animals were euthanized on D16 and the tumor weights were weighed. The statistical data of tumor weight and inhibition rate are shown in Table 6, and the schematic diagram of tumor weight inhibition rate is shown in FIG. 2.

**Table 6 Statistical data of tumor weight (x̅±s) and tumor weight inhibition rate (IR_{TW}, %)**

| Test sample/control | Tumor weight (g) | IR_{TW} (%) |
|---|---|---|
| Negative control | 1.844±0.475 | - |
| SB7+PCB | 0.387±0.227^{a} | 79.02^{▲} |
| 22-278 | 0.120±0.080^{a} | 93.48^{▲} |

Notes: 1. ${IR}_{TW} \left(%\right) = \left({W}_{solvent}-{W}_{treatment group}\right) / {W}_{solvent} \times 100 %$

### 2. " ▲" means that IR_{TW}%≥60%, and as for tumor weight, P≤0.05 relative to group 1.

### 3. a means P≤0.05 relative to group 1

It can be seen from Table 6 and FIG. 2 that the tumor weights of the SB7+PCB group and 22-278 group are significantly lower than the tumor weight of the negative control group and the IR_{TW}% is greater than 60%.

Conclusion: Under the experimental conditions, compound 22-278 administered by tail vein injection at a dose of 94.7 mg/kg has a significant tumor growth inhibitory effect on the subcutaneously transplanted tumor model of human colon cancer cell COLO-205. The combination of PCB at a dose of 3.5 mg/kg by intragastric administration and SB7 administered by tail vein injection at a dose of 4.0 mg/kg also has a significant growth inhibitory effect on the tumor model. The anti-tumor effect of Compound 22-278 is better than that of the SB7+PCB combination group.

Although the specific embodiments of this disclosure have been described in detail, those skilled in the art will understand that various modifications and changes can be made to the details according to all the teachings that have been disclosed, and these changes are within the protection scope of this disclosure. The full scope of the disclosure is given by the appended claims and any equivalents thereof.

## Claims

1. A polyethylene glycol conjugated drug of formula (I) or a pharmaceutically acceptable salt thereof; 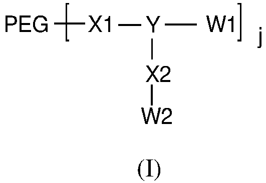 wherein PEG is a single-arm or multi-arm polyethylene glycol segment; j represents the number of arms of PEG1 (for example, 1, 2, 4, or 8);
X1 is selected from -NH-, 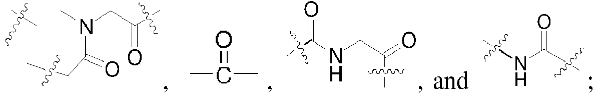 when j is greater than 1 (such as, 2, 4 or 8), there are multiple (such as, 2, 4 or 8) X1s at the same time, and in this case, the X1s are the same or different;
Y represents Lys (lysine residue) or Glu (glutamic acid residue); when j is greater than 1 (such as, 2, 4 or 8), there are multiple (such as, 2, 4 or 8) Ys at the same time, and in this case, the Ys are the same or different;
X2 is selected from 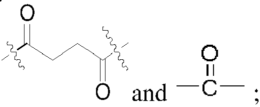 when j is greater than 1 (such as, 2, 4 or 8), there are multiple (such as, 2, 4 or 8) X2s at the same time, and in this case, the X2s are the same or different;
W1 is selected from N1-AC1, Q, 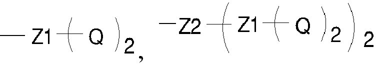 and 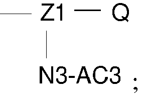 when j is greater than 1 (such as, 2, 4 or 8), there are multiple (such as, 2, 4 or 8) W1s at the same time, and in this case, the W1s are the same or different;
Q represents 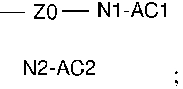 each of ZO, Z1, and Z2 is independently selected from 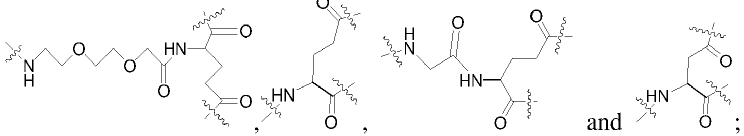
Z0, Z1 and Z2 are the same or different, and when there are multiple Z0s, multiple Z1s or multiple Z2s at the same time, the Z0s are the same or different, the Z1s are the same or different, or the Z2s are the same or different;
each of N1, N2 and N3 independently is G (glycine residue) or GFLG (glycine-phenylalanineleucine-glycine); N1, N2 and N3 are the same or different, and when there are multiple N1s, multiple N2s or multiple N3s at the same time, the N1s are the same or different, the N2s are the same or different, or the N3s are the same or different;
AC1, AC2 and AC3 are drug molecules (for example, drug molecules with anti-tumor activity); AC1, AC2 and AC3 are the same or different, and when there are multiple AC1s, multiple AC2s or multiple AC3s at the same time, the AC1s are the same or different, the AC2s are the same or different, or the AC3s are the same or different;
W2 is selected from N1'-AC1', Q', 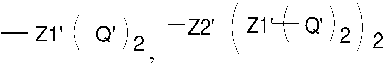 and 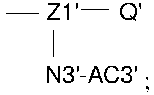 when j is greater than 1 (such as, 2, 4 or 8), there are multiple (such as, 2, 4 or 8) W2s at the same time, and in this case, the W2s are the same or different;
Q' represents 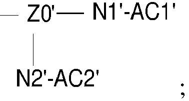 each of Z0', Z1' and Z2' is independently selected from: 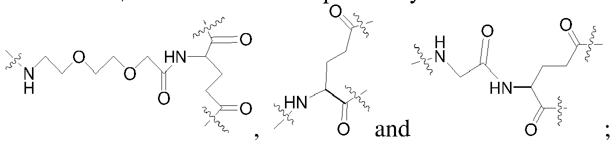
Z0', Z1' and Z2' are the same or different, and when there are multiple Z0's, multiple Zl's or multiple Z2's at the same time, the Z0's are the same or different, the Z1's are the same or different, or the Z2's are the same or different;
each of N1', N2', and N3' independently is G or GFLG; N1', N2', and N3' are the same or different, and when there are multiple N1's, multiple N2's, or multiple N3's at the same time, the N1's are the same or different, the N2's are the same or different, or the N3's are the same or different;
AC1', AC2' and AC3' are drug molecules (for example, drug molecules with anti-tumor activity); AC1', AC2' and AC3' are the same or different, and when there are multiple AC1's, multiple AC2's or multiple AC3's at the same time, the AC1's are the same or different, the AC2's are the same or different, or the AC3's are the same or different.

2. The polyethylene glycol conjugated drug or a pharmaceutically acceptable salt thereof according to claim 1, **characterized in** one or more of the following:
(1) PEG is a single-arm or four-arm polyethylene glycol segment;
(2) PEG has a number-average molecular weight of 5k-10k, 10k-20k or 20k-40k; and
(3) each of AC1, AC2, AC3, AC1', AC2', AC3' is independently selected from LPT, PCB, SB7, PKA, ABR, and SN38;
(4) when Y represents Lys, X1 is linked to an α-amino group on the Lys;
(5) when Y represents Lys, X1 is linked to an ε-amino group on the Lys;
(6) when Y represents Glu, X1 is linked to an α-carboxyl group on the Lys;
(7) when Y represents Glu, X1 is linked to a γ-carboxyl group on the Lys;
(8) N1 and N2 are both GFLG;
(9) N1, N2 and N3 are all GFLG;
(10) N1 and N2 are both G;
(11) N1, N2 and N3 are all G;
(12) N1' and N2' are both GFLG;
(13) N1', N2' and N3' are all GFLG;
(14) N1' and N2' are both G; and
(15) N1', N2' and N3' are all G.

3. The polyethylene glycol conjugated drug or a pharmaceutically acceptable salt thereof according to claim 1 or 2, **characterized in** one or more of the following:
(1) AC1 and AC2 are both PCB;
(2) AC1 and AC2 are both LPT;
(3) AC1 and AC2 are both SN38:
(4) AC1' and AC2' are both LPT;
(5) AC1' is SB7 and AC2' is LPT;
(6) AC1' and AC2' are both PCB; and
(7) AC1' and AC2' are both ABR.

4. The polyethylene glycol conjugated drug or a pharmaceutically acceptable salt thereof according to any one of claims 1 to 3, **characterized in** one or more of the following:
(1) AC1, AC2 and AC3 are all SN38; and
(2) AC1', AC2' and AC3' are all SN38.

5. The polyethylene glycol conjugated drug or a pharmaceutically acceptable salt thereof according to any one of claims 1 to 4, **characterized in** one or more of the following:
(1) N1 is GFLG and AC1 is SB7;
(2) N1 and N2 are both GFLG and AC1 and AC2 are both LPT;
(3) N1 and N2 are both GFLG and AC1 and AC2 are both PCB;
(4) N1, N2 and N3 are all G and AC1, AC2 and AC3 are all SN38;
(5) N1 is GFLG and AC1 is PCB;
(6) N1' and N2' are both GFLG and AC1' and AC2' are both LPT;
(7) N1' and N2' are both GFLG, AC1' is SB7, and AC2' is LPT;
(8) N1' and N2' are both GFLG and AC1' and AC2' are both PCB;
(9) N1' is GFLG and AC1' is PKA;
(10) N1' and N2' are both G and AC1' and AC2' are both ABR;
(11) N1', N2' and N3' are all G and AC1', AC2' and AC3' are all SN38;
(12) N1' and N2' are both GFLG, AC1' is SB7, and AC2' is PCB;
(13) N1 and N2 are both G and AC1 and AC2 are both SN38; and
(14) N1' is G and AC1' is SN38.

6. The polyethylene glycol conjugated drug or a pharmaceutically acceptable salt thereof according to any one of claims 1 to 5, wherein when the PEG is a single-arm polyethylene glycol segment, X1 is linked to an end of the PEG or in the PEG; preferably, when X1 is linked in PEG, X1 represents

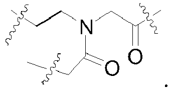

7. The polyethylene glycol conjugated drug or a pharmaceutically acceptable salt thereof according to any one of claims 1 to 6, wherein Y represents Lys.

8. The polyethylene glycol conjugated drug or a pharmaceutically acceptable salt thereof according to claim 7, wherein
X2 represents 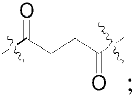
X1 is selected from 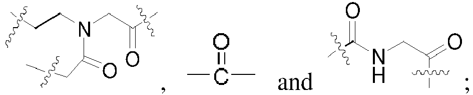
W1 is selected from N1-AC1, 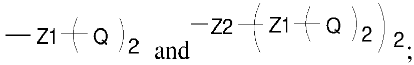
W2 is selected from Q', 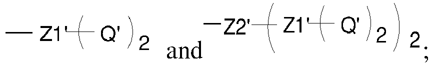
preferably, the PEG is a single-arm polyethylene glycol segment with a number-average molecular weight of 10k-20k or 20k-40k.
preferably, W1 is selected from:
(1) 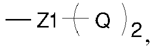 wherein Z1 is 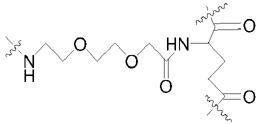 and Z0 is 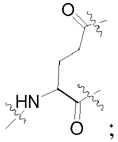 preferably, N1 and N2 are both GFLG and AC1 and AC2 are both LPT;
(2) 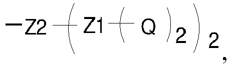 wherein Z2 is 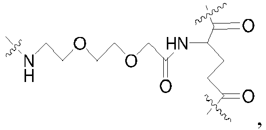 Z1 is 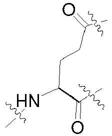 and Z0 is 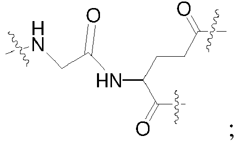 preferably, N1 and N2 are both GFLG and AC1 and AC2 are both LPT; and (3) N1-AC1, wherein N1 is GFLG; preferably, AC1 is SB7; preferably, W2 is selected from:
(1) 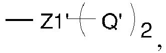 wherein Z1' is 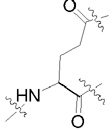 and Z0' is 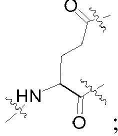 preferably, N1' and N2' are both GFLG and AC1' and AC2' are both LPT;
(2) Q', wherein Z0' is 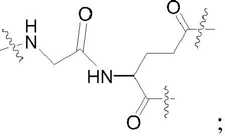 preferably, N1' and N2' are both GFLG and AC1' is SB7, and AC2' is LPT;
(3) 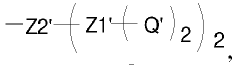 wherein Z2' is 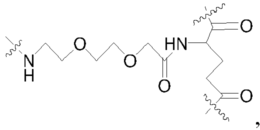 Z1' is 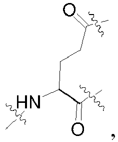 and Z0' is 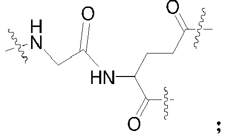 preferably, N1' and N2' are both GFLG and AC1' and AC2' are both LPT; and
(4) 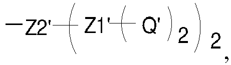 wherein Z2' is 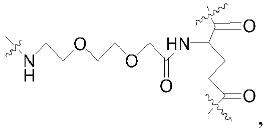 Z1' is 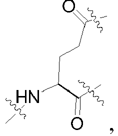 and Z0' is 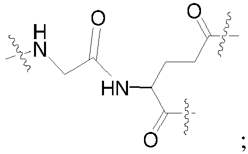 preferably, N1' and N2' are both G and AC1' and AC2' are both ABR; preferably, the polyethylene glycol conjugated drug has a structure selected from: 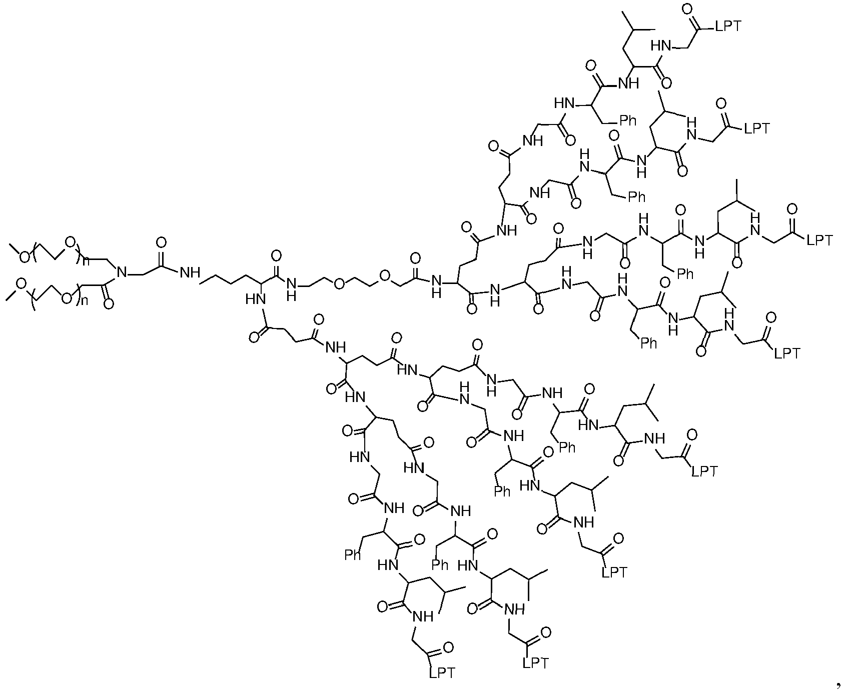 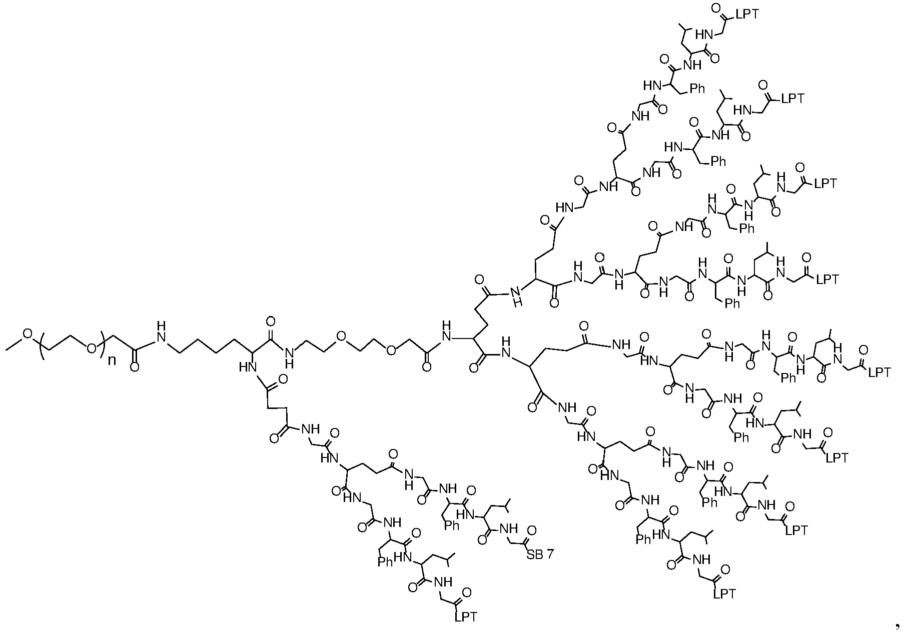 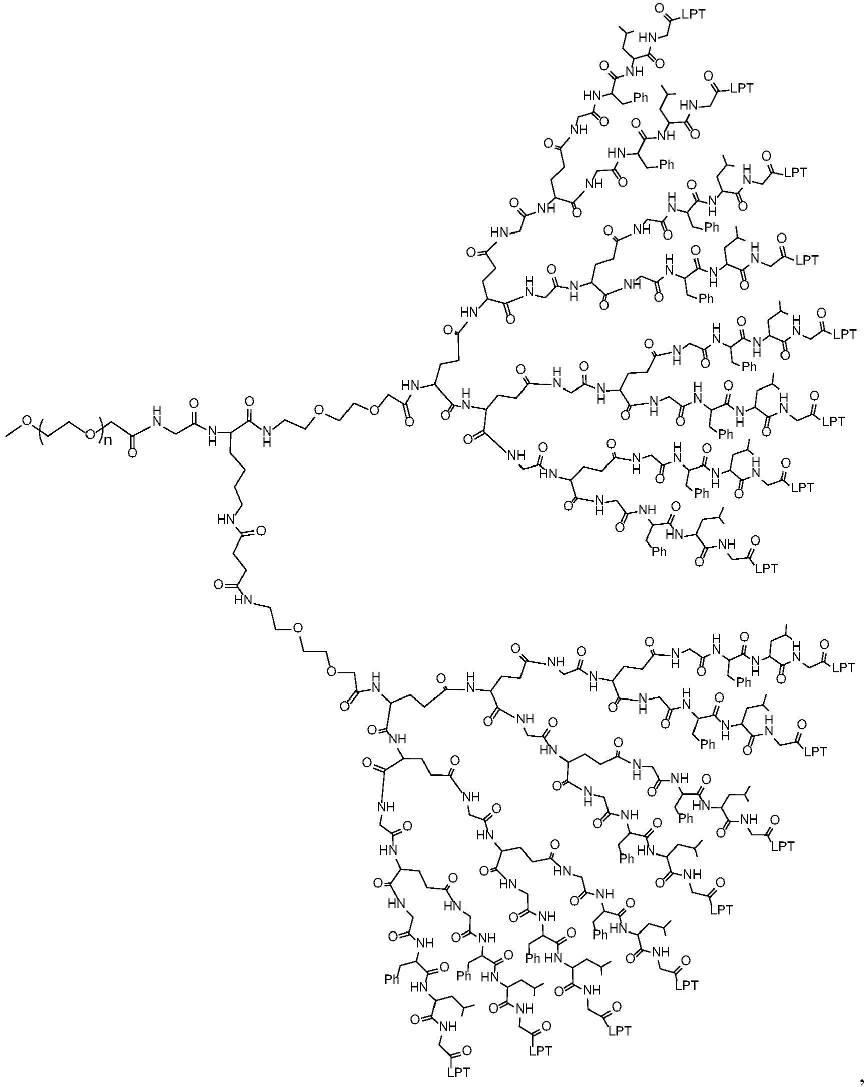 and

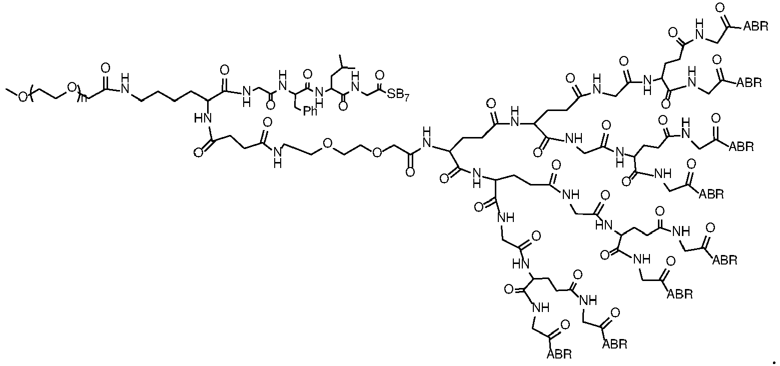

9. The polyethylene glycol conjugated drug or a pharmaceutically acceptable salt thereof according to claim 7, wherein
X2 represents 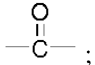
X1 is selected from 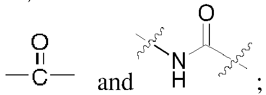
W1 is selected from N1-AC1, Q, 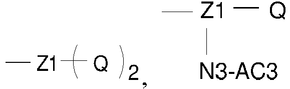 and 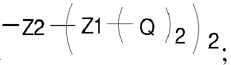
W2 is selected from N1'-AC1', Q' and 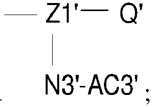
preferably, the PEG is a single-arm or four-arm polyethylene glycol segment with a number-average molecular weight of 5k-20k or 20k-40k;
preferably, W1 is selected from:
(1) Q, wherein Z0 is 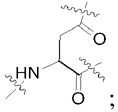 preferably, N1 and N2 are both GFLG and AC1 and AC2 are both PCB;
(2) 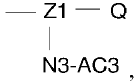 wherein Z1 is 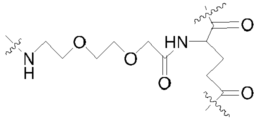 and Z0 is 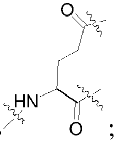 preferably, N1, N2 and N3 are all G and AC1, AC2 and AC3 are all SN38;
(3) N1-AC1, wherein N1 is GFLG; preferably, AC1 is PCB;
(4) 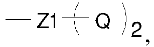 wherein Z1 is 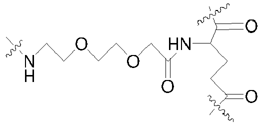 and Z0 is 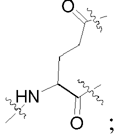 preferably, N1 and N2 are both GFLG and AC1 and AC2 are both PCB; and
(5) 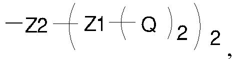 wherein Z2 and Z0 are both 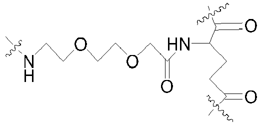 and Z1 is 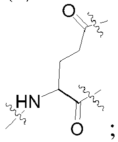 preferably, N1 and N2 are both GFLG and AC1 and AC2 are both PCB; preferably, W2 is selected from:
(1) N1'-AC1', wherein N1' is GFLG; preferably, AC1' is PKA;
(2) 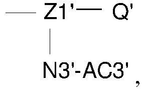 wherein Z1' is 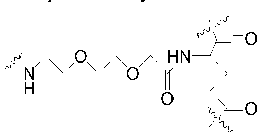 and Z0' is 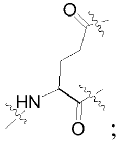 preferably, N1', N2' and N3' are all G and AC1', AC2' and AC3' are all SN38;
(3) N1'-AC1', wherein N1' is GFLG; preferably, AC1' is SB7; and
(4) Q', wherein Z0' is 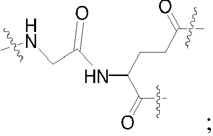 preferably, N1' and N2' are both GFLG, AC1' is SB7 and AC2' is PCB;
preferably, the polyethylene glycol conjugated drug has a structure selected from: 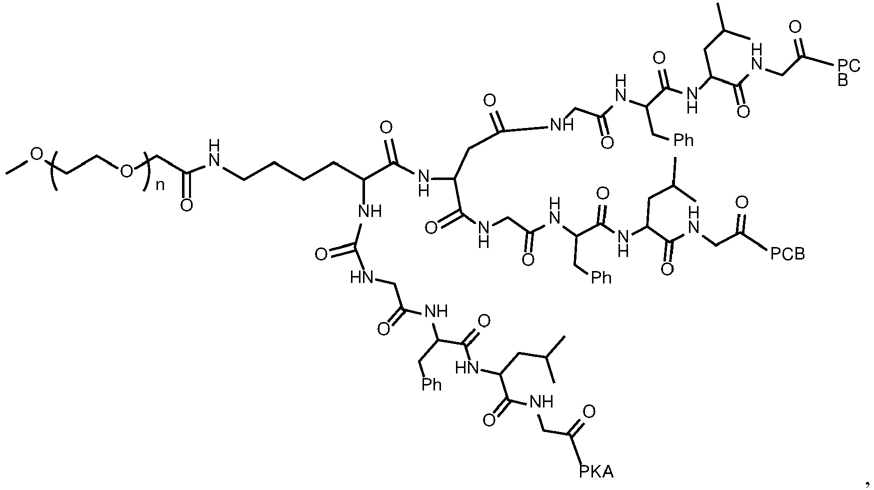 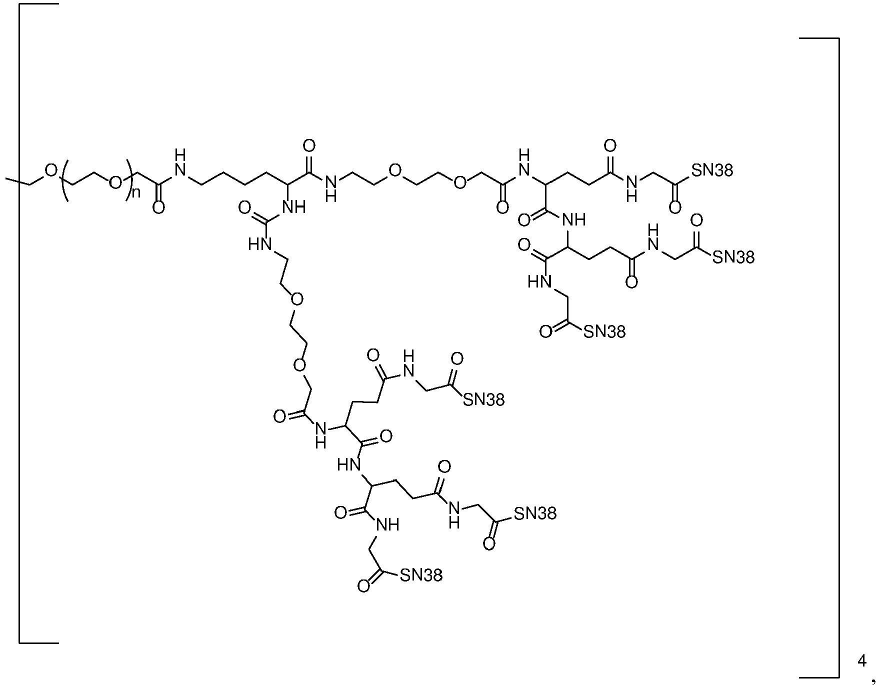 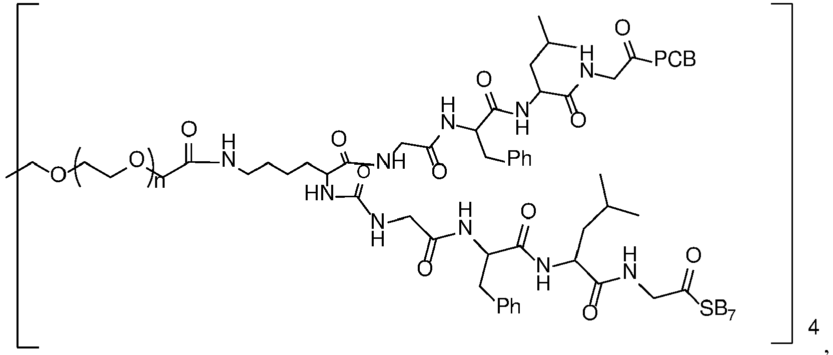 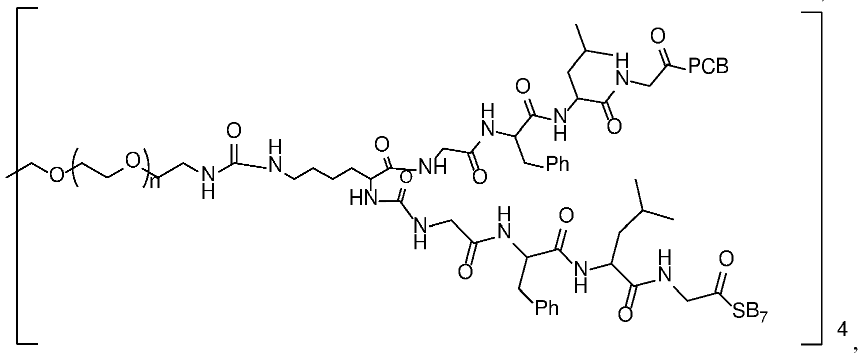 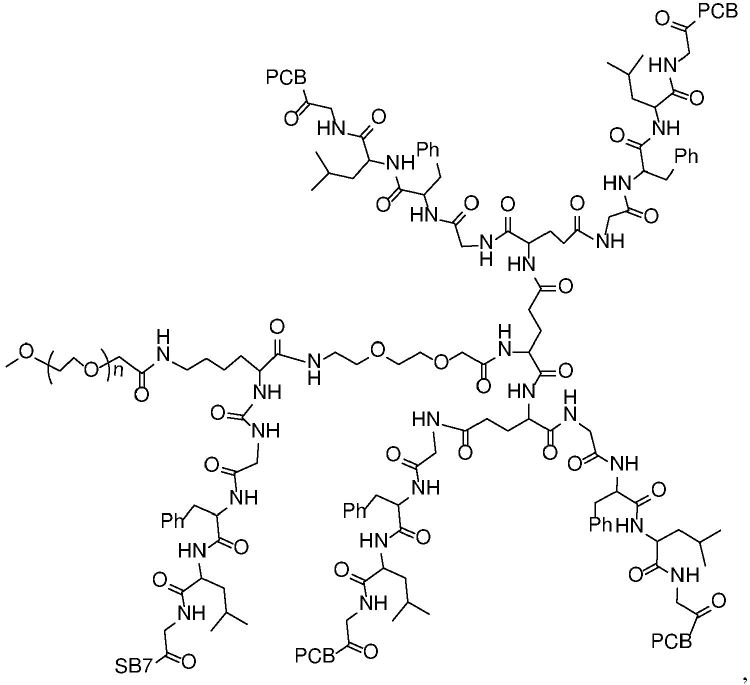 and

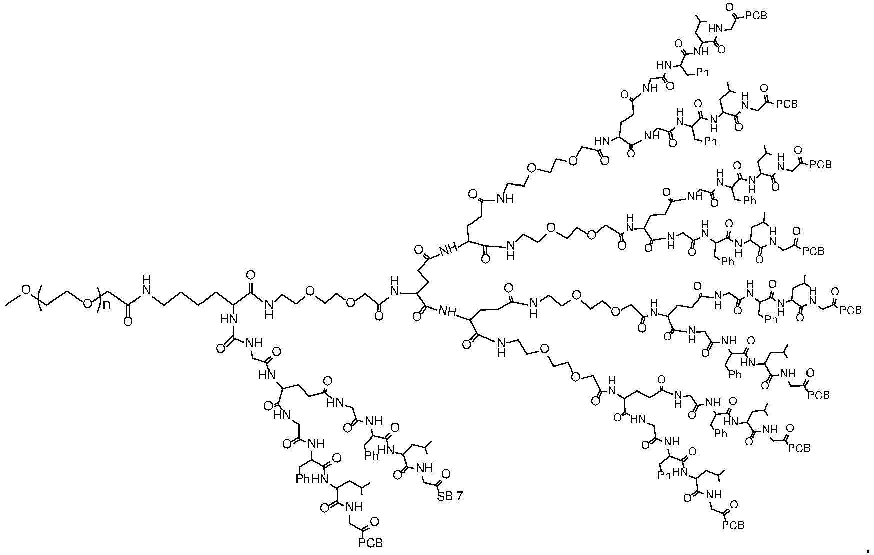

10. The polyethylene glycol conjugated drug or a pharmaceutically acceptable salt thereof according to any one of claims 1 to 6, wherein Y represents Glu.

11. The polyethylene glycol conjugated drug or a pharmaceutically acceptable salt thereof according to claim 10, wherein
X2 represents 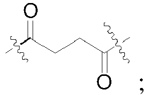 X1 represents -NH-; W1 represents N1-AC1; W2 represents 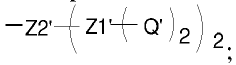
preferably, the PEG is a single-arm polyethylene glycol segment with a number-average molecular weight of 10k-20k;
preferably, N1 is GFLG and AC1 is SB7;
preferably, Z2' represents 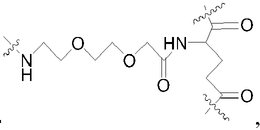 Z1' represents 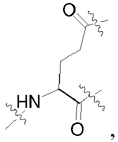 and Z0' represents 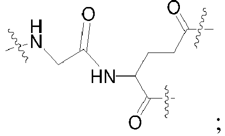
preferably, N1' and N2' are both GFLG and AC1' and AC2' are both LPT;
preferably, the polyethylene glycol conjugated drug has a structure as follows:

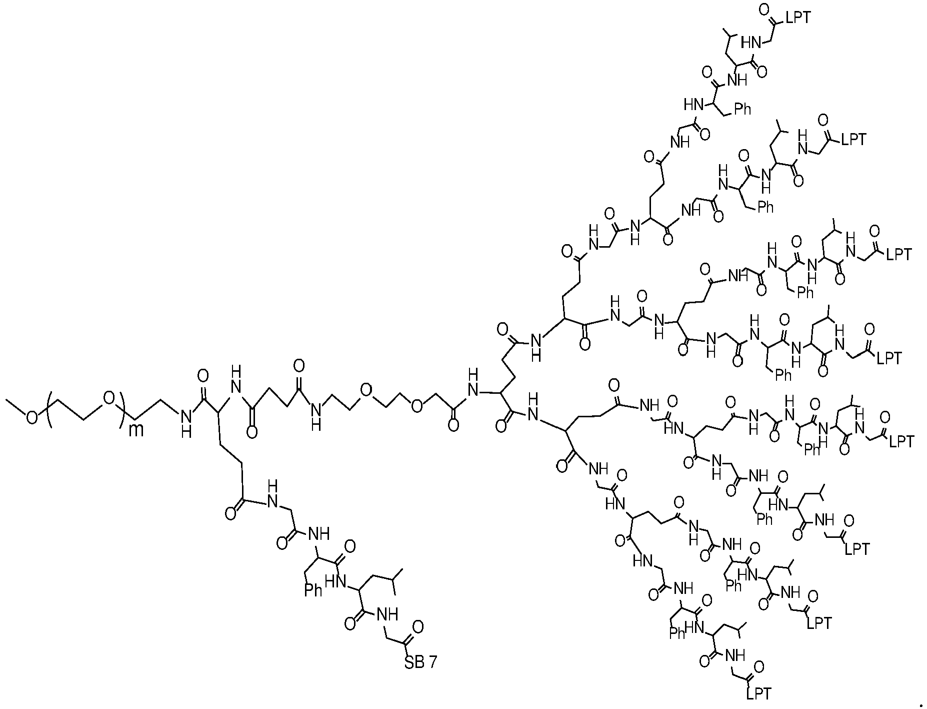

12. The polyethylene glycol conjugated drug or a pharmaceutically acceptable salt thereof according to claim 10, wherein
X2 represents 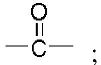 X1 represents -NH-; W1 represents N1-AC1; W2 represents 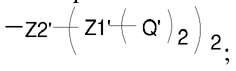
preferably, the PEG is a single-arm polyethylene glycol segment with a number-average molecular weight of 10k-20k;
preferably, N1 is GFLG and AC1 is SB7;
preferably, Z2' and Z0' both represent 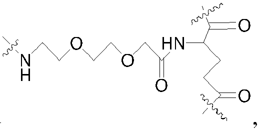 and Z1' represents 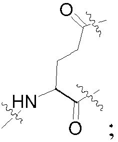
preferably, N1' and N2' are both GFLG and AC1' and AC2' are both PCB;
preferably, the polyethylene glycol conjugated drug has a structure as follows:

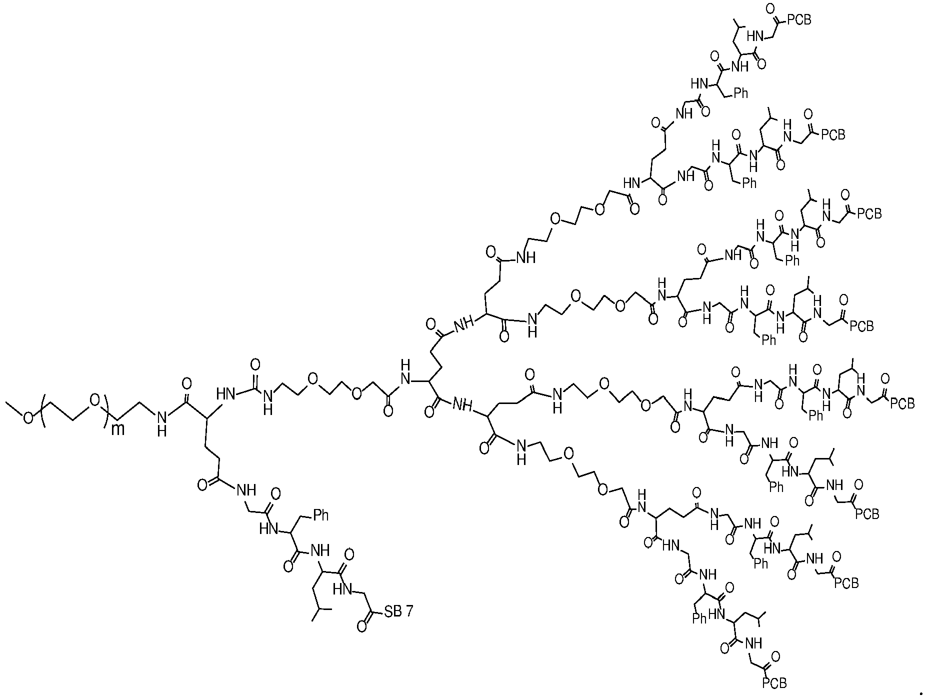

13. The polyethylene glycol conjugated drug or a pharmaceutically acceptable salt thereof according to claim 10, wherein
X2 represents 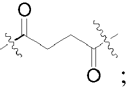 X1 represents -NH-; W1 represents Q; W2 represents N1'-AC1';
preferably, the PEG is a four-arm polyethylene glycol segment with a number-average molecular weight of 20k-40k;
preferably, N1 and N2 are both G and AC1 and AC2 are both SN38;
preferably, N1' is G and AC1' is SN38;
preferably, the polyethylene glycol conjugated drug has a structure as follows:

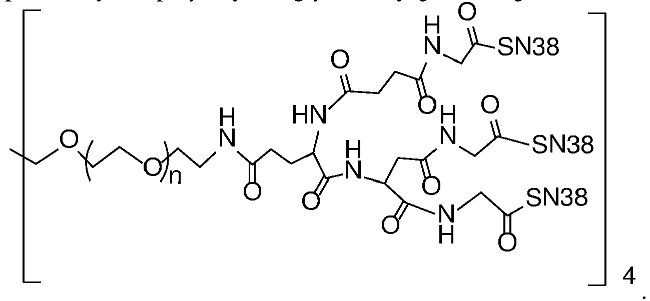

14. The polyethylene glycol conjugated drug or a pharmaceutically acceptable salt thereof, the polyethylene glycol conjugated drug being selected from
| | |
|---|---|
| 1 | 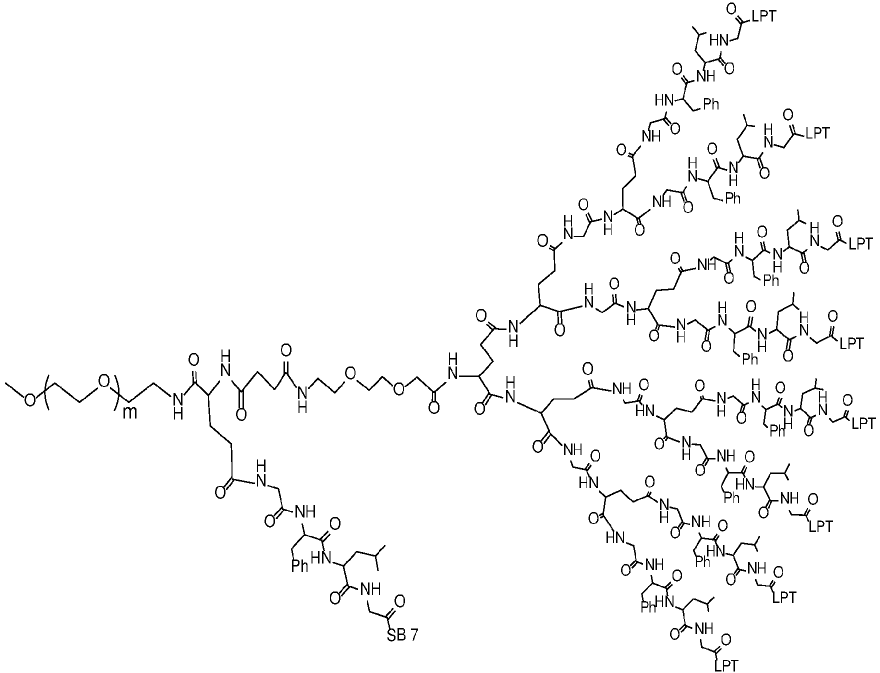 |
| | wherein PEG has a number-average molecular weight of 20k; |
| 2 | 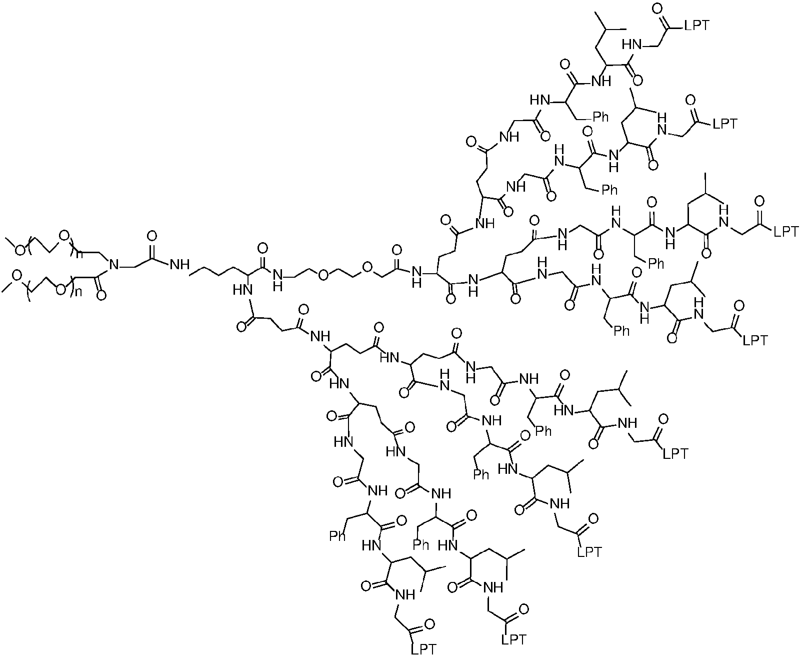 |
| | wherein PEG has a number-average molecular weight of 40k; |
| 3 | 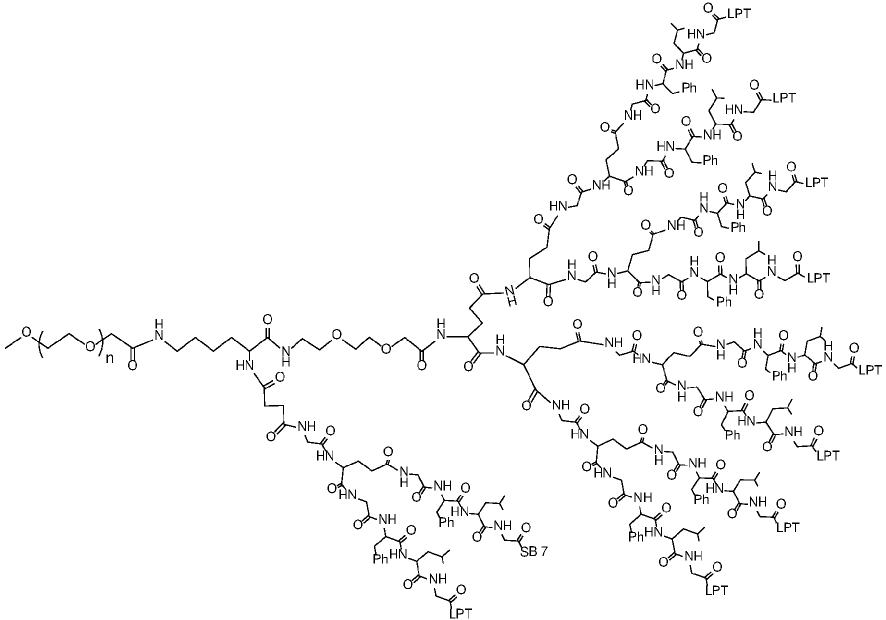 |
| | wherein PEG has a number-average molecular weight of 40k; |
| 4 | 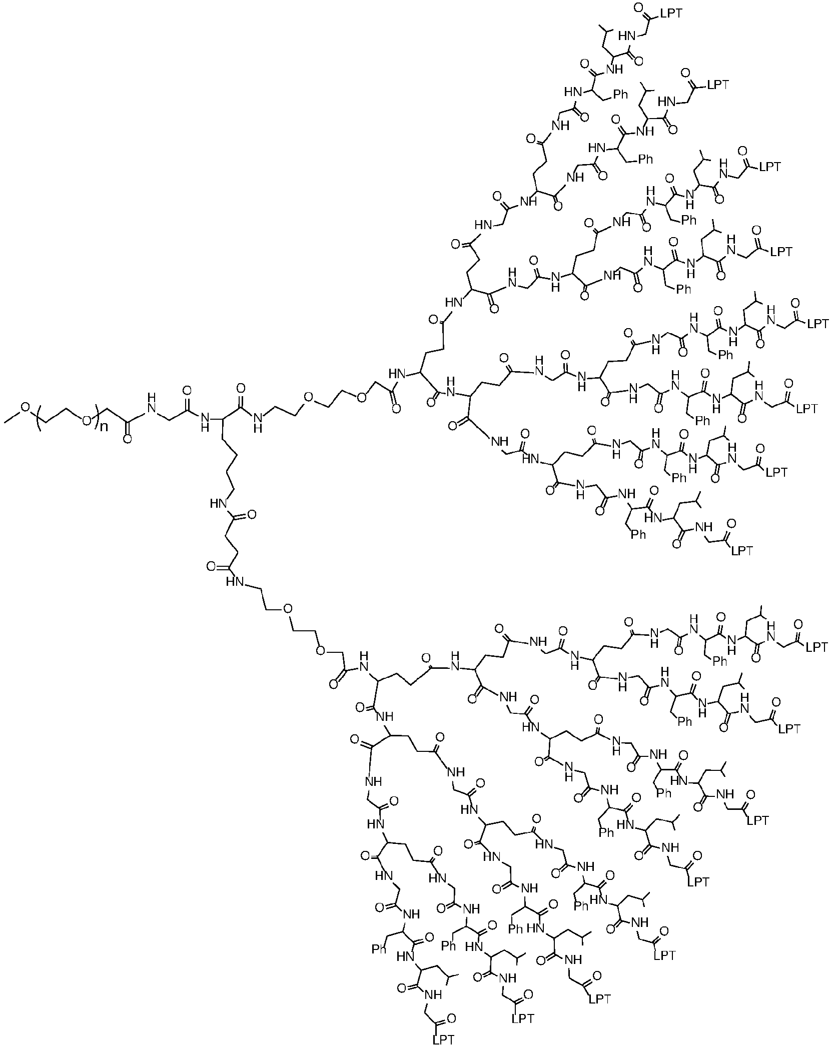 |
| | wherein PEG has a number-average molecular weight of 40k; |
| 5 | 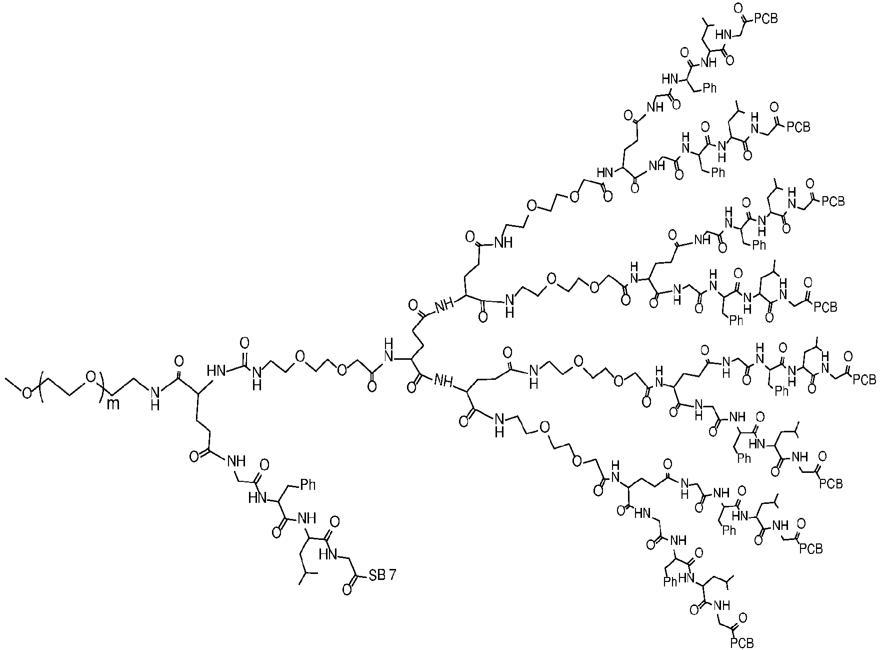 |
| | wherein PEG has a number-average molecular weight of 20k; |
| 6 | 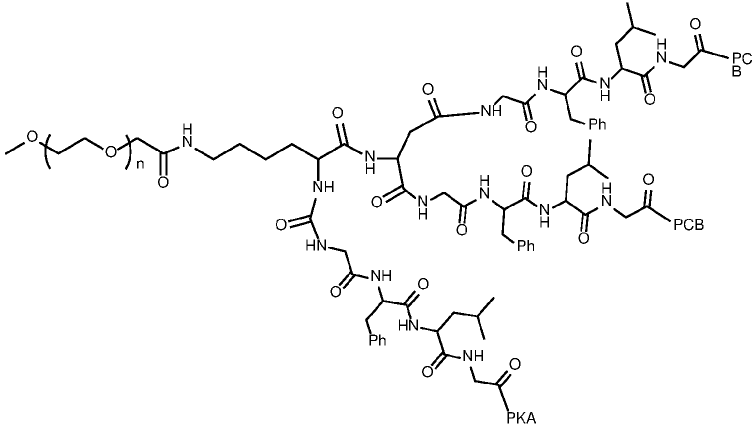 |
| | wherein PEG has a number-average molecular weight of 5k; |
| 7 | 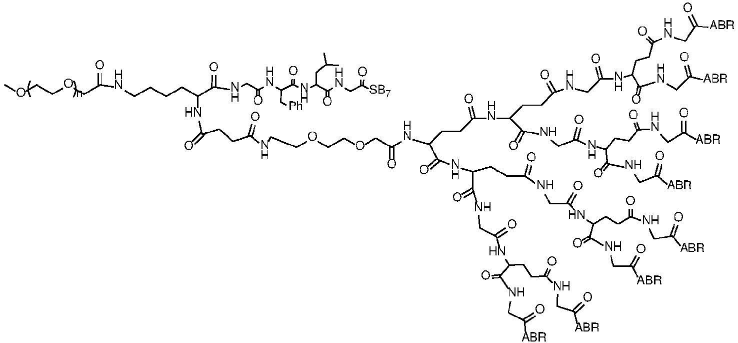 |
| | wherein PEG has a number-average molecular weight of 40k; |
| 8 | 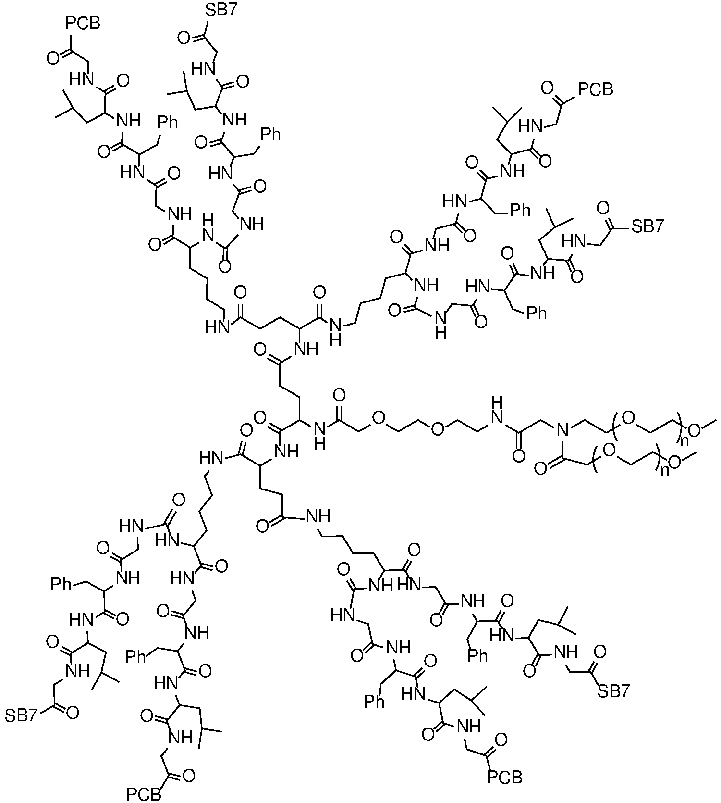 |
| | wherein PEG has a number-average molecular weight of 40k; |
| 9 | 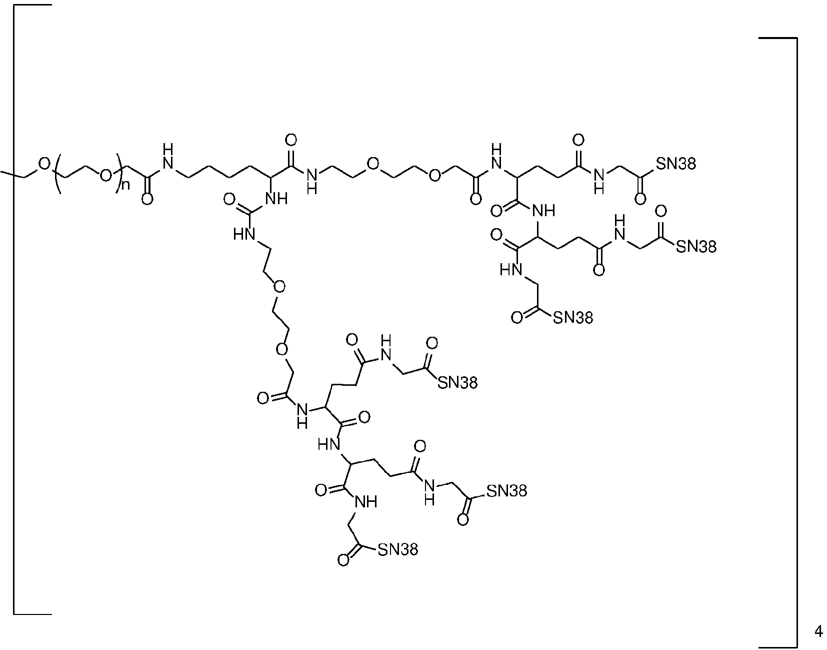 |
| | wherein PEG has a number-average molecular weight of 40k; |
| 10 | 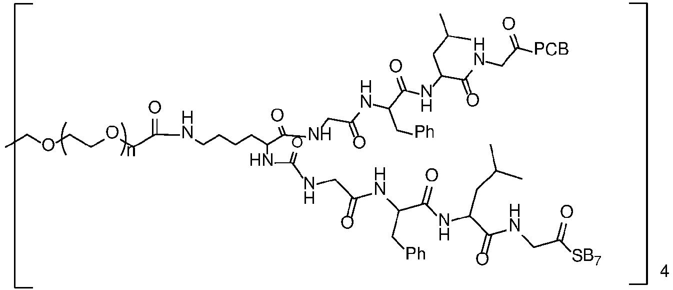 |
| | wherein PEG has a number-average molecular weight of 40k; |
| 11 | 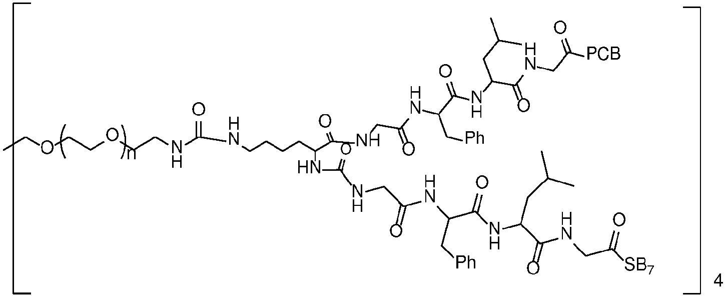 |
| | wherein PEG has a number-average molecular weight of 40k; |
| 12 | 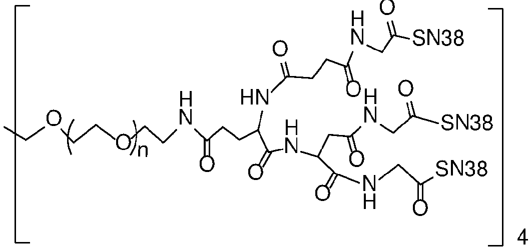 |
| | wherein PEG has a number-average molecular weight of 40k; |
| 13 | 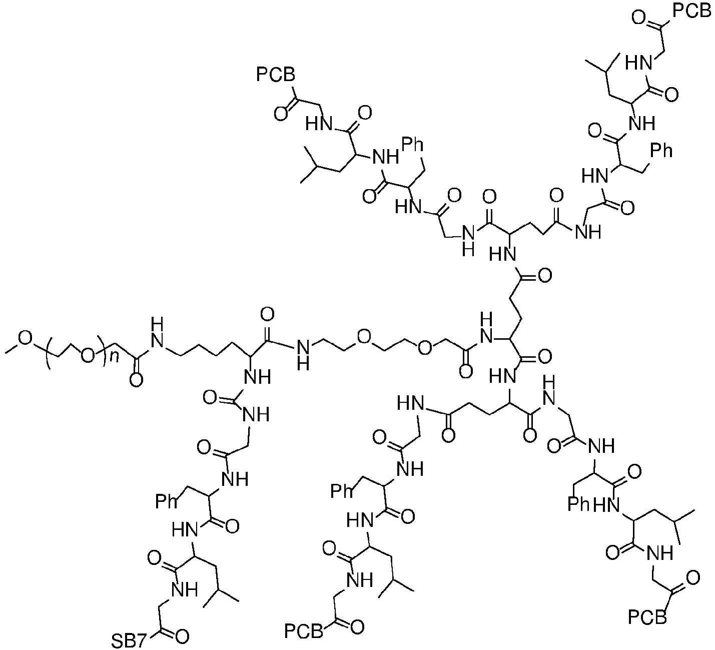 |
| | wherein PEG has a number-average molecular weight of 20k; and |
| 14 | 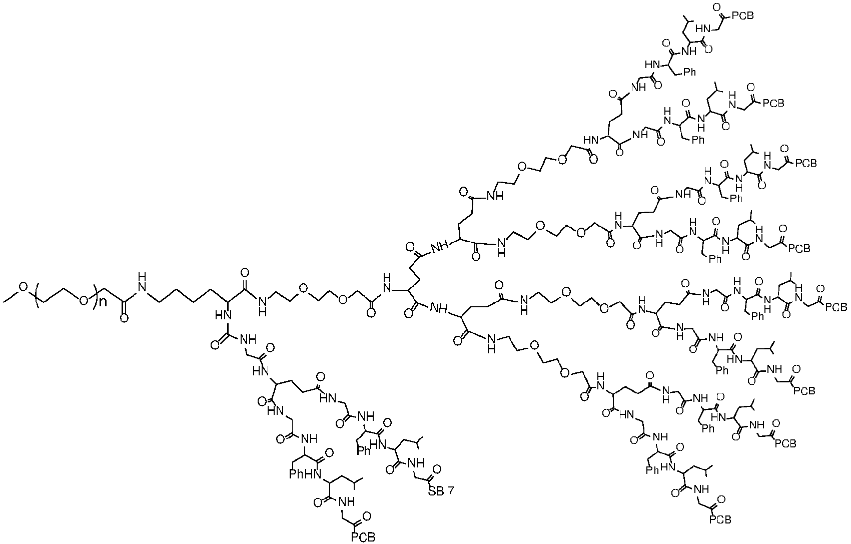 |
| | wherein PEG has a number-average molecular weight of 40k. |

15. A pharmaceutical composition, comprising a therapeutically and/or prophylactically effective amount of the polyethylene glycol conjugated drug or a pharmaceutically acceptable salt thereof according to any one of claims 1 to 14; preferably, the composition further comprises one or more pharmaceutically acceptable excipients;
preferably, the pharmaceutical composition is made into an injection preparation.

16. Use of the polyethylene glycol conjugated drug or a pharmaceutically acceptable salt thereof according to any one of claims 1 to 14 in preparation of a medicament for treating and/or preventing a disease (such as a cancer), wherein the disease refers to a disease treated by an active ingredient in the polyethylene glycol conjugated drug;
preferably, the cancer is selected from colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, and sarcoma, as well as metastasis of these cancers.

17. An injection solution, comprising the polyethylene glycol conjugated drug or a pharmaceutically acceptable salt thereof according to any one of claims 1 to 14, or the pharmaceutical composition according to claim 15; preferably, the injection solution uses saline as a carrier.

18. A method for preparing the polyethylene glycol conjugated drug or a pharmaceutically acceptable salt thereof according to any one of claims 1 to 13, comprising the following steps:
step 1: preparing an intermediate W1-Y-X2-W2, wherein Y has a free or activated carboxyl group; and
step 2: carrying out an amidation reaction so that PEG with amino groups is linked to Y on the intermediate W1-Y-X2-W2 to obtain a polyethylene glycol conjugated drug of formula (I),
wherein the number of amino groups is denoted as j, and the amino groups are free or activated amino groups;
wherein PEG, X2, Y, W1, W2, and j are as defined in any of claims 1 to 13;
preferably, in the polyethylene glycol conjugated drug, Y represents Glu, X1 represents -NH-, W1 represents N1-AC1, and W2 represents

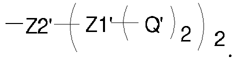

19. The method according to claim 18, wherein the intermediate W1-Y-X2-W2 is prepared by a method comprising the following steps:
step 1: preparing an intermediate W1-Y-X2 and an intermediate W2 respectively, wherein X2 has a free or activated carboxyl group, Y has a protected carboxyl group, and W2 has a free or activated amino group;
step 2: causing the free or activated carboxyl group on X2 to react with the free or activated amino group on W2 so that W1-Y-X2 is linked to W2; and
step 3: deprotecting the protected carboxyl group on Y and optionally carrying out activation to obtain the intermediate W1-Y-X2-W2.

20. A method for preparing the polyethylene glycol conjugated drug or a pharmaceutically acceptable salt thereof according to any one of claims 1 to 13, comprising the following steps:
step 1: preparing an intermediate W1-Y-X2-W2, wherein Y has a free or activated amino group; and
step 2: carrying out an amidation reaction so that PEG with carboxyl groups is linked to Y on the intermediate W1-Y-X2-W2 to obtain a polyethylene glycol conjugated drug of formula (I), wherein the number of carboxyl groups is denoted as j, and the carboxyl groups are free or activated carboxyl groups;
wherein PEG, X2, Y, W1, W2, and j are as defined in any one of claims 1 to 13;
preferably, in the polyethylene glycol conjugated drug, Y refers to Lys.

21. The method according to claim 20, wherein the intermediate W1-Y-X2-W2 is prepared by a method comprising the following steps:
step 1: preparing an intermediate W1-Y-X2 and an intermediate W2 respectively, wherein Y has a protected amino group, X2 has a free or activated carboxyl group, and W2 has a free or activated amino group;
step 2: causing the free or activated carboxyl group on X2 to react with the free or activated amino group on W2 so that W1-Y-X2 is linked to W2; and
step 3: deprotecting the protected amino group on Y and optionally carrying out activation to obtain the intermediate W1-Y-X2-W2;
preferably, in the polyethylene glycol conjugated drug, Y refers to Lys and X1 and X2 are both 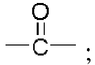
preferably, in the polyethylene glycol conjugated drug, Y refers to Lys, X1 is 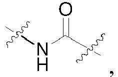 and X2 is 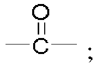
preferably, in the polyethylene glycol conjugated drug, Y refers to Lys, X1 is 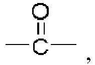 and X2 is

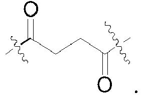

22. The method according to claim 20, wherein the intermediate W1-Y-X2-W2 is prepared by a method comprising the following steps:
step 1: preparing an intermediate W1-Y and an intermediate X2-W2 respectively, wherein Y has 2 amino groups, one of which is a free or activated amino group and the other is a protected amino group, and X2 has a free or activated carboxyl group;
step 2: causing the free or activated amino group on Y to react with the free or activated carboxyl group on X2 so that W1-Y is linked to X2-W2; and
step 3: deprotecting the protected amino group on Y and optionally carrying out activation to obtain the intermediate W1-Y-X2-W2;
preferably, in the polyethylene glycol conjugated drug, Y refers to Lys, X1 is 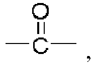 and X2 is

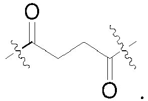

23. The method according to claim 20, wherein N-ACs on W1 and W2 are the same; the intermediate W1-Y-X2-W2 is prepared by a method comprising the following steps:
step 1: preparing intermediates N-AC, W1'-Y and X2-W2' respectively, wherein Y has 2 amino groups, one of which is a free or activated amino group and the other is a protected amino group, X2 has a free or activated carboxyl group, W1' and W2' are respectively precursors of W1 and W2 and said W1' and W2' have not been linked to N-AC, W1' and W2' have the same group M (e.g., hydroxyl group) that can react with N-AC, and M is protected;
step 2: causing the free or activated amino group on Y to react with the free or activated carboxyl group on X2 to obtain an intermediate W1'-Y-X2-W2';
step 3: deprotecting M but not deprotecting the protected amino group;
step 4: linking N-AC to W1' and W2'; and
step 5: deprotecting the protected amino group on Y and optionally carrying out activation to obtain the intermediate W1-Y-X2-W2
preferably, in the polyethylene glycol conjugated drug, Y refers to Lys, X1 is 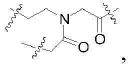 X2 is 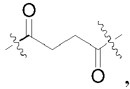 and the structure of the PEG is

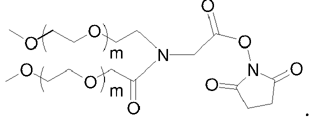

24. The method according to claim 20, wherein N-ACs on W1 and W2 are the same; the intermediate W1-Y-X2-W2 is prepared by a method comprising the following steps:
step 1: preparing intermediates N-AC, W1'-Y-X2 and W2' respectively, wherein Y has a protected amino group, X2 has a free or activated carboxyl group, W2' has a free or activated amino group, W1' and W2' are respectively precursors of W1 and W2 and said W1' and W2' have not been linked to N-AC, each of W1' and W2' has a group M (e.g., hydroxyl group) that can react with N-AC, and M is protected;
step 2: causing the free or activated amino group on W2' to react with the free or activated carboxyl group on X2 to obtain an intermediate W1'-Y-X2-W2';
step 3: deprotecting M but not deprotecting the protected amino group;
step 4: linking N-AC to W1' and W2'; and
step 5: deprotecting the protected amino group on Y and optionally carrying out activation to obtain the intermediate W1-Y-X2-W2.

25. A method for preparing the polyethylene glycol conjugated drug or a pharmaceutically acceptable salt thereof according to any one of claims 1 to 13, wherein N-ACs on W1 and W2 are the same, and X1 represents 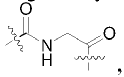 the method comprising the following steps:
step 1: preparing intermediates N-AC, W1'-Y-X2 and W2' respectively, wherein X2 has a free or activated carboxyl group, Y has a protected amino group, W2' has a free or activated amino group, W1' and W2' are respectively precursors of W1 and W2 and said W1' and W2' have not been linked to N-AC, each of W1' and W2' has a group M (e.g., hydroxyl group) that can react with N-AC, and M is protected;
step 2: causing the free or activated amino group on W2' to react with the free or activated carboxyl group on X2 to obtain an intermediate W1'-Y-X2-W2';
step 3: deprotecting the amino group on Y but not deprotecting the protected group of M;
step 4: causing the amino group on Y to react with a carboxyl group on Boc-Gly-OH (glycine with a protected amino group) to obtain an intermediate 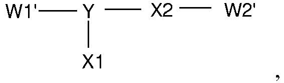 wherein X1 has a protected amino group;
step 5: deprotecting M but not deprotecting the protected amino group;
step 6: linking N-AC to W1' and W2';
step 7: deprotecting the protected amino group on X1 and optionally carrying out activation to obtain an intermediate 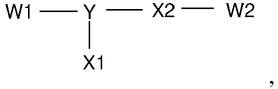 wherein X1 has a free or activated amino group; and
step 8: carrying out an amidation reaction so that PEG with carboxyl groups is linked to X1 on the intermediate 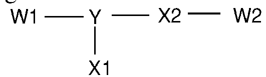 to obtain a polyethylene glycol conjugated drug of formula (I), wherein the number of carboxyl groups is denoted as j, and the carboxyl groups are free or activated carboxyl groups;
wherein PEG, X2, Y, W1, W2, and j are as defined in any of claims 1 to 13;
preferably, in the polyethylene glycol conjugated drug, Y refers to Lys;
preferably, in the polyethylene glycol conjugated drug, Y refers to Lys, X1 is 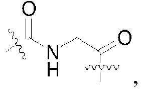 and X2 is

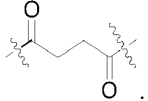

26. A method for preparing the polyethylene glycol conjugated drug or a pharmaceutically acceptable salt thereof according to any one of claims 1 to 13, wherein N-ACs on W1 and W2 are the same, the method comprising the following steps:
step 1: preparing intermediates N-AC', W1'-Y-X2 and W2' respectively, wherein X2 has a free or activated carboxyl group, Y has a protected carboxyl group, W2' has a free or activated amino group, W1' and W2' are respectively precursors of W1 and W2 and said W1' and W2' have not been linked to N-AC', each of W1' and W2' has a group M (e.g., hydroxyl group) that can react with N-AC', M is protected, AC' is a precursor of AC and said AC' is protected by a protecting group (e.g., TBDPS);
step 2: causing the free or activated amino group on W2' to react with the free or activated carboxyl group on X2 to obtain an intermediate W1'-Y-X2-W2';
step 3: deprotecting M but not deprotecting the protected carboxyl group;
step 4: linking N-AC' to W1' and W2';
step 5: deprotecting the protected carboxyl group on Y and optionally carrying out activation to obtain an intermediate W1"-Y-X2-W2", wherein Y has a free or activated carboxyl group, W1" is a precursor of W1 and W1" is the one without removing the protecting group on AC', and W2" is a precursor of W2 and W2" is the one without removing the protecting group on AC';
step 6: carrying out an amidation reaction so that PEG with amino groups is linked to the intermediate W1"-Y-X2-W2" to obtain an intermediate 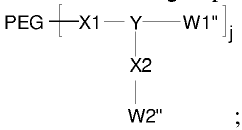 and
step 7: removing the protective groups of AC's on W1" and W2" to obtain a polyethylene glycol conjugated drug of formula (I),
wherein PEG, X1, X2, W1, W2, and Y are as defined in any of claims 1 to 13;
preferably, in the polyethylene glycol conjugated drug, Y refers to Glu, X1 is -NH-, and X2 is 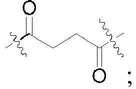
preferably, in the polyethylene glycol conjugated drug, W1 is Q and W2 is N1'-AC1';
preferably, in the polyethylene glycol conjugated drug, AC is SN38.

27. A method for preparing the polyethylene glycol conjugated drug or a pharmaceutically acceptable salt thereof according to any one of claims 1 to 13, wherein N-ACs on W1 and W2 are the same, the method comprising the following steps:
step 1: preparing intermediates N-AC', W1'-Y and X2-W2' respectively, wherein X2 has a free or activated carboxyl group, Y has two amino groups, one of which is a free or activated amino group and the other is a protected amino group, W1' and W2' are respectively precursors of W1 and W2 and said W1' and W2' have not been linked to N-AC', each of W1' and W2' has a group M (e.g.,
hydroxyl group) that can react with N-AC', M is protected, AC' is a precursor of AC and AC' is protected by a protecting group (e.g., TBDPS);
step 2: causing the free or activated carboxyl group on X2 to react with the free or activated amino group on Y to obtain an intermediate W1'-Y-X2-W2';
step 3: deprotecting M but not deprotecting the protected amino group;
step 4: linking N-AC' to W1' and W2';
step 5: deprotecting the protected amino group on Y and optionally carrying out activation to obtain an intermediate W1"-Y-X2-W2", wherein Y has a free or activated amino group, W1" is a precursor of W1 and W1" is the one without removing the protecting group on AC', and W2" is a precursor of W2 and W2" is the one without removing the protecting group on AC';
step 6: carrying out an amidation reaction so that PEG with carboxyl groups is linked to Y on the intermediate W1"-Y-X2-W2" to obtain an intermediate 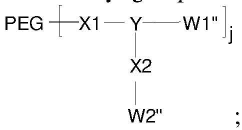 and
step 7: removing the protective groups of AC's on W1" and W2" to obtain a polyethylene glycol conjugated drug of formula (I),
wherein PEG, X1, X2, Y, W1, and W2 are as defined in any of claims 1 to 13;
preferably, in the polyethylene glycol conjugated drug, Y refers to Lys, and X1 and X2 both represent 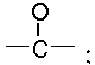
preferably, in the polyethylene glycol conjugated drug, W1 represents 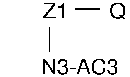 and W2
represents 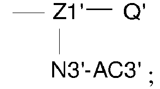 preferably, in the polyethylene glycol conjugated drug, AC is SN38.

28. A compound having a structure as follows: W1-Y-X2-W2, W1'-Y-X2-W2', W1"-Y-X2-W2", 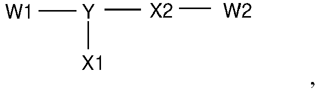 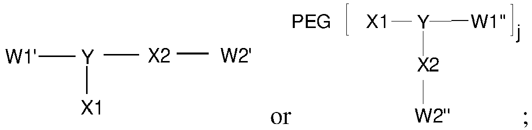 wherein X1, X2, Y, W1, W2, W1', W2', W1', W2' and PEG are as defined in any of claims 1 to 27.

29. A compound having a structure selected from
| | |
|---|---|
| 1 | 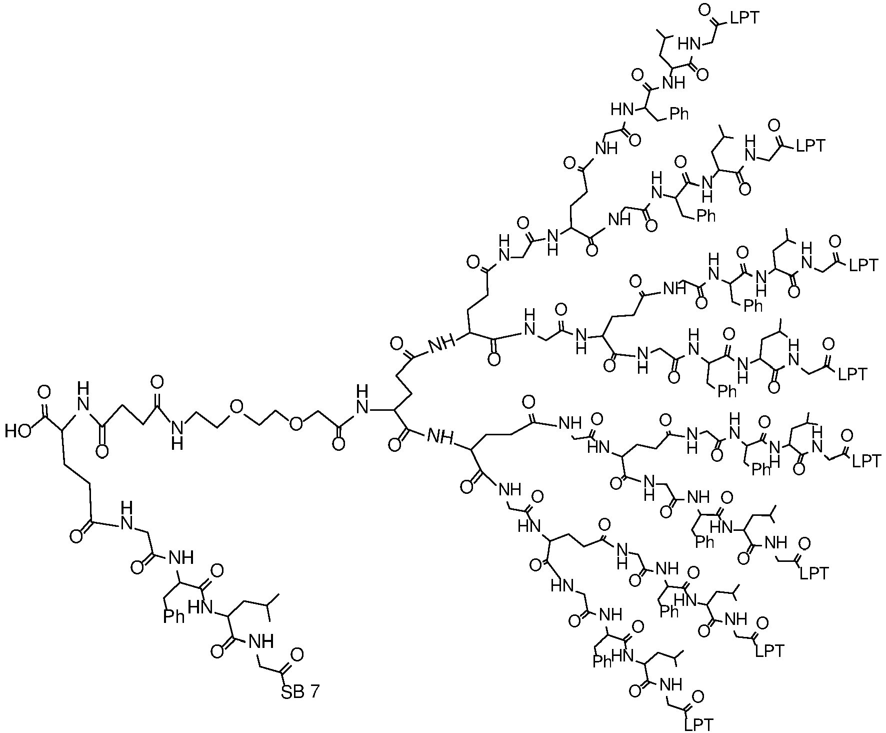 |
| 2 | 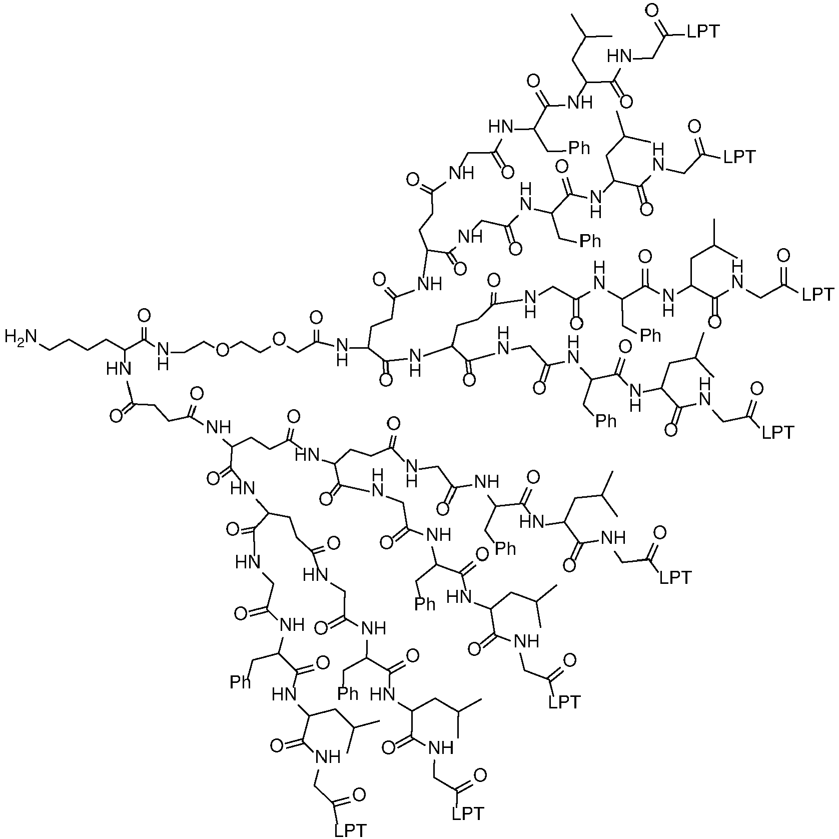 |
| 3 | 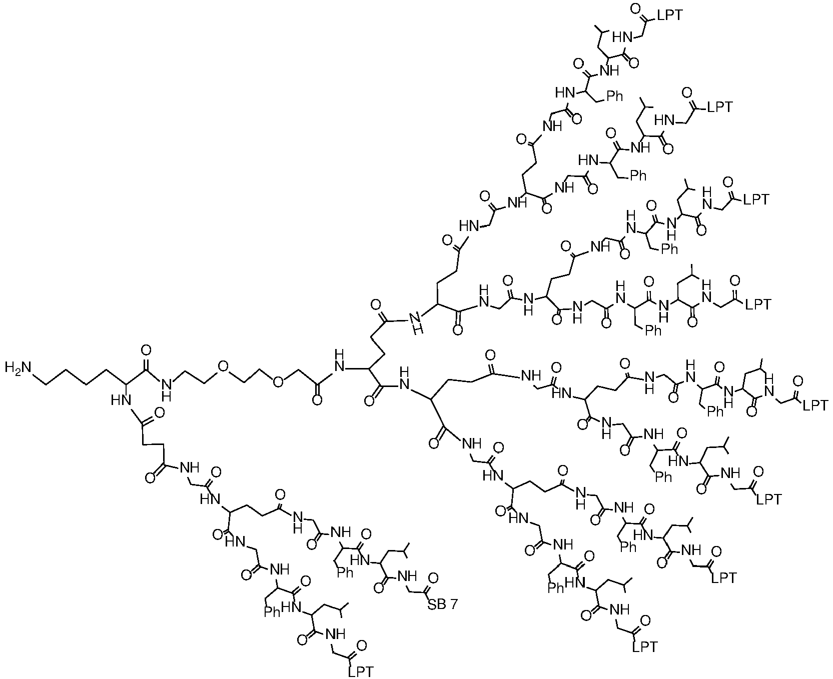 |
| 4 | 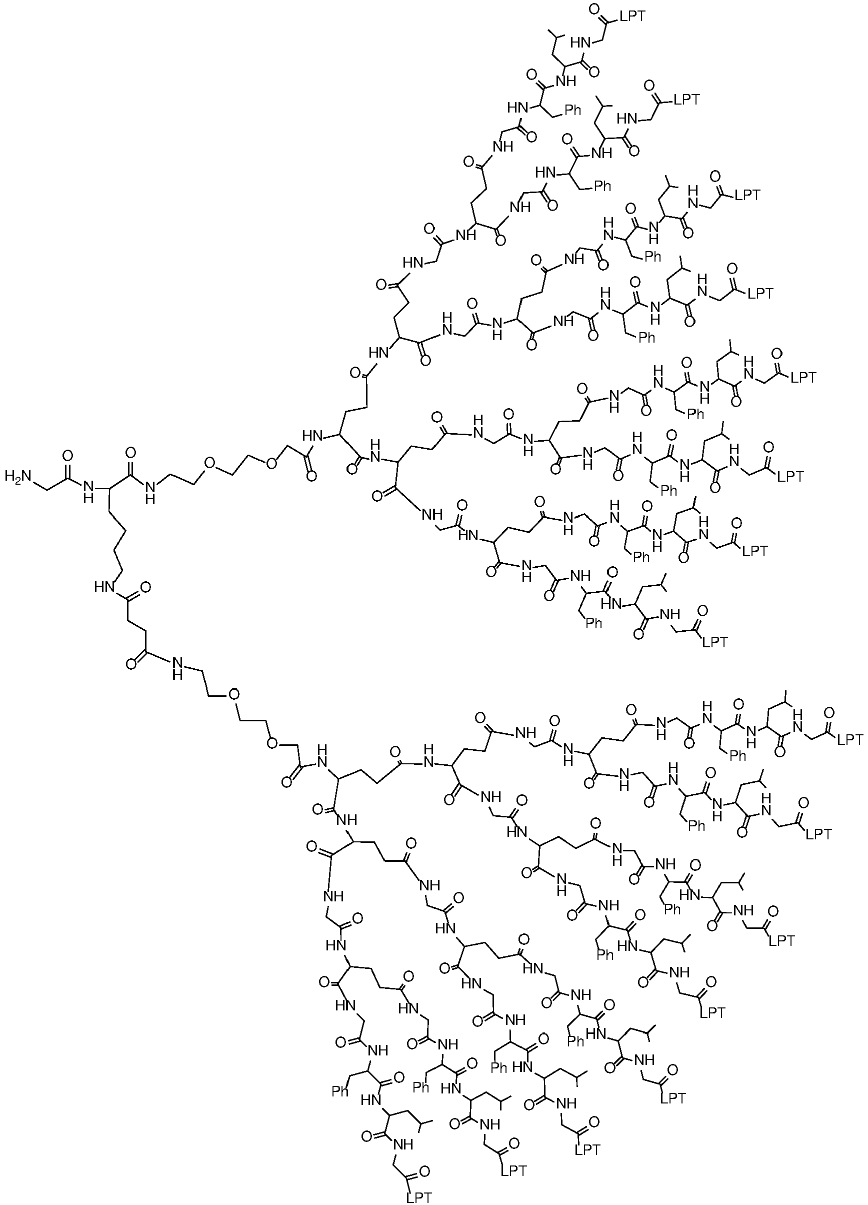 |
| 5 | 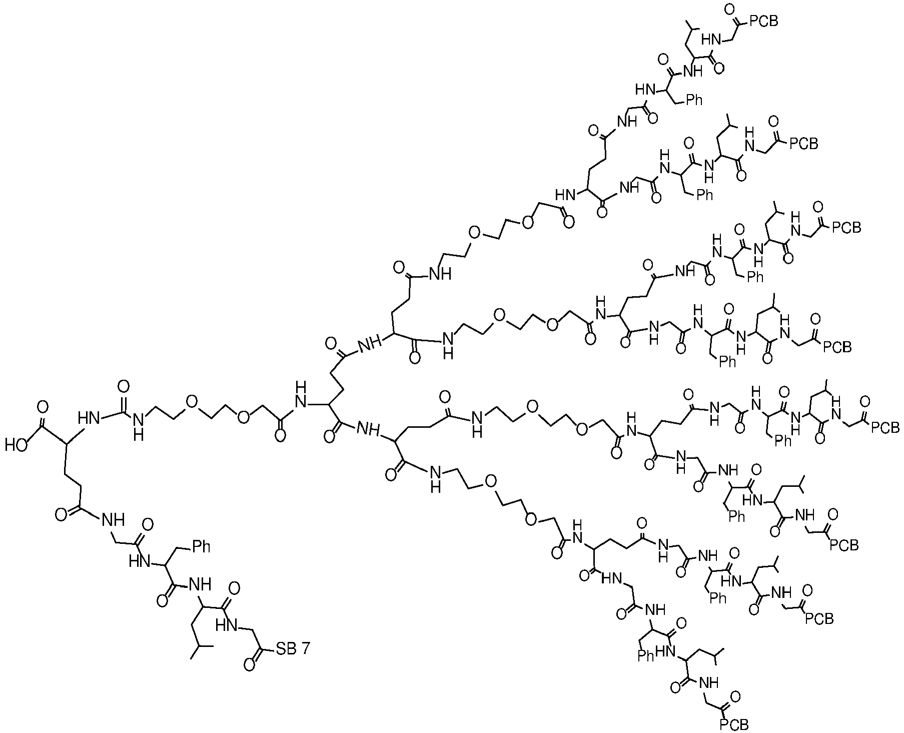 |
| 6 | 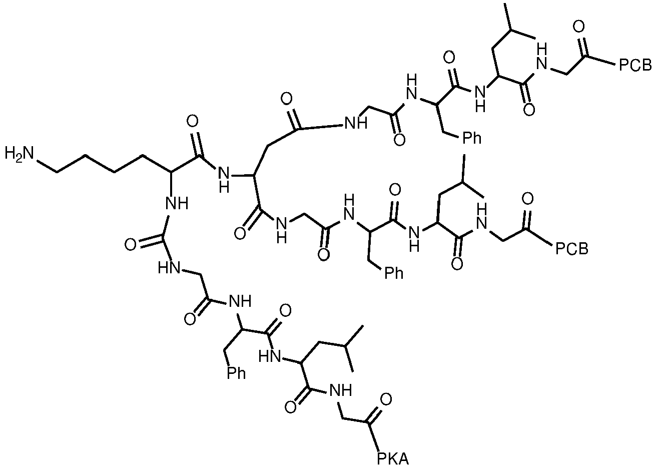 |
| 7 | 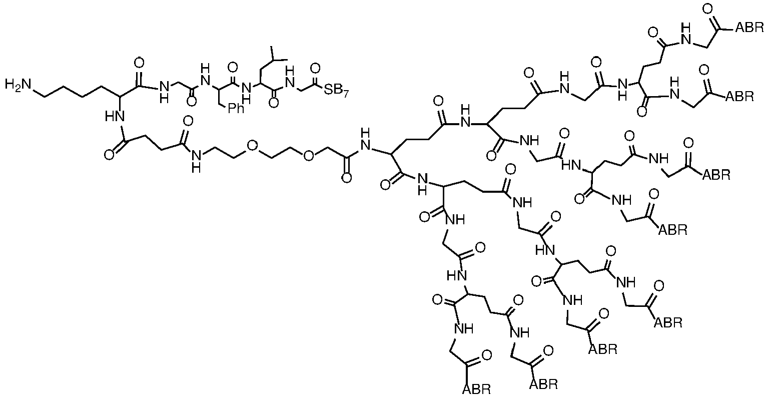 |
| 8 | 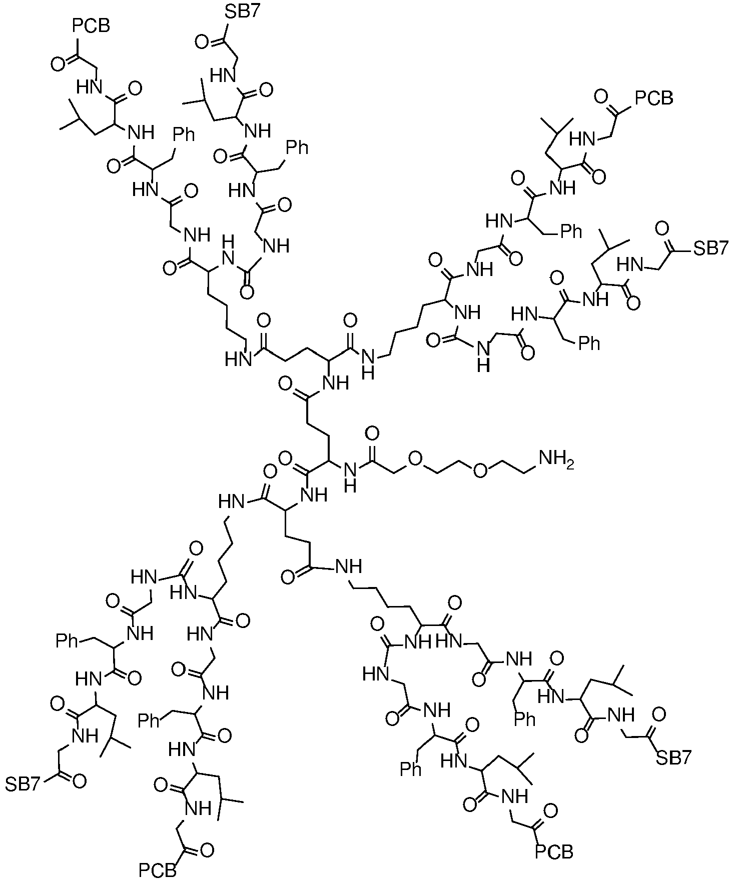 |
| 9 | 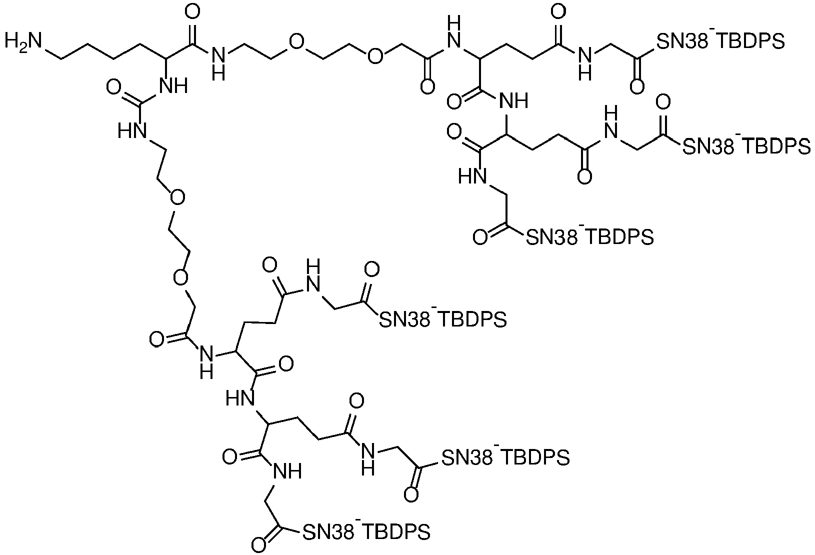 |
| 10 | 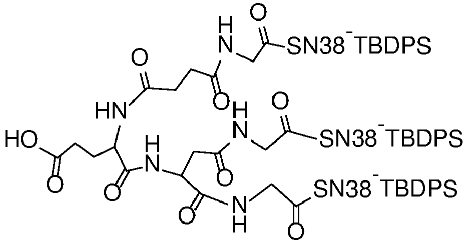 |
| 11 | 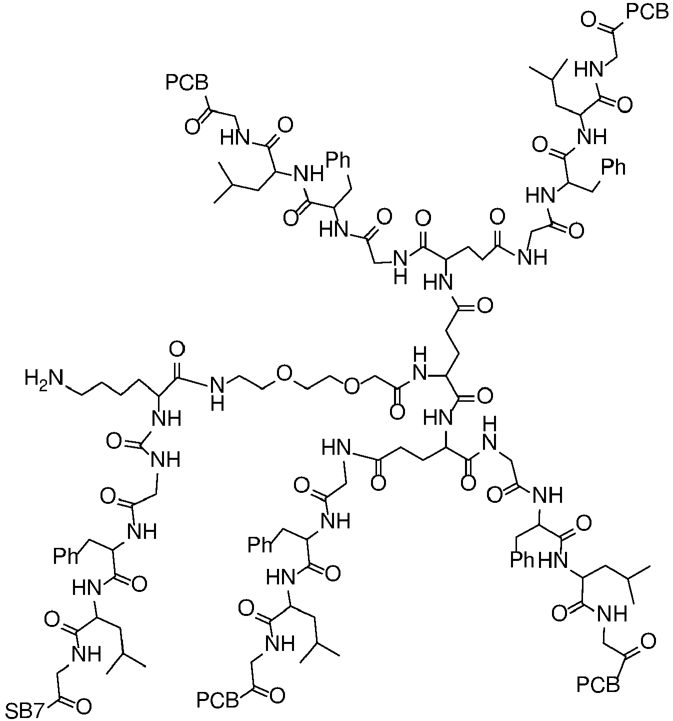 |
| 12 | 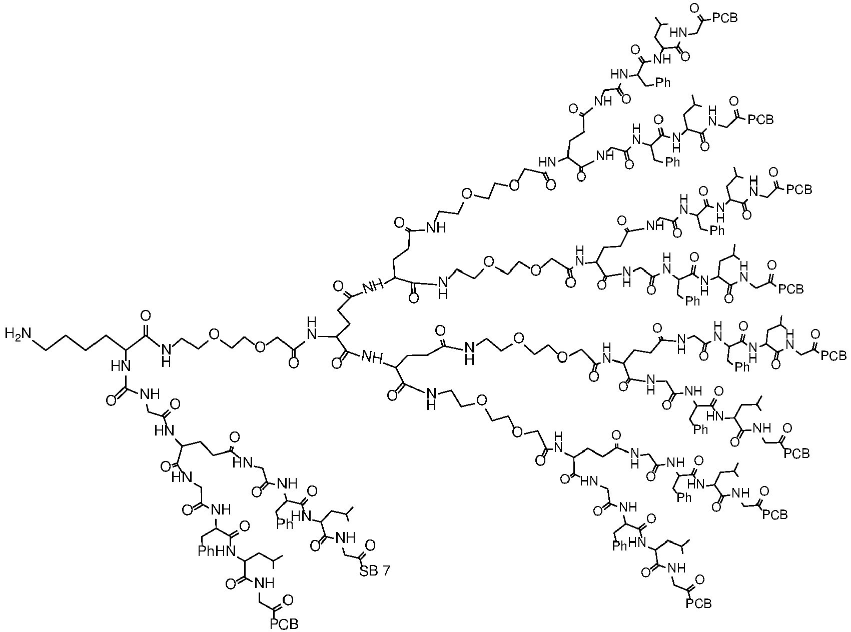 |
optionally, the free amino group or free carboxyl group in the compound is protected or activated.

30. Use of the compound according to claim 28 or 29 in preparation of the polyethylene glycol conjugated drug or a pharmaceutically acceptable salt thereof according to any one of claims 1 to 14.
